# EUROPEAN PATENT APPLICATION

(11) **EP 4 640 686 A1**
(43) Date of publication of application: **29.10.2025**
(21) Application number: 23895049.7
(22) Date of filing: 22.11.2023
(51) Int. Cl.: C07D 487/14, A61K 47/55, A61P 35/00, C07D 471/14, A61K 31/519

(54) **DEGRADER FOR DEGRADING BRD PROTEIN AND PHARMACEUTICAL COMPOSITION CONTAINING SAME**

(30) Priority: 22.11.2022 KR 20220157346
(71) Applicant: Innocure Therapeutics, Inc., Seongnam-si Gyeonggi-do 13488 (KR)
(72) Inventor: YOO, Hye Dong, Seongnam-si Gyeonggi-do 13488 (KR); SHIN, Young Jun, Seongnam-si Gyeonggi-do 13488 (KR); YANG, Dong Sik, Seongnam-si Gyeonggi-do 13488 (KR)
(74) Representative: Potter Clarkson
(86) International application number: PCT/KR2023/019003
(87) International publication number: WO 2024/112122

(57) **Abstract**

A TPD compound is disclosed.

The present invention relates to a PROTAC compound that binds to the BRD4 protein, and since it can bind to the BRD4 protein and degrade it, it can be useful for the treatment or prevention of diseases related to the BRD4 protein. The compound of the present invention has an excellent anticancer effect, and can also induce the degradation of BRD4 protein, which can show a therapeutic effect on BRD4 and related diseases.

## Description

### [Technical Fields]

The present invention relates to a novel PROTAC compound binding to a cMET protein and a pharmaceutical composition comprising the same.

### [Background Skills]

A PROTAC is a heterodimer molecule in which a ligand of a target protein and a ligand that binds to an E3 ligase are linked via a linker. PROTAC binds to both proteins simultaneously, bringing the target protein very close to E3 ligase, which recognizes the target protein as a substrate and triggers polyubiquitination and subsequent proteasome degradation. Since this principle can effectively remove specific proteins from cells, PROTAC can be used as a chemical probe to study the function of target proteins, and furthermore, it has a high potential as a treatment for diseases. The term TPD (Targeted Protein Degradation) is sometimes used instead of the term PROTAC.

In the present invention, a target protein-binding moiety and an E3 ligase-binding moiety binding moiety that bind to cMET proteins were prepared, and a novel PROTAC compound was completed by linking the E3 ligase-binding moiety and the cMET protein-targeted binding moiety through a linker.

### [Detailed explanation of the invention]

### [Technical Challenges]

The technical challenge intended to be achieved by the present invention relates to a PROTAC linked with a novel E3 ligase binding moiety and a cMET protein target binding moiety via a linker and a pharmaceutical composition comprising the same.

### [Technical Solution]

In order to accomplish the technical task, the present invention provides a TPD compound or a pharmaceutically acceptable salt thereof, expressed by the following formula 1: [Chemical Formula 1]

Compounds expressed with the following chemical formula 1 or their pharmaceutically acceptable salts:

[Chemical Formula 1] BRD4 target protein-binding moiety (P) - {linker (L)}ₚ - E3 ligase-binding moiety (E)

wherein,
E3 ligase binding moiety (E) is a structure denoted by the following chemical formulas 2 or 3;
BRD4 target protein-binding moiety (P) is the structure indicated by the following formula 4 or 5; and
p is an integer of 0 or 1, 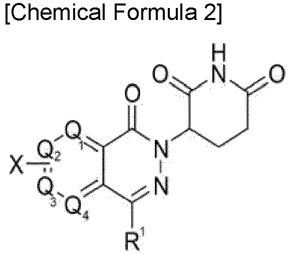 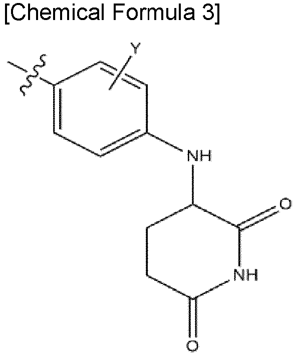 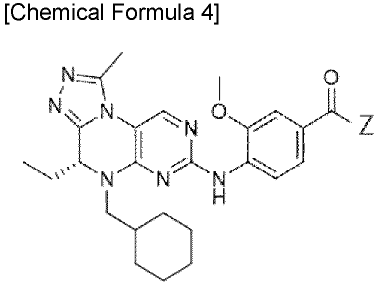 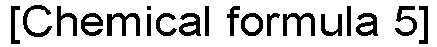 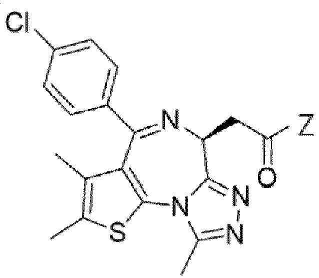
wherein,
X is hydrogen, halogen, amino, nitro, hydroxy, C1 to C6 straight or branched alkyloxy, or a heterocycle of 4 to 8 atoms containing oxygen or nitrogen;
Q₁ to Q₄ are independently C-F, C-Cl, C-H, C-X, or N, and at least any one of Q₁ to Q₄ is C-X;
Y is H, a halogen, or C1 to C6 straight or branched alkyl that is unsubstituted or optionally substitute with a halogen or cyclic alkyloxy;
Z is the part where one end of the linker connects; and
R¹ is hydrogen, nitro, amino, carbonyl, C1 to C6 straight or branched alkyl, C1 to C6 straight or branched alkyl substituted with halogen, or cycloalkyl.

In the present invention, the other end of the linker (L) is
(i) substitute X in the structure of the chemical formula 2 and connect with the structure of the chemical formula 1, or
(ii) bonded to a nitrogen atom or oxygen atom of X in the structure of chemical formula 2.

In the present invention, the linker (L) is 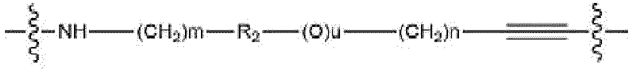 or 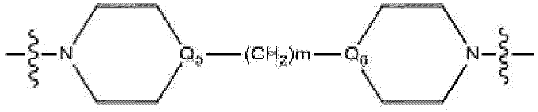 or 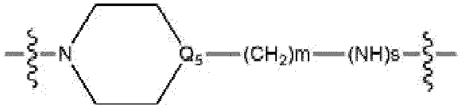 or 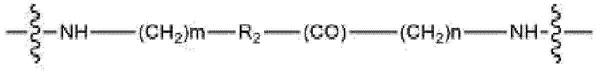 or 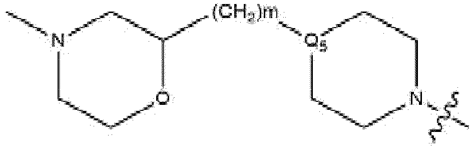 or 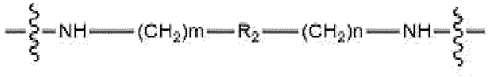 or 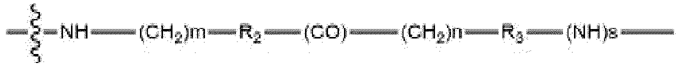 or 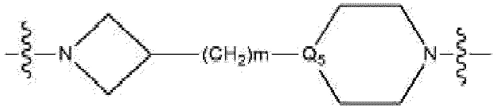 or 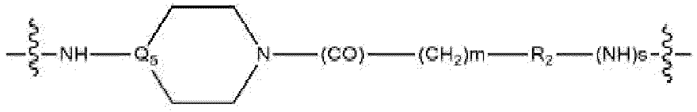 or 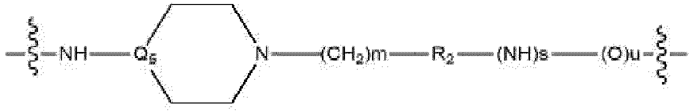 or 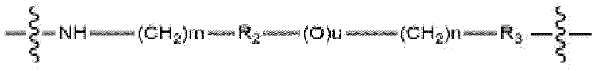 or a structure selected from the group of the following formulas: 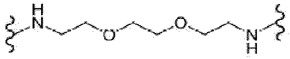 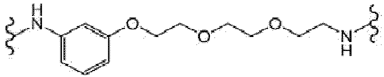 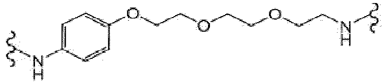 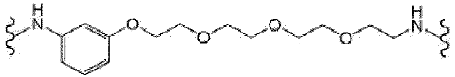 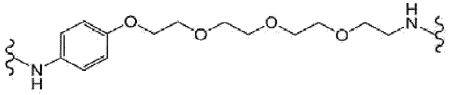 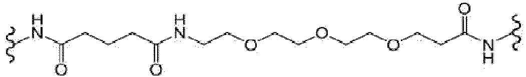
wherein, R₂ and R₃ are each independently unsubstituted or substituted C1 to C4 alkyl, piperidine, piperazine, -(CH₂)s-, -(CH₂)q-[O(C₂H₄)]ᵣ-, -O-(CH₂)-, benzene or morpholine;
Q₅ and Q₆ are independently carbon atoms or nitrogen atoms independently; and
m, n, q, r, s, t, and u are each independently an integer selected from 0 to 7.

In addition, the present invention relates to an anticancer composition comprising the above compounds.

### [Effect of Invention]

The present invention relates to a PROTAC compound that binds to a cMET protein, and since it can bind to a cMET protein and degrade it, it can be useful for the treatment or prevention of diseases related to cMET protein. The compound of the present invention has an excellent anticancer effect and can also induce the degradation of cMET proteins, which can show a therapeutic effect on cMET and related diseases.

### [Brief explanation of drawings]

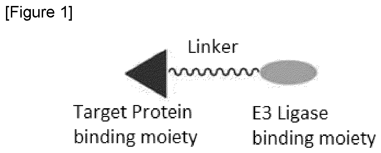

Figure 1 is a schematic of a PROTAC compound consisting of an E3 ligase-binding moiety, a linker, and a target protein-binding moiety.

### [Modes for the Practice of the Invention]

The present invention is described in more detail below. However, the present invention can be implemented in various different forms, and the present invention is not limited by the embodiments described herein.

The terms used herein are used merely to describe a specific embodiment and are not intended to qualify the present invention. Expressions in the singular include plural expressions, unless the context clearly means otherwise. The 'inclusion' of a component in the specification of the present invention means that it may include more other components rather than excluding other components, unless there is a specifically contrary statement.

PROTAC according to the present invention may be a compound denoted by the formula 1.

[Chemical Formula 1] cMET Targeted Protein-Binding Matrix (P) - [Linker (L)]p - E3 Ligase-Binding Matrix (E)

The basic framework of the E3 ligase binding moiety according to the present invention can be prepared by going through the reaction steps of the following Equation 1 or Equation 2: The basic framework of the E3 ligase binding moiety according to the present invention can be prepared by going through the reaction steps of the following Equation 1 or Equation 2:

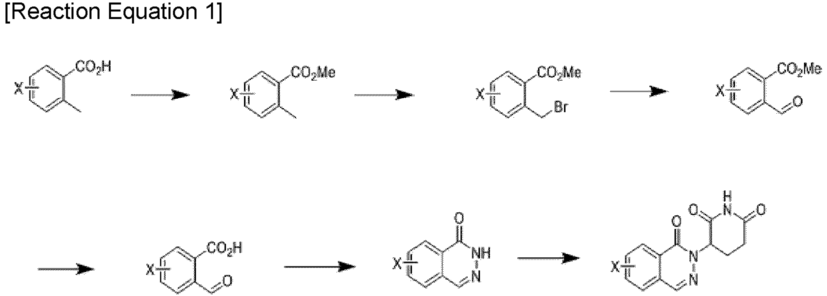

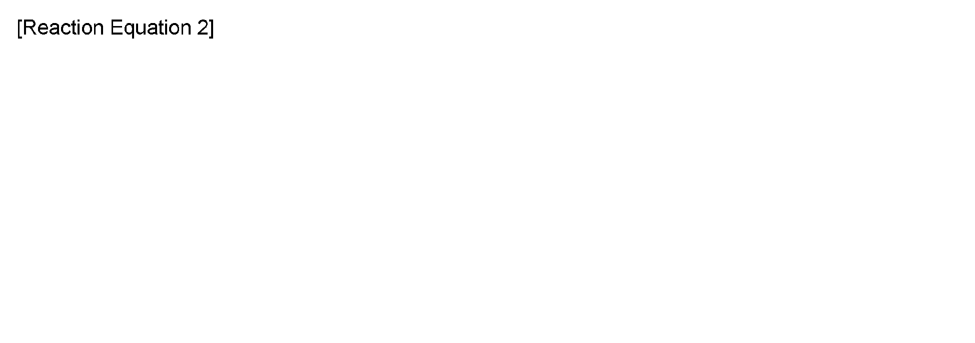

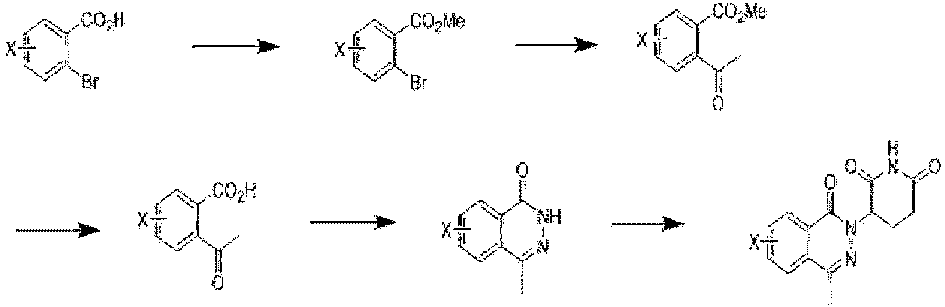

In the reaction equation 1 or 2, X is hydrogen, halogen, amino, nitro, hydroxy, piperazine group or an alkoxy of C1 to C4. However, for the convenience of explanation, in the above equation, the substituent that can be bound to carbon in Q₁ to Q₄ of the compounds of chemical formula 1 is omitted, and only simple substituents such as hydrogen or methyl are represented among the R¹ substituents.

In the following, a specific example of an E3 ligase-bound moiety compound is prepared using an embodiment.

### Example A: Preparation of an E3 ligase-coupled moiety compound

**Example A-1: Preparation of a compound** 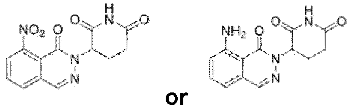 **(prepared according to Reaction Equation 1)**

### 1-1) Preparation of methyl 2-methyl-6-nitrobenzoate

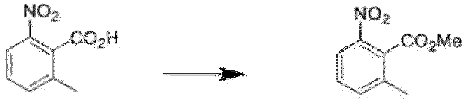

At room temperature, K₂CO₃ (19.1 g, 138 mmol) was added to a 2-methyl-6-nitrobenzoic acid (5 g, 27.6 mmol) solution in acetone (100 ml). After stirring for 30 minutes, methane iodide (19.6 g, 138 mmol) was added to the reactant and heated to 60°C for 6 hours. After cooling, the reactants were filtered and concentrated under reduced pressure. The product was used in the next reaction without additional purification. (5.45g, 98%) MS (ESI, m/z): [M+1]⁺ = [195.2].

### 1-2) Preparation of methyl 2-(bromomethyl)-6-nitrobenzoate

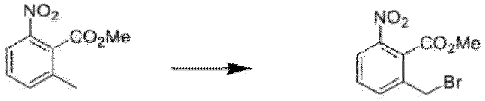

At room temperature, 1-bromomerolidine-2,5-dione (7.39 g, 41.4 mmol) and benzoyl benzene carboroxoate (0.67 g, 7.26 mmol) were sequentially added to a solution of methyl 2-methyl-6-nitrobenzoate (5.39 g, 27.6 mmol) in ClCH₂CH₂Cl (100 ml). The reactants were heated and refluxed for 3 hours. The point at which the red color disappears is the time when the reaction is completed. After cooling, the reactants were washed with water, dried with MgSO₄, and concentrated under reduced pressure. The crude product was used in the next reaction without additional purification. (7.34g, 98%) MS (ESI, m/z): [M+1]⁺ = [274.4].

### 1-3) Preparation of methyl 2-formyl-6-nitrobenzoate

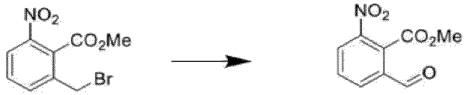

At room temperature, NMO (N-methylmorpholine N-oxide) (561mg, 4.87mmol) and 4°C molecular sieve were sequentially added to methyl 2-(bromomethyl)-6-nitrobenzoate (580mg, 2.12mmol) solution in DCM (30ml). The reactants were stirred at room temperature for 2 hours. Molecular sieve was filtered and washed with DCM (10ml). The DCM layer was washed with water (50ml), dried with MgSO₄, and concentrated under reduced pressure under reduced pressure. The residue was purified by column chromatography, and the title compound was obtained as a white solid. (350mg, 79.07%) MS (ESI, m/z): [M+1]⁺ = [210.0].

### 1-4) Preparation of 2-Formyl-6-Nitrobenzoic Acid

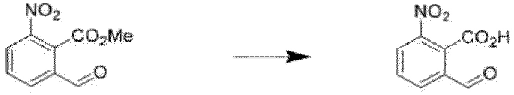

At room temperature, an aqueous solution (10 ml) of lithium (+1) hydroxyside (1.15 g, 47.8 mmol) was added to a solution of methyl 2-formyl-6-nitrobenzoate (2 g, 9.56 mmol) in THF (10 ml). After 2 h of agitation, the reactants were decompressively concentrated to dry the THF. After cooling to 0°C, the reactants were acidified with 1N HCl and the pH was adjusted to 4. The reactants were extracted twice with ethyl acetate (50ml). The combined ethyl acetate layer was dried with MgSO4, concentrated under reduced pressure to obtain white crystals (1.50 g, 80.3%). MS (ESI, m/z): [M+1]⁺ = [195.2].

### 1-5) Formulated with 8-Nitroptalazine-1(2H)-One

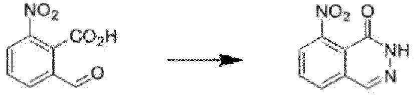

At room temperature, NH₂NH₂ monohydrate (417 mg, 8.33 mmol) was added to a 2-formyl-6-nitrobenzoic acid (1.2 g, 6.15 mmol) solution in methanol (10 ml). After stirring for 30 minutes, the reactants were heated to 80°C for 2 hours. The reactants were cooled to room temperature and concentrated under reduced pressure. The residue was poured into water (50ml) and extracted twice with ethyl acetate (50ml), and the combined ethyl acetate layer was dried with MgSO4, concentrated under reduced pressure, and white crystals were obtained (1g, 85.07%). MS (ESI, m/z): [M+1]⁺ = [191.8].

### 1-6) 3-(8-nitro-1-oxopthalazine-2(1H)-yl)piperidine-2,6-dione preparation

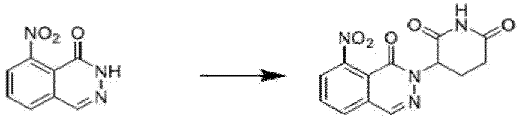

3-bromoperidin-2,6-dione (90 mg, 0.471 mmol) and K₂CO₃ (129 mg, 0.942 mmol) were sequentially added to 8-nitro-1,2-dihydrodroplasine-1-one (60 mg, 0.314 mmol) solution in DMF (1 ml). The reactants were heated to 85°C for 5 hours. After cold soaking, the reactant was poured into water (5 ml) and extracted twice with ethyl acetate (5 ml). The combined ethyl acetate layer was dried with MgSO4 and concentrated under reduced pressure. The residue was purified by column chromatography, and the title compound was obtained as a white crystal (68 mg, 71.7%). MS (ESI, m/z): [M+1]⁺ = [302.6].

### 1-7) 3-(8-nitro-1-oxopthalazine-2(1H)-yl)piperidine-2,6-dione preparation

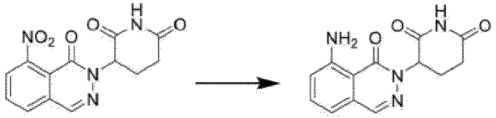

20 mg of 10% Pd/C (wet) (5 mg) of 20 mg was added to 3-(8-nitro-1-oxo-1,2-dihydrophopthalazine2-yl)piperidine-2,6-dione (25 mg, 0.82 mmol) solution in methanol (5 ml) and stirred for 1 hour under hydrogen in the balloon. The solids were filtered, the filtrate was decompressively concentrated, and the title compound was obtained at a yield of 99%. MS (ESI, m/z): [M+1]⁺ = [272.3].
[NMR] ¹H NMR (400 MHz, DMSO-d₆) δ 10.95 (s, 1H), 8.10 (s, 1H), 7.46 (t, J = 7.83 Hz, 1H), 7.22 (br s, 2H), 6.86 (d, J = 7.83 Hz, 1H), 6.81(d, J = 7,34 Hz, 1H), 5.61 (br dd, J = 5.2, 11.92 Hz, 1H), 2.77 - 2.89 (m, 1H), 2.46 - 2.57 (m, 2H), 1.95 - 2.08 (m, 1H)

**Example A-2: Preparation compound**

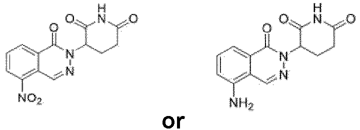

### 2-1) Preparation of methyl 2-methyl-3-nitrobenzoate

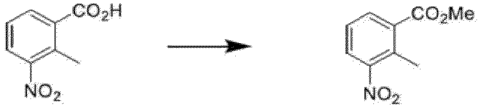

At room temperature, K2CO3 (19.1 g, 138 mmol) was added to a 2-methyl-3-nitrobenzoic acid (5 g, 27.6 mmol) solution of acetone (100 ml). After stirring for 30 minutes, methane iodide (19.6 g, 138 mmol) was added to the reactant and heated to 60°C for 6 hours. After cooling, the reactants were filtered and concentrated under reduced pressure. The product was used in the next reaction without additional purification. (5.45g, 98%) MS (ESI, m/z): [M+1]⁺ = [195.3].

### 2-2) Preparation of methyl 2-(bromomethyl)-3-nitrobenzoate

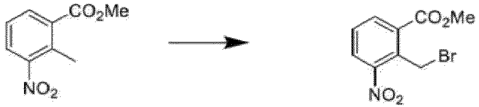

At room temperature, 1-bromophilidine-2,5-dione (7.39 g, 41.4 mmol) and benzoyl benzene carboforoxoate (0.67 g, 7.26 mmol) were sequentially added to a solution of methyl 2-methyl-3-nitrobenzoate (5.39 g, 27.6 mmol) in ClCH₂CH₂Cl (100 ml). The reactants were heated and refluxed for 3 hours. The point when the red color disappears is the time when the reaction is complete. After cooling, the reactants were washed with water, dried with MgSO₄, and concentrated under reduced pressure. The crude product was used in the next reaction without additional purification. (7.34g, 98%) MS (ESI, m/z): [M+1]⁺ = [274.4].

### 2-3) Preparation of methyl 2-formyl-3-nitrobenzoate

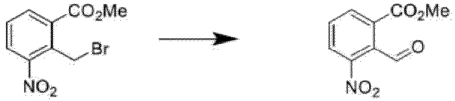

At room temperature, NMO (561mg, 4.87mmol) and 4Å molecular sieve were sequentially added to the methyl 2-(bromomethyl)-3-nitrobenzoate (580mg, 2.12mmol) solution in DCM (30ml). The reactants were stirred at room temperature for 2 hours. Molecular sieve was filtered and washed with DCM (10ml). The DCM layer was washed with water (50ml), dried with MgSO4, and concentrated under reduced pressure. The residue was purified by column chromatography, and the title compound was obtained as a white solid. (350mg, 79.07%) MS (ESI, m/z): [M+1]⁺ = [210.0].

### 2-4) Preparation of 2-formyl-3-nitrobenzoic acid

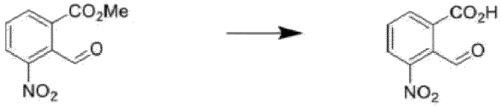

At room temperature, an aqueous solution of lithium (+1) hydroxide (1.15 g, 47.8 mmol) was added to a solution of methyl 2-formyl-3-nitrobenzoate (2 g, 9.56 mmol) in THF (10 ml). After 2 hours of agitation, the reactants were decompressively concentrated to dry the THF. After cooling to 0°C, the reactants were acidified with 1N HCl and the pH was adjusted to 4. The reactants were extracted twice with ethyl acetate (50ml). The combined ethyl acetate layer was dried with MgSO₄, concentrated under reduced pressure to obtain white crystals (1.50 g, 80.3%). MS (ESI, m/z): [M+1]⁺ = [195.2].

### 2-5) Preparation of 5-Nitroptalazine-1(2H)-one

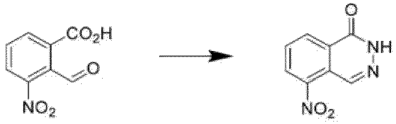

At room temperature, NH₂NH₂ monohydrate (417 mg, 8.33 mmol) was added to a 2-formyl-3-nitrobenzoic acid (1.2 g, 6.15 mmol) solution in methanol (10 ml). After stirring for 30 minutes, the reactants were heated to 80°C for 2 hours. The reactants were cooled to room temperature and concentrated under reduced pressure. The residue was poured into water (50ml) and extracted twice with ethyl acetate (50ml), and the combined ethyl acetate layer was dried with MgSO₄, concentrated under reduced pressure, and white crystals were obtained (1g, 85.07%). MS (ESI, m/z): [M+1]⁺ = [191.8].

### 2-6) Preparation of 3-(5-nitro-1-oxopthalazine-2(1H)-yl)piperidine-2,6-dione

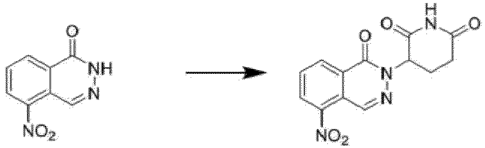

3-bromoperidin-2,6-dione (90 mg, 0.471 mmol) and K₂CO₃ (129 mg, 0.942 mmol) were sequentially added to a 5-nitrofthalazine-1(2H)-one (60 mg, 0.314 mmol) solution in DMF (1 ml). The reactants were heated to 85°C for 5 hours. After cooling, the reactants were poured into water (5 ml) and extracted twice with ethyl acetate (5 ml). The combined ethyl acetate layer was dried with MgSO₄ and concentrated under reduced pressure. The residue was purified by column chromatography, and the title compound was obtained as a white crystal (68 mg, 71.7%). MS (ESI, m/z): [M+1]⁺ = [302.4].

### 2-7) Preparation of 3-(5-amino-1-oxopthalazine-2(1H)-yl)piperidine-2,6-dione

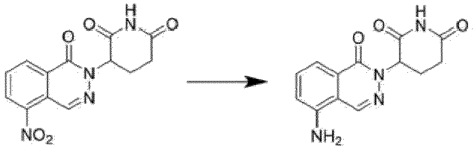

20 mg of 10% Pd/C (wet) (5 mg) of 10% Pd/C (5 mg) (5 mg) was added to a solution of 3-(5-nitro-1-oxopthalazine-2(1H)-yl) in methanol (5 ml) and stirred for 1 hour under hydrogen in the balloon. The solids were filtered, the filtrate was decompressively concentrated, and the title compound was obtained at a yield of 99%. MS (ESI, m/z): [M+1]⁺ = [272.3].
[NMR] ¹H NMR (400 MHz, DMSO-d₆) δ 10.95 (s, 1H), 8.20 -8.41 (m, 3H), 8.08 (s, 1H), 7.83 (d, J = 8.93 Hz, 1H), 6.95 (d, J = 10.5 Hz, 1H), 5.64 (br dd, J = 4.83, 11.31 Hz, 1H), 2.62 - 2.89 (m, 1H), 2.52 - 2.60 (m, 2H), 1.86 - 2.07 (m, 1H)

**Example A-3: Preparation of a compound**

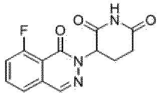

### 3-1) Preparation of methyl 2-fluoro-6-methylbenzoate

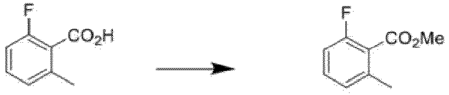

At room temperature, K₂CO₃ (44.8 g, 324 mmol) was added to a solution of 2-fluoro-6-methylbenzoic acid (10 g, 64.9 mmol) in acetone (200 ml). After stirring for 30 minutes, methane iodide (46 g, 324 mmol) was added to the reactant and heated to 60°C for 6 hours. After cooling, the reactants were filtered and concentrated under gamgam. The product was used in the next reaction without additional purification. (11.2g, yield 103%) MS (ESI, m/z): [M+1]⁺ = [168.3].

### 3-2) Preparation of methyl 2-(bromomethyl)-6-fluorobenzoate

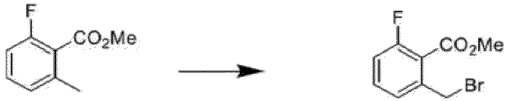

At room temperature, 1-bromomerrolidine-2,5-dione (24.7 g, 139 mmol) and benzoyl benzeperoxoate (1.53 g, 6.30 mmol) were sequentially added to a solution of methyl 2-fluoro-6-methylbenzoate (21.2 g, 126 mmol) in ClCH₂CH₂Cl (250 ml). The reactants were heated and refluxed for 16 hours. The point when the red color disappears is the time when the reaction is complete. After cooling, the reactants were washed with water, dried with MgSO₄, and concentrated under reduced pressure. The crude product was used in the next reaction without additional purification. (35g, yield 112%) MS (ESI, m/z): [M+1]⁺ = [245.9].

### 3-3) Preparation of methyl 2-fluoro-6-formylbenzoate

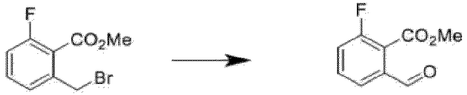

At room temperature, NMO (24.9 g, 212 mmol) was added to a solution of methyl 2-(bromomethyl)-6-fluorobenzoate (35 g, 142 mmol) in DCM (280 ml). The reactants were stirred at room temperature in the winter for 4 hours. The DCM layer was washed with water (200ml), dried with MgSO₄, and concentrated under reduced pressure. The residue was purified by column chromatography, and the title compound was obtained as a white solid. (11.52g, yield 45%) MS (ESI, m/z): [M+1]⁺ = [183.1].

### 3-4) Preparation of 2-fluoro-6-formylbenzoic acid

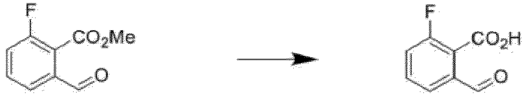

At room temperature, an aqueous solution (41 ml) of lithium (+1) hydroxide (7.57 g, 180 mmol) was added to a solution of methyl 2-fluoro-6-formylbenzoate (11.5 g, 63.2 mmol) in THF (82 ml). After 4 h of agitation, the reactants were decompressively concentrated and the THF was dried. After cooling to 0°C, the reactants were acidified with 1N HCl and the pH was adjusted to 4. The reactants were extracted twice with ethyl acetate (100ml). The combined ethyl acetate layer was dried with MgSO4, concentrated under reduced pressure, and white crystals were obtained (10.4 g, 97.8%). MS (ESI, m/z): [M+1]⁺ = [168.8].

### 3-5) Preparation of 8-Fluoroptalazine-1(2H)-one

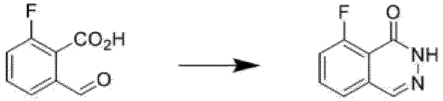

At room temperature, NH₂NH₂ monohydrate (3.72 mg, 61.9 mmol) was added to a 2-fluoro-6-formylbenzoic acid (10.4 g, 61.9 mmol) solution in methanol (120 ml) and stirred at room temperature for 16 hours. The white sediment was filtered and washed with methanol. The obtained white crystals were slurried with 100ml of ethyl acetate (EA) and filtered to obtain white crystals (3.05g, 30%). MS (ESI, m/z): [M+1]⁺ = [164.8].

### 3-6) Preparation of 3-(8-fluoro-1-oxopthalazine-2(1H)-yl)piperidine-2,6-dione

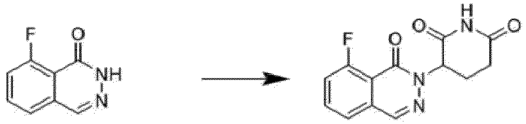

NaH (60%, 53.6mg, 1.34mmol) was added to 8-fluoroptalazine-1(2H)-one (200mg, 1.22mmol) solution in DMF (5ml) at 0°C and stirred for 30 minutes. 3-bromoperidin-2,6-dione (468mg, 2.44mmol) was added to the solution and stirred for 6 hours. The reaction was terminated with water, and extracted twice with ethyl acetate (20ml). The combined ethyl acetate layer was dried with MgSO₄ and concentrated under reduced pressure. The residue was purified by column chromatography, and the title compound was obtained as white crystals (70mg, 20.8%). MS (ESI, m/z): [M+1]⁺ = [276.1].
[NMR] ¹H NMR (400 MHz, DMSO-d₆) δ 11.06 (s, 1H), 8.48 (s, 1H), 7.99 (ddd, J = 4.6, 7.2, 8.2 Hz, 1H), 7.80 (d, J = 7.2 Hz, 1H), 7.67 (dd, J = 8.2, 11.4 Hz, 1H), 5.77 (dd, J=5.3, 12.0 Hz, 1H), 2.99-2.77 (m, 1H), 2.68-2.51 (m, 2H), 2.23-2.02 (m, 1H)

**Example A-4: Preparation of a compound**

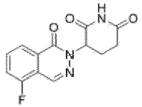

### 4-1) Preparation of methyl 5-fluoro-6-methylbenzoate

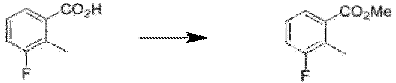

At room temperature, sulfuric acid (2 ml) was added to a solution of 3-fluoro-2-methylbenzoic acid (10 g, 64.9 mmol) in methanol (60 ml). It was heated to 80°C and stirred overnight. After cooling to room temperature, the reactants were evaporated, extracted with NaHCO₃ and ethyl acetate (EA), and dried with MgSO₄. The crude product was used in the next reaction without additional purification. (10.1g, yield 92%) MS (ESI, m/z): [M+1]⁺ = [168.3].

### 4-2) Preparation of methyl 2-(bromomethyl)-3-fluorobenzoate

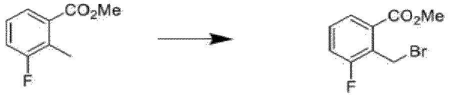

At room temperature, 1-bromomorphrolidine-2,5-dione (15.9 g, 89.2 mmol) and benzoyl benzeborosodium (0.72 g, 2.97 mmol) were sequentially added to a solution of methyl 3-fluoro-2-methylbenzoate (10 g, 59.5 mmol) in ClCH₂CH₂Cl (250 ml). The reactants were heated and refluxed for 16 hours. The point when the red color disappears is the time when the reaction is complete. After cold exposure, the reactants were washed with water, dried with MgSO₄, and concentrated under reduced pressure. The crude product was used in the next reaction without additional purification. (10.5g, yield 71%) MS (ESI, m/z): [M+1]⁺ = [245.9].

### 4-3) Preparation of methyl 3-fluoro-2-formylbenzoate

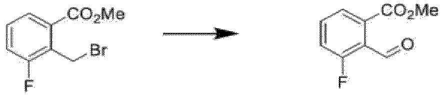

At room temperature, NMO (10.4 g, 89 mmol), 4Å molecular sieve was added to methyl 2-(bromomethyl)-3-fluorobenzoate (10 g, 40.5 mmol) solution in DCM (200 ml). The reactant water was stirred at room temperature for 4 hours. The DCM layer was washed with water (200ml), dried with MgSO₄, and concentrated under reduced pressure. The residue was refined by column chromatography and the headings were combined. (5.5g, 75%) MS (ESI, m/z): [M+1]⁺ = [183.1].

### 4-4) Preparation of 3-fluoro-2-formylbenzoic acid

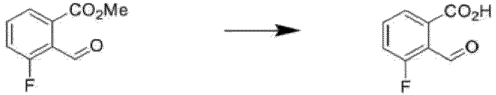

At room temperature, a solution of methyl 3-fluoro-2-formylbenzoate (2.5 g, 13.7 mmol) in THF (68 ml) was added with an aqueous solution (41 ml) of lithium (+1) hydroxyxide monohydrate (2.88 g, 68.6 mmol). After 4 hours of agitation, the reactants were concentrated under reduced pressure to dry the THF, cooled to 0°C, and acidified the reactants with 1N HCl to adjust the pH to 4. The semi-coagulated water was extracted twice with ethyl acetate (100ml). The combined ethyl acetate layer was dried with MgSO₄, concentrated under reduced pressure to obtain white crystals (2.5 g, 108%). MS (ESI, m/z): [M+1]⁺ = [168.8].

### 4-5) Preparation of 5-Fluoro-1,2-dihydrophthalazine-1-one

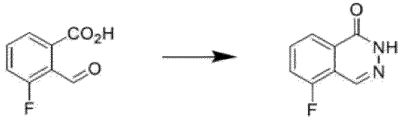

At room temperature, NH2NH2 monohydrate (1.22mg, 16.4mmol) was added to 3-fluoro-2-formylbenzoic acid (2.5g, 14.9mmol) solution in THF:water = 1:1 (70ml) and stirred for 16 hours. The mixture was acidified to pH 4 and the solid was filtered with hexane. Vacuum drying yielded the title compound (1.3 g, 53%). MS (ESI, m/z): [M+1]⁺ = [165.3]

### 4-6) Preparation of 3-(5-fluoro-1-oxo-1,2-dihydroptalazine-2-yl)piperidine-2,6-dione

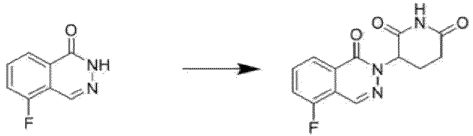

LDA (lithium diisopropylamide, 2.19 mmol) was added to 5-fluorine-1,2-dihydrophoptalazine (300 mg, 1.83 mmol) solution in DMF (10 ml) at 0°C and stirred for 30 minutes. 3-bromoperidin-2,6-dione (526 mg, 2.74 mmol) was added to the solution and stirred at 80°C for 6 hours. Once the reaction was completed, the reactant was cooled to room temperature, poured into water (100ml), pH was adjusted to 3~4 with 6N HCl, then extracted with ethyl acetate, dried with MgSO₄, and depressurized concentrated. The product was recrystallized into hexane, vacuum-dried, and the title compound was obtained. MS (ESI, m/z): [M+1]⁺ = [276.1].
[NMR] ¹H NMR (400 MHz, DMSO-d₆) δ 11.03 (s, 1H), 8.53 (s, 1H), 8.05 (d, J=7.82Hz, 1H), 7.76-7.90 (m, 2H), 5.78 (dd, J=12.23, 5.26Hz, 1H), 2.80-2.93 (m, 1H), 2.47-2.61 (m, 2H), 2.01-2.16 (m, 1H)

**Example A-5:** 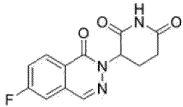 **Preparation of a compound**

### 5-1) Preparation of methyl 4-fluoro-6-methylbenzoate

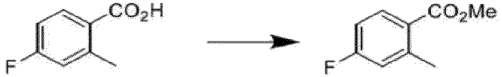

In accordance with the preparation method of Example 4-1, methyl 4-fluoro-2-methylbenzoate was prepared from 4-fluoro-2-methylbenzoate.

### 5-2) Preparation of methyl 2-(bromomethyl)-4-fluorobenzoate

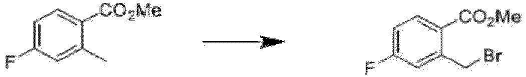

At room temperature, 1-bromophilolidin-2,5-dione (31.8 g, 178 mmol) and benzoyl benzeborocarbon (1.92 g, 5.95 mmol) were sequentially added to a solution of methyl 4-fluoro-2-methylbenzoate (20 g, 119 mmol) in ClCH₂CH₂Cl (100 ml). The reactants were heated and refluxed for 3 hours. The point at which the red color disappears is the time when the reaction is completed. After cooling, the reactants were washed with water, dried with MgSO₄, and concentrated under reduced pressure. Purify the product with MPLC (HX/EA EA 0 -> 5%) (22 g, yield 75%). MS (ESI, m/z): [M+1]⁺ = [248.4].

### 5-3) Preparation of methyl 3-fluoro-2-formylbenzoate

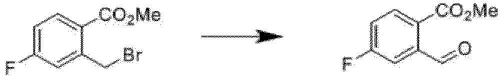

At room temperature, NMO (15.6 mg, 134 mmol) and 4Å molecular sieve were sequentially applied to methyl 2-(bromomethyl)-4-fluorobenzoate (22 g, 89 mmol) solution in DCM (100 ml). The reactants were stirred at room temperature for 2 hours. The molecular sieve was filtered and washed with DCM (50ml). The DCM layer was washed with water (200ml), dried with MgSO₄, and concentrated under reduced pressure. The residue was purified by column chromatography, and the title compound was obtained as a white solid. (12g, 73.98%) MS (ESI, m/z): [M+1]⁺ = [183.2].

### 5-4) Preparation of 4-fluoro-2-formylbenzoic acid

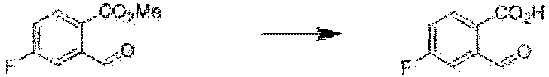

At room temperature, an aqueous solution of lithium (+1) hydroxyside (13.8 g, 329 mmol) was added to a solution of methyl 4-fluoro-6-formylbenzoate (12 g, 65.9 mmol) in THF (50 ml). After 2 h of agitation, the reactants were decompressively concentrated to dry the THF. After cooling to 0°C, the reactants were acidified with 1N HCl and the pH was adjusted to 4. The reactants were extracted twice with ethyl acetate (50ml). The combined ethyl acetate layer was dried with MgSO4, decompressively concentrated, and white crystals were obtained (10g, 90.3%). MS (ESI, m/z): [M+1]⁺ = [169.2].

### 5-5) Preparation of 6-fluoro-1,2-dihydrophthalazine-1-one

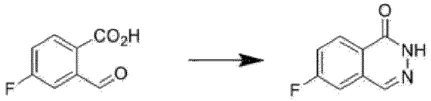

At room temperature, NH₂NH₂ monohydrate (2.02 g, 40.3 mmol) was added to a 4-fluoro-2-formylbenzoic acid (6.78 g, 40.3 mmol) solution in methanol (50 ml). After stirring for 30 minutes, the reactants were heated to 80°C for 2 hours. The reactants were cooled to room temperature and concentrated under reduced pressure. The residue was poured into water (150ml) and extracted twice with ethyl acetate (150ml). The combined ethyl acetate layer was dried with MgSO4, concentrated under reduced pressure, and white crystals were obtained (5.5g, 83.07%). MS (ESI, m/z): [M+1]⁺ = [165.3].

### 5-6) 3-(6-fluoro-1-oxopthalazine-2(1H)-yl)piperidine-2,6-dione preparation

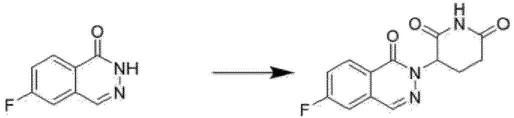

Sodium 2-methylpropane-2-oleate (175 mg, 0.914 mmol) was added to 6-fluorine-1,2-dihydrophoptalazin-1-one (100 mg, 0.609 mmol) solution in DMF (2 ml) at 0°C. After stirring for 30 minutes, 3-bromoperidin-2,6-dione (90 mg, 0.471 mmol) was added to the reaction mixture and stirred at room temperature for 6 hours. Water (20 ml) was poured into the reaction mixture, and ethyl acetate (20 ml) was extracted twice. The combined ethyl acetate was dried with MgSO₄ and concentrated under reduced pressure. The residue was purified by chromatography. The title compound was obtained as white crystals (110 mg, 65.5%). MS (ESI, m/z): [M+1]⁺ = [276.6].
[NMR] ¹H NMR (400 MHz, DMSO-d₆) δ 11.11 (s, 1H), 8.50 (s, 1H), 8.38 (dd, J=8.86, 5.44 Hz, 1H), 7.72 - 7.89 (m, 2H), 5.70 - 5.90 (m, 11H), 2.56 - 2.70 (m, 2H), 2.11 - 2.25 (m, 1H)

**Example A-6: Preparation of a compound**

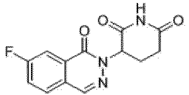

### 6-1) Preparation of methyl 4-fluoro-6-methylbenzoate

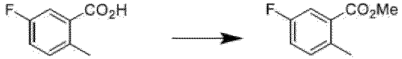

In accordance with the preparation method of Example 4-1, methyl 5-fluoro-2-methylbenzoate was prepared from 5-fluoro-2-methylbenzoate.

### 6-2) Preparation of methyl 2-(bromomethyl)-5-fluorobenzoate

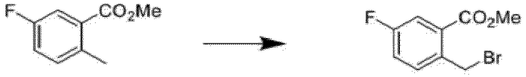

At room temperature, 1-bromophilolidine-2,5-dione (31.8 g, 178 mmol) and benzoyl benzene carboroxoate (1.92 g, 5.95 mmol) were sequentially added to a solution of methyl 5-fluoro-2-methylbenzoate (20 g, 119 mmol) in ClCH₂CH₂Cl (100 ml). The reactants were heated and refluxed for 3 hours. The point when the red color disappears is the time when the reaction is complete. After cooling, the reactants were washed with water, dried with MgSO₄, and concentrated under reduced pressure. Purified product with MPLC (HX/EA EA 0 -> 5%) (29 g, yield 98.8%). MS (ESI, m/z): [M+1]⁺ = [248.4].

### 6-3) Preparation of methyl 5-fluoro-2-formylbenzoate

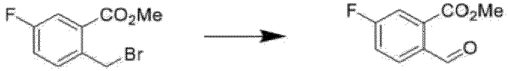

At room temperature, NMO (20.6mg, 176mmol) and 4Å molecular sieve were sequentially added to methyl 2-(bromomethyl)-5-fluorobenzoate (29g, 117mmol) solution in DCM (100ml). The reactants were stirred at room temperature for 2 hours. The molecular sieve was filtered and washed with DCM (50ml). The DCM layer was washed with water (200ml), dried with MgSO4, and concentrated under reduced pressure. The residue was purified by column chromatography, and the title compound was obtained as a white solid. (15g, yield 70.16%) MS (ESI, m/z): [M+1]⁺ = [183.2].

### 6-4) Preparation of 5-fluoro-2-formylbenzoic acid

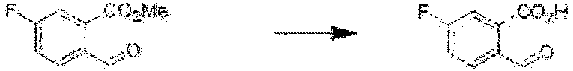

At room temperature, an aqueous solution of lithium (+1) hydroxide (17.3 g, 412 mmol) was added to a solution of methyl 5-fluoro-2-formylbenzoate (15 g, 82.3 mmol) in THF (50 ml). After 2 h of agitation, the reactants were decompressively concentrated to dry the THF. After cooling to 0°C, the reactants were acidified with 1N HCl and the pH was adjusted to 4. The reactants were extracted twice with ethyl acetate (50ml). The combined ethyl acetate layer was dried with MgSO4, concentrated under reduced pressure, and white crystals were obtained (12g, 86.67%). MS (ESI, m/z): [M+1]⁺ = [169.2].

### 6-5) Preparation of 7-Fluoro-1,2-dihydroptalazine-1-one

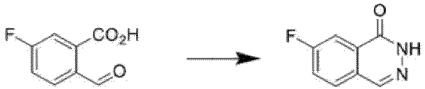

At room temperature, NH₂NH₂ monohydrate (3.74 g, 74.8 mmol) was added to a 5-fluoro-2-formylbenzoic acid (8.16 g, 48.5 mmol) solution in methanol (50 ml). After stirring for 30 minutes, the reactants were heated to 80°C for 2 hours. The reactants were cooled to room temperature and concentrated under reduced pressure. The residue was poured into water (150ml) and extracted twice with ethyl acetate (150ml). The combined ethyl acetate layer was dried with MgSO₄, concentrated under reduced pressure, and white crystals were obtained (6.5 g, 81.59%). MS (ESI, m/z): [M+1]⁺ = [165.3].

### 6-6) Preparation of 3-(7-fluoro-1-oxopthalazine-2(1H)-yl)piperidine-2,6-dione

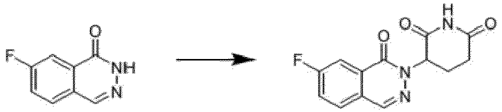

Sodium 2-methylpropane-2-oleate (586mg, 6.09mmol) was added to 7-fluorone-1,2-dihydrophoptalazin-1-one (500mg, 3.05mmol) solution in DMF (5ml) at 0°C. After stirring for 30 minutes, 3-bromoperidin-2,6-dione (1.05mg, 5.48mmol) was added to the reaction mixture and stirred at room temperature for 6 hours. The reaction mixture was poured into water (50ml) and extracted twice with ethyl acetate (50ml). The combined ethyl acetate was dried with MgSO₄ and concentrated under reduced pressure. The residues were purified by column chromatography. The title compound was obtained as white crystals (300 mg, 35.78%). MS (ESI, m/z): [M+1]⁺ = [276.6].
[NMR] ¹H NMR (400 MHz, DMSO-d₆) δ 11.09 (s, 1H), 8.53 (s, 1H), 8.13 (dd, J=8.74, 5.20 Hz, 1H), 7.87 - 8.00 (m, 2H), 5.76 - 5.88 (m, 11H), 2.54 - 2.68 (m, 2H), 2.10 - 2.20 (m ,1H)

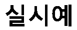 **A-7: Preparation of a compound** 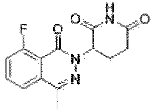 **(prepared according to Equation 2)**

### 7-1) Preparation of methyl 2-acetyl-6-fluorobenzoate

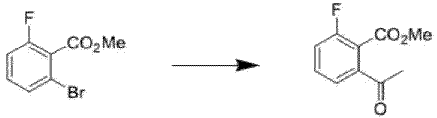

Tetrakis (triphenylphosphine)-palladium (0) (557 mg, 0.48 mmol) was added to a solution of methyl 2-acetyl-6-fluorobenzoate (1.12 g, 4.81 mmol) and tributyl (1-ethoxyvinyl) tin (1.91 g, 5.29 mmol) in toluene (20 ml) and stirred at 100°C for 16 hours. After cooling, 5 ml of 1N-HCl was added and stirred for 1 hour. The organic layer was dried with MgSO₄ and concentrated under reduced pressure. The residue was purified by silica column chromatography, and the heading compound was obtained as yellow oil. (820mg, yield 87%) MS (ESI, m/z): [M+1]⁺ = [196.8].

### 7-2) Preparation of 2-acetyl-6-fluorobenzoic acid

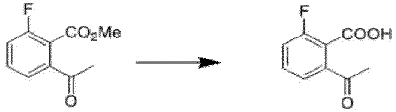

LiOH (494mg, 20.6mmol) was added to the solution of methyl 2-acetyl-6-fluorobenzoate (810mg, 4.13mmol) in THF (20ml) and water (10ml), and stirred for 20 hours at room temperature. The solution was acidified to 1N-HCl so that the pH was about 3. 100mL EA was added, the organic layer was dried with MgSO₄, and the title compound was obtained by decompression concentration. (810mg yield 108%) MS (ESI, m/z): [M+1]⁺ = [183.1].

### 7-3) Preparation of 8-fluoro-4-methylphthalazine-1(2H)-one

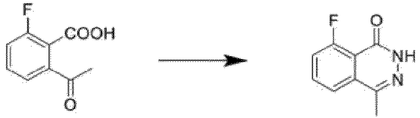

Hydrazine monohydrate (261 mg, 5.20 mmol) was added to a solution of 2-acetyl-6-fluorobenzoic acid (790 mg, 4.34 mmol) in methanol (23 ml) and stirred at room temperature for 16 hours. The sediment was filtered, washed with ACN (acetonitrile), and the title compound was obtained as a white solid. (612mg, yield 79.2%) MS (ESI, m/z): [M+1]⁺ = [179.1].

### 7-4) Preparation of 3-(8-fluoro-4-methyl-1-oxopthalazine-2(1H)-yl)piperidine-2,6-dione

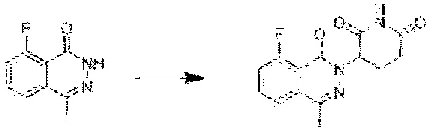

1M-Lithium diisopropylamide (3.6 ml, 3.6 mmol) was added to 8-fluoro-4-methylphthalazine-1(2H)-one (534 mg, 3 mmol) solution in THF (30 ml) at 0°C and stirred for 20 minutes. 3-brobopiperidine-2,6-dione (863 mg, 4.5 mmol) was added to the solution and stirred at 80°C for 2 hours. The reactants were concentrated under the reduced pressure and dried. Water (10 mL) was added and stirred for 1 hour, and then acidified with 1N-HCl to adjust the pH to 4. The sediment was filtered, washed with water, and the title compound was obtained as a white solid (760 mg, yield: 86.5%). MS (ESI, m/z): [M+1]⁺ = [289.8].
[NMR] ¹H NMR (400 MHz, DMSO-d₆) δ 11.05 (s, 1H), 8.04-7.94 (m, 1H), 7.79 (d, J = 7.9 Hz, 1H), 7.69 (dd, J = 7.9, 11.3 Hz, 1H), 5.71 (be dd, J = 4.9, 12.1 Hz, 1H), 3.0 - 2.83 (m, 1H), 2.64 - 2.56 (m, 2H), 2.55 (s, 3H), 2.17 - 2.05 (m, 1H).

**Example A-8: Preparation of a compound**

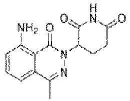

### 8-1) Preparation of 3-(8-((2,4-dimethoxybenzyl)amino)-4-methyl-1-oxopthalazine-2(1H)-yl)piperidine-2,6-dione

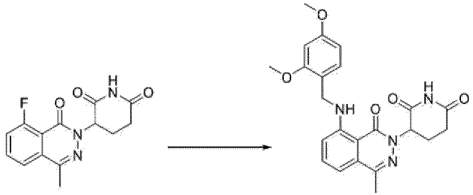

3-(8-fluoro-4-methyl-1-oxopthalazine-2(1H)-yl)piperidine-2,6-dione (0.104 mmol), 2,4-dimethoxybenzylamine (0.207 mmol) and DIPEA (N,N-diisopropylethylamine) (0.312 mmol) solutions in NMP (N-methyl-pyrrolidone) (1 mL) were stimulated with microwaves for 2 hours at 120°C. The reaction mixture was purified by reversed-phase chromatography (C18, water (0.1% FA) / ACN (0.1% FA), gradient) to obtain 33 mg white solid (33 mg, yield 72%). MS (ESI, m/z): [M+1]⁺ = [437.0]

### 8-2) Preparation of 3-(8-amino-4-methyl-1-oxopthalazine-2(1H)-yl)piperidine-2,6-dione

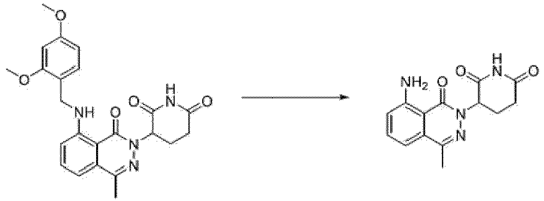

0.3ml of TFA (trifluoroacetic acid) was added to 3-(8-((2,4-dimethoxybenzyl)amino)4-methyl-1-oxotalasin-2(1H)-yl)piperidine-2,6-dione (14mg, 0.032mmol) in toluene (0.7mL) and stirred at 80°C for 1 hour. The reaction mixture was purified by reversed-phase column chromatography (C18, water (0.1% FA) / ACN (0.1% FA), gradient) yielded 7.5 mg of white solid (7.5 mg, yield 82%). MS (ESI, m/z): [M+1]⁺ = [287.0]
[NMR] ¹H NMR (400 MHz, DMSO-d₆) δ10.98 (s, 1H), 7.54 (t, J =8.0 Hz, 1H), 7.37 (brs, 2H), 6.95 (d, J = 8.2 Hz, 1H), 6.88 (d, J = 7.6 Hz, 1H), 5.62 (br dd, J = 5.3, 12.0 Hz, 1H), 3.0 - 2.82 (m, 1H), 2.66 - 2.52 (m,2H), 2.39 (s, 3H), 2.11 - 2.02 (m, 1H)

**Example A-9: Preparation of a compound**

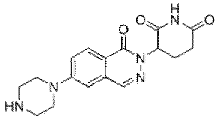

### 9-1) Preparation of tert-butyl 4-(2-(2,6-dioxopiperidine-3-yl)-1-oxo-1,2-dihydroptalazine-6-yl)piperazine-1-carboxylate

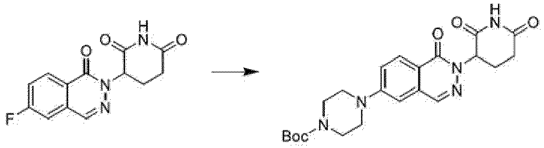

6-Fluoro-1,2-dihydroptalazine-1-one (100 mg, 0.36 mmol) and tert-butylpiperazine-1-carboxylate (81.2 mg, 0.36 mmol) were dissolved in NMP (1 mL), DIPEA (5 equivalents) was added to the reaction mixture, and stirred overnight at 120°C. The reaction of the reaction mixture was terminated with water, extracted with EA, and washed with NH4Cl saturated aqueous solution and brine solution. The organic layer was dried with MgSO₄. The reaction mixture was loaded into silica, separated by MPLC (HX/EA 30% -> 50% for 10 min), and the oil product was obtained (110 mg, yield 66%).

### 9-2) Preparation of 3-(1-oxo-6-(piperazine-1-yl)phthalazine-2(1H)-yl)piperidine-2,6-Dion

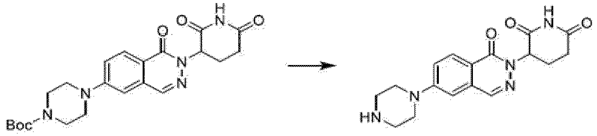

Tert-butyl 4-[2-(2,6-dioxopiperidine-3-yl)-1-oxo-1,2-dihydrophthalazine-6-yl]piperazine-1-carboxylate was dissolved in a 20% TFA solution in DCM (1 ml) and reacted for 2 hours at room temperature. The reaction was terminated with an aqueous solution saturated with NaHCO3 and extracted with EA. The organic layer was dried with MgSO4 and evaporated. By purifying the reaction mixture with MPLC (MC/ME Me 0 -> 10%). The product of white solids was obtained (40 mg, yield 86%) MS (ESI, m/z): [M+1]⁺ = [342.2].
[NMR] ¹H NMR (400 MHz, DMSO-d₆) δ 8.24 (s, 1 H) 8.02 (d, J=9.05 Hz, 1 H) 7.47 (dd, J=8.93, 2.57 Hz, 1 H) 7.31 (s, 0.5 H) 7.23 (d, J=2.45 Hz, 1 H) 7.13 (s, 0.5 H) 5.34 (dd, J=8.93, 4.52 Hz, 1 H) 3.28 - 3.44 (m, 6 H) 2.85 - 2.95 (m, 4 H) 2.30 - 2.40 (m, 1 H) 2.16 - 2.30 (m, 2 H)

**Example A-10:**

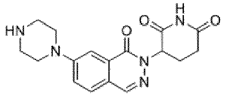

### 10-1) Preparation of tert-butyl-4-(3-(2,6-dioxopiperidine-3-yl)-4-oxo-3,4-dihydroptalazine-6-yl)piperazine-1-carboxylate

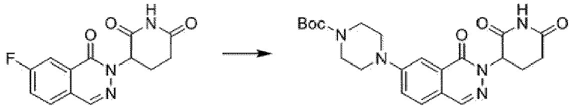

3-(7-fluoro-1-oxo-1,2-dihydroptalazine-2-yl)piperidine-2,6-dione (100 mg, 0.36 mmol) and tert-butylpiperazine-1-carboxylate (81.2 mg, 0.44 mmol) were dissolved in NMP (1 mL), DIPEA (5 equivalents) was added to the reaction mixture, and stirred overnight at 120°C. The reaction of the reaction mixture was terminated with water, extracted with EA, and washed with NH4Cl saturated aqueous solution and Brine solution. The organic layer was dried with MgSO4, the reaction mixture was loaded into silica, separated with MPLC (HX/EA 30% -> 50% for 10 min), and the product was obtained as oil (yield: 110mg, 66%).

### 10-2) Preparation of 3-(1-oxo-7-(piperazine-1-yl)phthalazine-2(1H)-yl)piperidine-2,6-dione

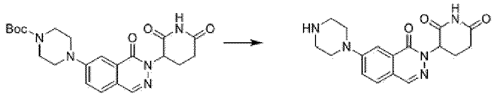

tert-butyl-4-[3-(2,6-dioxopiperidine-3-yl)-4-oxo-3,4-dihydrophthalazine-6-yl]piperazine-1-carboxylate (60 mg, 0.14 mmol) was added to 20% TFA in DCM (1 ml) and reacted for 2 hours at room temperature. The reaction was terminated with a NaHCO3 saturated aqueous solution, and extracted with EA. The organic layer was dried with MgSO4 and evaporated. The reaction mixture was purified with MPLC (MC/ME Me 0 -> 10%), and a white solid was obtained as the product (yield: 40 mg/ 86%). MS (ESI, m/z): [M+1]⁺ = [342.2].
[NMR] ¹H NMR (400 MHz, DMSO-d₆) δ 8.21 - 8.29 (m, 1 H) 7.76 (d, J=8.80 Hz, 1 H) 7.58 (dd, J=8.93, 2.57 Hz, 1 H) 7.47 (d, J=2.45 Hz, 1 H) 7.28 (s, 0.5 H) 7.13 (s, 0.5 H) 5.34 - 5.44 (m, 1 H) 3.26 - 3.33 (m, 4 H) 2.81 - 2.91 (m, 3 H) 2.62 - 2.69 (m, 1 H) 2.55 - 2.62 (m, 1 H) 2.31 - 2.44 (m, 1 H) 2.15 - 2.31 (m, 2 H)

### Example A-11: Preparation of 3-(8-methoxy-1-oxopthalazine-2(1H)-yl)piperidine-2,6-dione

**Preparation of a compound**

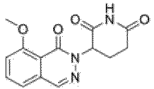

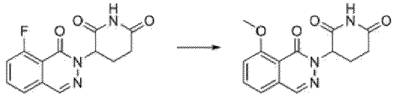

3-(6-fluoro-1-oxo-1,2-dihydroptalazine-2-yl)piperidine-2,6-dione (100 mg, 0.36 mmol) was added to DMSO (1 ml) and NaOMe (19.6 mg, 0.36 mmol) was added to the reaction mixture. The reaction mixture was reacted at room temperature for 1 hour. The organic solvent was removed, and extracted with EA and water. The mixture was purified with MPLC, and the product was obtained as a white solid (yield: 80 mg / 76%). MS (ESI, m/z): [M+1]⁺ = [288.2].
[NMR] ¹H NMR (400 MHz, DMSO-d₆) δ 11.04 (br. s., 1 H) 8.40 (s, 1 H) 8.14 - 8.25 (m, 1 H) 7.42 - 7.49 (m, 2 H) 5.80 (dd, J=12.23, 5.38 Hz, 1 H) 3.94 (s, 3 H) 2.86 - 2.99 (m, 1 H) 2.52 - 2.67 (m, 2 H) 2.07 - 2.17 (m, 1 H)

### Example A-12: Preparation of 3-(7-methoxy-1-oxopthalazine-2(1H)-yl)piperidine-2,6-dione

**Preparation of a compound**

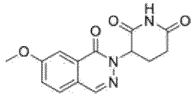

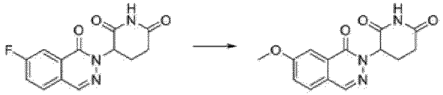

3-(7-fluoro-1-oxopthalazine-2(1H)-yl)piperidine-2,6-dione (100 mg, 0.36 mmol) was dissolved in DMSO (1 ml), and NaOMe (19.6 mg, 0.36 mmol) was added to the reaction mixture. The half-heung mixture was reacted at room temperature for 1 hour. The organic solvent was removed, and extracted with EA and water. The mixture was purified with MPLC, and the product was obtained as a white solid. (Yield: 78mg / 75%) MS (ESI, m/z): [M+1]⁺ = [288.2].
[NMR] ¹H NMR (400 MHz, DMSO-d₆) δ 11.01 - 11.11 (m, 1 H) 8.38 - 8.45 (m, 1 H) 7.90 - 7.96 (m, 1 H) 7.65 (d, J=2.69 Hz, 1 H) 7.56 (dd, J=8.68, 2.57 Hz, 1 H) 5.77 - 5.85 (m, 1 H) 3.93 - 3.98 (m, 3 H) 2.87 - 3.00 (m, 1 H) 2.53 - 2.70 (m, 2 H) 2.06 - 2.18 (m, 1 H)

### Example A-13: Preparation of 3-(6-methoxy-1-oxopthalazine-2(1H)-yl)piperidine-2,6-dione

**Preparation of a compound**

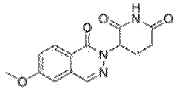

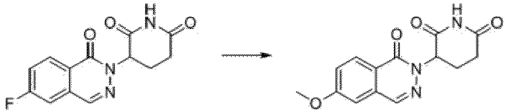

3-(6-fluoro-1-oxopthalazine-2(1H)-yl)piperidine-2,6-dione (100 mg, 0.36 mmol) was dissolved in DMSO (1 ml), and NaOMe (19.6 mg, 0.36 mmol) was added to the reaction mixture. The reaction mixture was reacted for 1 hour at room temperature. Organic solvents were removed, and EA and water were extracted. The mixture was purified with MPLC, and the product was obtained as a white solid. (Yield: 80mg / 76%) MS (ESI, m/z): [M+1]⁺ = [288.2].
[NMR] ¹H NMR (400 MHz, DMSO-d₆) δ 11.01 (s, 1 H) 8.32 (s, 1 H) 7.88 (t, J=8.01 Hz, 1 H) 7.40 (d, J=8.31 Hz, 1 H) 7.44 (d, J=7.70 Hz, 1 H) 5.64 (dd, J=11.49, 4.52 Hz, 1 H) 3.90 (s, 3 H) 2.83 - 2.96 (m, 1 H) 2.52 - 2.66 (m, 2 H) 2.03 - 2.13 (m, 1 H)

### Example A-14: Preparation of 3-(5-methoxy-1-oxopthalazine-2(1H)-yl)piperidine-2,6-dione

**Preparation of a compound**

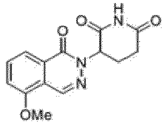

### 14-1) Preparation of Methyl 2-(Bromomethyl)-3-Methoxybenzoate

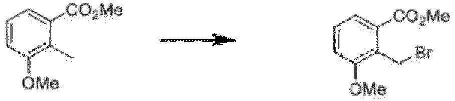

1-bromophilidine-2,5-dione (11.5 g, 64.94 mmol) and benzoyl benzene carboperoxate (786 mg, 3.24 mmol) were sequentially added to a solution of methyl 2-methyl-3-methoxybenzoate (11.7 g, 64.9 mmol) in ClCH2CH2Cl (250 ml) at room temperature. The reaction mixture was heated and refluxed for 3 hours. The point when the red color disappears is the time when the reaction is completed. After cooling, the reactants were washed with water, dried with MgSO4, and depressurized concentrated. The roughage was used in the next reaction without additional purification. (16.5 g, 98.2%) MS (ESI, m/z): [M+1]⁺= [259.4]

### 14-2) Preparation of Methyl 2-Formyl-3-Methoxybenzoate

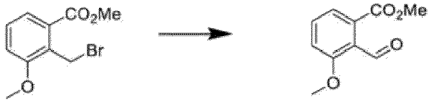

NMO (12.6 g, 108.1 mmol) and 4Å molecular sieve (50 g) were sequentially added to methyl 2-(bromomethyl)-3-methoxybenzoate (14.1 g, 54.1 mmol) solution in DCM (300 mL) at room temperature. The reactants were stirred at room temperature for 2 hours. The molecular sieve was filtered and washed with DCM (100 mL). The DCM layer was washed with water (250 mL), dried with MgSO₄, and depressurized concentrated. The residue was purified by column chromatography, and the title compound was obtained as a white solid. (8.3 g, 80.01%) MS (ESI, m/z): [M+1]⁺ = [195.0].

### 14-3) Preparation of 2-formyl-3-methoxybenzoic acid

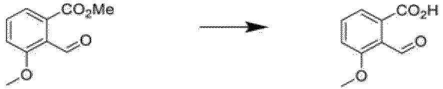

Lithium(1+) hydroxyside (2.4 g, 100.5 mmol) in H2O (100 mL) was added to a solution of methyl 2-formyl-3-methoxybenzoate (6.5 g, 33.6 mmol) in THF (100 mL) at room temperature. After 2 hours of agitation, the reactants were decompressively concentrated to evaporate the THF. After cooling to 0°C, the reactants were acidified with 1N HCl and the pH was adjusted to 4. The reactants were extracted twice with ethyl acetate (250 mL). The combined ethyl acetate layer was dried with MgSO₄, concentrated under reduced pressure, and white crystals were obtained. (11.2g, 82.6%) MS (ESI, m/z): [M+1]⁺ = [199.2].

### 14-4) Preparation of 5-methoxyphthalazine-1(2H)-one

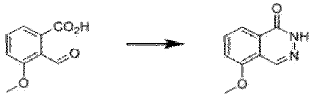

NH2NH2 monohydrate (10.3 g, 342 mmol) was added to 2-formyl-3-methoxybenzoic acid (8.2 g, 45.5 mmol) solution in MeOH (20 mL) at room temperature. After stirring for 2 hours, the reactants were decompressively concentrated, poured into water (50 ml), and extracted twice with ethyl acetate (150 mL). The combined ethyl acetate layer was dried with MgSO₄, and white crystals were obtained by decompression concentration. (9.2g, 76.35%) MS (ESI, m/z): [M+1]⁺ = [176.9].

### 14-5) Preparation of 3-(5-methoxy-1-oxopthalazine-2(1H)-yl)piperidine-2,6-dione

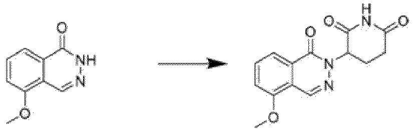

1.0 M LDA (37.4 mL) was dripped at 0°C in some suspension of 5-methoxyphthalazine-1(2H)-one (5.1 g, 28.9 mmol) in THF (250 mL). After stirring for 30 minutes, 3bromoperidin-2,6-dione was partially added to the reactants. The reactants were heated to 80°C and stirred for 2 hours. Once the reaction was finished, the reactant was cooled to room temperature, poured into water (100 ml), pH was adjusted to 3~4 with 6N HCl, extracted with ethyl acetate, dried with MgSO₄, and concentrated under reduced pressure. The residue was purified with MPLC and the title compound was obtained as a white crystal. (7.2g, yield 86.5%) MS (ESI, m/z): [M+1]⁺ = [288.3].
[NMR] 1H NMR (400 MHz, DMSO-d6) δ11.06 (s, 1H), 8.51 (s, 1H), 7.87-7.77 (m, 2H), 7.54-7.51 (m, 1H), 5.85 (m, 1H), 4.0 (s, 3H), 2.98 - 2.90 (m, 1H), 2.65 - 2.55 (m, 2H), 2.14 - 2.09 (m, 1H).

### Example A-15: Preparation of 3-(6-bromo-1-oxopthalazine-2(1H)-yl)piperidine-2,6-dione

**Preparation of a compound**

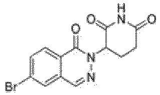

### 15-1) Preparation of 5-Bromo-3-hydroxyisobenzofuran-1(3H)-one

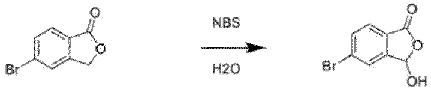

AIBN (401 mg, 2.44 mmol) was added to a mixture of 5-bromophthalide (5.2 g, 24.4 mmol) and N-bromosuccinicimide (5.6 g, 31.7 mmol) in 200 mL of ClCH₂CH₂Cl and refluxed for 8 hours. Following the reaction, TLC was carried out. The succinimide was filtered and the cake was washed with ClCH₂CH₂Cl (50 mL). The solvent was removed from the vacuum, leaving 5.2 g of residue, to which 50 ml of water was added. The mixture was stirred for 4 hours, then refluxed, the mixture was cooled, the product was filtered, neutralized and washed with water, and drying, and pale yellowish-white crystals were obtained. (4.85g, yield 86.7%) MS (ESI, m/z): [M+1]⁺ = [229.6] and [230.5]

### 15-2) Preparation of 6-bromophthalazine-1(2H)-one

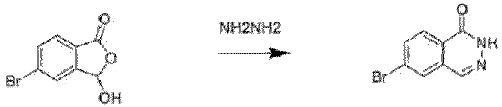

Added NH₂NH₂ H₂O (315 mg, 9.82 mmol) to 5-bromo-3-hydroxy-1,3-dihydro-2-benzofuran-1-one (1.5 g, 6.55 mmol) solution in MeOH (50 mL) at room temperature The reaction was refluxed for 5 hours. The reactants were cooled to room temperature and concentrated under reduced pressure. The resulting solids were tritured with ethyl acetate to obtain white crystals. (1.31g, 5.82 mmol) MS (ESI, m/z): [M+1]⁺ = [226.3].

### 15-3) Preparation of 3-(6-bromo-1-oxopthalazine-2(1H)-yl)piperidine-2,6 dione

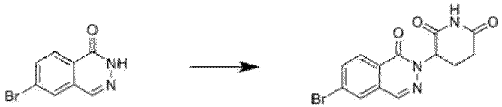

1.0 M LDA (7.33 mL) was dripped at 0°C in a 6-bromo-1,2-dihydroptalazine-1-one (1.31 g, 5.82 mmol) suspension of THF (150 mL). After stirring for 30 min, 3-bromoperidin-2,6-dione was partially added to the reactants. The reactants were heated to 80°C and stirred for 2 hours. When the reaction was finished, the reactant was cooled to room temperature, poured into water (100 ml), pH was adjusted to 3~4 with 6N HCl, extracted with ethyl acetate, dried with MgSO₄, and concentrated under reduced pressure. The resulting solids were filtered, washed with ethyl acetate, and white crystals were obtained. (1.73g, 5.15 mmol) MS (ESI, m/z): [M+1]⁺ = [337.2].
[NMR] 1H NMR (400 MHz, DMSO-d6) δ11.08 (s, 1H), 8.45 (s, 1H), 8.28 (s, 1H), 8.18-8.16 (m, 1H), 8.06-8.04 (m, 1H), 5.84-5.80 (m, 1H), 2.93 - 2.90 (m, 1H), 2.65 - 2.54 (m, 2H), 2.15 - 2.12 (m, 1H).

### Example A-16: Preparation of 3-(1-oxo-8-(piperazine-1-yl)phthalazine-2(1H)-yl)piperidine-2,6-dione 2HCl

**Preparation of a compound**

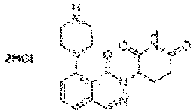

### 16-1) Preparation of 3-(1-oxo-8-(piperazine-1-yl)phthalazine-2(1H)-yl)piperidine-2,6-dione dihydrochloride

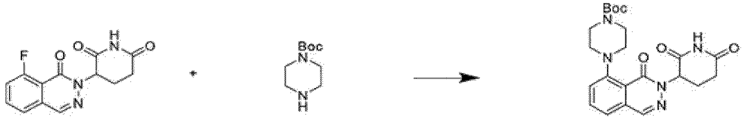

tert-butyl piperazine-1-carboxylate (244 mg, 1.13 mmol) and ethylbis (propane-2-yl)amine (423 mg, 3.24 mmol) were sequentially added to a 3-(8-fluoro-1-oxo-1,2-dihydrophthalazine-2-yl)piperidine-2,6-dione (0.3 g, 1.09 mmol) solution in DMA(4 mL). The mixture was stirred at 120°C for 5 hours. The reaction mixture was diluted with water and extracted with ethyl acetate. The organic layer was washed with brine solution, dried with Na₂SO₄, and concentrated. The residue was purified by column chromatography to obtain a pale yellowish-white oil. (415 mg, 86.2%)MS (ESI, m/z): [M+1]⁺ = [442.4]

### 16-2) Preparation of 3-(1-oxo-8-(piperazine-1-yl)phthalazine-2(1H)-yl)piperidine-2,6-dione dihydrochloride

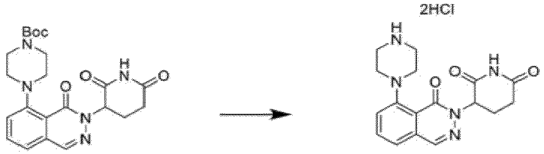

3-bromoperidin-2,6-dione (90 mg, 0.471 mmol) and K₂CO₃ (129 mg, 0.942 mmol) were sequentially added to 8-nitro-1,2-dihydrophthalazine-1-one (60 mg, 0.314 mmol) solution in DMF (1 mL). The reaction was heated to 85°C for 5 hours. After cooling, the reaction mixture was poured into water (5 mL) and extracted twice with ethyl acetate (5 mL). The combined ethyl acetate was extracted with MgSO₄ and concentrated under reduced pressure. The residue was purified by column chromatography, and the title compound was obtained as white crystals. (68.0 mg, yield 71.7%) MS (ESI, m/z): [M+1]⁺ = [342.6].
[NMR] 1H NMR (400 MHz, DMSO-d6) δ11.02 (s, 1H), 8.35 (s, 1H), 7.87-7.83 (m, 1H), 7.53-7.51 (m, 1H), 7.39-7.41 (m, 1H), 5.55 (m, 1H), 3.31- 3.27 (br, 8H), 2.88 - 2.80 (m, 1H), 2.64 - 2.57 (m, 2H), 2.50 (br, 1H), 2.13 - 2.08(m, 1H).

### Example B: Preparation of BRD4 PROTAC based on WNY derivatives

### Example B-1: Synthesis of 4-(((R)-5-(cyclohexylmethyl)-4-ethyl-1-methyl-4,5-dihydro-[1,2,4]triazolo[4,3-f]pteridine-7-yl)amino)N-(5-(2-(2-(2,6-dioxopiperidine-3-yl)-1-oxo-1,2-dihydroptalazine-6-yl)pent-4-phosphorus-1-yl)-3-methoxybenzamide (ICT-0001515)

### Step 1) Synthesis of methyl (R)-4-((5-(cyclohexylmethyl)-4-ethyl-1-methyl-4,5-dihydro-[1,2,4]triazolo[4,3-f]pteridine-7-yl)amino)-3-methoxybenzoate

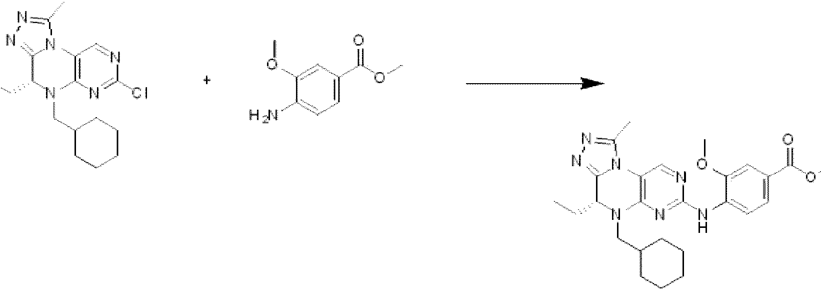

Dicyclohexyl [2',4',6'-Tris(propane-2-yl)-[1,1'-biphenyl]-2-yl]phospan (103 mg, 216 umol) was added to (4R)-7-chloro-5-(cyclohexyl)-4-ethyl-1-methyl-4H,5H-[1,2,4]triazol[4,3-f]pteridine (1.5 g, 4.32 mmol) and methyl 4-amino-3-methoxybenzoate (862 mg, 4.76 mmol) in toluene (10 ml) at room temperature. It was extracted with ethyl acetate, dried with MgSO₄, and then concentrated under reduced pressure. The residue was purified with MeOH/DCM (0-10%) MPLC to obtain the title compound (1.52 g). MS (ESI, m/z): [M+H]⁺ = [492.6].

### Step 2) Synthesis of (R)-4-((5-(cyclohexylmethyl)-4-ethyl-1-methyl-4,5-dihydro-[1,2,4]triazolo[4,3-f]pteridine-7-yl)amino)-3-methoxybenzoic acid

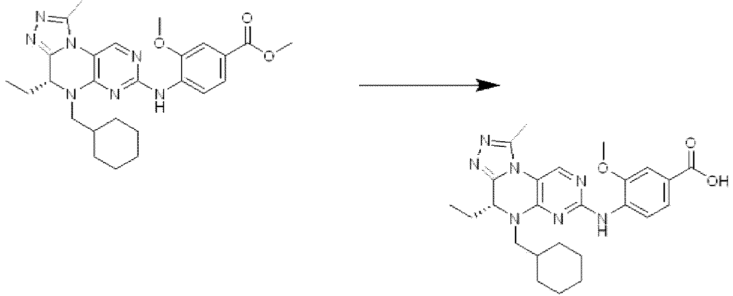

Lithium (1+) hydrate hydroxide (427 mg, 10.2 mol) in water (10 mL) was added to a solution of methyl 4-{[(4R)-5-(cyclohexylmethyl)-4-ethyl-1-methyl-4H,5H-[1,2,4]triazolo[4,3-f]pteridine-7-yl]amino}-3-methoxybenzoate (1.0 g, 2.03 mmol) in THF (10 mL) at room temperature. The reactants were stirred at 80°C for 16 hours. The reactants were cooled to room temperature and concentrated until the THF was removed. The generated residues were acidified to 1.0 M HCl and adjusted to pH=5. The resulting solids were filtered. The compound of purpose was obtained by vacuum drying (0.81 g). MS (ESI, m/z): [M+H]⁺= [478.6].

### Step 3) Synthesis of tert-butyl N-{5-[2-(2,6-dioxopiperidin-3-yl)-1-oxo-1,2-dihydroptalazine-6-yl]pent-4-phospho-1-yl}carbamate

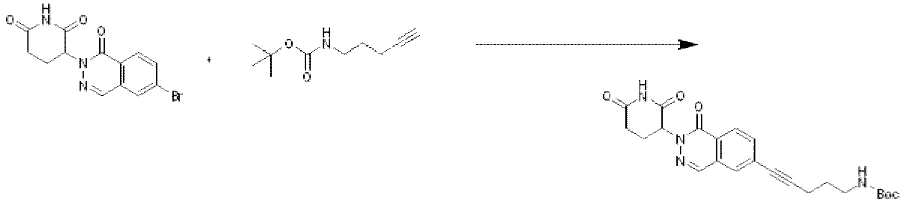

Cul (255 mg, 1.34 mmol) and Pd(PPh3)2Cl2 (940 mg, 1.34 mmol) were added to 3-(6-bromo-1-oxo-1,2-dihydroptallazine-2-yl) piperidine-2,6-dione (4.5 g, 13.4 mmol) solution in THF (150 mL), followed by TEA (4.06 g, 40.2 mmol). The mixture was stirred at 90°C for 3 hours. The reactants were diluted with water, extracted with ethyl acetate. The organic layer was washed with brine solution, dried with Na₂SO₄. It was concentrated. The residue was purified with EA/Hex (20~80%) MPLC to obtain the desired compound (3.9 g). MS (ESI, m/z): [M+H]⁺ = [439.5].

### Step 4) Synthesis of 3-[6-(5-aminopent-1-phosphorus-1-yl)-1-oxo-1,2-dihydroptalazine-2-yl]piperidine-2,6-dione

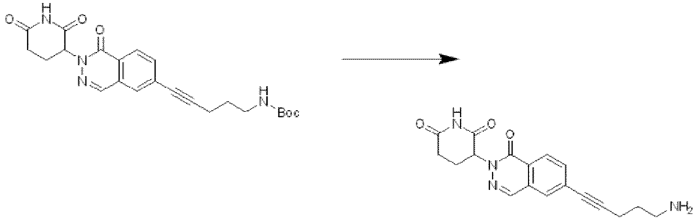

Tert-butyl 3-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-il}piperazine-1-yl)methyl]azetidine-1-carboxylate (3.9 g, 8.89 mmol) solution in 30% TFA in DCM were stirred at room temperature for 2 hours. After the reaction was completed, it was concentrated under reduced pressure. The residue was purified by DCM/MeOH (0~5%) amine column chromatography to obtain the title compound (3.0 g). MS (ESI, m/z): [M+H]+ = [339.4]

### Step 5) Synthesis of 4-(((R)-5-(cyclohexylmethyl)-4-ethyl-1-methyl-4,5-dihydro-[1,2,4]triazolo[4,3-f]pteridine-7-yl)amino)-N-(5-(2-(2,6-dioxopiperidin-3-yl)-1-oxo-1,2-dihydroptalazine-6-yl)pent-4-phosphorus-1-yl)-3-methoxybenzamide (ICT-0001515)

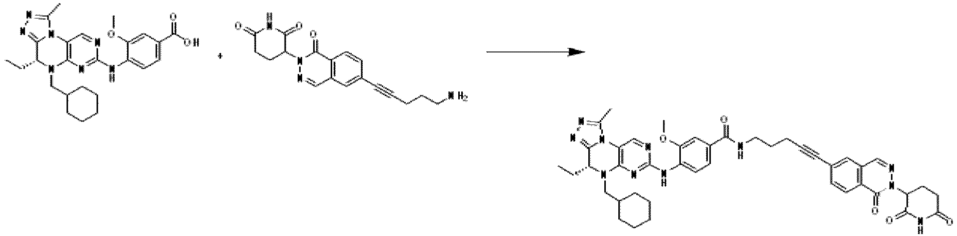

HATU (71.7 mg, 188 µmol) was added to 4-{[(4R)-5-(cyclohexylmethyl)-4-ethyl-1-methyl-4H,5H-[1,2,4]triazolo[4,3-f]pteridine-7-yl}-3-methoxybenzoic acid (75 mg, 157 µmol) and 3-[6-(5-aminopent-1-phospho-1-yl)-1-oxo-1,2-dihydropthalazine-2-yl]piperidine-2,6-dione (53.1 mg, 157 µmol) in 1 ml of DMF, followed by ethylbis (propane-2-yl)amine (40 mg, 314 µmol) at room temperature. The reactants were stirred for 6 hours. The reactants were poured into the water. It was extracted with ethyl acetate, dried with MgSO₄, and then concentrated under reduced pressure. The residue was purified with MeOH/DCM (0-10%) MPLC to obtain the title compound (89 mg). MS (ESI, m/z): [M+H]⁺= [798.9]
1H NMR (500 MHz, DMSO-d6) δ ppm 11.07 (s, 1 H) 8.47 - 8.53 (m, 1 H) 8.41 (s, 1 H) 8.34 (dd, J=8.16, 6.33 Hz, 1 H) 8.17 - 8.25 (m, 1 H) 7.95 (s, 1 H) 7.91 (d, J=5.80 Hz, 1 H) 7.79 - 7.87 (m, 1 H) 7.47 - 7.56 (m, 2 H) 5.76 - 5.86 (m, 1 H) 4.90 (dd, J=8.01, 3.59 Hz, 1 H) 4.11 (dd, J=13.28, 5.49 Hz, 1 H) 3.90 - 3.98 (m, 2 H) 3.45 (d, J=6.10 Hz, 2 H) 2.87 - 3.04 (m, 2 H) 2.70 (s, 2 H) 2.54 - 2.66 (m, 3 H) 2.06 - 2.19 (m, 1 H) 1.78 - 1.92 (m, 3 H) 1.63 - 1.74 (m, 4 H) 1.59 (br. s., 1 H) 1.23 (s, 1 H) 1.00 - 1.21 (m, 3 H) 0.90 - 0.99 (m, 1 H) 0.75 (t, J=7.25 Hz, 2 H)

### Example B-2: Synthesis of 3-(6-(4-((4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-il)methyl)piperidine-1-yl)4-methyl-1-oxopthalazine-2(1H)-yl)piperidine-2,6-dione (ICT-0001516)

### Step 1) Synthesis of benzyl 4-(piperidine-4-monomethyl)piperazine-1-carboxylate

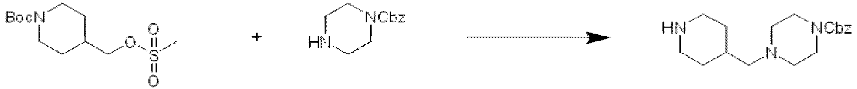

K₂CO₃ (16.4 g, 119.5 mmol) was added to tert-butyl 4-[(methanesulfonyoxy)methyl]piperidine-1-carboxylate (7.0 g, 23.9 mmol) and benzyl piperazine-1-carboxylate (5.26 g, 23.9 mmol) solution in 50 ml of acetonitrile at room temperature, and the reactants were stirred at 80°C for 6 hours. The Boc-protected compound was treated with 50% TFA in DCM (20 mL) and stirred for 5 hours. After the reaction was finished, the reactants were concentrated under reduced pressure and vacuum-dried. The residue was dissolved in DCM, passed through an NH-DM 1020 Chromatorex pad, and the title compound was obtained (6.01 g). MS (ESI, m/z): [M+H]⁺ = [318.4].

### Step 2) Synthesis of 3-(4-methyl-1-oxo-6-(4-(piperazine-1-ylmethyl)piperidine-1-yl)phthalazine-2(1H)-yl)piperidine-2,6-dione

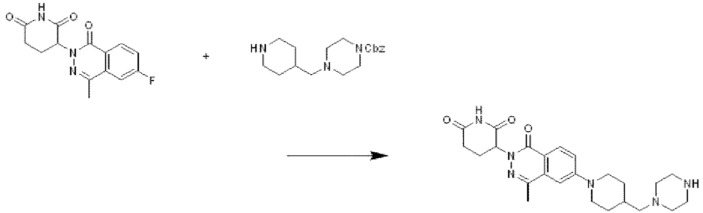

Ethylbis (propane-2-yl)amine (6.89 g, 20.7 mmol) was added to 3-(6-fluoro-4-methyl-1-oxo-1,2-dihydroptalazin-2-yl)piperidine-2,6-dion acid (2.0 g, 6.91 mmol) and 4-(piperazine-1-yl)aniline (2.19 g, 6.91 mmol) solutions in 5 ml of DMA at room temperature. The reactants were stirred at 130°C for 16 hours. The reactants were poured into water, extracted with ethyl acetate, dried with MgSO₄, and then concentrated under reduced pressure. The residues were purified with MPLC to obtain a CBZ-protected compound. The compound was dissolved in MeOH (50 m). 10% Pd/C (100 mg) was added. The reactants were stirred under an H2 balloon for 5 hours. The reactants were filtered, concentrated under the reduced pressure, and subjected compounds were obtained (2.3 g). MS (ESI, m/z): [M+H]⁺ = [453.7].

### Step 3) Synthesis of 3-(6-(4-((4-(R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-il)methyl)piperidine-1-yl)4-methyl-1-oxopthalazine-2(1H)-yl)piperidine-2,6-dione (ICT-0001516)

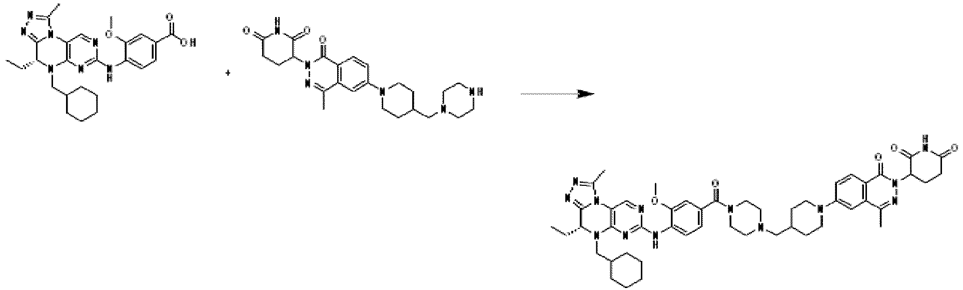

HATU (95.5 mg, 251 µmol) is added to 4-{[(4R)-5-(cyclohexylmethyl)-4-ethyl-1-methyl-4H,5H-[1,2,4]triazolo[4,3-f]pteridin-7-yl]amino}-3-methoxybenzoic acid (100 mg, 209 µmol) and 3-(4-methyl-1-ioxo-6-{4-[(piperazine-1-yl)methyl]piperidine-1-yl}-1,2-dihydroptalazin-2-yl}piperidine-2,6-dione (98.4 mg, 209 µmol) in 1 mL of DMF at room temperature, followed by ethylbis (propane-2-yl)amine (54.1 mg, 419 µmol). The reactants were stirred for 6 hours. The reactants were poured into water, extracted with ethyl acetate, dried with MgSO₄, and then concentrated under reduced pressure. The residue was purified with MeOH/DCM (0-10%) MPLC to obtain the title compound (130 mg). MS (ESI, m/z): [M+H]⁺= [913.1]
1H NMR (500 MHz, DMSO-d6) δ ppm 10.98 (s, 1 H) 8.36 - 8.43 (m, 1 H) 8.28 (d, J=8.24 Hz, 1 H) 8.05 (d, J=9.00 Hz, 1 H) 7.90 - 8.00 (m, 1 H) 7.48 (d, J=9.16 Hz, 1 H) 7.06 (d, J=5.80 Hz, 2 H) 6.96 (d, J=8.24 Hz, 1 H) 5.63 - 5.73 (m, 1 H) 4.89 (dd, J=8.09, 3.66 Hz, 1 H) 4.02 - 4.14 (m, 3 H) 3.91 (s, 3 H) 3.39 - 3.44 (m, 1 H) 2.89 - 3.00 (m, 4 H) 2.69 (s, 4 H) 2.53 - 2.64 (m, 3 H) 2.48 (s, 4 H) 2.39 (br. s., 3 H) 2.15 - 2.24 (m, 2 H) 2.01 - 2.12 (m, 1 H) 1.96 - 2.01 (m, 1 H) 1.93 (d, J=15.11 Hz, 1 H) 1.83 (d, J=9.00 Hz, 4 H) 1.63 - 1.73 (m, 5 H) 1.61 (br. s., 1 H) 1.19 - 1.25 (m, 4 H) 1.08 - 1.16 (m, 3 H) 0.74 (t, J=7.32 Hz, 3 H)

### Example B-3: Synthesis of 3-((4-(4-(4-((((R)-5-(cyclohexylmethyl)-4-ethyl-1-methyl-4,5-dihydro-[1,2,4]triazolo[4,3-f]pteridine-7-yl)amino)-3-methoxybenzoyl)piperazine-1-yl)-3-fluorophenyl)amino)piperidine-2,6-dione (ICT-0001517)

### Step 1) Synthesis of tert-butyl 4-(2-fluoro-4-nitrophenyl)piperazine-1-carboxylate

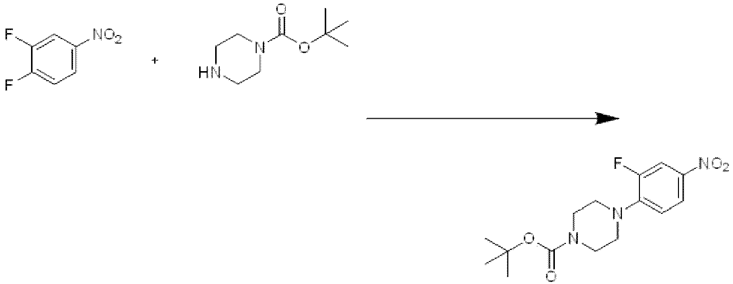

K₂CO₃ (17.4 g, 126 mmol) was added to tert-butylpiperazine-1-carboxylate (5 g, 31.4 mmol) and 1,2-difluoro-4-nitrobenzene (5.85 g, 31.4 mmol) solutions in acetonitrile (10 ml). The reactants were stirred at 80°C for 2 hours. After cooling to room temperature, the reactants were decompressively concentrated, and then purified by column chromatography to obtain the title compound (9.3 g). MS (ESI, m/z): [M+H]⁺= [326.3]

### Step 2) Synthesis of tert-butyl 4-(4-amino-2-fluorophenyl)piperazine-1-carboxylate

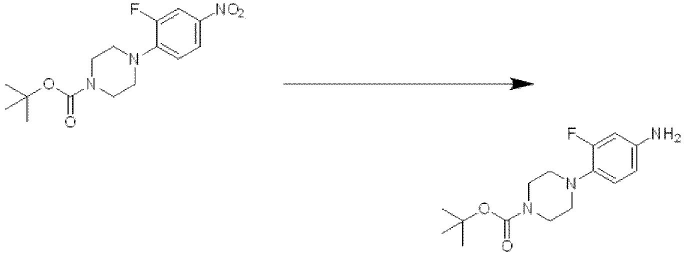

20 mL saturated NH₄Cl was added to tert-butyl 4-(2-fluoro-4-nitrophenyl)piperazine-1-carboxylate (3 g, 9.22 mmol) and iron (10.3 g, 1840 mmol) solutions in THF (2 ml). The reactants were stirred at 100°C for 2 hours. After cooling to room temperature, the reactants were filtered, the filtrate was poured into water, and extracted with ethyl acetate. The organic layer was dried with MgSO₄, concentrated under reduced pressure, and then purified by column chromatography to obtain the title compound (2.6 g) MS (ESI, m/z): [M+H]⁺= [296.4].

### Step 3) Synthesis of tert-butyl 4-(2-fluoro-4-nitrophenyl)piperazine-1-carboxylate

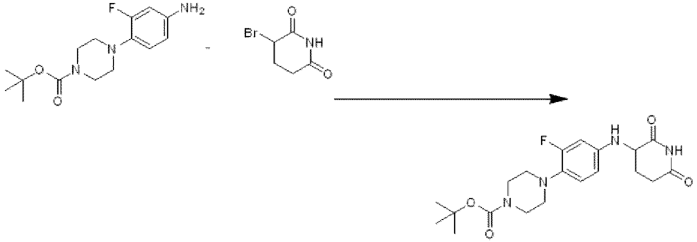

NaHCO₃ (4.27 g, 50.8 mmol) was added to tert-butyl 4-(4-amino-2-fluorophenyl)piperazine-1-carboxylate (3 g, 10.2 mmol) and 3-bromoperidin-2,6-dione (3.9 g, 20.3 mmol) solution in 10 ml of DMF at room temperature, followed by subsequently. The reactants were stirred at 80°C for 3 hours. The reactants were poured into water, extracted with ethyl acetate, dried with MgSO₄, and then concentrated under reduced pressure. The residues were purified with MeOH/DCM (0-10%) MPLC to obtain the heading compound (3.4 g) MS (ESI, m/z): [M+H]⁺= [407.5].

### Step 4) Synthesis of 3-{[3-fluoro-4-(piperazine-1-yl)phenyl]amino}piperidine-2,6-dione

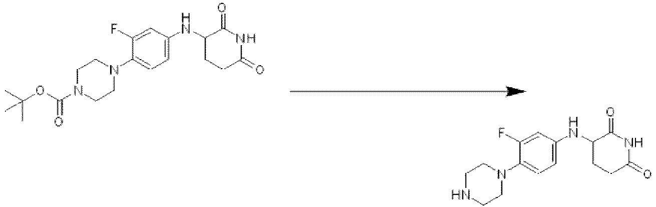

The reactants were stirred at room temperature for 2 hours in a solution of tert-butyl-4-{4-[(2,6-dioxopiperidine-3-yl)amino]-2-fluorophenyl}piperazine-1-carboxylate (3.4 g, 8.89 mmol) in 30% TFA in DCM. After the reaction was completed, it was concentrated under reduced pressure. The residue was purified by DCM/MeOH (0~5%) amine column chromatography to obtain the title compound (3.0 g). MS (ESI, m/z): [M+H]⁺= [307.3].

### Step 5) Synthesis of 3-((4-(4-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)-3-fluorophenyl)amino)piperidine-2,6-dione (ICT-0001517)

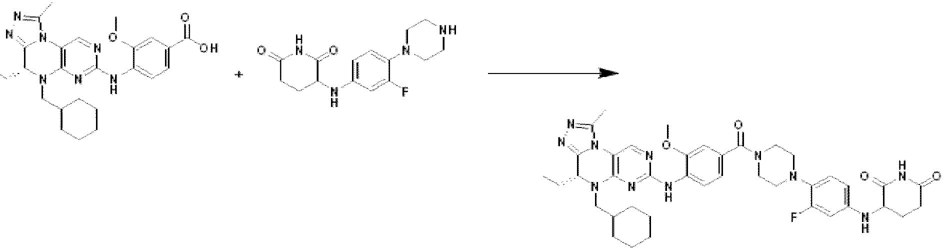

HATU (95.5 mg, 251 µmol) was added to 4-{[(4S)-5-(cyclohexylmethyl)-4-ethyl-1-methyl-4H,5H-[1,2,4]triazolo[4,3-f]pteridine-7-yl]amino}-3-methoxybenzoic acid (100 mg, 209 µmol) and 3-{[3-fluoro-4-(piperazine-1-yl)phenyl]amino}piperidine-2,6-dione (64.1 mg, 209 µmol) in 1 ml of DMF at room temperature. It was extracted with ethyl acetate, dried with MgSO₄, and then concentrated under reduced pressure. The residual acid was purified with MeOH/DCM (0~10%) MPLC to obtain the title compound (123 mg). MS (ESI, m/z): [M+H]⁺= [766.9]
1H NMR (500 MHz, DMSO-d6) δ ppm 10.79 (s, 1 H) 8.40 (s, 1 H) 8.30 (d, J=8.24 Hz, 1 H) 7.94 (s, 2 H) 7.07 - 7.13 (m, 2 H) 7.00 (d, J=8.24 Hz, 2 H) 6.87 (t, J=9.31 Hz, 1 H) 6.53 (dd, J=14.95, 2.29 Hz, 1 H) 6.44 (dd, J=8.70, 2.14 Hz, 1 H) 5.88 (d, J=7.78 Hz, 1 H) 4.89 (dd, J=8.24, 3.81 Hz, 2 H) 4.23 - 4.32 (m, 1 H) 4.10 (dd, J=13.58, 6.10 Hz, 1 H) 3.92 (s, 5 H) 2.68 - 2.80 (m, 6 H) 2.57 (dt, J=17.39, 3.89 Hz, 1 H) 2.04 - 2.14 (m, 2 H) 1.99 (s, 1 H) 1.75 - 1.92 (m, 4 H) 1.24 (s, 1 H) 1.01 - 1.21 (m, 5 H) 0.95 (d, J=11.90 Hz, 1 H) 0.75 (t, J=7.32 Hz, 5 H)

### Example B-4 : Synthesis of 3-((4-(4-((4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-il)methyl)piperidine-1-yl)-3-fluorophenyl)amino)piperidine-2,6-dione (ICT-0001518)

### Step 1) Synthesis of tert-butyl 4-{[1-(2-fluoro-4-nitrophenyl)piperidine-4-yl]methyl}piperazine-1-carboxylate synthesis

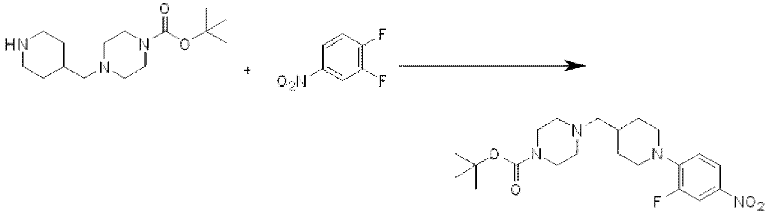

1,2-difluoro-4-nitrobenzene (5.61 g, 35.3 mmol) and potassium carbonate (24.4 g, 176 mmol) were added to tert-butyl 4-[(piperidine-4-yl)methyl]piperazine-1-carboxylate (10 g, 35.3 mmol) solution in DMF (200 mL). The reactants were stirred at 80°C for 2 hours. After the reaction was completed, the reactants were poured into water, extracted with ethyl acetate (2 times), dried with MgSO₄, and then concentrated under reduced pressure. The residues were purified by column chromatography to obtain the title compound (11 g). MS (ESI, m/z): [M+H]⁺ = 423.5

### Step 2) Synthesis of tert-butyl 4-{[1-(4-amino-2-fluorophenyl)piperidine-4-yl]methyl}piperazine-1-carboxylate

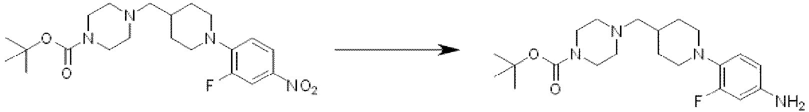

Iron powder (29.5 g, 521 mmol) was added to tert-butyl 4-{[1-(2-fluoro-4-nitrophenyl)piperidine-4-yl]methyl}piperazine-1-carboxylate (11 g, 26 mmol) in saturated NH4Cl/THF (1:1) (200 mL). The reactants were stirred at 80°C for 2 hours. After the end of the reaction, the reactants were decompressively concentrated. The crude substances were used in the next step without additional purification. (MS (ESI, m/z): [M+H]⁺= 393.5.

### Step 3) Synthesis of tert-butyl 4-[(1-{4-[(2,6-dioxopiperidine-3-yl)amino]-2-fluorophenyl}piperidine-4-yl)methyl]piperazine-1-carboxylate synthesis

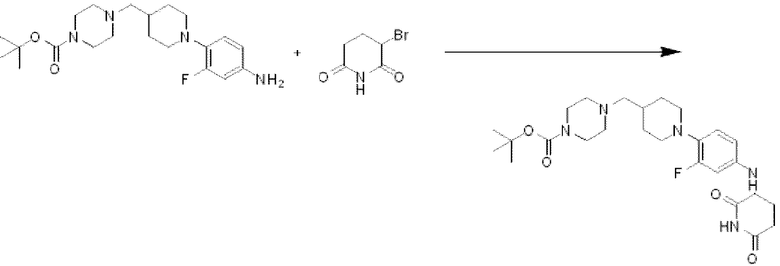

3-bromoperidin-2,6-dione (1.2 g, 6.1 mmol) was added to tert-butyl 4-{[1-(4-amino-2-fluorophenyl)piperidine-4-yl]methyl}piperazine-1-carboxylate (1.2 g, 3.1 mmol) in DMF (25 mL). The reactants were stirred at 85°C overnight. After the reaction was completed, the reactants were poured into water, extracted with ethyl acetate (twice), dried with MgSO₄, and then concentrated under reduced pressure. The residues were purified by column chromatography to obtain the title compound (1.3 g). MS (ESI, m/z): [M+H]⁺ = 504.6

### Step 4) Synthesis of 3-[(3-fluoro-4-{4-[(piperazine-1-yl)methyl]piperidine-1-yl}phenyl)amino]piperidine-2,6-dione

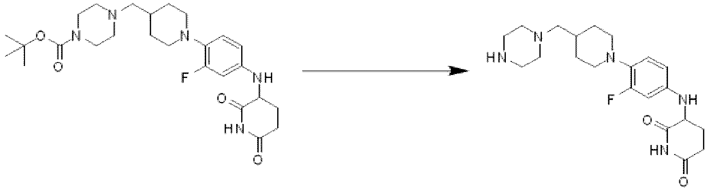

The reactants were stirred at room temperature for 2 hours in a solution of tert-butyl 4-[(1-{4-[(2,6-dioxopiperidine-3-yl)amino]-2-fluorophenyl}piperidine-4-yl)methyl]piperazine-1-carboxylate (650 mg, 1.29 mmol) in 30% TFA in DCM. After the reaction was completed, it was concentrated under reduced pressure. The residue was purified by DCM/MeOH (5-10%) amine column chromatography to obtain the title compound (485 mg). MS (ESI, m/z): [M+H]⁺ = 404.5.

### Step 5) Synthesis of 3-((4-(4-((4-(4-((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-il)methyl)piperidine-1-yl)-3-fluorophenyl)amino)piperidine-2,6-dione (ICT-0001518)

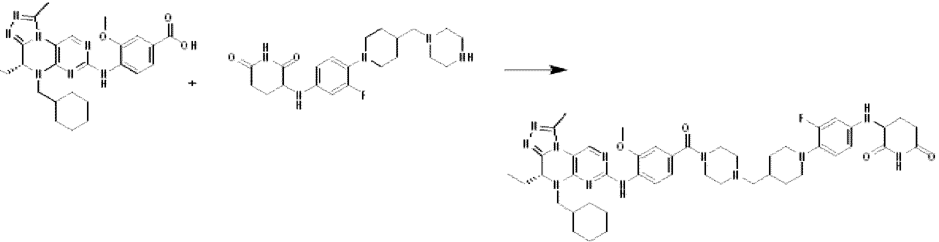

HATU (79.6 mg, 209 µmol) was added to 4-{[(4R)-5-(cyclohexylmethyl)-4-ethyl-1-methyl-4H,5H-[1,2,4]triazolo[4,3-f]pteridine-7-yl]amino}-3-methoxybenzoic acid (100 mg, 209 µmol) and 3-[6-(5-aminopent-1-phosphoin-1-yl)-1-oxo-1,2-dihydroptalazine-2-yl]piperidine-2,6-dione (84.5 mg, 209 µmol) in 1 ml of DMF at room temperature, followed by ethylbis (propane-2-yl)amine (51.4 mg, 419 µmol). The reactants were stirred for 6 hours. The reactants were poured into the water. It was extracted with ethyl acetate, dried with MgSO₄, and then concentrated under reduced pressure. The residual acid was purified with MeOH/DCM (0-10%) MPLC to obtain the title compound (111 mg). MS (ESI, m/z): [M+H]⁺ = [864.1]
1H NMR (500 MHz, DMSO-d6) δ ppm 10.79 (s, 1 H) 8.39 (s, 2 H) 8.28 (d, J=8.24 Hz, 2 H) 7.94 (s, 1 H) 7.06 (s, 2 H) 6.95 (d, J=8.24 Hz, 2 H) 6.83 (t, J=9.31 Hz, 2 H) 6.50 (dd, J=14.95, 2.14 Hz, 1 H) 6.41 (d, J=8.55 Hz, 1 H) 5.79 (d, J=7.63 Hz, 1 H) 4.89 (dd, J=8.09, 3.81 Hz, 1 H) 4.21 - 4.29 (m, 1 H) 3.99 - 4.14 (m, 2 H) 3.91 (s, 5 H) 3.11 (d, J=11.14 Hz, 3 H) 2.97 (dd, J=13.58, 8.39 Hz, 1 H) 2.70 (s, 5 H) 2.53 - 2.60 (m, 4 H) 2.21 (d, J=6.87 Hz, 3 H) 2.05 - 2.12 (m, 2 H) 1.98 - 2.01 (m, 1 H) 1.21 - 1.30 (m, 4 H) 1.13 (d, J=7.63 Hz, 3 H) 0.94 (d, J=11.75 Hz, 1 H) 0.75 (t, J=7.32 Hz, 5 H)

### Example B-5 : 4-(((R)-5-(cyclohexylmethyl)-4-ethyl-1-methyl-4,5-dihydro-[1,2,4]triazolo[4,3-f]pteridine-7-yl)amino)N-(1-(5-(2-(2,6-dioxopiperidin-3-yl)1-oxo-1,2-dihydroptalazine-6-yl)pent-4-phosphorus-1-yl)piperidine-4-yl)-3-methoxybenzamide (ICT-0001534) synthesis

### Step 1) Synthesis 3-[(3-fluoro-4-{4-[(piperazine-1-yl)methyl]piperidine-1-yl}phenyl)amino]piperidine-2,6-dione

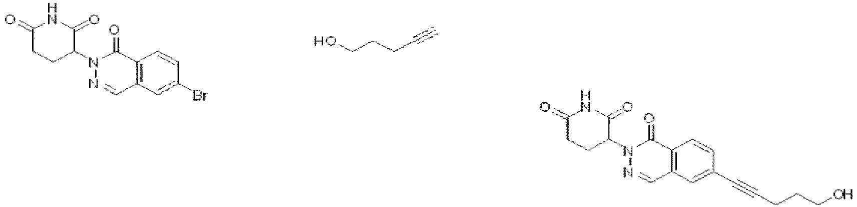

Cul (85 mg, 446 µmol) and Pd(PPh₃)₂Cl₂ (313 mg, 446 µmol) were added to 3-(6-bromo-1-oxo-1,2-dihydrophoptalazin-2-yl) piperidine-2,6-dione (1.5 g, 4.46 mmol) and pent-4-phospho-1-ol (488 mg, 5.8 mmol) solutions in DMF (5 mL), followed by TEA (1.35 g, 13.4 mmol). Ethyl acetate. The organic layer was washed with brine solution, dried with Na₂SO₄, and concentrated. The residue was purified with EA/Hexane (0-10%) MPLC to obtain the title compound (1.3 g). MS (ESI, m/z): [M+H]⁺= [340.4],

### Step 2) Synthesis of 5-(2-(2,6-dioxopiperidin-3-yl)-1-oxo-1,2-dihydroptalazine-6-yl)pent-4-phospho-1-yl methanesulfonate synthesis

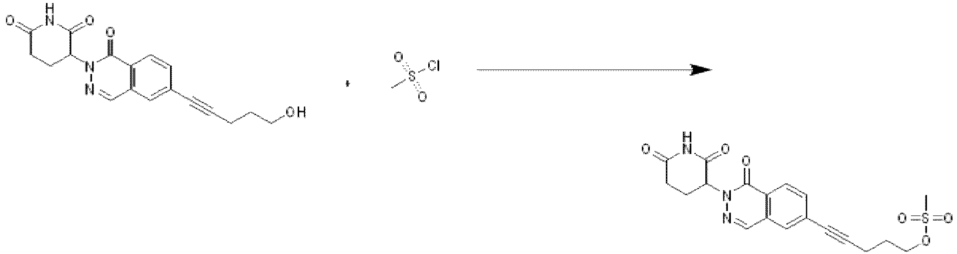

Methanesulfonyl chloride (1.32 g, 11.5 mmol) in CH₂Cl₂ (15 mL) was added to the [3-(5-(5-bromomolimidine-2-yl)phenyl]methanol (1.3 g, 3.83 mmol) solution in CH₂Cl₂ (50 mL), followed by trimethylamine (1.49 g, 11.5 mmol) at 0°C, and then stirred for 2 hours. The reactants were warmed to room temperature and stirred for an additional 2 hours. The reactants were poured into water (50 mL), extracted with dichloromethane (50 mL*3). The organic layers were combined, washed with brine solution (50 mL x 2), dried with sodium sulfate anhydrous, then filtered and concentrated. The residue was used in the next step without additional purification. MS (ESI, m/z): [M+H]⁺= [418.1],

### Step 3) Synthesis of 4-(((R)-5-(cyclohexylmethyl)-4-ethyl-1-methyl-4,5-dihydro-[1,2,4]triazolo[4,3-f]pteridine-7-yl)amino)N-(1-(5-(2-(2,6-dioxopiperidin-3-yl)1-oxo-1,2-dihydroptalazine-6-yl)pent-4-phosphorus-1-yl)piperidine-4-yl)-3-methoxybenzamide (ICT-0001534)

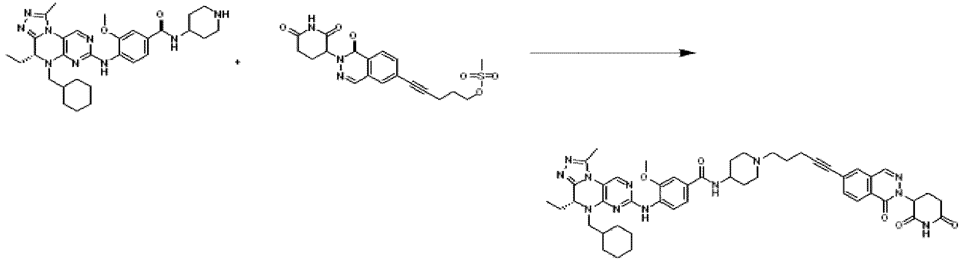

Ethylbis (18.5 mg, 143 µmol) was added to 4-{[(4R)-5-(cyclohexylmethyl)-4-ethyl-1-methyl-4H,5H-[1,2,4]triazolo[4,3-f]pteridine-7-yl]amino}-3-methoxy-N- (piperidine-4-yl)benzamide (40 mg, 71.5 µmol) and 5-[2-(2,6-dioxopiperidine-3-yl)-1-oxo-1,2-dihydroptalazine-5-yl]pent-4-phospholipidine-5-yl]pent-4-phospholipinate-1-yl methanesulfonate (29.8 mg, 71.5 µmol) at room temperature. The reactants were stirred at 65°C for 6 hours. The reactants were poured into the water. It was extracted with ethyl acetate, dried with MgSO₄, and then concentrated under reduced pressure. The residue was purified with MeOH/DCM (0-10%) MPLC to obtain the title compound (49 mg). MS (ESI, m/z): [M+H]⁺ = [882.1]
1H NMR (500 MHz, DMSO-d6) δ ppm 11.07 (s, 1 H) 8.47 - 8.53 (m, 1 H) 8.41 (s, 1 H) 8.34 (dd, J=8.16, 6.33 Hz, 1 H) 8.17 - 8.25 (m, 1 H) 7.95 (s, 1 H) 7.91 (d, J=5.80 Hz, 1 H) 7.79 - 7.87 (m, 1 H) 7.47 - 7.56 (m, 2 H) 5.76 - 5.86 (m, 1 H) 4.90 (dd, J=8.01, 3.59 Hz, 1 H) 4.11 (dd, J=13.28, 5.49 Hz, 1 H) 3.90 - 3.98 (m, 2 H) 3.45 (d, J=6.10 Hz, 2 H) 2.87 - 3.04 (m, 2 H) 2.70 (s, 2 H) 2.54 - 2.66 (m, 3 H) 2.06 - 2.19 (m, 1 H) 1.78 - 1.92 (m, 3 H) 1.63 - 1.74 (m, 4 H) 1.59 (br. s., 5 H) 1.23 (s, 5 H) 1.00 - 1.21 (m, 3 H) 0.90 - 0.99 (m, 1 H) 0.75 (t, J=7.25 Hz, 2 H)

### Example B-6: Synthesis of 4-(((R)-5-(cyclohexylmethyl)-4-ethyl-1-methyl-4,5-dihydro-[1,2,4]triazolo[4,3-f]pteridine-7-yl)amino)-N-(1-((3-(2,6-dioxopiperidin-3-yl)1-methyl-4-oxo-3,4-dihydroptalazin-5-yl)glycil)-4-methylpiperidine-4-yl)-3-methoxybenzamide (ICT-0001547)

### Step 1) Synthesis of (3-(2,6-dioxopiperidin-3-yl)-1-methyl-4-oxo-3,4-dihydroptalazine-5-yl)glycine

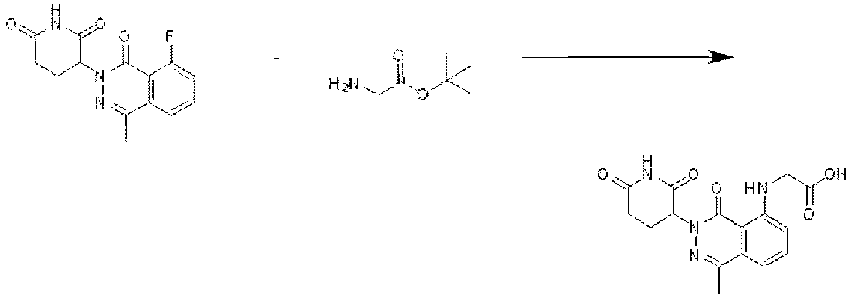

Tert-butyl 2-aminoacetate (118 mg, 899 umol) was added to 3-(8-fluoro-4-methyl-1-oxo-1,2-dihydroptalazine-2-yl)piperidine-2,6-dione (0.2 g, 691 umol) solution in DMA (2 mL) at room temperature, followed by ethylbis (propane-2-yl) amine (179 mg, 1.88 mmol). The reactants were heated to 110°C for 8 hours. The reactants were cooled to room temperature, poured into water (50 mL), and extracted with ethyl acetate (30 ml x 2). The bound organic layer was dried with MgSO₄, and concentrated under the reduced pressure. The residues were purified by column chromatography to obtain intermediates, which were treated with 50% TFA in CH₂Cl₂ (4 mL). The reactants were stirred for 2 hours, followed by decompression concentration, and the title compound was obtained (189 mg). MS (ESI, m/z): [M+H]⁺ = [345.3].

### Step 2) Synthesis of 3-(8-((2-(4-amino-4-methylpiperidin-1-yl)-2-oxoethyl)amino)-4-methyl-1-oxopthalazine-2(1H)-yl)piperidine-2,6-dione dihydrochloride

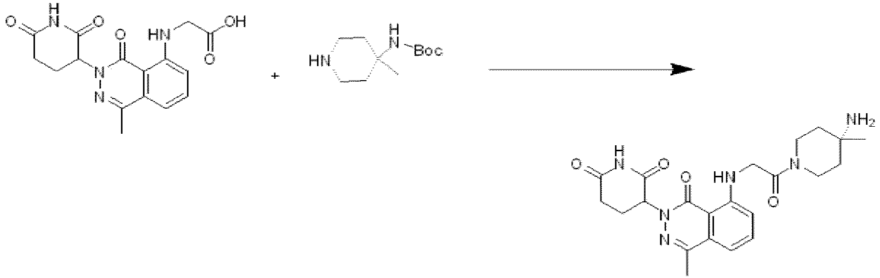

HATU was added to a solution of 2-{[3-(2,6-dioxopiperidine-3-yl)-1-methyl-4-oxo-3,4-dihydroptalazin-5-yl]amino}acetic acid (250 mg, 726 umol) and tert-butyl N-(4-methylpiperidine-4-yl)carbamate (156 mg, 726 umol) in 2 ml of DMF at room temperature, followed by ethylbis (propane-2-yl)amine (93.8 mg, 726 imol). The reactants were stirred for 6 hours. The reactants were poured into water, extracted with ethyl acetate, and dried with MgSO₄. Next, decompression and concentration were performed. The residue was purified with MeOH/DCM (0~10%) MPLC to obtain the title compound. The product was reacted with 4.0M HCl (10 eq) for 4 hours. The reactants were decompressively concentrated, the residues were dissolved in DCM, and the subject compounds were obtained by passing them through an NH-DM 1020 Chromatorex pad (6.01 g). MS (ESI, m/z): [M+H]⁺ = [441.2].

### Step 3) Synthesis of 4-(((R)-5-(cyclohexylmethyl)-4-ethyl-1-methyl-4,5-dihydro-[1,2,4]triazolo[4,3-f]pteridine-7-yl)amino)N-(1-((3-(2,6-dioxopiperidin-3-yl)1-methyl-4-oxo-3,4-dihydropetalazine-5-yl)glycy)-4-methylpiperidine-4-yl)-3-methoxybenzamide (ICT-0001547)

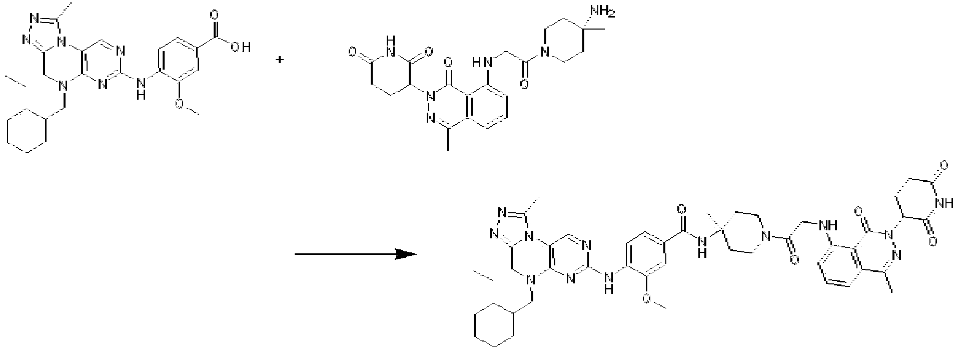

HATU (79.6 mg, 209 µmol) is added to 4-{[(4R)-5-(cyclohexylmethyl)-4-ethyl-1-methyl-4H,5H-[1,2,4]triazolo[4,3-f]pteridine-7-yl]amino}-3-methoxybenzoic acid (100 mg, 209 µmol) and 3-(8-((2-(4-(4-amino-4-methylpiperidin-1-yl)-2-oxoethyl)amino)4-methyl-1-oxopthalazine-2(1H)-yl)piperidine-2,6-dione (84.5 mg, 209 µmol), followed by ethylbis(propane-2-yl)amine (51.4 mg, 419 µmol). The reactants were stirred for 6 hours. The reactants were poured into water, extracted with ethyl acetate, dried with MgSO₄, and then concentrated under reduced pressure. The residual acid was purified with MeOH/DCM (0-10%) MPLC to obtain the title compound (111 mg). MS (ESI, m/z): [M+H]⁺= [864.1]
1H NMR (500 MHz, DMSO-d6) δ ppm 10.94 (s, 1 H) 8.99 - 9.04 (m, 1 H) 8.31 - 8.35 (m, 2 H) 8.19 - 8.26 (m, 2 H) 7.86 - 7.87 (m, 1 H) 7.60 (t, J=8.09 Hz, 1 H) 6.98 - 7.05 (m, 2 H) 6.83 - 6.94 (m, 4 H) 4.82 (dt, J=7.90, 3.76 Hz, 2 H) 3.98 - 4.08 (m, 2 H) 3.81 - 3.88 (m, 5 H) 3.39 (br. s., 1 H) 2.78 - 2.94 (m, 3 H) 2.58 (d, J=16.63 Hz, 2 H) 2.47 - 2.55 (m, 1 H) 2.28 - 2.39 (m, 4 H) 1.98 - 2.03 (m, 1 H) 1.73 (dd, J=6.94, 2.82 Hz, 1 H) 1.49 - 1.67 (m, 9 H) 1.13 - 1.24 (m, 2 H) 1.00 - 1.09 (m, 3 H) 0.67 (t, J=7.32 Hz, 5 H)

### Example B-7: Synthesis of 3-(6-(4-(((S)-4-(4-(R)-5-(cyclohexylmethyl)-4-ethyl-1-methyl-4,5-dihydro-[1,2,4]triazolo[4,3-f]pteridine-7-yl)amino)3-methoxybenzoyl)morpholine-2-il)methyl)piperazine-1-yl)4-methyl-1-oxopthalazine-2(1H)-yl)piperidine-2,6-dione (ICT-0001548) synthesis

### Step 1) Synthesis of tert-butyl(2S)-2-[(methanesulfonyoxy)methyl]morpholine-4-carboxylate

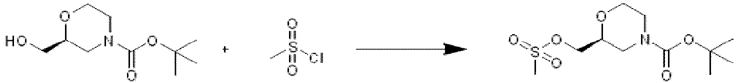

Methanesulfonyl chloride (4.43 g, 38.7 mmol) in CH2Cl2 (20 mL) was added to [tert-butyl(2S)-2-(hydroxymethyl)morpholine-4-carboxylate (7 g, 32.2 mmol) solution in DCM (100 mL), followed by triethylamine (4.89 g, 48.3 mmol) at 0°C, stirred for 2 hours. Reactants were poured into water (50 mL), extracted with dichloromethane (50 mLx3), organic layers were bonded, washed with brine solution (50 mL x 2). It was dried with sodium sulfate anhydrous, then filtered, and concentrated. The residue was used in the next step without additional purification. (8.9 g) MS (ESI, m/z): [M+H]⁺ = [296.4].

### Step 2) Synthesis of benzyl 4-{[(2S)-morpholine-2-yl]methyl}piperazine-1-carboxylate

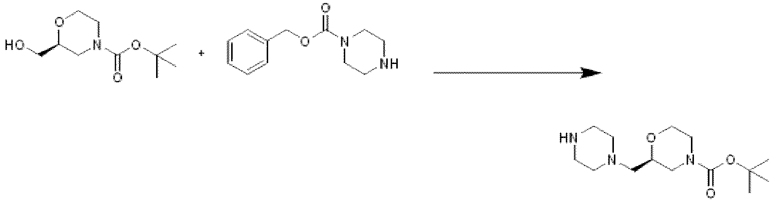

K₂CO₃ (8.32 g, 60.2 mmol) was added to tert-butyl(2S)-2-[(methanesulfonyoxy)methyl]morpholine-4-carboxylate (8.9 g, 30.1 mmol) and benzyl piperazine-1-carboxylate (6.63 g, 30.1 mmol) solution in 20 ml of DMF at room temperature, and then the reactants were stirred at 80°C for 6 hours. The Boc-protected compound was treated with 50% TFA in DCM (20 mL) and stirred for 5 hours. After the reaction was finished, the reactants were concentrated under reduced pressure and vacuum-dried. The residue was dissolved in DCM and passed through an NH-DM 1020 Chromatorex pad to obtain the heading compound (6.01 g). MS (ESI, m/z): [M+H]⁺ = [319.4].

### Step 3) Synthesis of Benzyl 4-{[(2S)-morpholine-2-yl]methyl}piperazine-1-carboxylate

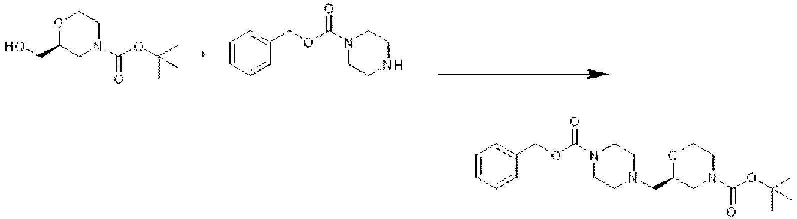

K₂CO₃ (8.32 g, 60.2 mmol) was added to tert-butyl(2S)-2-[(methanesulfonyoxy)methyl]morpholine-4-carboxylate (8.9 g, 30.1 mmol) and benzyl piperazine-1-carboxylate (6.63 g, 30.1 mmol) solution in 20 ml of DMF at room temperature, and then the reactants were stirred at 80°C for 6 hours. (6.01g). MS (ESI, m/z): [M+H]⁺ = [637.4].

### Step 4) Synthesis of Benzyl 4-{[(2S)-Morpholine-2-yl]methyl}piperazine-1-carboxylate

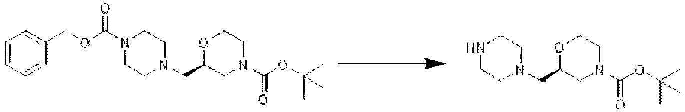

10% Pd/C (300 mg) was added to benzyl 4-{[(2S)-morpholine-2-yl]methyl}piperazine-1-carboxylate (6.01 g, 14.3 mmol) solution in MeOH (50 m). The reactants were stirred under an H₂ balloon for 5 hours. The reactants were filtered, decompressively concentrated, and subjective compounds were obtained (4.0 g). MS (ESI, m/z): [M+H]⁺ = [320.4].

### Step 5) Synthesis of 3-(4-methyl-6-(4-((R)-morpholine-2-yl)methyl)piperazine-1-yl)-1-oxopthalazine-2(1H)-yl)piperidine-2,6-dione

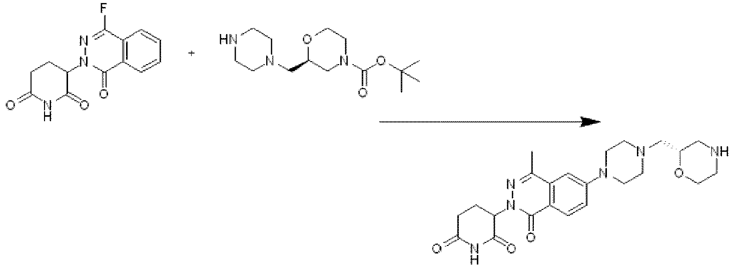

tert-butyl (2R)-2-[(piperazine-1-yl)methyl]morpholine-4-carboxylate (200 mg, 691 µmol), 3-(6-fluoro-4-methyl-1-oxopthalazine-2(1H)-yl)piperidine-2,6-dione (296 mg, 1.04 mmol), and DIPEA (268 mg, 2.07 mmol) solutions in NMP (2 mL) were stirred at 120°C for 5 hours. Stirred at room temperature for 2 hours. After the reaction was finished, the reactants were concentrated under reduced pressure and vacuum-dried. The residue was dissolved in DCM, passed through an NH-DM 1020 Chromatorex pad to obtain the heading compound, which was used in the next step without further purification. MS (ESI, m/z): [M+H]⁺ = [455.5 g/mol].

### Step 6) Synthesis of 3-(6-(4-(((S)-4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)morpholine-2-il)methyl)piperazine-1-yl)4-methyl-1-oxopthalazine-2(1H)-yl)piperidine-2,6-dione (ICT-0001548)

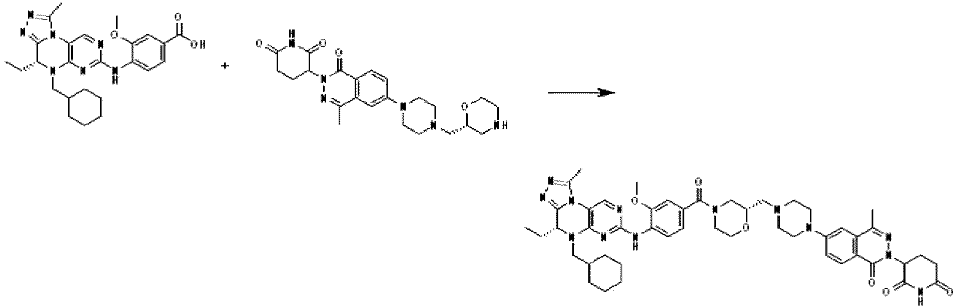

HATU (38.2 mg, 101 µmol) is added to 4-{[(4S)-5-(cyclohexylmethyl)-4-ethyl-1-methyl-4H,5H-[1,2,4]triazolo[4,3-f]pteridine-7-yl]amino}-3-methoxybenzoic acid (50 mg, 105 µmol) and 3-[4-methyl-6-(4-{(2S)-morpholine-2-yl]methyl}piperazine-1-yl)-1-oxo-1,2-dihydroplopthalazine-2-yl]piperidine-2,6-dione (38.1 mg, 83.8 µmol) in 1 ml of DMF at room temperature, followed by ethylbis (propane-2-yl)amine (21.7 mg, 168 µmol). The reactants were stirred for 6 hours. The reactants were poured into water, extracted with ethyl acetate, dried with MgSO₄, and then concentrated under reduced pressure. The residue was purified with MeOH/DCM (0-10%) MPLC to obtain the title compound (52 mg). MS (ESI, m/z): [M+H]⁺ = [915.1]
1H NMR (500 MHz, DMSO-d6) δ ppm 10.91 (s, 1 H) 8.28 - 8.34 (m, 2 H) 8.25 (d, J=8.09 Hz, 1 H) 7.85 (s, 2 H) 6.98 - 7.06 (m, 2 H) 6.92 (d, J=8.39 Hz, 1 H) 4.81 (br. s., 1 H) 4.01 (dd, J=13.35, 6.48 Hz, 1 H) 3.79 - 3.90 (m, 5 H) 2.78 - 2.93 (m, 2 H) 2.59 - 2.63 (m, 5 H) 2.47 - 2.59 (m, 4 H) 2.40 (br. s., 3 H) 1.95 - 2.04 (m, 1 H) 1.58 (d, J=9.77 Hz, 4 H) 1.05 (d, J=7.63 Hz, 2 H) 0.60 - 0.71 (m, 5 H) 0.00 (s, 1 H)

### Example B-8: Synthesis of 4-(((R)-5-(cyclohexylmethyl)-4-ethyl-1-methyl-4,5-dihydro-[1,2,4]triazolo[4,3-f]pteridine-7-yl)amino)N-(2-(2-(2-(2-((3-(2,6-dioxopiperidin-3-yl)-4-oxo-3,4-dihydroptalazin-5-yl)amino)ethoxy)ethoxy)ethoxy)ethoxy)ethoxy)ethyl)ethyl)3-methoxybenzamide (ICT-0001549)

### Step 1) Synthesis of 3-(8-((2-(2-(2-(2-(2-(2-(2-(2-Aminoethoxy)ethoxy)ethoxy)ethyl)amino)-1-oxopthalazine-2(1H)-yl)piperidine-2,6-dion hydrochloride

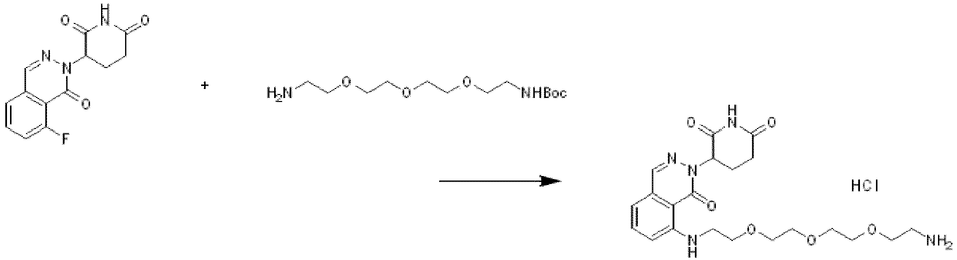

Ethyl bis(propane-2-yl)amine (285 mg, 2.2 mol) was added to 3-(8-fluoro-4-methyl-1-oxo-1,2-dihydroptallazine-2-yl)piperidine-2,6-dione (0.2 g, 724 umol) and tert-butyl N-(2-{2-[2-(2-aminoethoxy)ethoxy}ethyl)carbamate (387 mg, 1.32 mmol) in 1 ml of DMA at room temperature. The reactants were stirred at 120°C for 3 hours. The reactants were poured into water, extracted with ethyl acetate, and dried with MgSO₄, and concentrated under reduced pressure. The residue was purified with MeOH/DCM (0-10%) MPLC to obtain the title compound. The intermediate was dissolved in dioxane (1 mL), and 5 mL of 4.0 M HCl of dioxane was added. After stirring for 3 hours, the reactants were decompressively concentrated. The resulting solids were used in the next step without additional tablets (245 mg). MS (ESI, m/z): [M+H]⁺ = [448.1].

### Step 2) Synthesis of 4-(((R)-5-(cyclohexylmethyl)-4-ethyl-1-methyl-4,5-dihydro-[1,2,4]triazolo[4,3-f]pteridine-7-yl)amino)N-(2-(2-(2-(2-(2-((3-(2,6-dioxopiperidin-3-yl)4-oxo-3,4-dihydroptalazine-5-yl)amino)ethoxy)ethoxy)ethoxy)ethoxy)ethyl)3-methoxybenzamide (ICT-0001549)

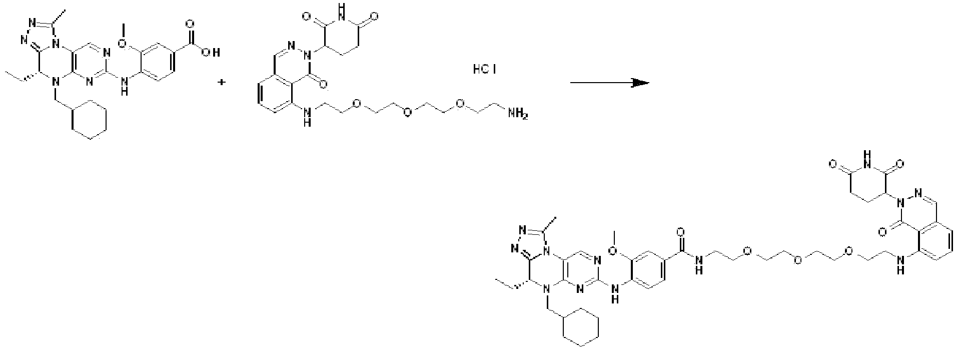

HATU (38.2 mg, 101 µmol) is added to a solution of 4-{[(4S)-5-(cyclohexylmethyl)-4-ethyl-1-methyl-4H,5H-[1,2,4]triazolo[4,3-f]pteridine-7-yl}-methoxybenzoic acid (50 mg, 105 µmol) and 3-[8-(12-amino-4,7,10-trioxa-1-azadodecane-1-yl)-1-oxo-1,2-dihydroptalazine-2-yl]piperidine-2,6-dione hydrochloride (37.5 mg, 83.8 uoml) in 1 ml of DMF at room temperature, followed by ethylbis (propane-2-yl)amine (21.7 mg, 168 µmol). The reactants were stirred for 6 hours. The reactants were poured into water, extracted with ethyl acetate, dried with MgSO₄, and then concentrated under reduced pressure. The residual acid was purified with MeOH/DCM (0~10%) MPLC to obtain the title compound (38 mg). MS (ESI, m/z): [M+H]⁺= [908.0]
1H NMR (500 MHz, DMSO-d6) δ ppm 11.03 (s, 1 H) 8.81 (t, J=5.04 Hz, 1 H) 8.46 (t, J=5.57 Hz, 1 H) 8.41 (s, 1 H) 8.35 (d, J=8.39 Hz, 1 H) 8.22 (s, 1 H) 7.92 (s, 1 H) 7.62 (t, J=8.01 Hz, 1 H) 7.54 (s, 1 H) 7.49 (d, J=8.55 Hz, 1 H) 6.91 (d, J=7.48 Hz, 1 H) 6.88 (d, J=8.54 Hz, 1 H) 5.71 (d, J=7.17 Hz, 1 H) 4.89 (dd, J=8.24, 3.81 Hz, 1 H) 4.11 (dd, J=13.58, 6.26 Hz, 1 H) 3.94 (s, 3 H) 3.65 (t, J=5.26 Hz, 2 H) 3.42 (q, J=5.59 Hz, 2 H) 2.85 - 3.04 (m, 2 H) 2.70 (s, 3 H) 2.61 (d, J=17.70 Hz, 1 H) 2.03 - 2.12 (m, 1 H) 1.99 (s, 1 H) 1.78 - 1.84 (m, 2 H) 1.63 - 1.71 (m, 5 H) 1.24 (br. s., 2 H) 1.01 - 1.20 (m, 5 H) 0.95 (d, J=10.83 Hz, 1 H) 0.74 (t, J=7.32 Hz, 3 H)

### Example B-9: Synthesis of 4-(((R)-5-(cyclohexylmethyl)-4-ethyl-1-methyl-4,5-dihydro-[1,2,4]triazolo[4,3-f]pteridine-7-yl)amino)N-(1-(3-(4-(2-(2,6-dioxopiperidin-3-yl)-4-methyl-1-oxo-1-dihydropthalazine-6-yl)piperazine-1-il)propanoyl)piperidine-4-yl)piperidine-4-yl)-3-methoxybenzamide (ICT-0001551)

### Step 1) Synthesis of 3-[1-oxo-6-(piperazine-1-yl)-1,2-dihydroptalazine-2-yl]piperidine-2,6-dione

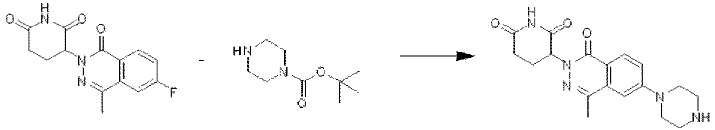

3-(6-fluoro-4-methyl-1-oxo-1,2-dihydroptalazine-2-yl)piperidine-2,6-dione (500 mg, 1.73 mmol), tert-butyl piperazine-1-carboxylate (483 mg, 2.59 mmol), and DIPEA (670 mg, 5.19 mmol) solutions in NMP (2 mL) were stirred at 120°C for 5 hours. The reaction mixture was purified by column chromatography to obtain the Boc-protected compound, dissolved in 1 ml DCM, followed by 0.2 ml TFA, and stirred at room temperature for 2 hours. The reactants were concentrated under reduced pressure and vacuum-dried. The residue was dissolved in DCM, passed through an NH-DM 1020 Chromatorex pad to obtain the heading compound, which was used in the next step without further purification. MS (ESI, m/z): [M+H]⁺ = [356.5].

### Step 2) Synthesis of 3-(4-(2-(2,6-dioxopiperidin-3-yl)-4-methyl-1-oxo-1,2-dihydroptalazine-6-yl)piperazine-1-yl)propanoic acid synthesis

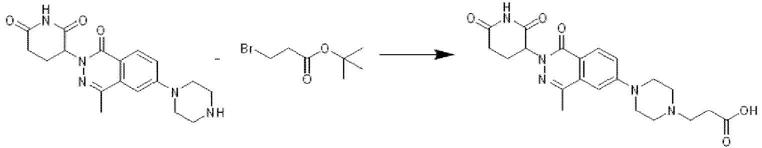

3-[1-oxo-6-(piperazine-1-yl)-1,2-dihydroptalazine-2-yl]piperidine-2,6-dione (200 mg, 586 µmol), tert-butyl 3-bromopropanoate (245 mg, 1.17 mmol), and DIPEA (379 mg, 2.93 mmol) solutions in acetonitrile (10 mL) were stirred at 80°C for 5 hours. The reaction mixture was purified by column chromatography to obtain the Boc-protected compound, dissolved in 1 ml DCM, followed by 0.2 ml TFA, and stirred at room temperature for 2 hours. The reactants were concentrated under reduced pressure and vacuum-dried. The residue was dissolved in DCM, passed through an NH-DM 1020 Chromatorex pad to obtain the heading compound, which was used in the next step without further purification. (170 mg) MS (ESI, m/z): [M+H]⁺ = [470.5].

### Step 3) Synthesis of 4-(((R)-5-(cyclohexylmethyl)-4-ethyl-1-methyl-4,5-dihydro-[1,2,4]triazolo[4,3-f]pteridine-7-yl)amino)N-(1-(3-(4-(2-(2,6-dioxopiperidin-3-yl)-4-methyl-1-oxo-1,2-dihydroptalazine-6-yl)piperazine-1-day)profanoyl)piperidine-4-yl)-3-methoxybenzamide (ICT-0001551)

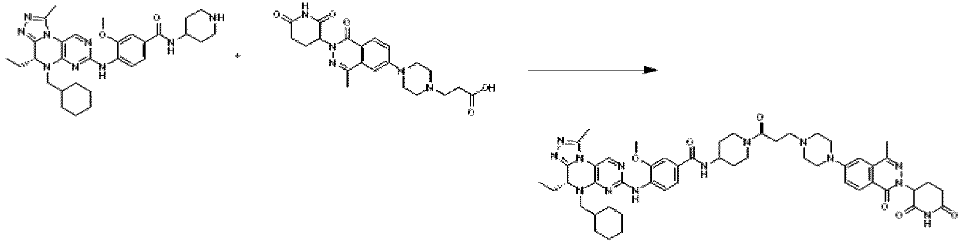

HATU (47.8 mg, 126 µmol) is added to 4-{[(4R)-5-(cyclohexylmethyl)-4-ethyl-1-methyl-4H,5H-[1 ,2,4]triazolo[4,3-f]pteridine-7-yl]amino}-3-methoxy-N- (piperidine-4-yl)benzamide (58.6 mg, 105 µmol) and 3-{4-[2-(2,6-dioxopiperidine-3-yl)-4-methyl-1-oxo-1,2-dihydroptalazine-6-yl]piperazine-1-yl}propanoic acid (44.8 mg, 126 µmol), followed by ethylbis (propane-2-yl)amine (27.1 mg, 209 µmol). The reactants were stirred for 6 hours. The reactants were poured into water, extracted with ethyl acetate, dried with MgSO₄, and then concentrated under reduced pressure. The residue was purified with MeOH/DCM (0~10%) MPLC to obtain the title compound (54 mg). MS (ESI, m/z): [M+H]⁺ = [970.2]
1H NMR (500 MHz, DMSO-d6) δ ppm 10.91 (s, 1 H) 8.34 (s, 1 H) 8.25 - 8.30 (m, 1 H) 8.06 - 8.13 (m, 1 H) 8.00 (d, J=9.00 Hz, 1 H) 7.85 - 7.89 (m, 1 H) 7.40 - 7.44 (m, 2 H) 7.02 (s, 1 H) 5.57 - 5.65 (m, 1 H) 4.82 (dd, J=8.16, 3.89 Hz, 1 H) 4.34 (d, J=12.51 Hz, 1 H) 3.95 - 4.08 (m, 2 H) 3.84 - 3.93 (m, 4 H) 3.42 - 3.51 (m, 1 H) 3.38 (br. s., 3 H) 3.20 (br. s., 1 H) 3.07 (t, J=12.59 Hz, 1 H) 2.85 - 2.97 (m, 2 H) 2.78 - 2.85 (m, 3 H) 2.59 - 2.66 (m, 4 H) 2.49 - 2.58 (m, 7 H) 2.46 - 2.48 (m, 1 H) 1.94 - 2.05 (m, 1 H) 1.83 (d, J=14.04 Hz, 1 H) 1.67 - 1.79 (m, 3 H) 1.56 - 1.67 (m, 5 H) 1.53 (br. s., 2 H) 1.28 - 1.48 (m, 2 H) 1.16 (br. s., 1 H) 1.05 (br. s., 3 H) 0.98 (d, J=9.61 Hz, 1 H) 0.80 - 0.93 (m, 1 H) 0.67 (t, J=7.32 Hz, 3 H)

### Example B-10: Synthesis of 3-(6-(4-(1-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperidine-4-yl)piperazine-1-yl)4-methyl-1-oxopthalazine-2(1H)-yl)piperidine-2,6-dione (ICT-0001553) synthesis

### Step 1) Synthesis of tert-butyl 4-[(piperazine-1-yl)methyl]piperidine-1-carboxylate

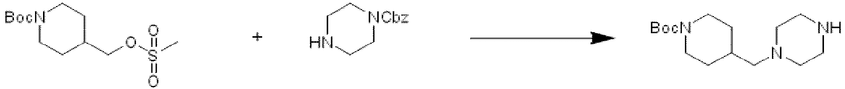

K₂CO₃ (16.4 g, 119.5 mmol) was added to the solution of tert-butyl 4-[(methanesulfonyoxy)methyl]piperidine-1-carboxylate (7.0 g, 23.9 mmol) and benzyl piperazine-1-carboxylate (5.26 g, 23.9 mmol) in 50 ml of acetonitrile at room temperature, and then the reactants were stirred at 80°C for 6 hours. The reactants were filtered, decompressively concentrated, and then the residues were purified with MPLC to obtain the subjective compound. The compound was dissolved in MeOH (50 m) and 10% Pd/C (100 mg) was added. The reactants were stirred under an H2 balloon for 5 hours. The reactants were filtered. Concentrated under pressure, the title compound was obtained (4.3 g). MS (ESI, m/z): [M+H]⁺ = [284.2].

### Step 2) Synthesis of 3-(4-methyl-1-oxo-6-(4-(piperazine-1-ylmethyl)piperidine-1-yl)phthalazine-2(1H)-yl)piperidine-2,6-dione

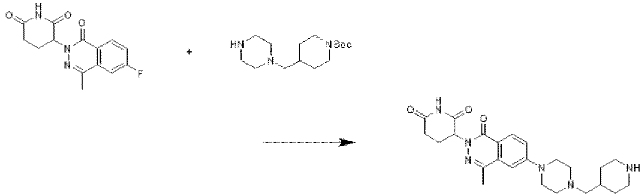

Ethylbis (propane-2-yl)amine (1.34 g, 10.4 mmol) was added to 3-(7-fluoro-4-methyl-1-oxo-1,2-dihydroptalazin-2-yl)piperidine-2-one (500 mg, 3.46 mmol) and tert-butyl 4-[(piperazine-1-yl)methyl]piperidine-1-carboxylate (580 mg, 3.46 mmol) in 3 ml of DMF at room temperature. The reactants were stirred at 130°C for 16 hours. The reactants were poured into water, extracted with ethyl acetate, dried with MgSO₄, and then concentrated under reduced pressure. The residues were purified with MPLC to obtain CBZ-protected compounds. It was dissolved in 2 ml DCM, followed by 0.2 ml TFA, and stirred at room temperature for 2 hours. After the reaction was finished, the reactants were concentrated under reduced pressure and vacuum-dried. The residue was dissolved in DCM, passed through an NH-DM 1020 Chromatorex pad to obtain the heading compound, which was used in the next step without further purification. (370 mg) MS (ESI, m/z): [M+H]⁺ = [453.6 g/mol].

### Step 3) Synthesis of 3-(6-(4-(1-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperidine-4-yl)piperazine-1-yl)4-methyl-1-oxopthalazine-2(1H)-yl)piperidine-2,6-dione (ICT-0001553)

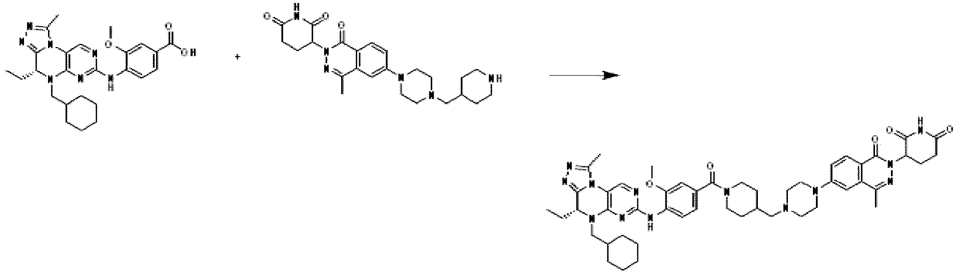

HATU (66.9 mg, 176 umol) is added to 4-{[(4R)-5-(cyclohexylmethyl)-4-ethyl-1-methyl-4H,5H-[1,2,4]triazolo[4,3-f]pteridine-7-yl]amino}-3-methoxybenzoic acid (70 mg, 147 umol) and 3-(7-fluoro-4-methyl-1-oxo-6-{4-[(piperidine-4-yl)methyl]piperazine-1-yl}-1,2-dihydroptalazin-2-yl)piperidine-2,6-dione (66.3 mg, 147 umol) in 1 ml of DMF at room temperature, followed by ethyl bis(propane-2-yl)amine (37.9 mg, 293 umol). The reactants were stirred for 6 hours. The reactants were poured into water, extracted with ethyl acetate, dried with MgSO₄, and then concentrated under reduced pressure. The residue was purified with MeOH/DCM (0~10%) MPLC to obtain the title compound (89 mg). MS (ESI, m/z): [M+H]⁺ = [913.2]
1H NMR (500 MHz, DMSO-d6) δ ppm 10.92 (s, 1 H) 9.90 (s, 1 H) 8.32 - 8.38 (m, 2 H) 8.04 (d, J=9.00 Hz, 1 H) 7.91 (s, 1 H) 7.51 - 7.58 (m, 4 H) 7.11 (s, 1 H) 6.97 (d, J=9.00 Hz, 2 H) 5.63 (d, J=7.32 Hz, 1 H) 4.84 (dd, J=8.09, 3.81 Hz, 1 H) 4.07 (dd, J=13.43, 6.41 Hz, 1 H) 3.93 (s, 3 H) 3.56 (br. s., 4 H) 2.94 (dd, J=13.81, 8.16 Hz, 1 H) 2.78 - 2.90 (m, 2 H) 2.64 (s, 4 H) 2.47 - 2.57 (m, 2 H) 1.96 - 2.05 (m, 1 H) 1.69 - 1.80 (m, 1 H) 1.57 - 1.66 (m, 4 H) 0.97 - 1.14 (m, 4 H) 0.62 - 0.73 (m, 4 H)

### Example B-11 : Synthesis of 3-({4-[4-(4-{[(4S)-5-(cyclohexylmethyl)-4-ethyl-1-methyl-4H,5H-[1,2,4]triazolo[4,3-f]pteridine-7-yl]amino}-2-methoxybenzoyl)piperazine-1-yl]-3-fluorophenyl}amino)-1-methylpiperidine-2,6-dione (ICT-0001554)

### Step 1) Synthesis of 3-{[3-fluoro-4-(piperazine-1-yl)phenyl]amino}-1-methylpiperidine-2,6-dione

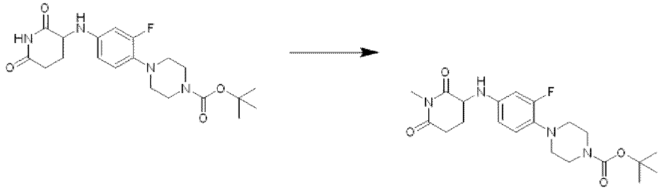

tert-butyl-4-{4-[(2,6-dioxopiperidin-3-yl)amino]-2-fluorophenyl} piperazine-1-carboxylate (0.2 g, 492 µmol) was dissolved in methanol (1% acetic acid) (10 ml), formaldehyde (73.8 mg, 2.46 mmol, in 35% water) and NaBH4 (46.5 mg, 1.23 mmol) were added, and the mixture was stirred at room temperature for 1 hour. The solvent was evaporated, the residue was suspended in a saturated Na₂CO₃ solution, and extracted 3 times with CH₂Cl₂. The bound organic layer was dried with Na₂SO₄ and the solvent was evaporated. The residue was purified by flash chromatography on a silica gel (DCM/MeOH = 99/1) and the compound was obtained (200 mg). MS (ESI, m/z): [M+H]+ = [421.5].

### Step 2) Synthesis of 3-{[3-fluoro-4-(piperazine-1-yl)phenyl]amino}piperidine-2,6-dione

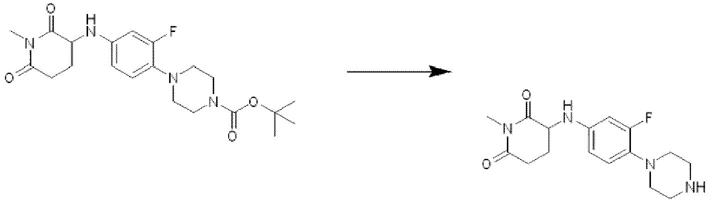

The reactants were stirred at room temperature for 1 hr in a solution of 3-{[3-fluoro-4-(piperazine-1-yl)phenyl]amino}-1-methylpiperidine-2,6-dione (0.2 g, 492 µmol) in 30% TFA in DCM. After the reaction was completed, it was concentrated under reduced pressure. The residues were purified by DCM/MeOH (0~5%) amine column chromatography to obtain the title compound (120 mg). MS (ESI, m/z): [M+H]+ = [321.5].

### Step 3) Synthesis of 3-({4-[4-(4-{[(4S)-5-(cyclohexylmethyl)-4-ethyl-1-methyl-4H,5H-[1,2,4]triazolo[4,3-f]pteridine-7-yl]amino}-2-methoxybenzoyl)piperazine-1-yl]-3-fluorophenyl}amino)-1-methylpiperidine-2,6-dione (ICT-0001554)

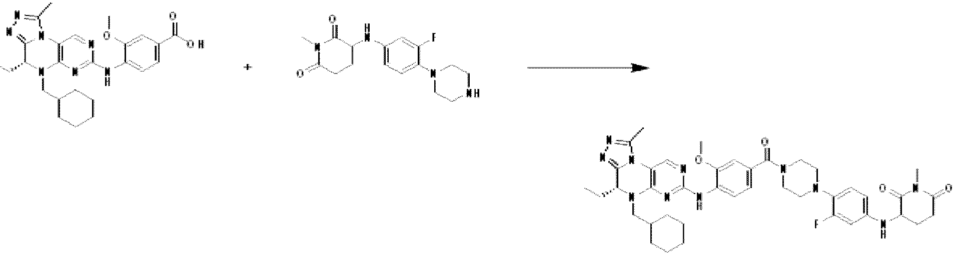

HATU (71.2 mg, 187 µmol) was added to 4-{[(4S)-5-(cyclohexylmethyl)-4-ethyl-1-methyl-4H,5H-[1,2,4]triazolo[4,3-f]pteridine-7-yl]amino}-2-methoxybenzoic acid (59.6 mg, 125 µmol) and 3-{[3-fluoro-4-(piperazine-1-yl)phenyl]amino}-1-methylpiperidine-2,6-dione (40.0 mg, 125 µmol) in 1 ml of DMF at room temperature. It was extracted with ethyl acetate, dried with MgSO₄, and then concentrated under reduced pressure. The residual acid was purified with MeOH/DCM (0~10%) MPLC to obtain the title compound (123 mg). MS (ESI, m/z): [M+H]⁺ = [780.9]
1H NMR (500 MHz, DMSO-d6) δ ppm 8.32 (s, 2 H) 8.23 (s, 1 H) 7.87 (s, 1 H) 7.03 (s, 2 H) 6.93 (d, J=8.09 Hz, 1 H) 6.78 - 6.81 (m, 1 H) 6.48 (s, 1 H) 6.45 (s, 1 H) 6.37 (d, J=8.70 Hz, 2 H) 5.87 (d, J=7.93 Hz, 2 H) 4.82 (dd, J=7.86, 3.59 Hz, 2 H) 4.24 - 4.30 (m, 2 H) 4.03 (dd, J=13.89, 6.41 Hz, 1 H) 3.85 (s, 5 H) 2.86 - 2.92 (m, 3 H) 2.71 - 2.78 (m, 2 H) 2.65 - 2.69 (m, 1 H) 2.62 (s, 4 H) 2.29 (s, 1 H) 1.74 (d, J=3.97 Hz, 1 H) 1.56 - 1.66 (m, 4 H) 1.05 (br. s., 3 H) 0.95 - 1.03 (m, 1 H) 0.87 (d, J=6.56 Hz, 1 H) 0.67 (t, J=7.32 Hz, 5 H)

### Example B-12: Synthesis of 3-(6-(4-(1-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperidine-4-yl)piperazine-1-yl)4-methyl-1-oxopthalazine-2(1H)-yl)piperidine-2,6-dione (ICT-0001556)

### Step 1) Synthesis of benzyl 4-{1-[(tert-butoxy)carbonyl]piperidine-4-yl}piperazine-1-carboxylate

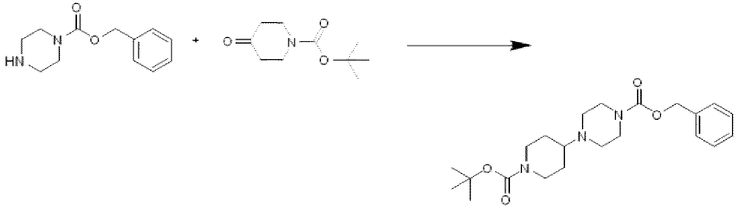

AcOH (3mL) was added to benzyl piperazine-1-carboxylate (3.0 g, 13.6 mmol) and tert-butyl 4-oxopiperidine-1-carboxylate (2.71 g, 13.6 mmol) solutions in MeOH (50 mL). After stirring at 25°C for 1 hour, NaBH3CN (2.61 g, 42.2 mmol) was added to the mixture, and the resulting mixture was stirred at 25°C for 16 hours. The mixture reaction was terminated with aqueous NaHCO₃ and extracted with EtOAc. The organic layer was washed with brine solution, Na₂SO₄ and concentrated. The residue was purified on silica gel by PE: EtOAc (5:1) column chromatography to obtain the title compound. (3.6 g) MS (ESI, m/z): [M+H]⁺ = [404.5],

### Step 2) Synthesis of tert-butyl 4-(piperazine-1-yl)piperidine-1-carboxylate

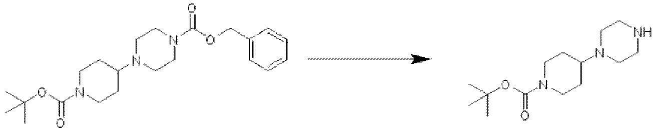

Benzyl 4-{1-[(tert-butoxy)carbonyl]piperidine-4-yl}piperazine-1-carboxylate (3.6 g, 8.92 mmol) was dissolved in EtOH (50 ml) and 10% Pd/C (360 mg) was added. The reactants were stirred under an H₂ balloon for 5 hours. The reactants were filtered, decompressively concentrated to obtain the subjective compound (2.7 g). MS (ESI, m/z): [M+H]⁺ = [270.2].

### Step 3) Synthesis of 3-(4-methyl-1-oxo-6-(4-(piperidine-4-yl)piperazine-1-yl)phthalazine-2(1H)-yl)piperidine-2,6-dione

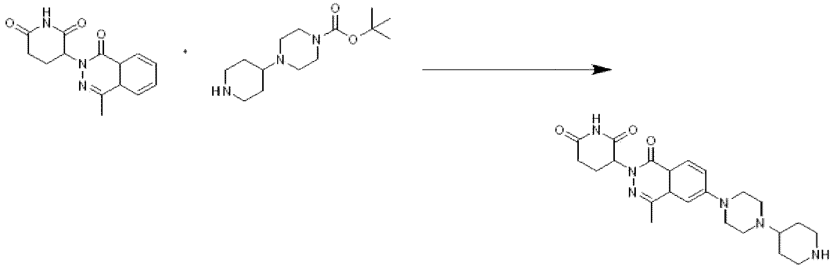

tert-butyl 4-(piperazine-1-yl)piperidine-1-carboxylate (2.7 g, 10.0 mmol), 3-(6-fluoro-4-methyl-1-oxo-1,2-dihydroptallazine-2-yl)piperidine-2,6-dione (2.7 g, 1.04 mmol), and DIPEA (3.8 g, 2.07 mmol) solutions in NMP (5 mL) were stirred at 130°C for 5 hours. The reaction mixture was purified by column chromatography to obtain the Boc-protected compound, which was dissolved in 5 ml DCM, followed by 0.5 ml TFA, and stirred at room temperature for 1 hour. After the end of the reaction. The reactants were concentrated under reduced pressure and vacuum-dried. The residue was dissolved in DCM, passed through an NH-DM 1020 Chromatorex pad to obtain the heading compound, which was used in the next step without further purification. (3.1 g) MS (ESI, m/z): [M+H]⁺ = [441.2 g/mol].

### Step 4) Synthesis of 3-(6-(4-(1-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperidine-4-yl)piperazine-1-yl)4-methyl-1-oxopthalazine-2(1H)-yl)piperidine-2,6-dione (ICT-0001556)

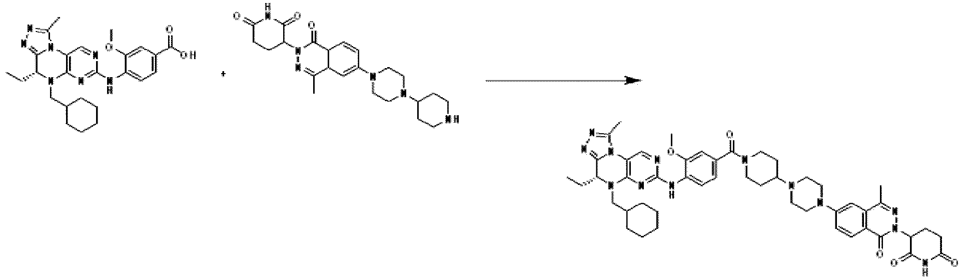

HATU (47.8 mg, 126 µmol) was added to 4-{[(4R)-5-(cyclohexylmethyl)-4-ethyl-1-methyl-4H,5H-[1,2,4]triazolo[4,3-f]pteridine-7-yl]amino}-3-methoxybenzoic acid (50 mg, 105 µmol) and 3-{1-oxo-6-[4-(piperidine-4-yl)piperazine-1-yl]-1,2-dihydroptalazin-2-yl}piperidine-2,6-dione (45.9 mg, 105 µmol) in 1 ml of DMF at room temperature, followed by ethylbis (propane-2-yl)amine (27.1 mg, 209 µmol). The reactants were stirred for 6 hours. The reactants were poured into water, extracted with ethyl acetate, dried with MgSO₄, and then concentrated under reduced pressure. The residual acid was purified with MeOH/DCM (0~10%) MPLC to obtain the title compound (81 mg). MS (ESI, m/z): [M+H]⁺ = [899.1]
1H NMR (500 MHz, DMSO-d6) δ ppm 10.91 (s, 1 H) 8.32 (s, 2 H) 8.21 (d, J=7.63 Hz, 1 H) 8.02 (d, J=8.55 Hz, 1 H) 7.87 (s, 2 H) 7.01 (s, 1 H) 6.90 (d, J=7.78 Hz, 1 H) 5.56 - 5.67 (m, 1 H) 4.82 (dd, J=8.09, 3.66 Hz, 2 H) 4.03 (dd, J=13.35, 6.18 Hz, 2 H) 3.84 (s, 5 H) 2.79 - 2.95 (m, 3 H) 2.62 (s, 6 H) 2.46 - 2.58 (m, 2 H) 1.95 - 2.07 (m, 1 H) 1.70 - 1.80 (m, 2 H) 1.51 - 1.67 (m, 8 H) 1.19 (t, J=7.02 Hz, 1 H) 1.11 (d, J=7.63 Hz, 1 H) 1.05 (br. s., 4 H) 0.93 - 1.03 (m, 2 H) 0.87 (d, J=10.22 Hz, 1 H) 0.67 (t, J=7.32 Hz, 6 H)

### Example B-13: Synthesis of 3-(6-(4-((1-(4-(((R)-5-(cyclohexylmethyl)-4-ethyl-1-methyl-4,5-dihydro-[1,2,4]triazolo[4,3-f]pteridine-7-yl)amino)3-methoxybenzoyl)azetidine-3-yl)methyl)piperazine-1-yl)4-methyl-1-oxopthalazine-2(1H)-yl)piperidine-2,6-dione (ICT-0001557)

### Step 1) Synthesis of 1-Boc-Azetidine-3-yl-Methanol

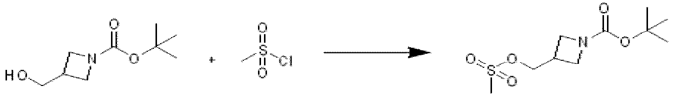

Methanesulfonyl chloride (5.11 g, 44.8 mmol) in CH₂Cl₂ (20 mL) was added to 1-Bocazetidine-3-yl-methanol (5.0 g, 37.4 mmol) solution in CH₂Cl₂ (100 mL), followed by triethylamine (5.67 g, 56.1 mmol) at 0°C, stirred for 2 hours. Reactants were poured into water (50 mL), extracted with dichloromethane (50 mLx3), organic layers were bonded, washed with brine solution (50 mLx2), dried with sodium sulfate anhydrous. Then it was filtered and concentrated. The residue was used in the next step without additional purification. (6.5 g) MS (ESI, m/z): [M+H]⁺ = [380.1].

### Step 2) Synthesis of 3-(6-(4-(azetidine-3-ylmethyl)piperazine-1-yl)-4-methyl-1-oxopthalazine-2(1H)-yl)piperidine-2,6-dione

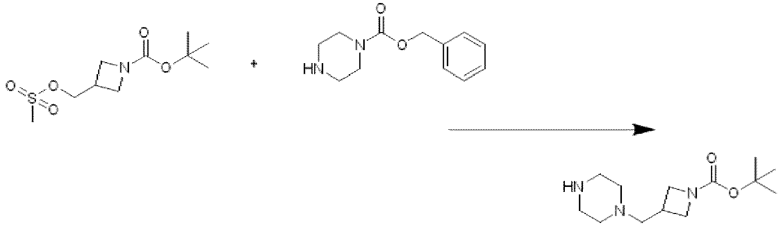

K₂CO₃ (10.1 g, 73.5 mmol) was added to the solution of 1-Boc-azetidine-3-yl-methanol (6.5 g, 24.5 mmol) and 1-Z-piperazine (5.40 g, 24.5 mmol) in 50 ml of acetonitrile at room temperature, and then the reactants were stirred at 80°C for 6 hours. The reactants were filtered, decompressively concentrated, and then the residues were purified with MPLC to obtain the subjective compounds. The resulting compounds were dissolved in (7.1 g, 18.2 mmol) EtOH (50 ml), and 10% Pd/C (710 mg) was added. The reactants were stirred under an H₂ balloon for 5 hours. The reactants were filtered and the filtrate was concentrated to yield a heading compound (5.4 g). MS (ESI, m/z): [M+H]⁺ = [256.2].

### Step 3) Synthesis of 3-(6-{4-[(Azetidine-3-yl)methyl]piperazine-1-yl}-4-methyl-1-oxo-1,2-dihydroptalazine-2-yl)piperidine-2,6-dione

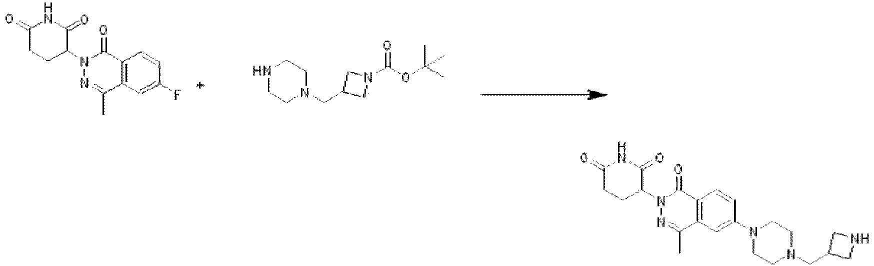

3-(6-fluoro-4-methyl-1-oxo-1,2-dihydroptallazine-2-yl)piperidine-2,6-dione (500 mg, 1.73 mmol), 3-(6-(4-(azetidine-3-ylmethyl)piperazine-1-yl)4-methyl-1-oxopthalazine-2(1H)-yl)piperidine-2,6-dione (441 mg, 1.73 mmol), and DIPEA (670 mg, 5.19 mmol) solutions were stirred at 120°C for 5 hours. Then, 0.2 ml TFA was added and stirred at room temperature for 2 hours. After the reaction was finished, the reactants were concentrated under reduced pressure and vacuum-dried. The residues were dissolved in DCM, passed through an NH-DM 1020 Chromatorex pad to obtain the heading compound, which was used in the next step without further purification. (420 mg) MS (ESI, m/z): [M+H]⁺ = [425.2].

### Step 4) Synthesis of 3-(6-(4-((1-(4-(((R)-5-(cyclohexylmethyl)-4-ethyl-1-methyl-4,5-dihydro-[1,2,4]triazolo[4,3-f]pteridine-7-yl)amino)3-methoxybenzoyl)azetidine-3-il)methyl)piperazine-1-yl)4-methyl-1-oxopthalazine-2(1H)-yl)piperidine-2,6-dione (ICT-0001557)

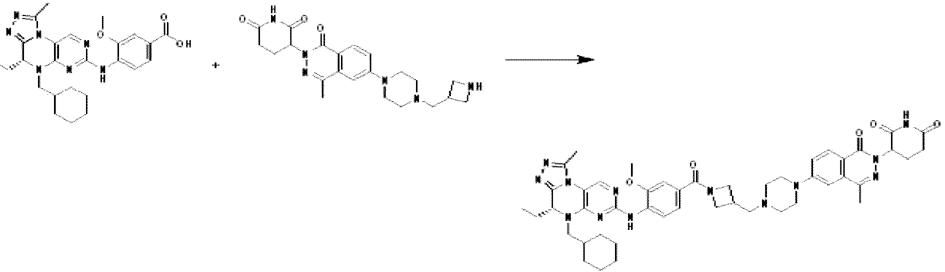

HATU (47.8 mg, 126 µmol) is added to 4-{[(4R)-5-(cyclohexylmethyl)-4-ethyl-1-methyl-4H,5H-[1,2,4]triazolo[4,3-f]pteridine-7-yl]amino}-3-methoxybenzoic acid (50 mg, 105 µmol) and 3-(6-{4-[(azetidine-3-yl)methyl]piperazine-1-yl}-4-methyl-1-oxo-1,2-dihydroptalazin-2-yl)piperidine-2,6-dione (44.4 mg, 105 µmol) in 1 ml of DMF at room temperature, followed by ethylbis (propane-2-yl)amine (27.1 mg, 209 µmol). The reactants were stirred for 6 hours. The reactants were poured into water, extracted with ethyl acetate, dried with MgSO₄, and then concentrated under reduced pressure. The residue was purified with MeOH/DCM (0~10%) MPLC to obtain the title compound (63 mg). MS (ESI, m/z): [M+H]⁺ = [885.1]
1H NMR (500 MHz, DMSO-d6) δ ppm 10.91 (s, 1 H) 8.33 (s, 2 H) 7.90 (s, 1 H) 7.20 (s, 2 H) 7.14 (d, J=8.09 Hz, 2 H) 4.75 - 4.92 (m, 2 H) 4.03 (dd, J=13.28, 6.10 Hz, 1 H) 3.86 (s, 5 H) 2.78 - 2.98 (m, 1 H) 2.62 (s, 6 H) 2.49 - 2.58 (m, 1 H) 2.47 (s, 1 H) 2.29 (br. s., 1 H) 1.54 - 1.66 (m, 8 H) 1.15 - 1.22 (m, 4 H) 0.67 (t, J=7.25 Hz, 6 H)

### Example B-14: Synthesis of 4-(((R)-5-(cyclohexylmethyl)-4-ethyl-1-methyl-4,5-dihydro-[1,2,4]triazolo[4,3-f]pteridine-7-yl)amino)N-(2-((3-(2,6-dioxopiperidin-3-yl)4-oxo-3,4-dihydroptalazine-5-yl)amino)ethyl)-3-methoxybenzamide (ICT-0001558)

### Step 1) Synthesis of 3-(8-((2-aminoethyl)amino)-1-oxopthalazine-2(1H)-yl)piperidine-2,6-dione

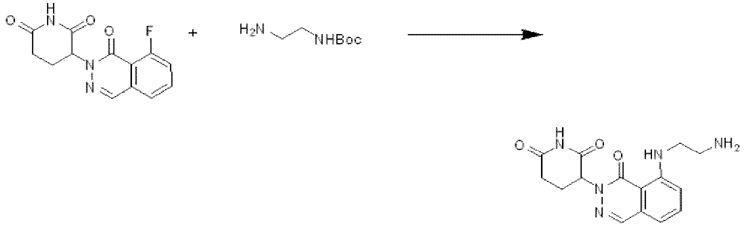

3-(8-fluoro-4-methyl-1-oxo-1,2-dihydroptalazin-2-yl)piperidine-2,6-dione (500 mg, 1.73 mmol), N-tert-Boc-ethylenediamine (277 mg, 1.73 mmol), and DIPEA (670 mg, 3.46 mmol) solutions in NMP (2 mL) were stirred at 110°C for 5 hours. The reaction mixture was purified by column chromatography to obtain the Boc-protected compound, which was dissolved in 1 ml DCM, followed by 0.2 ml TFA, and stirred at room temperature for 2 hours. The reactants were concentrated under reduced pressure and vacuum-dried. The residue was dissolved in DCM, passed through an NH-DM 1020 Chromatorex pad to obtain the heading compound, which was used in the next step without further purification. (380 mg) MS (ESI, m/z): [M+H]⁺ = [316.1].

### Step 2) Synthesis of 4-(((R)-5-(cyclohexylmethyl)-4-ethyl-1-methyl-4,5-dihydro-[1,2,4]triazolo[4,3-f]pteridine-7-yl)amino)N-(2-((3-(2,6-dioxopiperidin-3-yl)4-oxo-3,4-dihydropthalazine-5-yl)amino)ethyl)-3-methoxybenzamide (ICT-0001558)

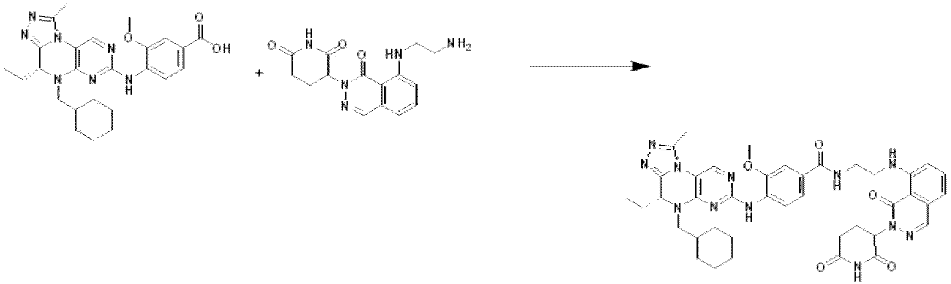

HATU (34.3 mg, 146 µmol) is added to 4-{[(4R)-5-(cyclohexylmethyl)-4-ethyl-1-methyl-4H,5H-[1,2,4]triazolo[4,3-f]pteridine-7-yl]amino}-3-methoxybenzoic acid (58 mg, 121 µmol) and 3-{8-[(2-aminoethyl)amino]-1-oxo-1,2-dihydroptalazin-2-yl}piperidine-2,6-dione (38.3 mg, 121 µmol) in 1 ml of DMF at room temperature, followed by ethylbis (propane-2-yl)amine (31.4 mg, 243 µmol). The reactants were stirred for 6 hours. The reactants were poured into water, extracted with ethyl acetate, dried with MgSO₄, and then concentrated under reduced pressure. The residual acid was purified with MeOH/DCM (0~10%) MPLC to obtain the title compound (63.3 mg). MS (ESI, m/z): [M+H]⁺ = [775.9]
1H NMR (500 MHz, DMSO-d6) δ ppm 11.03 (s, 1 H) 8.42 (s, 1 H) 8.36 (d, J=8.55 Hz, 1 H) 8.24 (s, 1 H) 7.93 (s, 1 H) 7.68 (t, J=7.93 Hz, 1 H) 7.51 - 7.55 (m, 1 H) 7.49 (d, J=8.54 Hz, 1 H) 7.13 (d, J=8.85 Hz, 1 H) 6.94 (d, J=7.63 Hz, 1 H) 4.86 - 4.97 (m, 1 H) 4.12 (dd, J=13.43, 6.26 Hz, 1 H) 3.95 (s, 4 H) 3.49 (br. s., 4 H) 2.98 - 3.05 (m, 1 H) 2.70 (s, 5 H) 2.61 - 2.67 (m, 1 H) 2.55 (br. s., 1 H) 2.37 (s, 1 H) 2.09 (s, 2 H) 1.64 - 1.72 (m, 2 H) 0.75 (t, J=7.32 Hz, 5 H)

### Example B-15: Synthesis of 4-(((R)-5-(cyclohexylmethyl)-4-ethyl-1-methyl-4,5-dihydro-[1,2,4]triazolo[4,3-f]pteridine-7-yl)amino)N-(1-(2-(2-(2-(2-((2-(2-(2-(2-(2-(2-(2-(2,6-dioxopiperidin-3-yl)-1-oxo-1,2-dihydropthalazine-6-yl)amino)ethoxy)ethoxy)ethoxy)ethoxy)acetyl)piperidine-4-yl)-3-methoxybenzamide (ICT-0001559)

### Step 1) Synthesis of 2-(2-(2-(2-((2-(2-(2-(2,6-dioxopiperidin-3-yl)1-oxo-1,2-dihydroptalazin-6-yl)amino)ethoxy)ethoxy)ethoxy)ethoxy)ethoxy)acetic acid

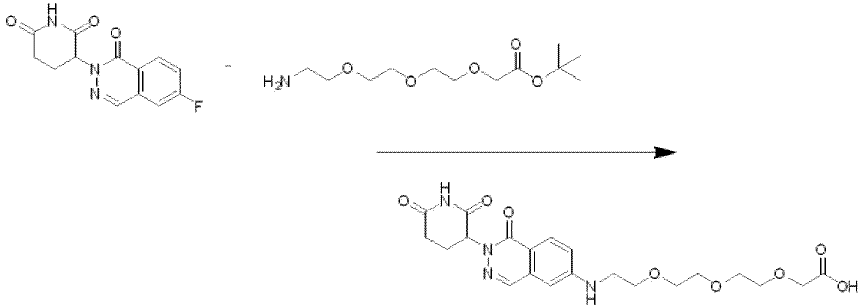

6-Fluoro-1,2-dihydrophthalazine-1-one (300 mg, 1.09 mmol) and 2-{2-[2-(2-aminoethoxy)ethoxy]ethoxy}acetic acid (407 mg, 1.09 mmol) were dissolved in NMP (3 mL), and DIPEA (5 equivalent) was added to the reaction mixture. Stirred overnight at 130°C. The reactants were terminated with water, extracted with DCM, NH₄Cl and Brine solution. Dried with MgSO4. The reaction mixture was loaded with silica curry and separated into MPLC (HX/EA 30%-> 50% for 10 min). The product was obtained as oil. The compound was dissolved in 1 ml DCM, followed by 0.2 ml TFA, and stirred at room temperature for 2 hours. After the reaction was finished, the reactants were concentrated under reduced pressure and vacuum-dried. The residue was dissolved in DCM, passed through an NH-DM 1020 Chromatorex pad to obtain the heading compound, which was used in the next step without further purification. (170 mg) MS (ESI, m/z): [M+H]⁺ = [463.5],

### Step 2) Synthesis of 4-(((R)-5-(cyclohexylmethyl)-4-ethyl-1-methyl-4,5-dihydro-[1,2,4]triazolo[4,3-f]pteridine-7-yl)amino)N-(1-(2-(2-(2-(2-((2-(2-(2-(2-(2-(2-(2,6-dioxopiperidin-3-yl)1-oxo-1,2-dihydrophthalazine-6-yl)amino)ethoxy)ethoxy)ethoxy)ethoxy)acetyl)piperidine-4-yl)-3-methoxybenzamide (ICT-0001559)

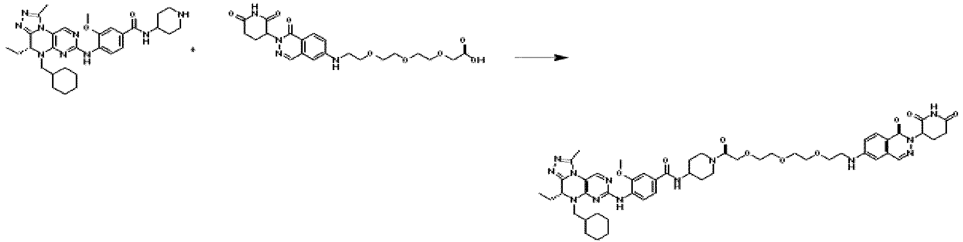

HATU (47.8 mg, 126 µmol) is added to 4-{[(4R)-5-(cyclohexylmethyl)-4-ethyl-1-methyl-4H,5H-[1,2,4]triazolo[4,3-f]phteridine-7-yl]amino}-3-methoxy-N- (piperidine-4-yl)benzamide (70 mg, 125 µmol) and 2-{2-[2-(2-{[2-(2,6-dioxopiperidin-3-yl)-1-oxo-1,2-dihydropthalazine-6-yl]amino}ethoxy)ethoxy]ethoxy}acetic acid (48.4 mg, 105 µmol). Ethylbis (propane-2-yl)amine was then added. The reactants were stirred for 6 hours. The reactants were poured into water, extracted with ethyl acetate, dried with MgSO₄, and then concentrated under reduced pressure. The residue was purified with MeOH/DCM (0-10%) MPLC to obtain the title compound (72 mg). MS (ESI, m/z): [M+H]⁺ = [1005.2]
1H NMR (500 MHz, DMSO-d6) δ ppm 10.92 (s, 1 H) 8.32 (s, 2 H) 8.20 (d, J=8.24 Hz, 1 H) 8.06 - 8.11 (m, 1 H) 7.81 - 7.89 (m, 3 H) 7.52 (d, J=8.09 Hz, 1 H) 7.03 (dd, J=8.85, 2.14 Hz, 1 H) 6.93 - 6.99 (m, 2 H) 6.86 (d, J=9.61 Hz, 2 H) 6.68 - 6.75 (m, 1 H) 4.75 - 4.89 (m, 2 H) 3.95 - 4.06 (m, 2 H) 3.78 - 3.89 (m, 9 H) 3.45 - 3.55 (m, 13 H) 2.77 - 2.91 (m, 4 H) 2.62 (s, 5 H) 2.47 - 2.58 (m, 1 H) 1.67 - 1.76 (m, 1 H) 1.53 - 1.66 (m, 4 H) 1.04 (d, J=6.56 Hz, 2 H) 0.88 (d, J=6.41 Hz, 2 H) 0.67 (t, J=7.32 Hz, 5 H)

### Example B-16 : Synthesis of 4-{[(4S)-5-(cyclohexylmethyl)-4-ethyl-1-methyl-4H,5H-[1,2,4]triazolo[4,3-f]pteridine-7-yl]amino}-N-[1-(2-{4-[2-(2,6-dioxopiperidine-3-yl)-4-methyl-1-oxo-1,2-dihydroptalazine-6-yl]piperazine-1-yl}ethyl)piperidine-4-yl]-3-methoxybenzamide (ICT-0001560)

### Step 1) Synthesis of tert-butyl N-[1-(2-{4-[2-(2,6-dioxopiperidine-3-yl)-4-methyl-1-oxo-1,2-dihydroptalazine-6-yl]piperazine-1-yl}ethyl)piperidine-4-yl]carbamate

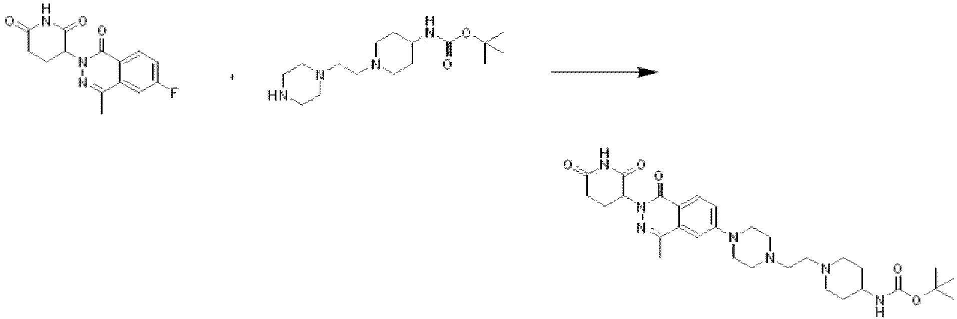

tert-butyl N-{1-[2-(piperazine-1-yl)piperidine-4-yl}carbamate (0.1 g, 320 µmol) and ethylbis (propane-2-yl)amine (279 µL, 1.6 mmol) were added to 3-(6-fluoro-4-methyl-1-oxo-1,2-dihydroptalazine-2-yl) (92.6 mg, 320 µmol) solution in NMP (2 mL). Next, decompression and concentration were performed. The residue was purified with hexane/ethyl acetate (50~90%) column chromatography to obtain the title compound (150 mg). MS (ESI, m/z): [M+H]+ = 582.7.

### Step 2) Synthesis of 3-(6-{4-[2-(4-aminopiperidine-1-yl)ethyl]piperazine-1-yl}-4-methyl-1-oxo-1,2-dihydroptalazine-2-yl)piperidine-2,6-dione

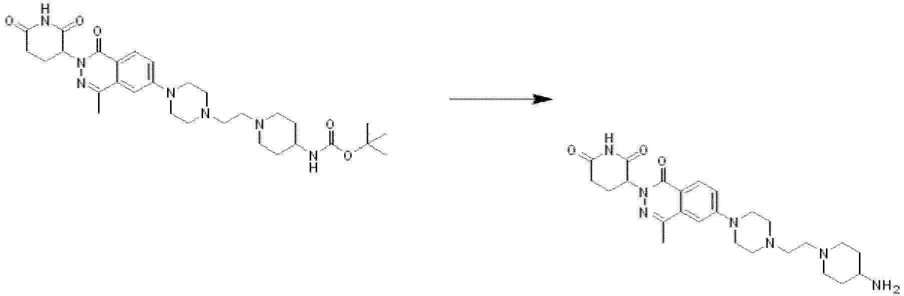

tert-butyl N-[1-(2-{4-[2-(2,6-dioxopiperidine-3-yl)-4-methyl-1-oxo-1,2-dihydroptalazine-6-yl]piperazine-1-yl}ethyl)piperidine-4-yl]carbamate (150 mg, 258 µmol) solution reactants in 30% TFA in DCM (1 mL) were stirred at room temperature for 30 min. After the reaction was completed, it was concentrated under reduced pressure. After the reaction was completed, the reactant was poured into water, extracted with ethyl acetate (twice), dried with MgSO₄, and then concentrated under reduced pressure. The residues were purified by DCM/MeOH (0~5%) amine column chromatography to obtain the title compound (120 mg). MS (ESI, m/z): [M+H]⁺ = 482.6.

### Step 3) Synthesis of 3-(6-{4-[2-(4-aminopiperidine-1-yl)ethyl]piperazine-1-yl}-4-methyl-1-oxo-1,2-dihydroptalazine-2-yl)piperidine-2,6-dione (ICT-0001560)

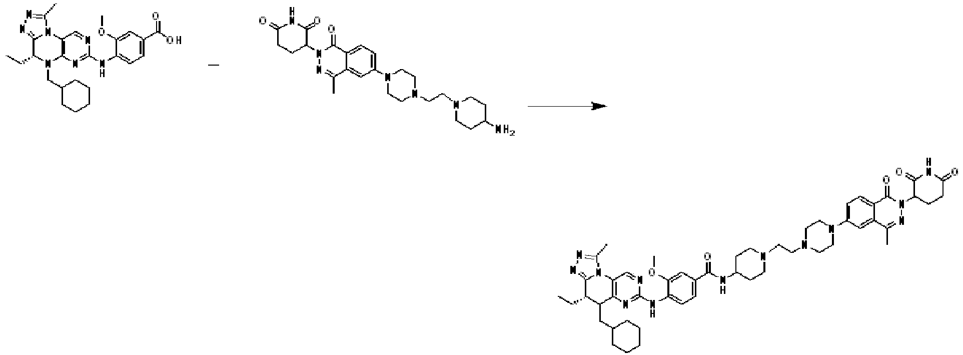

3-(6-{[(4S)-5-(cyclohexylmethyl)-4-ethyl-1-methyl-4H,5H-[1,2,4]triazolo[4,3-f]phteridine-7-yl]amino}-3-methoxybenzoic acid (123 mg, 257 µmol) in DMF (2 mL) in a solution of 3-(6-{4-[2-(4-aminopiperidine-1-yl)ethyl]piperazine-1-yl}-4-methyl-1-oxo-1,2-dihydroptalazine-2-yl)piperidine-2,6-dione (124 mg, 257 µmol), HATU (196 mg, 515 µmol) and ethylbis(propane-2-yl)amine (333 mg, 2.57 mmol). The reactants were stirred at room temperature for 2 hours. After the reaction was completed, the reactant was poured into water, extracted with DCM (twice), dried with MgSO₄, and then concentrated under reduced pressure. The residue was purified by DCM/MeOH (0~5%) column chromatography to obtain the title compound (82 mg). MS (ESI, m/z): [M+H]⁺ = 942.2
1H NMR (500 MHz, DMSO-d6) δ ppm 10.98 (s, 1 H) 8.41 (s, 1 H) 8.34 (d, J=8.09 Hz, 1 H) 8.11 - 8.21 (m, 1 H) 8.08 (d, J=8.85 Hz, 1 H) 7.93 (s, 1 H) 7.45 - 7.55 (m, 3 H) 7.10 (s, 1 H) 5.64 - 5.77 (m, 1 H) 4.90 (dd, J=8.24, 3.81 Hz, 1 H) 4.12 (dd, J=13.58, 6.87 Hz, 1 H) 3.93 - 3.98 (m, 3 H) 3.42 - 3.48 (m, 5 H) 2.86 - 3.04 (m, 4 H) 2.70 (s, 3 H) 2.56 - 2.66 (m, 7 H) 2.37 (s, 1 H) 1.96 - 2.13 (m, 4 H) 1.89 - 1.94 (m, 1 H) 1.74 - 1.86 (m, 4 H) 1.58 - 1.74 (m, 8 H) 1.22 - 1.26 (m, 2 H) 1.05 - 1.18 (m, 4 H) 0.93 - 1.00 (m, 1 H) 0.86 (t, J=7.17 Hz, 1 H) 0.75 (t, J=7.32 Hz, 3 H)

### Example B-17: Synthesis of 4-{[(4S)-5-(cyclohexylmethyl)-4-ethyl-1-methyl-4H,5H-[1,2,4]triazolo[4,3-f]pteridine-7-yl]amino}-N-{1-[2-(4-{4-[(2,6-dioxopiperidin-3-yl)amino]-2-fluorophenyl}piperazine-1-il)ethyl]piperidine-4-yl}-3-methoxybenzamide (ICT-0001561)

### Step 1) Synthesis of Tert-Butyl N-(1-{2-[4-(2-fluoro-4-nitrophenyl)piperazine-1-day]ethyl}piperidine-4-yl)carbamate

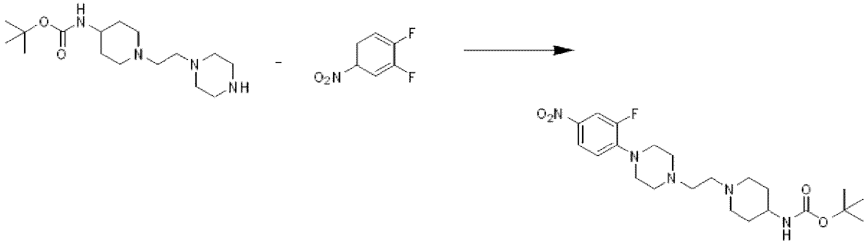

tert-butyl N-{1-[2-(piperazine-1-yl)ethyl]piperidine-4-yl} carbamate (0.1 g, 320 µmol) and dipotassium carbonate (221 mg, 1.6 mmol) were added to a solution of 1,2-difluoro-4-nitrobenzene (51.6 mg, 320 µmol) in DMF (1 mL). The reactants were stirred at 85°C for 2 hours. After the end of the reaction, the reactants were poured into water, extracted with ethyl acetate (2 times), dried with MgSO₄, and then concentrated under reduced pressure. The residues were purified by hexane/ethyl acetate (0~60%) column chromatography to obtain the title compound (145 mg). MS (ESI, m/z): [M+H]⁺ = 452.5.

### Step 2) Synthesis of tert-butyl N-(1-{2-[4-(4-amino-2-fluorophenyl)piperazine-1-day]ethyl}piperidine-4-yl)carbamate

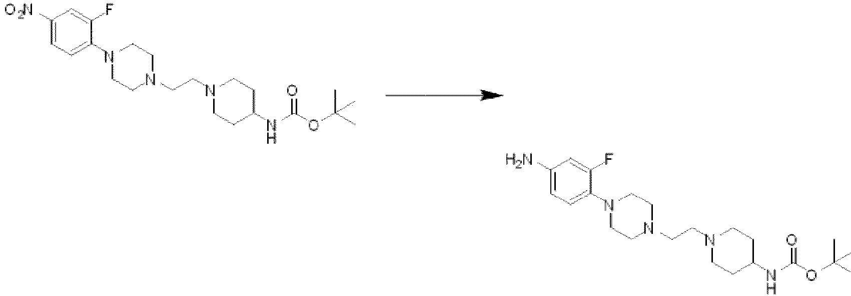

Iron powder (179 mg, 3.2 mmol) was added to tert-butyl N-(1-{2-[4-(2-(2-fluoro-4-nitrophenyl)piperazine-1-yl]ethyl}piperidine-4-yl)carbamate (145 mg, 3.2 µmol) in saturated NH4Cl/THF (1:1) (2 mL). The reactants were stirred at 85°C for 2 hours. After the reaction was completed, the reactants were filtered and decompressively concentrated. The residues were purified with DCM/MeOH (0~5%) reversed-phase chromatography to obtain the headline compound (0.65 g). (ESI, m/z): [M+H]⁺ = 422.5.

### Step 3) Synthesis of tert-butyl N-{1-[2-(4-{4-[(2,6-dioxopiperidine-3-yl)amino]-2-fluorophenyl}piperazine-1-yl)ethyl]piperidine-4-yl}carbamate

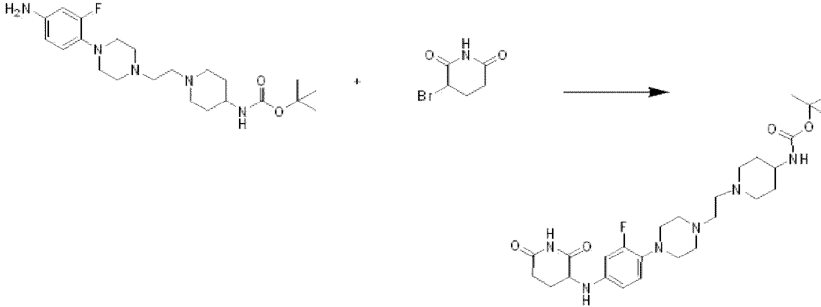

3-bromoperidin-2,6-dione (182 mg, 949 µmol) and sodium bicarbonate (239 mg, 2.85 mmol) were added to tert-butyl N-(1-{2-[4-(4-amino-2-fluorophenyl)piperazine-1-yl]ethyl}piperidine-4-yl)carbamate (0.2 g, 474 µmol) in DMF (3 mL). 3-bromopiperidine-2,6-dione (182 mg, 949 µmol) and sodium bicarbonate (239 mg, 2.85 mmol) were added to the solution of tert-butyl N-(1-{2-[4-amino-2-fluorophenyl)piperazine-1-yl]ethyl}, and dried with MgSO₄. Next, decompression and concentration were performed. The residue was purified by DCM/MeOH (0~5%) column chromatography to obtain the title compound (65 mg). MS (ESI, m/z): [M+H]⁺ = 533.7.

### Step 4) Synthesis of 3-[(4-{4-[2-(4-aminopiperidine-1-yl)ethyl]piperazine-1-yl}-3-fluorophenyl)amino]piperidine-2,6-dione

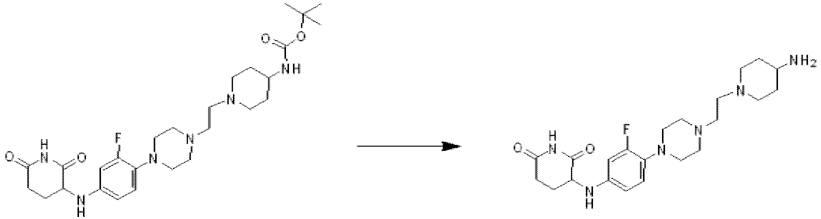

The reactants of tert-butyl N-{1-[2-(4-{4-[(2,6-dioxopiperidine-3-yl)amino]-2-fluorophenyl}piperazine-1-yl)ethyl]piperidine-4-yl}carbamate (65 mg, 122 µmol) in 30% TFA in DCM (5 mL) were stirred at room temperature for 30 minutes. After the end of the reaction, the reactants were poured into water, extracted with ethyl acetate (twice), dried with MgSO₄, and then concentrated under reduced pressure. The residues were purified by DCM/MeOH (0~5%) amine column chromatography to obtain the title compound (110 mg). MS (ESI, m/z): [M+H]⁺ = 433.5.

### Step 5) Synthesis of 4-{[(4S)-5-(cyclohexylmethyl)-4-ethyl-1-methyl-4H,5H-[1,2,4]triazolo[4,3-f]phteridine-7-yl]amino}-N-{1-[2-(4-{4-[(2,6-dioxopiperidine-3-yl)amino]-2-fluorophenyl}piperazine-1-yl)ethyl]piperidine-4-yl}-3-methoxybenzamide (ICT-0001561)

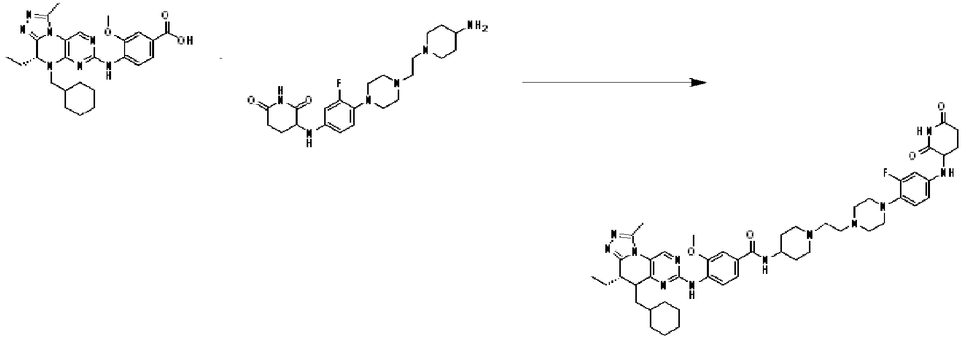

3-[(4S)-5-(cyclohexylmethyl)-4-ethyl-1-methyl-4H,5H-[1,2,4]triazolo[4,3-f]phteridine-7-yl}amino}-3-methoxybenzoic acid (123 mg, 257 µmol) in DMF (2 mL) in 3-[(4-{4-[2-(4-aminopiperidine-1-yl)ethyl]piperazine-1-yl}-3-fluorophenyl)amino]piperidine-2,6-dione (111 mg, 257 µmol), [(dimethylamino)({3H-[1,2,3]triazolo[4,5-b]pyridine-3-yloxy})methylidene]dimethylazanium hexafluoro-λ-phosphanoid (196 mg, 515 µmol) and ethylbis (propane-2-yl) amine (333 mg, 2.57 mmol). The reactants were stirred at room temperature for 2 hours. After the reaction was completed, the reactant was poured into water, extracted with DCM (twice), dried with MgSO₄, and then concentrated under reduced pressure. The residue was purified by DCM/MeOH (0~5%) column chromatography to obtain the title compound (130 mg). MS (ESI, m/z): [M+H]⁺ = 893.1
1H NMR (500 MHz, DMSO-d6) δ ppm 10.79 (s, 1 H) 8.61 (d, J=3.51 Hz, 2 H) 8.38 - 8.43 (m, 4 H) 8.35 (d, J=8.39 Hz, 2 H) 8.23 - 8.26 (m, 2 H) 7.94 (s, 2 H) 7.48 - 7.54 (m, 5 H) 7.40 (dd, J=8.32, 4.20 Hz, 2 H) 6.83 (t, J=9.31 Hz, 1 H) 6.52 (dd, J=14.88, 1.75 Hz, 1 H) 6.39 - 6.47 (m, 1 H) 5.84 (d, J=7.48 Hz, 1 H) 4.90 (dd, J=8.16, 3.74 Hz, 2 H) 4.26 (br. s., 1 H) 4.12 (dd, J=13.58, 6.26 Hz, 2 H) 3.96 (s, 6 H) 2.96 - 3.04 (m, 4 H) 2.85 - 2.90 (m, 3 H) 2.69 - 2.72 (m, 7 H) 1.21 - 1.27 (m, 5 H) 0.95 (d, J=10.53 Hz, 1 H) 0.75 (t, J=7.32 Hz, 7 H)

### Example B-18: Synthesis of 4-{[(4S)-5-(cyclohexylmethyl)-4-ethyl-1-methyl-4H,5H-[1,2,4]triazolo[4,3-f]pteridine-7-yl]amino}-N-{1-[2-(1-{4-[(2,6-dioxopiperidin-3-yl)amino]-2-fluorophenyl}piperidine-4-yl)ethyl]piperidine-4-yl}-3-methoxybenzamide (ICT-0001562)

### Step 1) Synthesis of tert-butyl N-[1-(2-{4-[2-(2,6-dioxopiperidine-3-yl)-4-methyl-1-oxo-1,2-dihydroptalazine-6-yl]piperazine-1-yl}ethyl)piperidine-4-yl]carbamate

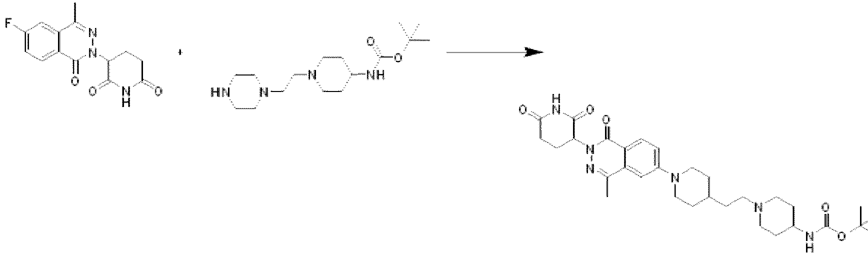

tert-butyl N- (1-{2-[1-(4-amino-2-fluorophenyl)piperidine-4-yl]ethyl}piperidine-4-yl) and ethylbis(propane-2-yl)amine (207 mg, 1.61 mmol) were added to 3-(6-fluoro-4-oxo-1,2-dihydroptalazine-2-yl)piperidine-2,6-dione (93 mg, 321 µmol) solution in NMP (2 mL). It was dried with MgSO₄ and then concentrated under reduced pressure. The residue was purified with hexane/ethyl acetate (50~90%) column chromatography to obtain the title compound (150 mg). MS (ESI, m/z): [M+H]⁺ = 581.7.

### Step 2) Synthesis of 3-[(4-{4-[2-(4-aminopiperidine-1-yl)ethyl]piperidine-1-yl}-3-fluorophenyl)amino]piperidine-2,6-dione

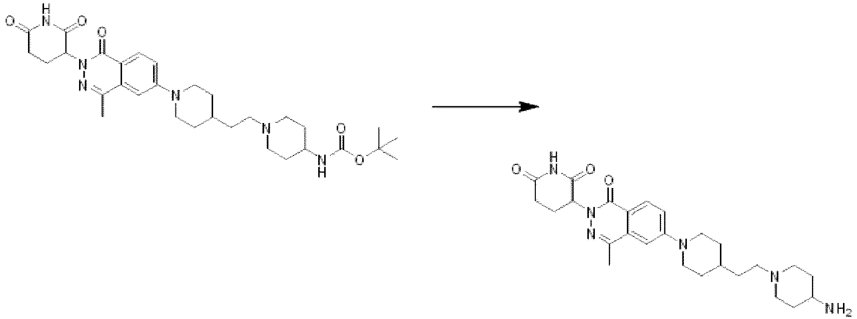

The reactants of tert-butyl N-{1-[2-(1-{4-[(2,6-dioxopiperidine-3-yl)amino]-2-fluorophenyl}piperidine-4-yl)ethyl]piperidine-4-yl}carbamate (64 mg mg, 120 µmol) in 30% TFA in DCM (1 mL) were stirred at room temperature for 30 min. After the reaction was completed, it was concentrated under reduced pressure. After the reaction was completed, the reactant was poured into water, extracted with ethyl acetate (twice), dried with MgSO₄, and then concentrated under reduced pressure. The residues were purified by DCM/MeOH (0~5%) amine column chromatography to obtain the title compound (120 mg). MS (ESI, m/z): [M+H]⁺ = 432.6.

### Step 3) Synthesis of 3-(6-{4-[2-(4-aminopiperidine-1-yl)ethyl]piperazine-1-yl}-4-methyl-1-oxo-1,2-dihydroptalazine-2-yl)piperidine-2,6-dione (ICT-0001562)

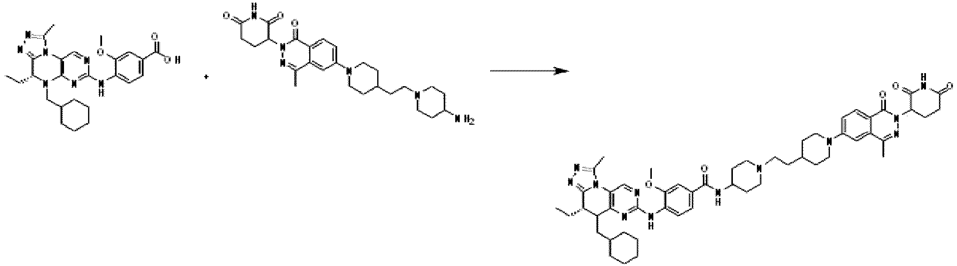

3-[(4S)-5-(cyclohexylmethyl)-4-ethyl-1-methyl-4H,5H-[1,2,4]triazolo[4,3-f]pteridine-7-yl]amino}-3-methoxybenzoic acid (56 mg, 116 µmol) in DMF (2 mL) were added with 3-[(4-{4-[2-(4-aminopiperidine-1-yl)ethyl]piperidine-1-yl}-3-fluorophenyl)amino]piperidine-2,6-dione (50 mg, 116 µmol), HATU (88.1 mg, 232 µmol) and ethylbis (propane-2-yl)amine (74.9 mg, 579 µmol). The reactants were stirred at room temperature for 2 hours. After the end of the reaction, The reactants were poured into water, extracted with DCM (twice), dried with MgSO₄, and then decompressed and concentrated. The residue was purified by DCM/MeOH (0~5%) column chromatography to obtain the title compound (38 mg). MS (ESI, m/z): [M+H]⁺ = 942.2
1H NMR (500 MHz, DMSO-d6) δ ppm 10.79 (s, 1 H) 8.40 - 8.47 (m, 2 H) 8.35 (d, J=8.55 Hz, 2 H) 7.95 (s, 1 H) 7.49 - 7.55 (m, 3 H) 6.84 (t, J=9.23 Hz, 2 H) 6.47 - 6.56 (m, 2 H) 6.42 (d, J=8.54 Hz, 1 H) 5.81 (d, J=7.78 Hz, 1 H) 4.90 (dd, J=7.86, 3.43 Hz, 2 H) 4.20 - 4.30 (m, 1 H) 4.12 (dd, J=13.66, 6.18 Hz, 2 H) 3.96 (s, 6 H) 3.12 (d, J=10.22 Hz, 3 H) 3.00 (dd, J=13.50, 8.32 Hz, 2 H) 2.70 (s, 6 H) 2.64 (s, 1 H) 2.53 - 2.62 (m, 4 H) 2.37 (s, 1 H) 2.06 - 2.11 (m, 1 H) 1.79 - 1.89 (m, 1 H) 1.74 (d, J=10.53 Hz, 1 H) 1.66 - 1.70 (m, 5 H) 1.34 (br. s., 1 H) 1.21 - 1.27 (m, 2 H) 1.10 - 1.19 (m, 3 H) 0.75 (t, J=7.32 Hz, 6 H)

### Example B-19: Synthesis of 4-(((R)-5-(cyclohexylmethyl)-4-ethyl-1-methyl-4,5-dihydro-[1,2,4]triazolo[4,3-f]pteridine-7-yl)amino)N-(1-(2-(4-(2-(2-(2-(2,6-dioxopiperidin-3-yl)-4-methyl-1-oxo-1,2-dihydroptalazin-6-yl)piperazine-1-il)acetyl)piperidine-4-yl)-3-methoxybenzamide (ICT-0001563)

### Step 1) Synthesis of 2-(4-(2-(2,6-dioxopiperidin-3-yl)-4-methyl-1-oxo-1,2-dihydroptalazine-6-yl)piperazine-1-yl)acetic acid

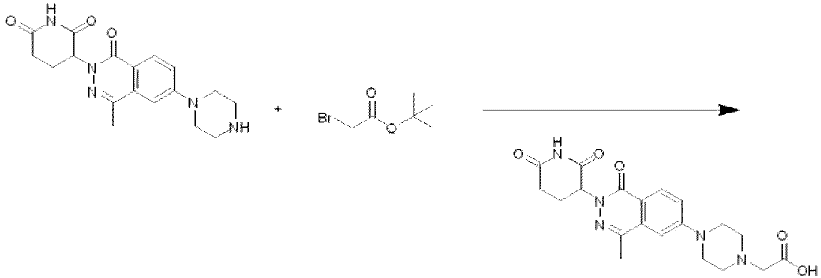

Under a nitrogen atmosphere, a mixture solution of 3-[4-methyl-1-oxo-6-(piperazine-1-yl)-1,2-dihydropetalazine-2-yl]piperidine-2,6-dione (200 mg, 563 µmol) and tert-butyl 2-bromoacetate (121 mg, 619 µmol), DIPEA (109 mg, 844 µ mol) in DCM were stirred for 5 hours at 35°C. The residues were purified by column chromatography (EA/Hexane 50%). The compound was dissolved in 1 ml DCM, followed by 0.2 ml TFA. Stirred at room temperature for 2 hours. After the reaction was finished, the reactants were concentrated under reduced pressure and vacuum-dried. The residue was dissolved in DCM, passed through an NH-DM 1020 Chromatorex pad to obtain the heading compound, which was used in the next step without further purification. (170 mg) MS (ESI, m/z): [M+H]⁺ = [470.5],

### Step 2) Synthesis of 4-(((R)-5-(cyclohexylmethyl)-4-ethyl-1-methyl-4,5-dihydro-[1,2,4]triazolo[4,3-f]pteridine-7-yl)amino)N-(1-(2-(4-(2-(2-(2-(2,6-dioxopiperidin-3-yl)-4-methyl-1-oxo-1,2-dihydroptalazine-6-yl)piperazine-1-yl)acetyl)piperidine-4-yl)-3-methoxybenzamide (ICT-0001563)

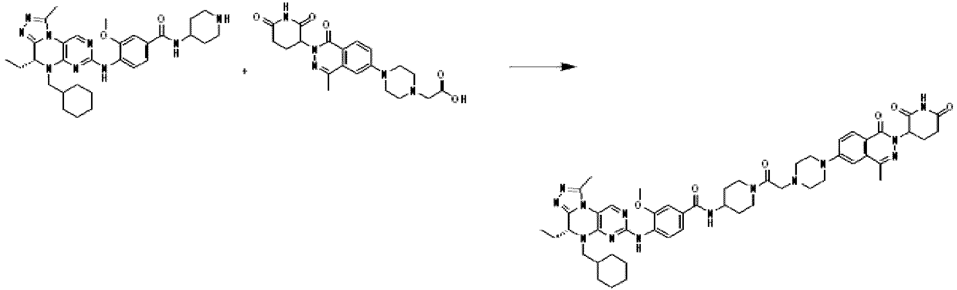

HATU was added to 4-{[(4R)-5-(cyclohexylmethyl)-4-ethyl-1-methyl-4H,5H-[1,2,4]triazolo[4,3-f]phteridine-7-yl]amino}-3-methoxy-N- (piperidine-4-yl)benzamide (70 mg, 125 µmol) and 3-(6-{4-[(azetidine-3-yl)methyl]piperazine-1-yl}-4-methyl-1-oxo-1,2-dihydroptalazine-2-ylpiperidine-2,6-dione (43.3 mg, 105 µmol) in 1 ml of DMF at room temperature. It was extracted with ethyl acetate, dried with MgSO₄, and then concentrated under reduced pressure. The residue was purified with MeOH/DCM (0-10%) MPLC to obtain the title compound (69 mg). MS (ESI, m/z): [M+H]⁺ = [956.2].
1H NMR (500 MHz, DMSO-d6) δ ppm 10.92 (s, 1 H) 8.32 (s, 2 H) 8.17 - 8.24 (m, 1 H) 8.05 (d, J=8.70 Hz, 1 H) 7.90 (br. s., 1 H) 7.47 (d, J=8.85 Hz, 1 H) 6.97 - 6.99 (m, 1 H) 6.88 (d, J=8.09 Hz, 1 H) 5.63 (d, J=7.93 Hz, 1 H) 4.82 (dd, J=8.09, 3.51 Hz, 1 H) 4.02 (dd, J=13.66, 5.87 Hz, 1 H) 3.84 (s, 4 H) 2.80 - 2.94 (m, 2 H) 2.62 (s, 13 H) 2.46 - 2.59 (m, 2 H) 2.43 (m, 11H) 1.96 - 2.05 (m, 1 H) 1.69 - 1.79 (m, 2 H) 1.53 - 1.65 (m, 4 H) 1.39 (br. s., 1 H) 1.12 - 1.24 (m, 1 H) 1.05 (br. s., 2 H) 0.99 (d, J=11.60 Hz, 1 H) 0.87 (d, J=11.60 Hz, 1 H) 0.68 (t, J=7.32 Hz, 5 H)

### Example B-20 : Synthesis of 4-{[(4S)-5-(cyclohexylmethyl)-4-ethyl-1-methyl-4H,5H-[1,[1,2,4]Triazolo[4,3-f]phteridine-7-yl]amino}-N-{1-[2-(1-{4-[(2,6-dioxopiperidin-3-yl)amino]-2-fluorophenyl}piperidine-4-yl)ethyl]piperidin-4-yl}-3-methoxybenzamide 4-{[(4S)-5-(cyclohexylmethyl)-4-ethyl-1-methyl-4H,5H-[1,2,4]triazolo[4,3-f]pteridine-7-yl]amino}-N-{1-[2-(1-{4-[(2,6-dioxopiperidin-3-yl)amino]-2-fluorophenyl}piperidine-4-yl)ethyl]piperidine-4-yl}-3-methoxybenzamide (ICT-0001564)

### Step 1) Synthesis of tert-butyl N-(1-{2-[1-(2-fluoro-4-nitrophenyl)piperidine-4-yl]ethyl}piperidine-4-yl)

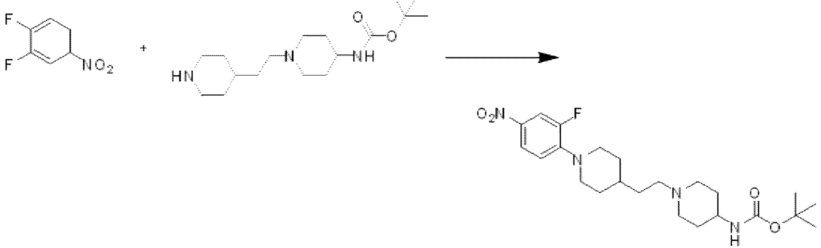

tert-butyl N-{1-[2-(piperidine-4-yl)ethyl]piperidine-4-yl} carbamate (0.1 g, 642 µmol) and dipotassium carbonate (444 mg, 3.21 mmol) were added to a solution of 1,2-difluoro-4-nitrobenzene (200 mg, 642 µmol) in DMF (1 mL). The reactants were stirred at 85°C for 2 hours. After the end of the reaction, the reactants were poured into water, extracted with ethyl acetate (twice), dried with MgSO₄, and then concentrated under reduced pressure. The residues were purified by hexane/ethyl acetate (0~60%) column chromatography to obtain the title compound (145 mg). MS (ESI, m/z): [M+H]⁺ = 451.6

### Step 2) Synthesis of tert-butyl N-(1-{2-[1-(4-amino-2-fluorophenyl)piperidine-4-yl]ethyl}piperidine-4-yl)carbamate

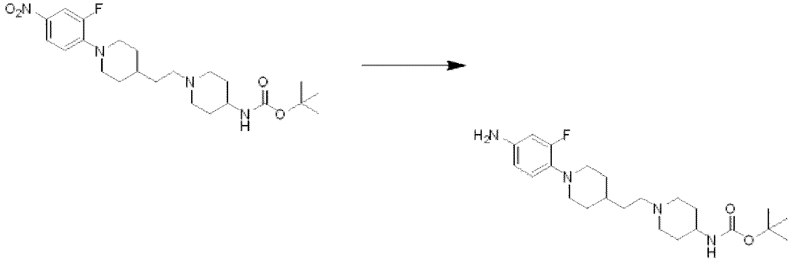

Iron powder (174 mg, 3.1 mmol) was added to tert-butyl N-(1-{2-[4-(2-fluoro-4-nitrophenyl)piperazine-1-yl]ethyl}piperidine-4-yl) carbamate (145 mg, 3.1 mmol) in saturated NH4Cl/THF (1:1) (2 mL). The reactants were stirred at 85°C for 2 hours. After the reaction was completed, the reactants were filtered and decompressively concentrated. The residues were purified with DCM/MeOH (0~5%) reversed-phase chromatography to obtain the headline compound (180 mg). (ESI, m/z): [M+H]+ = 421.6.

### Step 3) Synthesis of tert-butyl N-{1-[2-(1-{4-[(2,6-dioxopiperidin-3-yl)amino]-2-fluorophenyl}piperidine-4-yl)ethyl]piperidine-4-yl}carbamate

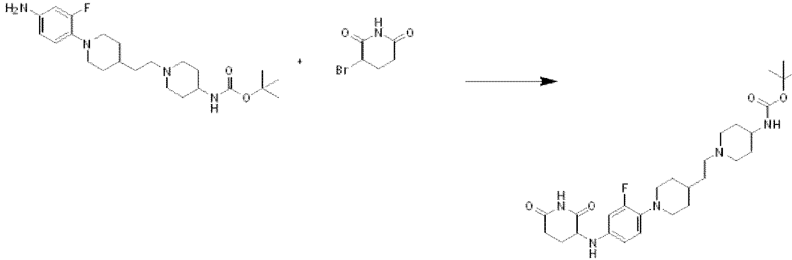

3-bromoperidin-2,6-dione (164 mg, 856 µmol) and sodium bicarbonate (216 mg, 2.57 mmol) were added to tert-butyl N-(1-{2-[1-(4-amino-2-fluorophenyl)piperidine-4-yl]ethyl}piperidine-4-yl) carbamate (180 mg, 428 µmol) in DMF (2 mL). The reactants were stirred at 85°C for 3 hours. After the reaction was completed, the reactants were poured into water, extracted with ethyl acetate (2 times), dried with MgSO₄, and concentrated under reduced pressure. The residue was purified by DCM/MeOH (0~5%) column chromatography to obtain the title compound (125 mg). MS (ESI, m/z): [M+H]⁺ = 532.7.

### Step 4) Synthesis of 3-[(4-{4-[2-(4-aminopiperidine-1-yl)ethyl]piperidine-1-il}-3-fluorophenyl)amino]piperidine-2,6-dione

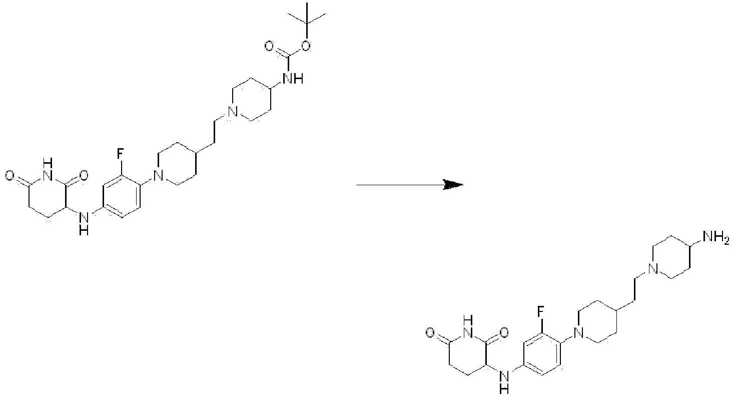

tert-butyl N-{1-[2-(4-{4-[(2,6-dioxopiperidine-3-yl)amino]-2-fluorophenyl}piperazine-1-yl)ethyl]piperidine-4-yl}carbamate (125 mg, 235 µmol) solution reactants in 30% TFA in DCM (5 mL) were stirred at room temperature for 30 minutes. After the reaction was completed, the reactants were poured into water, extracted with ethyl acetate (2 times), dried with MgSO₄, and then concentrated under reduced pressure. The residues were purified with DCM/MeOH (0~5%) amine column chromatography to obtain the title compound (101 mg). MS (ESI, m/z): [M+H]⁺ = 432.6.

### Step 5) Synthesis of 4-{[(4S)-5-(cyclohexylmethyl)-4-ethyl-1-methyl-4H,5H-[1,2,4]triazolo[4,3-f]pteridine-7-yl]amino}-N-{1-[2-(1-{4-[(2,6-dioxopiperidin-3-yl)amino]-2-fluorophenyl}piperidine-4-yl)ethyl]piperidine-4-yl}-3-methoxybenzamide (ICT-0001564)

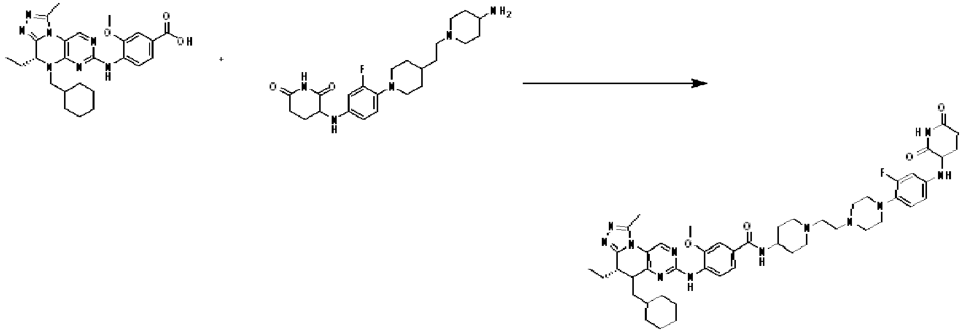

3-[(4S)-5-(cyclohexylmethyl)-4-ethyl-1-methyl-4H,5H-[1,2,4]triazolo[4,3-f]pteridin-7-yl]amino}-3-methoxybenzoic acid (55 mg, 116 µmol) in DMF (2 mL) was added to 3-[(4-{4-[2-(4-aminopiperidine-1-yl)ethyl]piperidine-1-yl}-3-fluorophenyl)amino]piperidine-2,6-dione (50 mg, 116 µmol), HATU (88 mg, 232 µmol) and ethylbis (propane-2-yl)amine (75 mg, 579 µmol). The reactants were stirred at room temperature for 2 hours. The reactants were poured into water, extracted with DCM (twice), dried with MgSO₄, and then concentrated under reduced pressure. The residue was purified by DCM/MeOH (0~5%) column chromatography to obtain the title compound (62 mg). MS (ESI, m/z): [M+H]⁺ = 892.1
1H NMR (500 MHz, DMSO-d6) δ ppm 10.98 (s, 1 H) 8.41 (s, 1 H) 8.35 (d, J=8.39 Hz, 1 H) 8.06 (d, J=9.00 Hz, 1 H) 7.94 (s, 1 H) 7.47 - 7.55 (m, 3 H) 7.07 (s, 1 H) 5.68 (d, J=7.78 Hz, 1 H) 4.90 (dd, J=8.16, 3.74 Hz, 1 H) 4.04 - 4.15 (m, 3 H) 3.96 (s, 3 H) 2.87 - 3.04 (m, 5 H) 2.70 (s, 3 H) 2.53 - 2.67 (m, 3 H) 2.49 (s, 3 H) 2.02 - 2.11 (m, 1 H) 1.81 (s, 3 H) 1.79 (s, 2 H) 1.64 - 1.73 (m, 5 H) 1.61 (br. s., 4 H) 1.26 (d, J=6.56 Hz, 8 H) 1.03 - 1.17 (m, 3 H) 0.95 (d, J=10.83 Hz, 1 H) 0.75 (t, J=7.32 Hz, 3 H)

### Example B-21 : Synthesis of 4-{[(4R)-5-(cyclohexylmethyl)-4-ethyl-1-methyl-4H,5H-[1,2,4]triazolo[4,3-f]pteridine-7-yl]amino}-N-(1-{4-[(2,6-dioxopiperidine-3-yl)amino]-2-fluorophenyl}piperidine-4-yl)-3-methoxybenzamide (ICT-0001566) synthesis Step 1) Synthesis of tert-butyl N-[1-(4-amino-2-fluorophenyl)piperidine-4-yl]carbamate

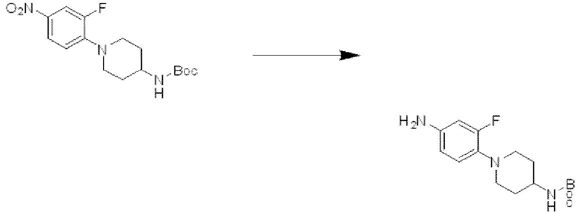

Iron powder (658 mg, 11.8 mmol) was added to tert-butyl N-[1-(2-fluoro-4-nitrophenyl)piperidine-4-yl]carbamate (0.4 g, 1.2 mmol) in saturated NH₄Cl/THF (1:1) (10 mL). The reactants were stirred at 85°C for 2 hours. After the reaction was completed, the reactants were filtered and concentrated under reduced pressure. The residue was purified with DCM/MeOH (0~5%) reversed-phase chromatography to obtain the title compound (0.3 g). (ESI, m/z): [M+H]⁺ = 310.4.

### Step 3) Synthesis of tert-butyl N-(1-{4-[(2,6-dioxopiperidin-3-yl)amino]-2-fluorophenyl}piperidine-4-yl)carbamate

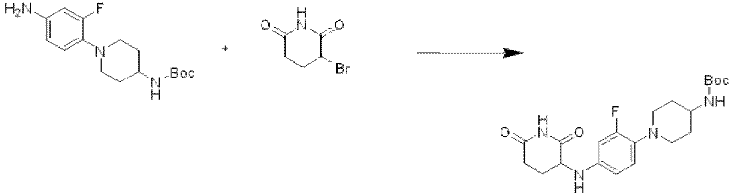

3-bromoperidin-2,6-dione (372 mg, 1.94 mmol) and sodium bicarbonate (670 mg, 4.85 mmol) were added to tert-butyl N-[1-(4-amino-2-fluorophenyl)piperidine-4-yl]carbamate (0.3 g, 970 µmol) solution in DMF (3 mL). 3-bromoperidin-2,6-dione (372 mg, 1.94 mmol) and sodium bicarbonate (670 mg, 4.85 mmol) were added to the reactant at 85°C for 3 hours. After the end of the reaction, the reactant was poured into water, extracted with ethyl acetate (2 times), dried with MgSO₄, and then concentrated under reduced pressure. The residue was purified by DCM/MeOH (0-5%) column chromatography to obtain the title compound (393 mg). MS (ESI, m/z): [M+H]⁺ = 421.5.

### Step 4) Synthesis of 3-[4-(4-aminopiperidine-1-yl)-3-fluorophenyl]piperidine-2,6-dione synthesis

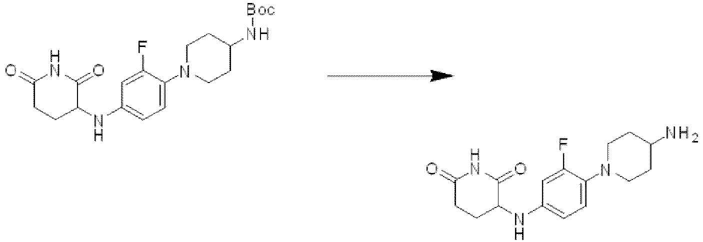

The reactants of tert-butyl N-(1-{4-[(2,6-dioxopiperidine-3-yl)amino]-2-fluorophenyl}piperidine-4-yl)carbamate (408 mg, 970 µmol) solution in 30% TFA in DCM (30 mL) were stirred at room temperature for 30 min. After the end of the reaction, the reactants were poured into water, extracted with ethyl acetate (twice), dried with MgSO₄, and then concentrated under reduced pressure. The residues were purified by DCM/MeOH (0~5%) amine column chromatography to obtain the title compound (310 mg). MS (ESI, m/z): [M+H]⁺ = 321.4.

### Step 5) Synthesis of 4-{[(4R)-5-(cyclohexylmethyl)-4-ethyl-1-methyl-4H,5H-[1,2,4]triazolo[4,3-f]pteridine-7-yl]amino}-N-(1-{4-[(2,6-dioxopiperidine-3-yl)amino]-2-fluorophenyl}piperidine-4-yl)-3-methoxybenzamide (ICT-0001566)

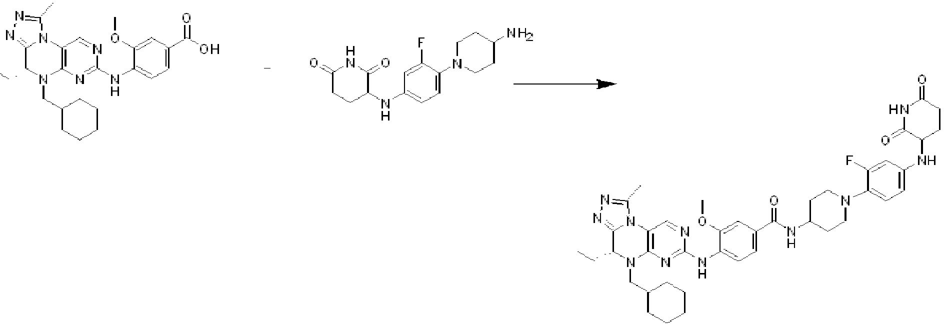

4-{[(4R)-5-(cyclohexylmethyl)-4-ethyl-1-methyl-4H,5H-[1,2,4]triazolo[4,3-f]pteridine-7-yl]amino}-3-methoxybenzoic acid (74.5 mg, 156 µmol) in DMF (1 mL) in 3-{[4-(4-(4-aminopiperidine-1-yl)-3-fluorophenyl]amino}piperidine-2,6-dione (50 mg, 156 µmol), [(dimethylamino)({3H-[1,2,3]triazolo[4,5-b]pyridine-3-yloxy})methylidene]dimethylazanium (119 mg, 312 µmol) and ethylbis (propane-2-yl)amine (202 mg, 1.56 mmol). The reactants were stirred at room temperature for 2 hours. After the reaction was completed, the reactants were poured into water, extracted with DCM (twice), dried with MgSO₄, and then concentrated under reduced pressure. The residues were purified by DCM/MeOH (0~5%) column chromatography to obtain the title compound (130 mg). MS (ESI, m/z): [M+H]⁺ = 780.9
1H NMR (500 MHz, DMSO-d6) δ ppm 10.72 (s, 1 H) 8.34 (s, 1 H) 8.28 (d, J=8.39 Hz, 1 H) 8.13 (d, J=7.93 Hz, 1 H) 7.86 (s, 1 H) 7.40 - 7.49 (m, 2 H) 6.81 (t, J=9.31 Hz, 1 H) 6.45 (dd, J=15.03, 1.91 Hz, 1 H) 6.36 (d, J=8.55 Hz, 1 H) 5.75 (d, J=7.63 Hz, 1 H) 4.83 (dd, J=7.86, 3.74 Hz, 1 H) 4.16 - 4.24 (m, 1 H) 4.05 (dd, J=13.66, 6.18 Hz, 1 H) 3.89 (s, 3 H) 3.79 - 3.87 (m, 1 H) 3.10 (d, J=11.14 Hz, 2 H) 2.92 (dd, J=13.43, 8.24 Hz, 1 H) 2.54 - 2.72 (m, 7 H) 2.46 - 2.54 (m, 1 H) 1.98 - 2.04 (m, 1 H) 1.80 - 1.83 (m, 2 H) 1.65 - 1.78 (m, 5 H) 1.58 - 1.63 (m, 4 H) 1.17 (s, 1 H) 0.94 - 1.14 (m, 4 H) 0.89 (d, J=10.22 Hz, 1 H) 0.68 (t, J=7.32 Hz, 3 H)

### Example B-22 : Synthesis of 4-{[(4R)-5-(cyclohexylmethyl)-4-ethyl-1-methyl-4H,5H-[1,2,4]triazolo[4,3-f]pteridine-7-yl]amino}-N-{1-[2-(2,6-dioxopiperidine-3-yl)-4-methyl-1-oxo-1,2-dihydroptalazine-6-yl]piperidine-4-yl}-3-methoxybenzamide (ICT-0001567)

### Step 1) Synthesis of 3-[6-(4-aminopiperidine-1-yl)-4-methyl-1-oxo-1,2-dihydroptalazine-2-yl]piperidine-2,6-dione

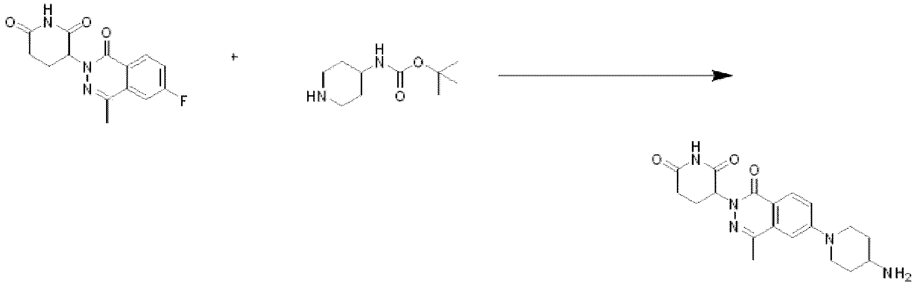

tert-butyl (2R)-2-[(piperazine-1-yl)methyl]morpholine-4-carboxylate (300 mg, 1.04 mmol), 3-(6-fluoro-4-methyl-1-oxo-1,2-dihydroptalazin-2-yl)piperidine-2,6-dione (208 mg, 1.04 mmol), and DIPEA (402 mg, 3.11 mmol) solutions in NMP (2 mL) were stirred at 140°C for 6 hours. Stirred at room temperature for 2 hours. After the reaction was finished, the reactants were concentrated under reduced pressure and vacuum-dried. The residue was decentralized with DCM, passed through an NH-DM 1020 Chromatorex pad to obtain the heading compound, which was used in the next step without further purification. (130 mg) MS (ESI, m/z): [M+H]⁺ = [370.4 g/mol].

### Step 2) Synthesis of 4-{[(4R)-5-(cyclohexylmethyl)-4-ethyl-1-methyl-4H,5H-[1,2,4]triazolo[4,3-f]pteridine-7-yl]amino}-N-{1-[2-(2,6-dioxopiperidine-3-yl)-4-methyl-1-oxo-1,2-dihydropthalazine-6-yl]piperidine-4-yl}-3-methoxybenzamide (ICT-0001567)

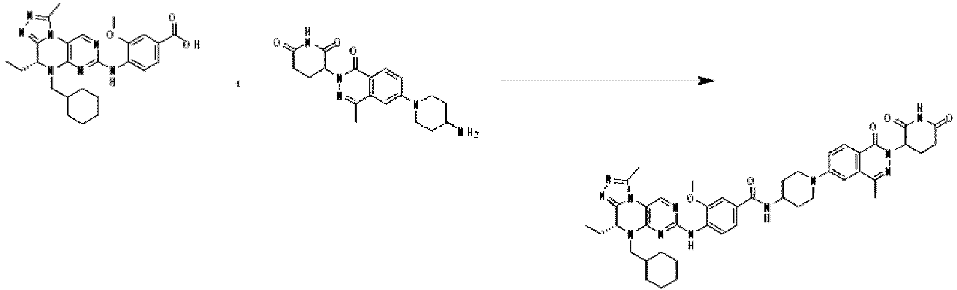

HATU (103 mg, 271 µmol) was added to 3-[6-(4-aminopiperidine-1-yl)-4-methyl-1-oxo-1,2-dihydroptalazin-2-yl]piperidine-2,6-dione (50 mg, 135 µmol) and 4-{[(4R)-5-(cyclohexylmethyl)-4-ethyl-1-methyl-4H,5H-[1,2,4]triazolo[4,3-f]pteridine-7-yl]amino}-3-methoxybenzoic acid (64.6 mg, 135 µmol) in 1 ml of DMF at room temperature. It was extracted with ethyl acetate, dried with MgSO₄, and then concentrated under reduced pressure. The residue was purified with MeOH/DCM (0~10%) MPLC to obtain the title compound (69 mg). MS (ESI, m/z): [M+H]⁺ = [830.0]
1H NMR (500 MHz, DMSO-d6) δ ppm 10.72 (s, 1 H) 8.34 (s, 1 H) 8.28 (d, J=8.39 Hz, 1 H) 8.13 (d, J=7.93 Hz, 1 H) 7.86 (s, 1 H) 7.40 - 7.49 (m, 2 H) 6.81 (t, J=9.31 Hz, 1 H) 6.45 (dd, J=15.03, 1.91 Hz, 1 H) 6.36 (d, J=8.55 Hz, 1 H) 5.75 (d, J=7.63 Hz, 1 H) 4.83 (dd, J=7.86, 3.74 Hz, 1 H) 4.16 - 4.24 (m, 1 H) 4.05 (dd, J=13.66, 6.18 Hz, 1 H) 3.89 (s, 3 H) 3.79 - 3.87 (m, 1 H) 3.10 (d, J=11.14 Hz, 2 H) 2.92 (dd, J=13.43, 8.24 Hz, 1 H) 2.54 - 2.72 (m, 7 H) 2.46 - 2.54 (m, 1 H) 1.98 - 2.04 (m, 1 H) 1.80 - 1.83 (m, 2 H) 1.65 - 1.78 (m, 5 H) 1.58 - 1.63 (m, 4 H) 1.17 (s, 1 H) 0.94 - 1.14 (m, 4 H)

### Example B-23 : Synthesis of 4-{[(4R)-5-(cyclohexylmethyl)-4-ethyl-1-methyl-4H,5H-[1,2,4]triazolo[4,3-f]phteridine-7-yl]amino}-N-[1-(3-{4-[2-(2,6-dioxopiperidin-3-yl)-4-methyl-1-oxo-1,2-dihydroptalazine-6-yl}piperazine-1-il}piperidine-4-yl]piperidine-4-yl]-3-methoxybenzamide (ICT-0001568)

### Step 1) Synthesis of 2-(4-(2-(2,6-dioxopiperidin-3-yl)-4-methyl-1-oxo-1,2-dihydroptalazine-6-yl)piperazine-1-yl)acetic acid

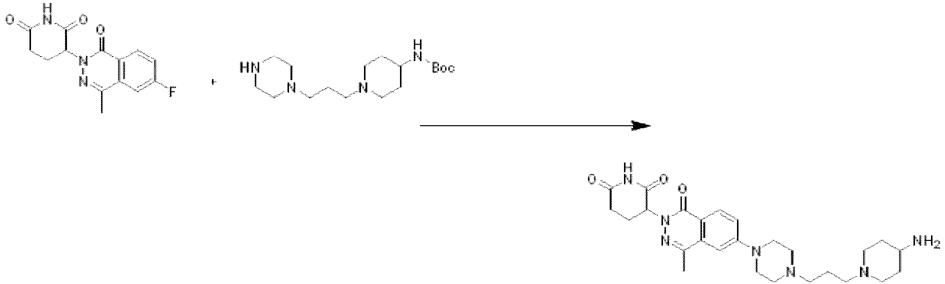

Under a nitrogen atmosphere, a mixture of 3-[4-methyl-1-oxo-6-(piperazine-1-yl)-1,2-dihydroptalazine-2,6-dione (113 mg, 613 µmol) and tert-butyl N-{1-[3-(piperazine-1-yl)propyl]piperidine-4-yl}carbamate (200 mg, 613 µmol), DIPEA (396 mg, 3.06 mmol) in NMP were stirred for 8 hours at 140°C. The residues were purified by column chromatography (EA/Hexane 50%). The compound was dissolved in 1 ml DCM. Then, 0.2 ml TFA was added and stirred at room temperature for 2 hours. After the reaction was finished, the reactants were concentrated under reduced pressure and vacuum-dried. The residue was dissolved in DCM, passed through an NH-DM 1020 Chromatorex pad to obtain the heading compound, which was used in the next step without further purification. (110 mg) MS (ESI, m/z): [M+H]⁺ = [596.7],

### Step 2) Synthesis of 4-{[(4R)-5-(cyclohexylmethyl)-4-ethyl-1-methyl-4H,5H-[1,2,4]triazolo[4,3-f]phteridine-7-yl]amino}-N-{1-[2-(2,6-dioxopiperidin-3-yl)-4-methyl-1-oxo-1,2-dihydroptalazin-6-yl]piperidine-4-yl}-3-methoxybenzamide (ICT-0001568)

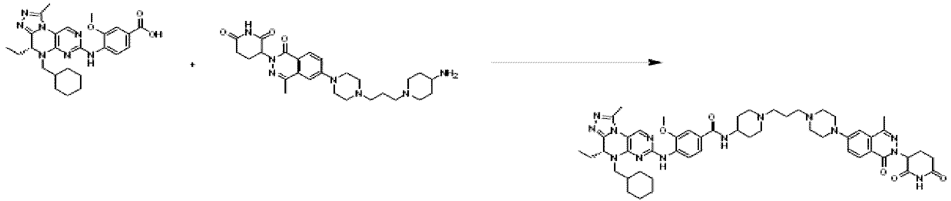

HATU (115 mg, 303 µmol) was added to 3 -[6-(4-aminopiperidine-1-yl)-4-methyl-1-oxo-1,2-dihydroptalazin-2-yl]piperidine-2,6-dione (75 mg, 151 µmol) and 4-{[(4R)-5-(cyclohexylmethyl)-4-ethyl-1-methyl-4H,5H-[1,2,4]triazolo[4,3-f]pteridin-7-yl]amino}-3-methoxybenzoic acid (72.3 mg, 151 µmol), followed by ethylbis (propane-2-yl)amine (97.8 mg, 757 µmol) in 1 ml of DMF at room temperature. It was extracted with ethyl acetate, dried with MgSO₄, and then concentrated under reduced pressure. The residue was purified with MeOH/DCM (0~10%) MPLC to obtain the title compound (69 mg). MS (ESI, m/z): [M+H]⁺ = [956.2]
1H NMR (500 MHz, DMSO-d6) δ ppm 10.72 (s, 1 H) 8.34 (s, 1 H) 8.27 (d, J=8.39 Hz, 1 H) 7.86 (s, 1 H) 7.40 - 7.44 (m, 2 H) 6.83 (t, J=9.31 Hz, 1 H) 6.52 (dd, J=14.88, 1.75 Hz, 1 H) 6.40 - 6.47 (m, 1 H) 4.83 (dd, J=8.24, 3.66 Hz, 1 H) 4.26 (br. s., 1 H) 4.12 (dd, J=13.58, 6.26 Hz, 2 H) 4.04 (dd, J=13.73, 6.10 Hz, 1 H) 3.94 (s, 6 H) 3.88 (s, 3 H) 2.96 - 3.04 (m, 4 H) 2.92 (dd, J=13.50, 8.16 Hz, 1 H) 2.85 - 2.90 (m, 4 H) 2.69 - 2.73 (m, 7 H) 2.60 - 2.67 (m, 4 H) 2.49 - 2.54 (m, 1 H) 2.01 - 2.03 (m, 3 H) 1.56 - 1.63 (m, 4 H) 1.21 - 1.26 (m, 5 H) 1.16 - 1.21 (m, 4 H) 0.96 (s, 1 H) 0.67 (t, J=7.32 Hz, 3 H)

### Example B-24 : Synthesis of 4-{[(4R)-5-(cyclohexylmethyl)-4-ethyl-1-methyl-4H,5H-[1,2,4]triazolo[4,3-f]pteridine-7-yl]amino}-N-[1-({4-[(2,6-dioxopiperidin-3-yl)amino]phenyl}methyl)piperidine-4-yl]-3-methoxybenzamide (ICT-0001569)

### Step 1) Synthesis of Tert-butyl N-{1-[(4-nitrophenyl)methyl]piperidine-4-yl}carbamate

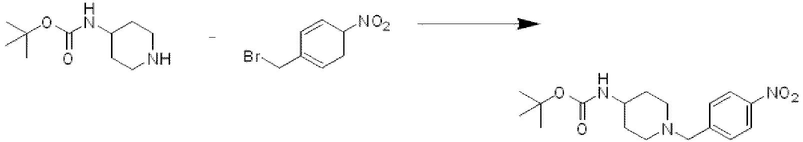

tert-butyl N- (piperidine-4-yl) carbamate (1.21 g, 6.02 mmol) and triethylamine (937 mg, 9.26 mmol) were added to a solution of 1-(bromomethyl)-4-nitrobenzene (1 g, 4.63 mmol) in CH₂Cl₂ (1 mL). The reactants were stirred at room temperature for 6 hours. After the reaction was finished, the reactants were poured with water and washed twice. The organic layer was dried with MgSO₄, followed by concentration under reduced pressure. The residues were purified with hexane/ethyl acetate (0~60%) column chromatography to obtain the title compound (1.55 g). MS (ESI, m/z): [M+H]⁺ = 336.4.

### Step 2) Synthesis of Tert-butyl N-{1-[(4-aminophenyl)methyl]piperidine-4-yl}carbamate

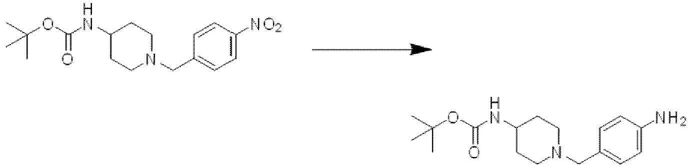

Iron powder (3.75 g, 67.1 mmol) was added to tert-butyl N-{1-[(4-nitrophenyl)methyl]piperidine-4-yl} carbamate (1.5 g, 67.1 mmol) in saturated NH4Cl/THF (1:1) (20 mL). The reactants were stirred at 85°C for 2 hours. After the reaction ends, the reactants are filtered and concentrated under reduced pressure. The residue was purified by DCM/MeOH (0-5%) reversed-phase chromatography to obtain the title compound (1.60 g). (ESI, m/z): [M+H]+ = 306.4

### Step 3) Synthesis of tert-butyl N-{1-[2-(4-{4-[(2,6-dioxopiperidine-3-yl)amino]-2-fluorophenyl}piperazine-1-yl)ethyl]piperidine-4-yl}carbamate

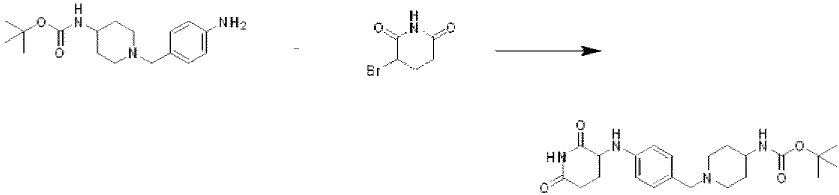

3-bromoperidin-2,6-dione (8.05 g, 41.9 mmol) and sodium bicarbonate (4.4 g, 52.4 mmol) were added to tert-butyl N-{1-[(4-aminophenyl)methyl]piperidine-4-yl}carbamate (1.6 g, 5.24 mmol) solution in DMF (10 mL). 3-bromoperidin-2,6-dione (8.05 g, 41.9 mmol) and sodium bicarbonate (4.4 g, 52.4 mmol) were stirred at 85°C for 3 hours. After the reaction was finished, the reactants were poured into water, extracted with ethyl acetate (2 times), dried with MgSO4, and then concentrated under reduced pressure. The residues were purified by DCM/MeOH (0~5%) column chromatography to obtain the title compound (2.4 g). MS (ESI, m/z): [M+H]⁺ = 417.5.

### Step 4) Synthesis of 3-[(4-{4-[2-(4-aminopiperidine-1-yl)ethyl]piperazine-1-yl}-3-fluorophenyl)amino]piperidine-2,6-dione

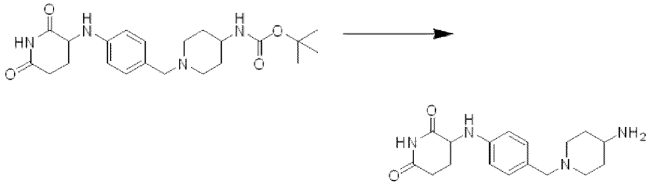

tert-butyl N-[1-({4-[(2,6-dioxopiperidine-3-yl)amino]phenyl}methyl)piperidine-4-yl]carbamate (2.46 g, 5.91 mmol) in 30% TFA DCM (5 mL). The reactants were stirred at room temperature for 30 minutes. After the end of the reaction, the reactants were poured with water, extracted with ethyl acetate (twice), dried with MgSO₄, and then concentrated under reduced pressure. The residues were purified and amine column chromatography yielded with DCM/MeOH (0~5%) heading compound (1.66 g). MS (ESI, m/z): [M+H]⁺ = 317.4

### Step 5) Synthesis of 4-{[(4R)-5-(cyclohexylmethyl)-4-ethyl-1-methyl-4H,5H-[1,2,4]triazolo[4,3-f]pteridine-7-yl]amino}-N-[1-({4-[(2,6-dioxopiperidine-3-yl)amino]phenyl}methyl)piperidine-4-yl]-3-methoxybenzamide (ICT-0001569)

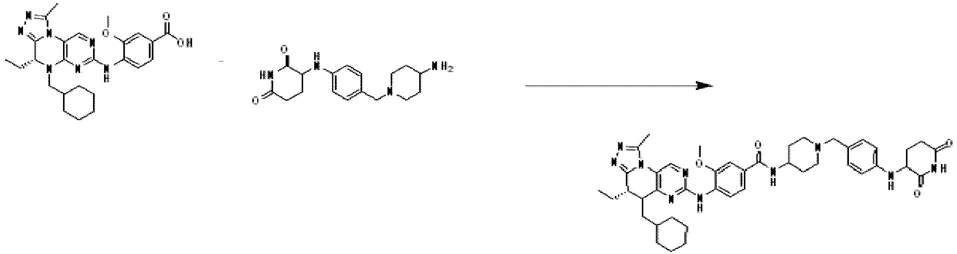

3-({4-[(4R)-5-(cyclohexylmethyl)-4-ethyl-1-methyl-4H,5H-[1,2,4]triazolo[4,3-f]phteridine-7-yl]amino}-3-methoxybenzoic acid (50 mg, 105 µmol) in DMF (2 mL) was added with 3-({4-[(4-aminopiperidine-1-yl)methyl}phenyl}amino)piperidine-2,6-dione ((49.7 mg, 157 µmol), HATU (119 mg, 314 µmol) and ethylbis (propane-2-yl)amine (27 mg, 209 µmol). The reactants were stirred at room temperature for 2 hours. The reactants were poured into water, extracted with DCM (twice), dried with MgSO₄, and then concentrated under reduced pressure. The residue was purified by DCM/MeOH (0~5%) column chromatography to obtain the title compound (35 mg). MS (ESI, m/z): [M+H]⁺ = 770.1
1H NMR (500 MHz, DMSO-d6) δ ppm 10.81 (s, 1 H) 8.38 - 8.46 (m, 1 H) 8.34 (d, J=8.39 Hz, 1 H) 8.21 (d, J=6.71 Hz, 1 H) 7.87 - 7.99 (m, 1 H) 7.44 - 7.54 (m, 2 H) 7.07 (m, J=7.93 Hz, 2 H) 6.66 (m, J=8.39 Hz, 2 H) 4.90 (dd, J=8.24, 3.81 Hz, 1 H) 4.27 - 4.37 (m, 1 H) 4.12 (dd, J=13.66, 6.33 Hz, 1 H) 3.92 - 3.99 (m, 3 H) 3.85 (br. s., 1 H) 3.55 - 3.67 (m, 2 H) 3.51 (br. s., 1 H) 2.99 (dd, J=13.58, 8.24 Hz, 2 H) 2.65 - 2.80 (m, 5 H) 2.06 - 2.15 (m, 1 H) 1.76 - 1.99 (m, 4 H) 1.63 - 1.73 (m, 4 H) 1.60 (br. s., 2 H) 1.04 - 1.28 (m, 10 H) 0.75 (t, J=7.40 Hz, 3 H)

### Example B-25: Synthesis of 4-{[(4R)-5-(cyclohexylmethyl)-4-ethyl-1-methyl-4H,5H-[1,2,4]triazolo[4,3-f]pteridine-7-yl]amino}-N-{1-[2-(2-{4-[(2,6-dioxopiperidin-3-yl)amino]-2-fluorophenoxy}ethoxy)ethyl]piperidine-4-yl}-3-methoxybenzamide (ICT-0001570)

### Step 1) Synthesis of 2-[2-(2-fluoro-4-nitrophenoxy)ethoxy]ethane-1-ol

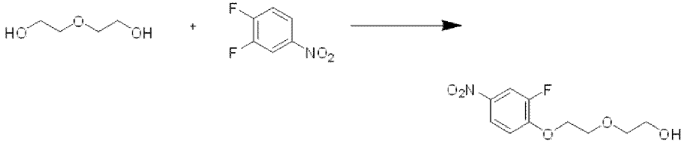

Potassium 2-methylpropane-2-oleate (354 mg, 3.14 mmol) was added in part to 2-(2-hydroxyethoxy)ethane-1-ol (667 mg, 6.29 mmol) solution in stirred THF (3 mL) under nitrogen at 0°C. The mixture was stirred at 0°C for 30 minutes, followed by 3-chloro-4-fluoronitrobenzene (200 mg, 1.26 mmol) (the reactants turn dark) slowly. The mixture was stirred at 0°C for 10 minutes, followed by the removal of the ice bath. The reactants were warmed to room temperature (10 min) and stirred for 1 hour. The solvent was removed under vacuum, and the residues were distributed between EtOAc (50 mL) and H₂O (50 mL). The aqueous layer was extracted with EtOAc (2 x 50 mL), the combined organic layer was dried (Na₂SO₄), filtered, and the solvent was removed under vacuum, leaving the product behind. The residues were purified with hexane/ethyl acetate (0-60%) column chromatography to obtain the title compound (0.4 g). MS (ESI, m/z): [M+H]⁺ = 246.2.

### Step 2) Synthesis of 2-[2-(2-fluoro-4-nitrophenoxy)ethoxy]ethyl methanesulfonate

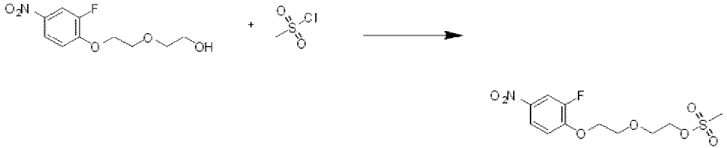

Methanesulfonyl chloride (374 mg, 3.26 mmol) in CH2Cl2 (15 mL) was added to 2-[2-(2-(2-fluoro-4-nitrophenoxy)ethoxy]ethane-1-ol (400 mg, 1.63 mmol) in CH2Cl2 (50 mL), followed by trimethylamine (413 mg, 4.08 mmol) at 0°C, and then stirred for 2 hours. The reactants were warmed to room temperature and stirred for an additional 2 hours. The reactants were poured into water (50 mL), extracted as dichloromethane (50 mLx3). The organic layers were combined, washed with brine solution (50 mLx2), dried with sodium sulfate anhydrous, then filtered and concentrated. The residue was used in the next step without additional purification. (527 mg), MS (ESI, m/z): [M+H]⁺ = [551.0],

### Step 3) Synthesis of tert-butyl N-(1-{2-[2-(2-fluoro-4-nitrophenoxy)ethoxy]ethyl}piperidine-4-yl)carbamate

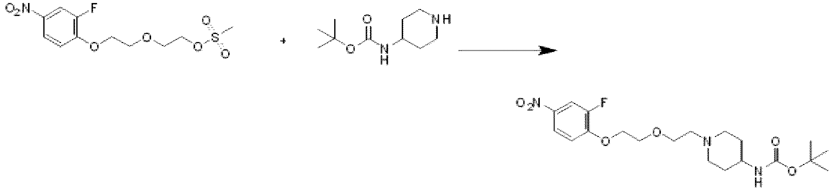

Ethyl bis (propane-2-yl)amine (1.05 g, 8.16 mmol) was added to a solution of 2-[2-(2-(2-fluoro-4-nitrophenoxy)ethoxy]ethyl methanesulfonate (527 mg, 1.63 mmol) tert-butyl N-(piperidine-4-yl)carbamate (427 mg, 4.08 mmol) in 50 ml of acetonitrile at room temperature, and the reactants were stirred at 80°C for 6 hours. (6.01g). MS (ESI, m/z): [M+H]⁺ = [318.4].

### Step 4) Synthesis of tert-butyl N-(1-{2-[2-(4-amino-2-fluorophenoxy)ethoxy]ethyl}piperidine-4-yl)carbamate

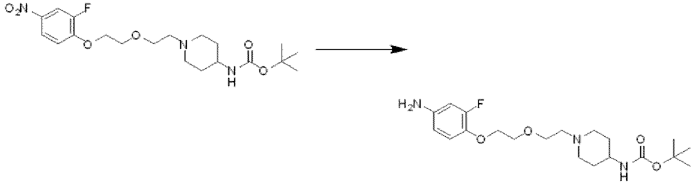

10 mL saturated NH4Cl was added to a solution of tert-butyl N- (1-{2-[2-(2-(2-(2-nitrophenoxy)ethoxy]ethyl}piperidine-4-yl)carbamate (0.9 g, 2.11 mmol) and iron (2.35 g, 42.1 mmol) in THF (10 ml). The reactants were stirred at 100°C for 2 hours. After cooling to room temperature, the reactants were filtered, the filtrate was poured into water, and extracted with ethyl acetate. The organic layer was dried with MgSO₄, concentrated under reduced pressure, and then purified by column chromatography to obtain the heading compound (0.75 g) MS (ESI, m/z): [M+H]⁺ = [398.5].

### Step 5) Synthesis of tert-butyl N-{1-[2-(2-{4-[(2,6-dioxopiperidine-3-yl)amino]-2-fluorophenoxy}ethoxy)ethyl]piperidine-4-yl}carbamate

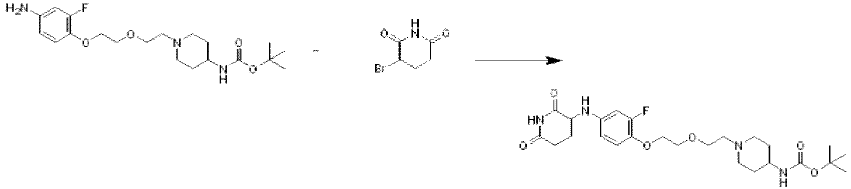

3-bromoperidin-2,6-dione (2.9 g, 15.1 mmol) and sodium bicarbonate (1.59 g, 18.9 mmol) were added to tert-butyl N-(1-{2-[2-(4-amino-2-fluorophenoxy)ethoxy]ethyl}piperidine-4-yl)carbamate (750 mg, 1.89 mmol) in DMF (10 mL). 3-bromopiperidin-2,6-dione (2.9 g, 15.1 mmol) and sodium bicarbonate (1.59 g, 18.9 mmol) were added to the reactant in DMF (10 mL). Next, decompression and concentration were performed. The residue was purified by DCM/MeOH (0~5%) column chromatography to obtain the title compound (960 mg). MS (ESI, m/z): [M+H]⁺ = 509.6

### Step 6) Synthesis of 3-[(4-{2-[2-(4-aminopiperidine-1-yl)ethoxy]ethoxy}-3-fluorophenyl)amino]piperidine-2,6-dione

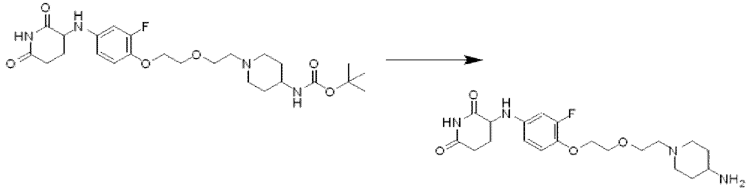

The reactants of tert-butyl N-{1-[2-(2-{4-[(2,6-dioxopiperidin-3-yl)amino]-2-fluorophenoxy}ethoxy)ethyl]piperidine-4-yl}carbamate (750 mg, 1.89 mmol) in 30% TFA in DCM (5 mL) were stirred at room temperature for 30 min. After the end of the reaction, the reactants were poured into water, extracted with ethyl acetate (twice), dried with MgSO₄, and then concentrated under reduced pressure. The residues were purified by DCM/MeOH (0~5%) amine column chromatography to obtain the title compound (560 mg). MS (ESI, m/z): [M+H]⁺ = 409.5

### Step 7) Synthesis of 4-{[(4R)-5-(cyclohexylmethyl)-4-ethyl-1-methyl-4H,5H-[1,2,4]triazolo[4,3-f]pteridine-7-yl]amino}-N-{1-[2-(2-{4-[(2,6-dioxopiperidin-3-yl)amino]-2-fluorophenoxy}ethoxy)ethyl]piperidine-4-yl}-3-methoxybenzamide (ICT-0001570)

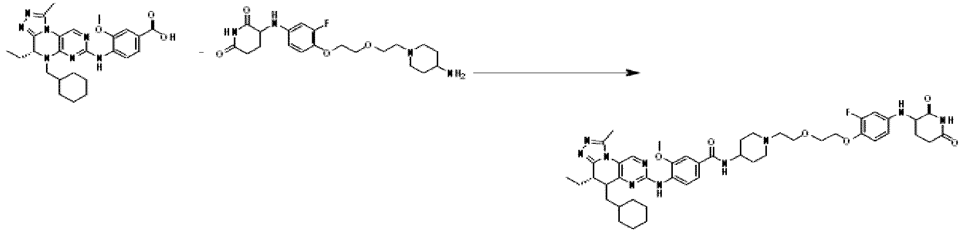

4-{[(4R)-5-(cyclohexylmethyl)-4-ethyl-1-methyl-4H,5H-[1,2,4]triazolo[4,3-f]pteridine-7-yl]amino}-3-methoxybenzoic acid (50 mg, 105 µmol), HATU (99.5 mg, 262 µ mol) and ethylbis (propane-2-yl)amine (27 mg, 209 µmol) were added to a solution of 3-[(4R)-5-(cyclohexylmethyl)-4-ethyl-1-methyl-4H,5H-[1,2,4]triazolo[4,3-f]pteridine-7-yl]amino}-3-methoxybenzoic acid (50 mg, 105 µmol), HATU (99.5 mg, 262mol) and ethylbis (propane-2-yl)amine (27 mg, 209 µmol). The reactants were stirred at room temperature for 2 hours. The reactants were poured into water, extracted with DCM (twice), dried with MgSO₄, and then concentrated under reduced pressure. The residue was purified by DCM/MeOH (0~5%) column chromatography to obtain the title compound (38 mg). MS (ESI, m/z): [M+H]⁺ = 869.0
1H NMR (500 MHz, DMSO-d6) δ ppm 10.72 (s, 1 H) 8.49 (d, J=3.97 Hz, 1 H) 8.24 - 8.37 (m, 2 H) 8.17 (d, J=7.48 Hz, 1 H) 7.86 (s, 1 H) 7.40 - 7.47 (m, 2 H) 7.30 (dd, J=8.32, 4.35 Hz, 1 H) 6.86 (t, J=9.31 Hz, 1 H) 6.51 (dd, J=14.19, 2.59 Hz, 1 H) 6.35 (d, J=9.00 Hz, 1 H) 5.73 (d, J=7.78 Hz, 1 H) 4.83 (dd, J=8.24, 3.81 Hz, 1 H) 4.14 - 4.24 (m, 1 H) 4.05 (dd, J=13.43, 6.41 Hz, 1 H) 3.93 - 4.00 (m, 2 H) 3.88 (s, 3 H) 3.57 - 3.68 (m, 4 H) 3.32 - 3.45 (m, 8 H) 2.92 (dd, J=13.73, 8.24 Hz, 1 H) 2.59 - 2.69 (m, 4 H) 1.98 - 2.07 (m, 1 H) 1.89 (d, J=16.94 Hz, 2 H) 1.78 - 1.84 (m, 2 H) 1.70 - 1.77 (m, 2 H) 1.56 - 1.69 (m, 5 H) 1.11 - 1.22 (m, 2 H) 1.01 - 1.10 (m, 2 H) 0.68 (t, J=7.32 Hz, 3 H)

### Example B-26: Synthesis of 4-{[(4R)-5-(cyclohexylmethyl)-4-ethyl-1-methyl-4H,5H-[1,2,4]triazolo[4,3-f]pteridine-7-yl]amino}-N-(1-[3-(4-(4-[(2,6-dioxopiperidine-3-yl)amino]-2-fluorophenyl}piperazine-1-yl)propanoyl]piperidine-4-yl}-3-methoxybenzamide (ICT-0001571) synthesis

### Step 1) Synthesis of tert-butyl 3-(4-{4-[(2,6-dioxopiperidin-3-yl)amino]-2-fluorophenyl}piperazine-1-yl)propanoate

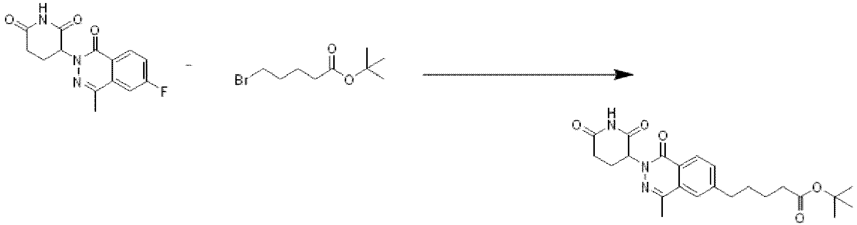

tert-butyl 3-bromopropanoate (232 mg, 979 µmol) and ethylbis (propane-2-yl)amine (169 mg, 1.31 mmol) were added to 3-{[3-fluoro-4-(piperazine-1-yl)phenyl]amino}piperidine-2,6-dione (0.2 g, 653 µmol) solution in DCM (5 mL). tert-butyl 3-bromopropanoate (232 mg, 979 µmol) and ethylbis (propane-2-yl)amine (169 mg, 1.31 mmol) were stirred overnight. After the reaction was completed, the reactant was poured into water, extracted with ethyl acetate (twice), dried with MgSO4, and then concentrated under reduced pressure. The residue was purified by DCM/MeOH (0~5%) amine column chromatography to obtain the title compound (205 mg). MS (ESI, m/z): [M+H]⁺ = 463.6.

### Step 2) Synthesis of 5-(4-{4-[(2,6-dioxopiperidine-3-yl)amino]-2-fluorophenyl}piperazine-1-yl)pentanoic acid

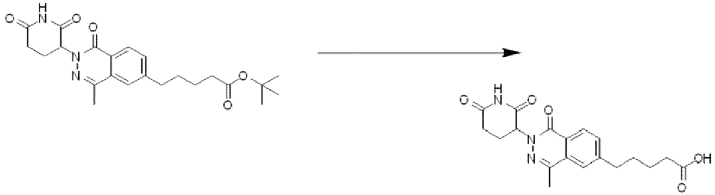

The reactants of tert-butyl 5-(4-{4-[(2,6-dioxopiperidine-3-yl)amino]-2-fluorophenyl}piperazine-1-yl) pentanoate (200 mg, 432 µmol) solution in 30% TFA in DCM (100 mL) were stirred at room temperature for 30 min. After the end of the reaction, the reactants were poured into water, extracted with ethyl acetate (twice), dried with MgSO₄, and then concentrated under reduced pressure. The residues were purified with DCM/MeOH (0~5%) amine column chromatography to obtain the title compound (402 mg). MS (ESI, m/z): [M+H]⁺ = 407.5.

### Step 3) Synthesis of 4-{[(4R)-5-(cyclohexylmethyl)-4-ethyl-1-methyl-4H,5H-[1,2,4]triazolo[4,3-f]pteridine-7-yl]amino}-N-{1-[5-(4-{4-[(2,6-dioxopiperidin-3-yl)amino]-2-fluorophenyl}piperazine-1-yl)pentanoyl]piperidine-4-yl}-3-methoxybenzamide (ICT-0001571)

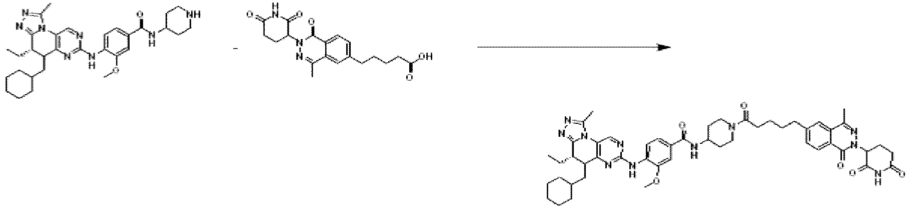

5-(4R)-5-(cyclohexylmethyl)-4-ethyl-1-methyl-4H,5H-[1,2,4]triazolo[4,3-f]pteridine-7-yl]amino}-3-methoxy-N- (piperidine-4-yl)benzamide (110 mg, 197 µmol) in DMF (2 mL) was added with 5-(4-(4-((2,6-dioxopiperidine-3-yl)amino)-2-fluorophenyl)piperazine-1-yl) pentanoic acid (80 mg, 197 µmol), HATU (149 mg, 393 µmol) and ethylbis (propane-2-yl)amine (127 mg, 983 µmol). The reactants were stirred at room temperature for 2 hours. After the end of the reaction. The reactants were poured into water, extracted with DCM (twice), dried with MgSO₄, and then concentrated under reduced pressure. The residues were purified by DCM/MeOH (0~5%) column chromatography to obtain the title compound (141 mg). MS (ESI, m/z): [M+H]⁺ = 949.2.

### Example B-27: Synthesis of 4-{[(4R)-5-(cyclohexylmethyl)-4-ethyl-1-methyl-4H,5H-[1,2,4]triazolo[4,3-f]pteridine-7-yl]amino}-N-{1-[3-(4-{4-[(2,6-dioxopiperidin-3-yl)amino]-2-fluorophenyl}piperazine-1-yl)propyl]piperidine-4-yl}-3-methoxybenzamide (ICT-0001572)

### Step 1) Synthesis of Tert-butyl (1-(3-(4-(2-fluoro-4-nitrophenyl)piperazine-1-il)propyl)piperidine-4-yl)carbamate

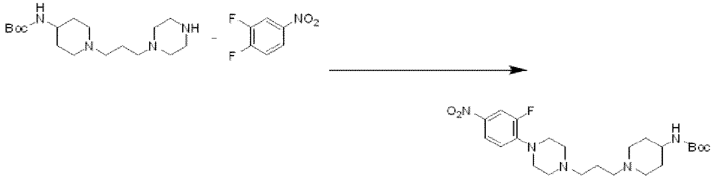

Tert-butyl N-{1-[3-(piperazine-1-yl)propyl]piperidine-4-yl}carbamate (200 mg, 613 µmol) and tert-butyl N-{1-[3-(piperazine-1-yl)propyl]piperidine-4-yl}carbamate (99 mg, 613 µmol), DIPEA (423 mg, 3.06 mmol) solution reactants were stirred at 85°C for 2 hours. After the end of the reaction, the reactants were poured into water, extracted with ethyl acetate (2 times), dried with MgSO₄, and then concentrated under reduced pressure. The residues were purified by hexane/ethyl acetate (0-60%) column chromatography to obtain the title compound (210 mg). MS (ESI, m/z): [M+H]⁺ = 466.6

### Step 2) Synthesis of 5-(4-{4-[(2,6-dioxopiperidine-3-yl)amino]-2-fluorophenyl}piperazine-1-yl)pentanoic acid

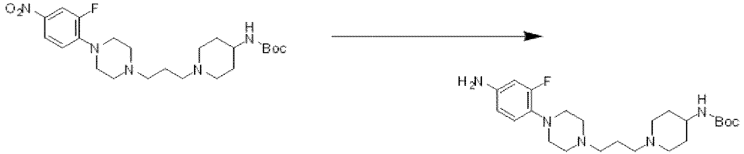

20 mL saturated NH4Cl was added to tert-butyl 4-(2-fluoro-4-nitrophenyl)piperazine-1-carboxylate (3 g, 9.22 mmol) and iron (210 mg, 451 µmol) solutions in THF (2 ml). The reactants were stirred at 100°C for 2 hours. After cooling to room temperature, the reactants were filtered, the filtrate was poured into water, and extracted with ethyl acetate. The organic layer was dried with MgSO₄, concentrated under reduced pressure, and then purified by column chromatography to obtain the title compound (190 mg) MS (ESI, m/z): [M+H]⁺ = [436.6].

### Step 3) Synthesis of tert-butyl N-{1-[3-(4-{4-[(2,6-dioxopiperidine-3-yl)amino]-2-fluorophenyl}piperazine-1-yl)propyl]piperidine-4-yl}carbamate

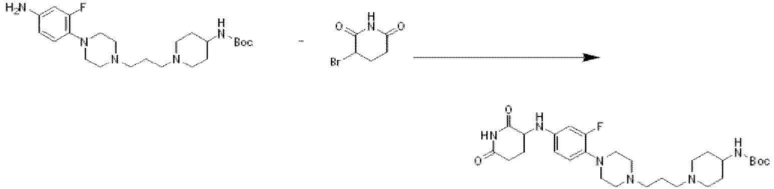

NaHCO3 (96 mg, 1.15 mmol) was added to tert-butyl N-(1-{3-[4-(4-amino-2-fluorophenyl)piperazine-1-yl]propyl}piperidine-4-yl)carbamate (100 mg, 230 µmol) and 3-bromoperidin-2,6-dione (88 mg, 459 µmol) in 10 ml of DMF at room temperature, and then the reactants were stirred at 80°C for 3 hours. The reactants were poured into water, extracted with ethyl acetate, dried with MgSO₄, and then concentrated under reduced pressure. The residue was purified with MeOH/DCM (0~10%) MPLC to obtain the title compound (140 mg) MS (ESI, m/z): [M+H]⁺ = [463.6].

### Step 4) Synthesis of 5-(4-{4-[(2,6-dioxopiperidin-3-yl)amino]-2-fluorophenyl}piperazine-1-yl)pentanoic acid

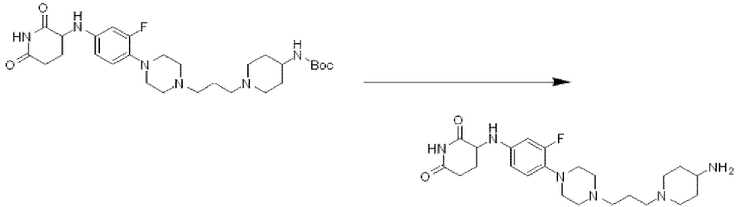

The reactants of tert-butyl N-{1-[3-(4-{4-[(2,6-dioxopiperidine-3-yl)amino]-2-fluorophenyl}piperazine-1-yl)propyl]piperidine-4-yl}carbamate (140 mg, 256 µmol) in 30% TFA in DCM were stirred at room temperature for 2 hours. After the reaction was completed, it was concentrated under reduced pressure. The residue was purified by DCM/MeOH (0~5%) amine column chromatography to obtain the title compound (110 mg). MS (ESI, m/z): [M+H]⁺ = [447.6].

### Step 5) Synthesis of 4-{[(4R)-5-(cyclohexylmethyl)-4-ethyl-1-methyl-4H,5H-[1,2,4]triazolo[4,3-f]pteridine-7-yl]amino}-N-{1-[3-(4-{4-[(2,6-dioxopiperidine-3-yl)amino]-2-fluorophenyl}piperazine-1-yl)propyl]piperidine-4-yl}-3-methoxybenzamide (ICT-0001572)

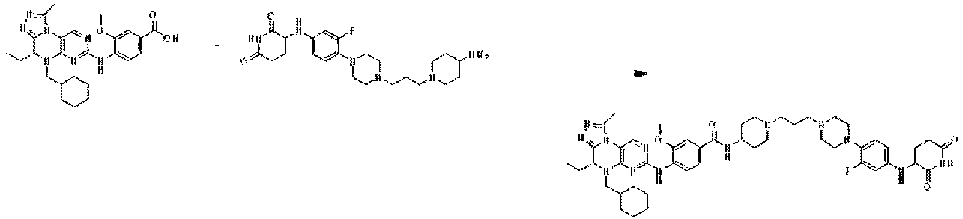

HATU (79.6 mgmg, 209 µmol) is added to 4-{[(4R)-5-(cyclohexylmethyl)-4-ethyl-1-methyl-4H,5H-[1,2,4]triazolo[4,3-f]phteridine-7-yl]amino}-3-methoxybenzoic acid (50 mg, 105 µmol) and 3-[(4-{4-[3-(4-aminopiperidine-1-yl)propyl]piperazine-1-yl}-3-fluorophenyl)amino]piperidine-2,6-dione (46.8 mg, 105 µmol) in 1 ml of DMF at room temperature, followed by ethylbis (propane-2-yl)amine (67.7 mg, 523 µmol). The reactants were stirred for 6 hours. The reactants were poured into water, extracted with ethyl acetate, dried with MgSO₄, and then concentrated under reduced pressure. The residue was purified with MeOH/DCM (0~10%) MPLC to obtain the title compound (45 mg). MS (ESI, m/z): [M+H]⁺ = [907.1]

### Example B-28: Synthesis of 3-{6-[4-(4-{(4R)-5-(cyclohexylmethyl)-4-ethyl-1-methyl-4H,5H-[1,2,4]triazolo[4,3-f]pteridine-7-yl]amino}-3-methoxybenzoyl)piperazine-1-yl]-4-methyl-1-oxo-1,2-dihydroptalazine-2-yl}piperidine-2,6-dione (ICT-0001576)

### Step 1) Synthesis of 3-{6-[4-(4-{[(4R)-5-(cyclohexylmethyl)-4-ethyl-1-methyl-4H,5H-[1,2,4]triazolo[4,3-f]pteridine-7-yl]amino}-3-methoxybenzoyl)piperazine-1-yl]-4-methyl-1-oxo-1,2-dihydroptalazine-2-yl}piperidine-2,6-dione (ICT-0001576)

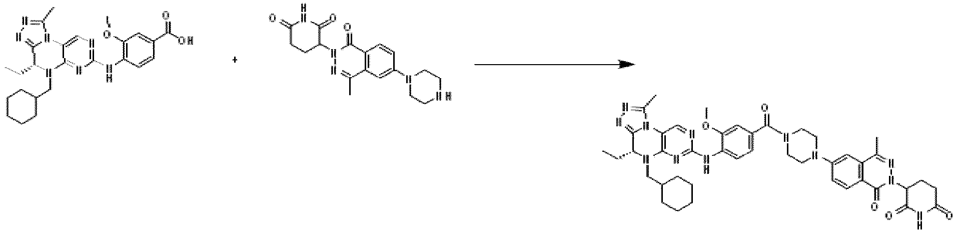

HATU was added to 3-[4-methyl-1-oxo-6-(piperazine-1-yl)-1,2-dihydroptalazin-2-yl]piperidine-2,6-dione (50 mg, 141 µmol) and 4-{[(4R)-5-(cyclohexylmethyl)-4-ethyl-1-methyl-4H,5H-[1,2,4]triazolo[4,3-f]pteridine-7-yl]amino}-3-methoxybenzoic acid (67.2 mg, 141 µmol) in 1 ml of DMF at room temperature. It was extracted with ethyl acetate, dried with MgSO₄, and then concentrated under reduced pressure. The residue was purified with MeOH/DCM (0~10%) MPLC to obtain the subject compound (38 mg). MS (ESI, m/z): [M+H]⁺ = [816]
1H NMR (400 MHz, DMSO-d6) δ = 11.03 (s, 1H), 10.30 (s, 1H), 8.83(t, J = 5.14 Hz, 1H), 8.22 (s, 1H), 7.62 (t, J = 8.01 Hz, 1H), 7.49 (d, J= 8.6 Hz, 2H), 7.43 (d, J= 8.6 Hz, 2H), 7.36 (s, 1H), 7.23 - 7.11 (m, 2H), 6.91(d, J = 7.95 Hz, 2H), 6.62 (d, J = 7.49 Hz, 1H), 5.76 - 5.66 (m, 1H), 4.60 (t, J = 7.09 Hz, 1H), 3.76 (m, 2H), 3.67 (m, 2H), 3.60 (m, 4H), 3.50 (m, 2H), 3.38 (m 2H), 2.93 - 2.82 (m, 1H), 2.63 - 2.53 (m, 5H), 2.48 - 2.38 (m, 4H), 2.10 (m, 1H), 1.63 (s, 3H).

### Example B-29: Synthesis of 3-{6-[4-(4-{(4R)-5-(cyclohexylmethyl)-4-ethyl-1-methyl-4H,5H-[1,2,4]triazolo[4,3-f]pteridine-7-yl]amino}-3-methoxybenzoyl)piperazine-1-yl]-1-oxo-1,2-dihydropetalazine-2-yl}piperidine-2,6-dione (ICT-0001577)

### Step 1) Synthesis of 3-{6-[4-(4-{(4R)-5-(cyclohexylmethyl)-4-ethyl-1-methyl-4H,5H-[1,2,4]triazolo[4,3-f]pteridine-7-yl]amino}-3-methoxybenzoyl)piperazine-1-yl]-1-oxo-1,2-dihydropetalazine-2-yl}piperidine-2,6-dione (ICT-0001577)

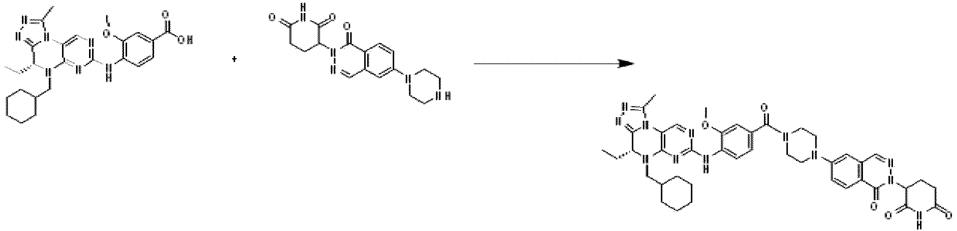

HATU was added to 3-[1-oxo-6-(piperazine-1-yl)-1,2-dihydroptalazin-2-yl]piperidine-2,6-dione (50 mg, 146 µmol) and 4-{[(4R)-5-(cyclohexylmethyl)-4-ethyl-1-methyl-4H,5H-[1,2,4]triazolo[4,3-f]pteridine-7-yl]amino}-3-methoxybenzoic acid (35 mg, 73.2 µmol) in 1 ml of DMF at room temperature. It was extracted with ethyl acetate, dried with MgSO₄, and then concentrated under reduced pressure. The residue was purified with MeOH/DCM (0-10%) MPLC to obtain the title compound (17 mg). MS (ESI, m/z): [M+H]⁺ = [801.9]
1H NMR (500 MHz, DMSO-d6) δ ppm 10.95 (s, 1 H) 8.34 (s, 1 H) 8.26 (d, J=8.24 Hz, 1 H) 8.19 (s, 1 H) 8.01 (d, J=9.00 Hz, 1 H) 7.88 (s, 1 H) 7.44 (dd, J=9.08, 2.06 Hz, 1 H) 7.18 - 7.25 (m, 1 H) 7.07 (s, 1 H) 6.97 (d, J=8.24 Hz, 1 H) 5.69 (dd, J=11.83, 5.11 Hz, 1 H) 4.83 (dd, J=8.16, 3.74 Hz, 1 H) 4.04 (dd, J=13.58, 6.10 Hz, 1 H) 3.86 (s, 3 H) 3.46 (br. s., 3 H) 2.80 - 2.95 (m, 2 H) 2.63 (s, 3 H) 2.46 - 2.58 (m, 2 H) 1.99 - 2.06 (m, 1 H) 1.69 - 1.80 (m, 2 H) 1.56 - 1.67 (m, 5 H) 1.54 (br. s., 1 H) 0.95 - 1.11 (m, 3 H) 0.83 - 0.94 (m, 1 H) 0.68 (t, J=7.32 Hz, 3 H)

### Example B-30 : Synthesis of 4-{[(4R)-5-(cyclohexylmethyl)-4-ethyl-1-methyl-4H,5H-[1,2,4]triazolo[4,3-f]pteridine-7-yl]amino}-N-{1-[3-(4-{4-[(2,6-dioxopiperidine-3-yl)amino]-2-fluorophenyl}piperazine-1-il)propanoyl]piperidine-4-yl}-3-methoxybenzamide (ICT-0001578)

### Step 1) Synthesis of Tert-butyl 3-(4-{4-[(2,6-dioxopiperidine-3-yl)amino]-2-fluorophenyl}piperazine-1-yl)propanoate

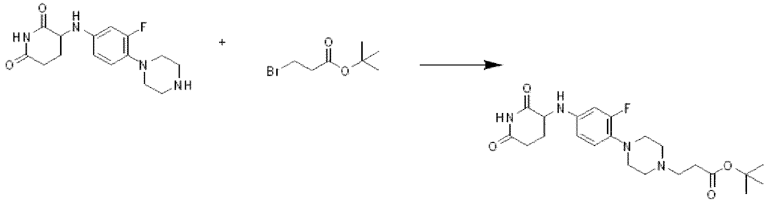

tert-butyl 3-bromopropanoate (164 mg, 783 µmol) and ethylbis (propane-2-yl)amine (422 mg, 3.3 mmol) were added to 3-{[3-fluoro-4-(piperazine-1-yl)phenyl]amino}piperidine-2,6-dione (0.2 g, 653 µmol) solution in DCM (5 mL). tert-butyl 3-bromopropanoate (164 mg, 783 µmol) and ethylbis (propane-2-yl)amine (422 mg, 3.3 mmol) were stirred overnight. After the reaction was completed, the reactant was poured into water, extracted with ethyl acetate (twice), dried with MgSO₄, and then concentrated under reduced pressure. The residue was purified by DCM/MeOH (0-5%) amine column chromatography to obtain the title compound (90 mg). MS (ESI, m/z): [M+H]⁺ = 435.5.

### Step 2) Synthesis of 3-(4-{4-[(2,6-dioxopiperidin-3-yl)amino]-2-fluorophenyl}piperazine-1-yl)propanoic acid

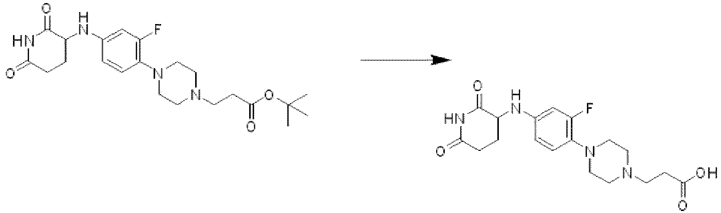

tert-butyl 3-(4-{4-[(2,6-dioxopiperidine-3-yl)amino]-2-fluorophenyl}piperazine-1-yl)piperazine-1-yl) in 30% TFA in DCM (100 mL) was stirred at room temperature for 30 min. After the end of the reaction, the reactants were poured into water, extracted with ethyl acetate (twice), dried with MgSO4, and then concentrated under reduced pressure. The residues were purified by DCM/MeOH (0~5%) amine column chromatography to obtain the title compound (70 mg). MS (ESI, m/z): [M+H]⁺ = 379.4.

### Step 3) Synthesis of 4-{[(4R)-5-(cyclohexylmethyl)-4-ethyl-1-methyl-4H,5H-[1,2,4]triazolo[4,3-f]pteridine-7-yl]amino}-N-{1-[3-(4-{4-[(2,6-dioxopiperidin-3-yl)amino]-2-fluorophenyl}piperazine-1-yl)propanoyl]piperidine-4-yl}-3-methoxybenzamide (ICT-0001578)

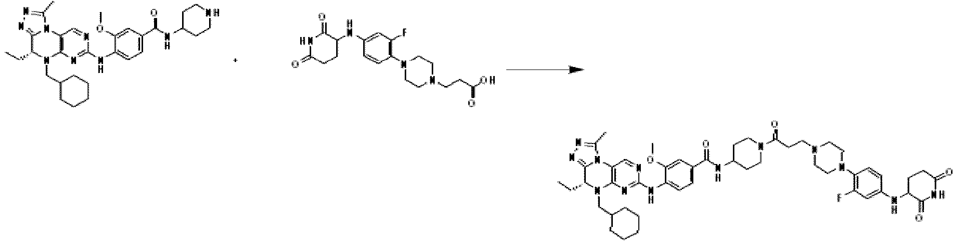

4-{[(4R)-5-(cyclohexylmethyl)-4-ethyl-1-methyl-4H, in DMF (2 mL) 5H-[1,2,4]triazolo[4,3-f]phteridine-7-yl]amino}-3-methoxy-N- (piperidine-4-yl)benzamide (104 mg, 185 µmol) in a solution of 3-(4-{4-[(2,6-dioxopiperidine-3-yl)amino]-2-fluorophenyl}piperazine-1-yl)propanoic acid (70 mg, 185 µmol), [(dimethylamino)({3H-[1,2,3]triazolo[4,5-b]pyridine-3-yloxy})methylidene]dimethylazanium (141 mg, 370 µmol) and ethyl bis(propane-2-yl)amine (239 mg, 1.85 mmol). The reactants were stirred at room temperature for 2 hours. After the reaction was completed, the reactants were poured into water, extracted with DCM (twice), dried with MgSO₄, and then concentrated under reduced pressure. The residues were purified by DCM/MeOH (0-5%) column chromatography to obtain the title compound (141 mg). MS (ESI, m/z): [M+H]⁺ = 921.1
1H NMR (500 MHz, DMSO-d6) δ ppm 10.72 (s, 1 H) 8.34 (s, 1 H) 8.27 (d, J=8.39 Hz, 1 H) 7.86 (s, 1 H) 7.40 - 7.44 (m, 2 H) 6.83 (t, J=9.31 Hz, 1 H) 6.52 (dd, J=14.88, 1.75 Hz, 1 H) 6.40 - 6.47 (m, 1 H) 4.83 (dd, J=8.24, 3.66 Hz, 1 H) 4.26 (br. s., 1 H) 4.12 (dd, J=13.58, 6.26 Hz, 2 H) 4.04 (dd, J=13.73, 6.10 Hz, 1 H) 3.94 (s, 6 H) 3.88 (s, 3 H) 2.96 - 3.04 (m, 4 H) 2.92 (dd, J=13.50, 8.16 Hz, 1 H) 2.85 - 2.90 (m, 4 H) 2.69 - 2.73 (m, 7 H) 2.60 - 2.67 (m, 4 H) 2.49 - 2.54 (m, 1 H) 2.01 - 2.03 (m, 1 H) 1.56 - 1.63 (m, 4 H) 1.21 - 1.26 (m, 5 H) 1.16 - 1.21 (m, 4 H) 0.96 (s, 1 H) 0.67 (t, J=7.32 Hz, 3 H)

### Example B-31: Synthesis of 3-(8-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)4-methyl-1-oxopthalazine-2(1H)-yl)piperidine-2,6-dione (ICT-0001583)

### Step 1) Synthesis of 3-(4-methyl-1-oxo-8-((2-(piperazine-1-yl)ethyl)amino)phthalazine-2(1H)-yl)piperidine-2,6-dione

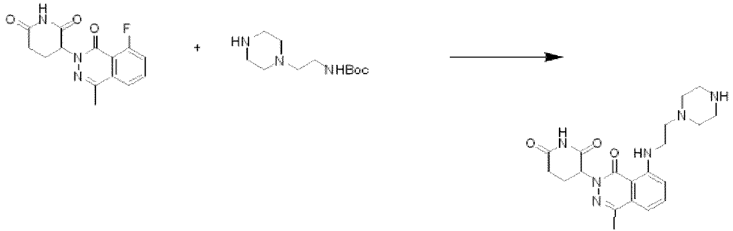

3-(8-fluoro-4-methyl-1-oxo-1,2-dihydroptalazin-2-yl)piperidine-2,6-dione (500 mg, 1.73 mmol), tert-butyl 4-(2-aminoethyl)piperazine-1-carboxylate (595 mg, 2.57 mmol), and DIPEA (447 mg, 3.46 mmol) solutions in NMP (2 mL) were stirred at 120°C for 5 hours. The reaction mixture was purified by column chromatography to obtain the Boc-protected compound, dissolved in 1 ml DCM, followed by 0.2 ml TFA, and stirred at room temperature for 2 hours. The reactants were concentrated under reduced pressure and vacuum-dried. The residue was dissolved in DCM, passed through an NH-DM 1020 Chromatorex pad to obtain the heading compound, which was used in the next step without further purification. MS (ESI, m/z): [M+H]⁺ = [399.5].

### Step 2) Synthesis of 3-(8-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)4-methyl-1-oxopthalazine-2(1H)-yl)piperidine-2,6-dione (ICT-0001583)

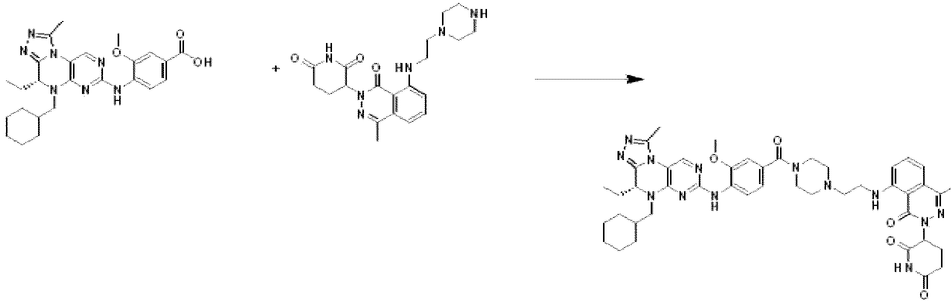

HATU (66.9 mg, 176 umol) is added to 4-{[(4R)-5-(cyclohexylmethyl)-4-ethyl-1-methyl-4H,5H-[1,2,4]triazolo[4,3-f]phteridine-7-yl]amino}-3-methoxybenzoic acid (70 mg, 147 umol) and 3-(4-methyl-1-oxo-8-{[2-(piperazine-1-yl)ethyl]amino}-1,2-dihydroptalazin-2-yl)piperidine-2,6-dion hydrate (61 mg, 147 umol) in 1 ml of DMF at room temperature, followed by ethyl bis(propane-2-yl)amine (37.9 mg, 293 umol). The reactants were stirred for 6 hours. The reactants were poured into water, extracted with ethyl acetate, dried with MgSO₄, and then concentrated under reduced pressure. The residue was purified with MeOH/DCM (0~10%) MPLC to obtain the title compound (69 mg). MS (ESI, m/z): [M+H]⁺ = [859.1].
1H NMR (500 MHz, DMSO-d6) δ ppm 10.94 (s, 1 H) 8.99 - 9.04 (m, 1 H) 8.31 - 8.35 (m, 2 H) 8.19 - 8.26 (m, 2 H) 7.86 - 7.87 (m, 1 H) 7.60 (t, J=8.09 Hz, 1 H) 6.98 - 7.05 (m, 2 H) 6.83 - 6.94 (m, 4 H) 4.82 (dt, J=7.90, 3.76 Hz, 2 H) 3.98 - 4.08 (m, 2 H) 3.81 - 3.88 (m, 5 H) 3.39 (br. s., 1 H) 2.78 - 2.94 (m, 3 H) 2.58 (d, J=16.63 Hz, 2 H) 2.47 - 2.55 (m, 1 H) 2.28 - 2.39 (m, 4 H) 1.98 - 2.03 (m, 1 H) 1.73 (dd, J=6.94, 2.82 Hz, 1 H) 1.49 - 1.67 (m, 9 H) 1.13 - 1.24 (m, 2 H) 1.00 - 1.09 (m, 3 H) 0.67 (t, J=7.32 Hz, 5 H)

### Example B-32: Synthesis of 3-(8-(4-(4-(((R)-5-(cyclohexylmethyl)-4-ethyl-1-methyl-4,5-dihydro-[1,2,4]triazolo[4,3-f]pteridine-7-yl)amino)-3-methoxybenzoyl)piperazine-1-yl)-4-methyl-1-oxopthalazine-2(1H)-yl)piperidine-2,6-dione (ICT-0001584)

### Step 1) Synthesis of 3-(4-methyl-1-oxo-8-(piperazine-1-yl)phthalazine-2(1H)-yl)piperidine-2,6-dione

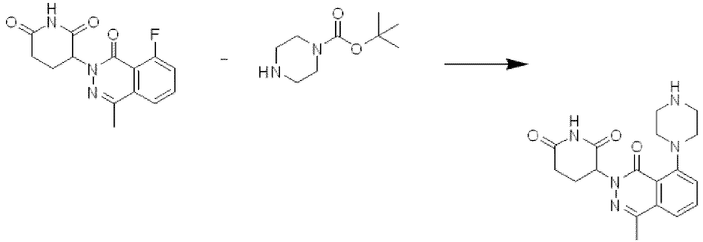

3-(8-fluoro-4-methyl-1-oxopthalazine-2(1H)-yl)piperidine-2,6-dione (500 mg, 1.73 mmol), tert-butylpiperazine-1-carboxylate (483 mg, 2.59 mmol), and DIPEA (670 mg, 5.19 mmol) solutions in NMP (2 mL) were stirred at 120°C for 5 hours. The reaction mixture was purified by column chromatography to obtain the Boc-protected compound, dissolved in 1 ml DCM, followed by 0.2 ml TFA, and stirred at room temperature for 2 hours. The reactants were concentrated under reduced pressure and vacuum-dried. The residue was dissolved in DCM, passed through an NH-DM 1020 Chromatorex pad to obtain the heading compound, which was used in the next step without further purification. (420 mg), MS (ESI, m/z): [M+H]⁺ = [356.5].

### Step 2) Synthesis of 3-(8-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)4-methyl-1-oxopthalazine-2(1H)-yl)piperidine-2,6-dione (ICT-0001584)

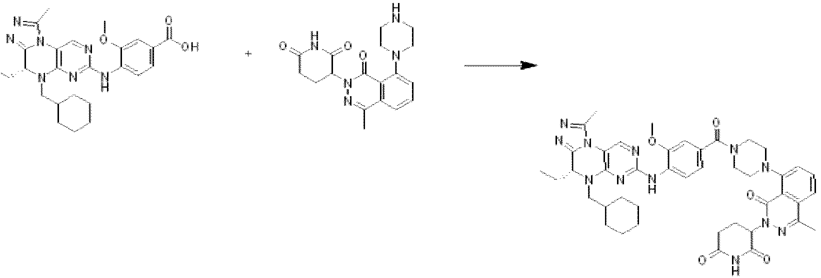

HATU (83.6 mg, 220 umol) was added to 4-{[(4R)-5-(cyclohexylmethyl)-4-ethyl-1-methyl-4H,5H-[1,2,4]triazolo[4,3-f]pteridine-7-yl]amino}-3-methoxybenzoic acid (70 mg, 147 umol) and 3-[4-methyl-1-oxo-8-(piperazine-1-yl)-1,2-dihydroptalazine-2-yl]piperidine-2,6-dione (52.1 mmg, 147 umol) in 1 ml of DMF at room temperature, followed by ethylbis (propane-2-yl)amine (37.9 mg, 293 mmol). The reactant was stirred for 6 hours. The reactant was poured into the water. It was extracted with ethyl acetate, dried with MgSO₄, and then concentrated under reduced pressure. The residue was purified with MeOH/DCM (0~10%) MPLC to obtain the title compound (86 mg). [M+1]⁺ = [816.5]
1H NMR (500 MHz, DMSO-d6) δ ppm 10.98 (s, 1 H) 8.40 (s, 1 H) 8.30 (dd, J=8.16, 6.03 Hz, 1 H) 7.94 (d, J=2.29 Hz, 1 H) 7.84 (t, J=8.01 Hz, 1 H) 7.49 (d, J=7.93 Hz, 1 H) 7.39 (d, J=8.24 Hz, 1 H) 7.13 (d, J=1.53 Hz, 1 H) 7.02 (d, J=8.39 Hz, 1 H) 4.89 (dd, J=8.09, 3.66 Hz, 1 H) 4.07 - 4.15 (m, 1 H) 3.88 - 3.97 (m, 4 H) 2.85 - 3.01 (m, 2 H) 2.70 (s, 4 H) 2.54 - 2.67 (m, 2 H) 2.49 (s, 3 H) 2.04 - 2.12 (m, 1 H) 1.81 (d, J=6.71 Hz, 1 H) 1.62 - 1.75 (m, 5 H) 1.59 (br. s., 1 H) 1.02 - 1.19 (m, 4 H) 0.86 - 1.02 (m, 2 H) 0.75 (t, J=7.32 Hz, 4 H)

### Example B-33: Synthesis of 4-(((R)-5-(cyclohexylmethyl)-4-ethyl-1-methyl-4,5-dihydro-[1,2,4]triazolo[4,3-f]pteridine-7-yl)amino)-N-(1-(1-(3-(2,6-dioxopiperidin-3-yl)-1-methyl-4-oxo-3,4-dihydrophthalazine-6-yl)piperidine-4-carbonyl)piperidine-4-carbonyl)piperidine-4-yl)-3-methoxybenzamide (ICT-0001592)

### Step 1) Synthesis of 1-(3-(2,6-dioxopiperidine-3-yl)-1-methyl-4-oxo-3,4-dihydroptalazine-6-yl)piperidine-4-carboxylic acid

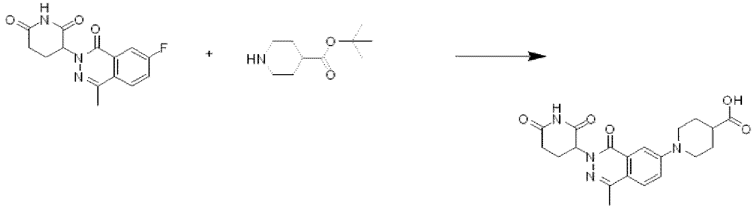

3-(7-fluoro-4-methyl-1-oxo-1,2-dihydroptalazine-2-yl)piperidine-2,6-dione (1.0 g, 3.46 mmol), tert-butylpiperidine-4-carboxylate (769 mg, 4.15 mmol), and DIPEA (894 mg, 6.91 mmol) solutions in NMP (2 mL) were stirred at 120°C for 5 hours. The reaction mixture was purified by column chromatography to obtain the Boc-protected compound, which was dissolved in 1 ml DCM, followed by 0.2 ml TFA, and stirred at room temperature for 2 hours. The reactants were concentrated under reduced pressure and vacuum-dried. The residuals were dissolved in DCM, passed through an NH-DM 1020 Chromatorex pad to obtain the heading compound, which was used in the next step without further purification (1.10 g). MS (ESI, m/z): [M+H]⁺ = [399.5].

### Step 2) Synthesis of 4-(((R)-5-(cyclohexylmethyl)-4-ethyl-1-methyl-4,5-dihydro-[1,2,4]triazolo[4,3-f]pteridine-7-yl)amino)N-(1-(1-(3-(2,6-dioxopiperidin-3-yl)-1-methyl-4-oxo-3,4-dihydroptalazine-6-yl)piperidine-4-carbonyl)piperidine-4-yl)piperidine-4-yl)-3-methoxybenzamide (ICT-0001592)

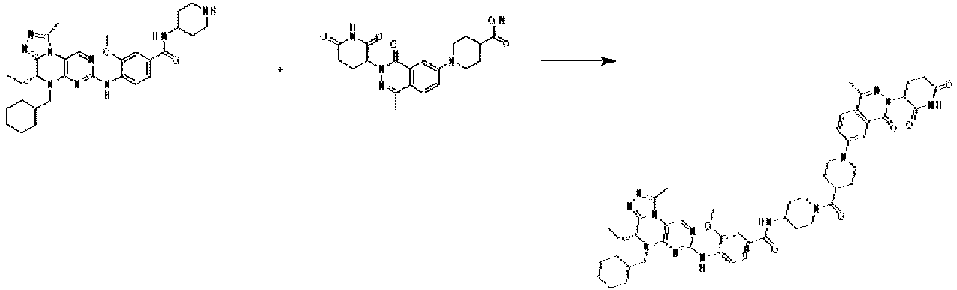

4-{[(4R)-5-(cyclohexylmethyl)-4-ethyl-1-methyl-4H, in 1 ml of DMF at room temperature HATU (66.9 mg, 176 umol) was added to a solution of 5H-[1,2,4]triazolo[4,3-f]pteridine-7-yl]amino}-3-methoxy-N- (piperidine-4-yl)benzamide (70 mg, 125 umol) and 1-[3-(2,6-dioxopiperidine-3-yl)-1-methyl-4-oxo-3,4-dihydroptalazin-6-yl]piperidine-4-carboxylic acid (58.4 mg, 147 umol) solution, followed by ethylbis (propane-2-yl)amine (28.4 mg, 220 umol). The reactants were stirred for 6 hours. It was extracted with ethyl acetate, dried with MgSO₄, and then concentrated under reduced pressure. The residual acid was purified with MeOH/DCM (0~10%) MPLC to obtain the title compound (78 mg). MS (ESI, m/z): [M+H]⁺ = [941.1]
1H NMR (500 MHz, DMSO-d6) δ ppm 10.90 (s, 1 H) 8.32 (s, 1 H) 8.19 (d, J=8.09 Hz, 1 H) 7.98 (d, J=9.00 Hz, 1 H) 7.91 (br. s., 1 H) 7.79 (d, J=7.48 Hz, 1 H) 7.39 - 7.47 (m, 1 H) 7.00 (s, 1 H) 6.97 (s, 1 H) 6.86 (d, J=8.24 Hz, 1 H) 5.61 (d, J=6.56 Hz, 1 H) 4.82 (dd, J=8.16, 3.59 Hz, 1 H) 3.98 - 4.07 (m, 3 H) 3.84 (s, 3 H) 3.75 (br. s., 1 H) 2.78 - 2.95 (m, 4 H) 2.62 (s, 3 H) 2.45 - 2.60 (m, 3 H) 2.27 - 2.42 (m, 4 H) 1.95 - 2.04 (m, 1 H) 1.73 (s, 2 H) 1.71 (s, 3 H) 1.50 - 1.68 (m, 8 H) 1.05 (d, J=7.32 Hz, 2 H) 0.99 (d, J=10.83 Hz, 1 H) 0.87 (d, J=11.60 Hz, 1 H) 0.67 (t, J=7.32 Hz, 3 H) 0.00 (s, 1 H)

### Example B-34: Synthesis of 3-(6-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)4-methyl-1-oxopthalazine-2(1H)-yl)piperidine-2,6-dione (ICT-0001599)

### Step 1) Synthesis of 3-(4-methyl-1-oxo-6-((2-(piperazine-1-yl)ethyl)amino)phthalazine-2(1H)-yl)piperidine-2,6-dione

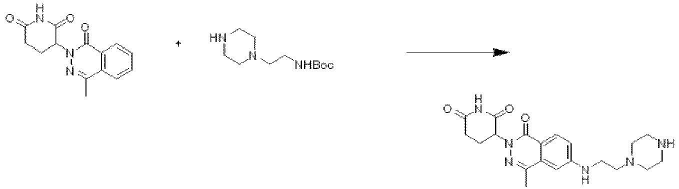

3-(6-fluoro-4-methyl-1-oxopthalazine-2(1H)-yl)piperidine-2,6-dione (500 mg, 1.73 mmol), tert-butyl 4-(2-aminoethyl)piperazine-1-carboxylate (595 mg, 2.57 mmol), and DIPEA (447 mg, 3.46 mmol) solutions in NMP (2 mL) were stirred at 120°C for 5 hours. The reaction mixture was purified by column chromatography to obtain the Boc-protected compound, dissolved in 1 ml DCM, followed by 0.2 ml TFA, and stirred at room temperature for 2 hours. The reactants were concentrated under reduced pressure and vacuum-dried. The residue was dissolved in DCM, passed through an NH-DM 1020 Chromatorex pad to obtain the heading compound, which was used in the next step without further purification. MS (ESI, m/z): [M+H]⁺ = [399.5].

### Step 2) Synthesis of 3-(6-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)4-methyl-1-oxopthalazine-2(1H)-yl)piperidine-2,6-dione (ICT-0001599)

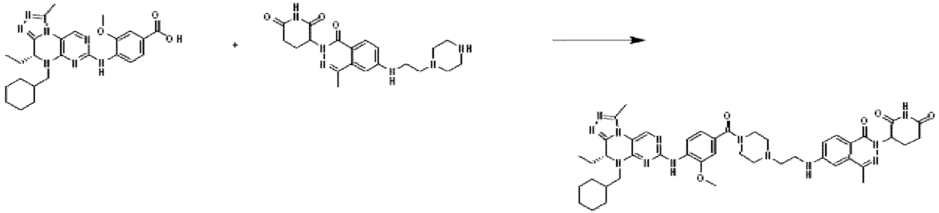

HATU (66.9 mg, 176 umol) is added to the solution of (R)-4-((5-(cyclohexylmethyl)-4-ethyl-1-methyl-4,5-dihydro-[1,2,4]triazolo[4,3-f]phteridine-7-yl)amino)-3-methoxybenzoic acid (70 mg, 147 umol) and 3-(4-methyl-1-oxo-6-(((2-(piperazine-1-yl)ethyl)amino)phthalazine-2(1H)-yl)piperidine-2,6-dione (61 mg, 147 umol) in 1 ml of DMF, followed by ethylbis (propane-2-yl)amine (37.9 mg, 293 umol) at room temperature. The reactants were stirred for 6 hours. The reactants were poured into water, extracted with ethyl acetate, dried with MgSO₄, and then concentrated under reduced pressure. The residue was purified with MeOH/DCM (0~10%) MPLC to obtain the title compound (69 mg). MS (ESI, m/z): [M+H]⁺ = [859.1]
1H NMR (500 MHz, DMSO-d6) δ ppm 10.84 - 10.93 (m, 1 H) 8.32 (s, 1 H) .22 (d, J=8.24 Hz, 1 H) 7.82 - 7.94 (m, 2 H) 7.05 (dd, J=8.85, 1.83 Hz, 1 H) 7.00 (s, 1 H) 6.90 (d, J=7.93 Hz, 1 H) 6.60 - 6.78 (m, 2 H) 5.59 (d, J=6.41 Hz, 1 H) 4.82 (dd, J=8.16, 3.74 Hz, 1 H) 4.02 (dd, J=13.50, 6.03 Hz, 1 H) 3.79 - 3.89 (m, 3 H) 2.77 - 2.99 (m, 2 H) 2.58 - 2.68 (m, 10 H) 2.35 - 2.56 (m, 10 H) 1.93 - 2.05 (m, 1 H) 1.68 - 1.81 (m, 2 H) 1.47 - 1.68 (m, 6 H) 0.93 - 1.14 (m, 3 H) 0.80 - 0.93 (m, 1 H) 0.67 (t, J=7.40 Hz, 3 H)

### Example B-35 : Synthesis of 3-(7-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)4-methyl-1-oxopthalazine-2(1H)-yl)piperidine-2,6-dione (ICT-0001600)

### Step 1) Synthesis of 3-(4-methyl-1-oxo-7-((2-(piperazine-1-yl)ethyl)amino)phthalazine-2(1H)-yl)piperidine-2,6-dione

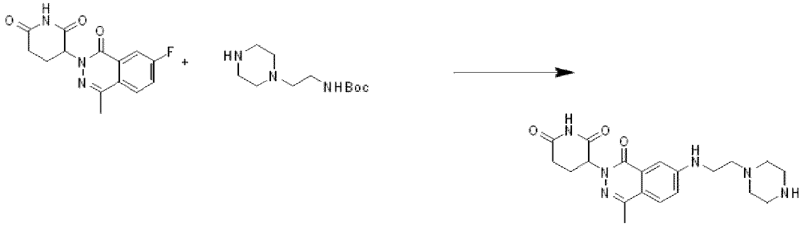

3-(7-fluoro-4-methyl-1-oxopthalazine-2(1H)-yl)piperidine-2,6-dione (500 mg, 1.73 mmol), tert-butyl 4-(2-aminoethyl)piperazine-1-carboxylate (595 mg, 2.57 mmol), and DIPEA (447 mg, 3.46 mmol) solutions in NMP (2 mL) were stirred at 120°C for 5 hours. The reaction mixture was purified by column chromatography to obtain the Boc-protected compound, dissolved in 1 ml DCM, followed by 0.2 ml TFA, and stirred at room temperature for 2 hours. The reactants were concentrated under reduced pressure and vacuum-dried. The residuals were dissolved in DCM, and the compound titled NH-DM 1020 Chromatorex pad was obtained by passing it through the body, which was used in the next step without further purification. MS (ESI, m/z): [M+H]⁺ = [399.5].

### Step 2) Synthesis of 3-(7-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)4-methyl-1-oxopthalazine-2(1H)-yl)piperidine-2,6-dione (ICT-0001600)

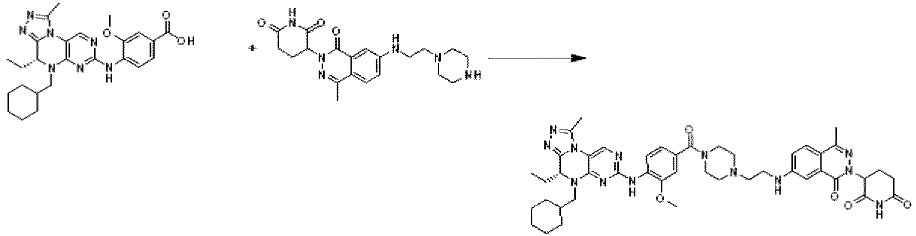

HATU (66.9 mg, 176 umol) is added to (R)-4-((5-(cyclohexylmethyl)-4-ethyl-1-methyl-4,5-dihydro-[1,2,4]triazolo[4,3-f]pteridine-7-yl)amino)-3-methoxybenzoic acid (70 mg, 147 umol) and 3-(4-methyl-1-oxo-7-(((2-(piperazine-1-yl)ethyl)amino)phthalazine-2(1H)-yl)piperidine-2,6-dione (61 mg, 147 umol) in 1 ml of DMF at room temperature, followed by ethylbis (propane-2-yl)amine (37.9 mg, 293 umol). The reactants were stirred for 6 hours. The reactants were poured into water, extracted with ethyl acetate, dried with MgSO₄, and then concentrated under reduced pressure. The residue was purified with MeOH/DCM (0~10%) MPLC to obtain the title compound (69 mg). MS (ESI, m/z): [M+H]⁺ = [859.1]
1H NMR (500 MHz, DMSO-d6) δ ppm 10.90 (s, 1 H) 8.32 (s, 1 H) 8.21 (d, J=8.09 Hz, 1 H) 7.86 (s, 1 H) 7.60 (d, J=8.54 Hz, 1 H) 7.10 - 7.22 (m, 2 H) 7.00 (s, 1 H) 6.89 (d, J=8.24 Hz, 1 H) 6.69 (br. s., 1 H) 4.81 (dd, J=8.01, 3.59 Hz, 1 H) 4.02 (dd, J=13.50, 5.87 Hz, 1 H) 3.84 (s, 3 H) 2.78 - 2.93 (m, 2 H) 2.47 - 2.67 (m, 10 H) 2.35 - 2.45 (s, 10 H) 1.92 - 2.05 (m, 1 H) 1.67 - 1.78 (m, 2 H) 1.47 - 1.67 (m, 6 H) 0.93 - 1.12 (m, 4 H) 0.80 - 0.93 (m, 1 H) 0.67 (t, J=7.25 Hz, 3 H)

### Example B-36: Synthesis of 3-(8-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-il)ethyl)amino)1-oxopthalazine-2(1H)-yl)piperidine-2,6-dione (ICT-0001601)

### Step 1) Synthesis of 3-(1-oxo-8-((2-(piperazine-1-yl)ethyl)amino)phthalazine-2(1H)-yl)piperidine-2,6-dione

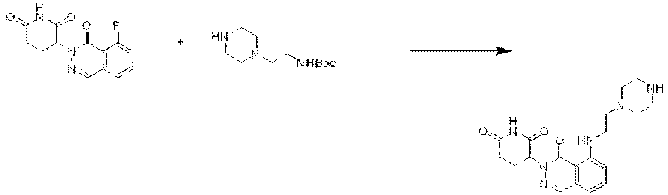

3-(8-fluoro-1-oxopthalazine-2(1H)-yl)piperidine-2,6-dione (500 mg, 1.73 mmol), tert-butyl 4-(2-aminoethyl)piperazine-1-carboxylate (595 mg, 2.57 mmol), and DIPEA (447 mg, 3.46 mmol) solutions in NMP (2 mL) were stirred at 120°C for 5 hours. The reaction mixture was purified by column chromatography to obtain the Boc-protected compound, dissolved in 1 ml DCM, followed by 0.2 ml TFA, and stirred at room temperature for 2 hours. The reactants were concentrated under reduced pressure and vacuum-dried. The residue was dissolved in DCM, passed through an NH-DM 1020 Chromatorex pad to obtain the heading compound, which was used in the next step without further purification. MS (ESI, m/z): [M+H]⁺ = [385.5].

### Step 2) Synthesis of 3-(8-((2-(4-(4-(((R)-5-(cyclohexylmethyl)-4-ethyl-1-methyl-4,5-dihydro-[1,2,4]triazolo[4,3-f]phteridine-7-yl)amino)3-methoxybenzoyl)piperazine-1-yl)ethyl)amino)1-oxopthalazine-2(1H)-yl)piperidine-2,6-dione (ICT-0001601)

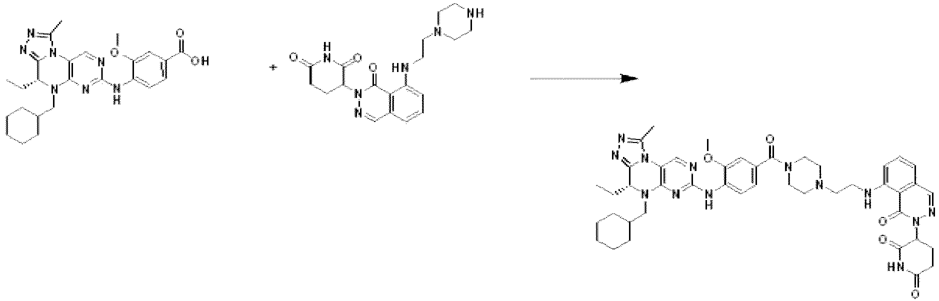

HATU (66.9 mg, 176 umol) is added to (R)-4-((5-(cyclohexylmethyl)-4-ethyl-1-methyl-4,5-dihydro-[1,2,4]triazolo[4,3-f]pteridine-7-yl)amino)-3-methoxybenzoic acid (70 mg, 147 umol) and 3-(1-oxo-8-(((2-(piperazine-1-yl)ethyl)amino)phthalazine-2(1H)-yl)piperidine-2,6-dione (61 mg, 147 umol) in 1 ml of DMF at room temperature, followed by ethylbis (propane-2-yl)amine (37.9 mg, 293 umol). The reactants were stirred for 6 hours. The reactants were poured into water, extracted with ethyl acetate, dried with MgSO₄, and then concentrated under reduced pressure. The residue was purified with MeOH/DCM (0~10%) MPLC to obtain the title compound (69 mg). MS (ESI, m/z): [M+H]⁺ = [845.1]
1H NMR (500 MHz, DMSO-d6) δ ppm 10.97 (s, 1 H) 8.75 - 8.86 (m, 1 H) 8.32 (s, 1 H) 8.21 (d, J=8.24 Hz, 1 H) 8.15 (s, 1 H) 7.86 (s, 1 H) 7.58 (t, J=7.93 Hz, 1 H) 7.00 (s, 1 H) 6.77 - 6.93 (m, 3 H) 4.81 (dd, J=8.09, 3.66 Hz, 1 H) 4.02 (dd, J=13.66, 6.33 Hz, 1 H) 3.84 (s, 3 H) 2.76 - 2.96 (m, 2 H) 2.50 - 2.68 (m, 10 H) 2.34- 2.47 (m, 10 H) 1.97 - 2.07 (m, 1 H) 1.67 - 1.80 (m, 2 H) 1.45 - 1.67 (m, 6 H) 0.93 - 1.10 (m, 2 H) 0.86 (d, J=11.75 Hz, 1 H) 0.67 (t, J=7.32 Hz, 3 H)

### Example B-37: Synthesis of 3-(5-((2-(4-(4-((((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)1-oxopthalazine-2(1H)-yl)piperidine-2,6-dione (ICT-0001602)

### Step 1) Synthesis of 3-(1-oxo-5-((2-(piperazine-1-yl)ethyl)amino)phthalazine-2(1H)-yl)piperidine-2,6-dione

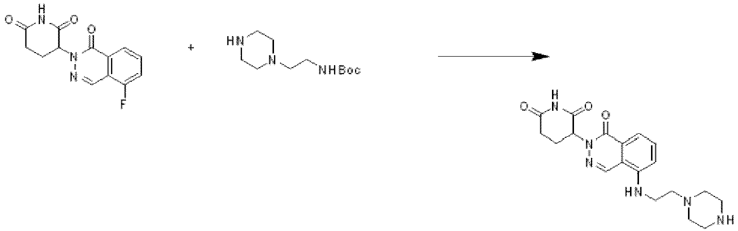

3-(5-fluoro-1-oxopthalazine-2(1H)-yl)piperidine-2,6-dione (500 mg, 1.73 mmol), tert-butyl 4-(2-aminoethyl)piperazine-1-carboxylate (595 mg, 2.57 mmol), and DIPEA (447 mg, 3.46 mmol) solutions in NMP (2 mL) were stirred at 120°C for 5 hours. The reaction mixture was purified by column chromatography to obtain the Boc-protected compound, which was dissolved in 1 ml DCM, followed by 0.2 ml TFA, and stirred at room temperature for 2 hours. The reactants were concentrated under reduced pressure and vacuum-dried. The residue was dissolved in DCM, passed through an NH-DM 1020 Chromatorex pad to obtain the heading compound, which was used in the next step without further purification. MS (ESI, m/z): [M+H]⁺ = [385.5].

### Step 2) Synthesis of 3-(5-((2-(4-(4-((((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)1-oxopthalazine-2(1H)-yl)piperidine-2,6-dione (ICT-0001602)

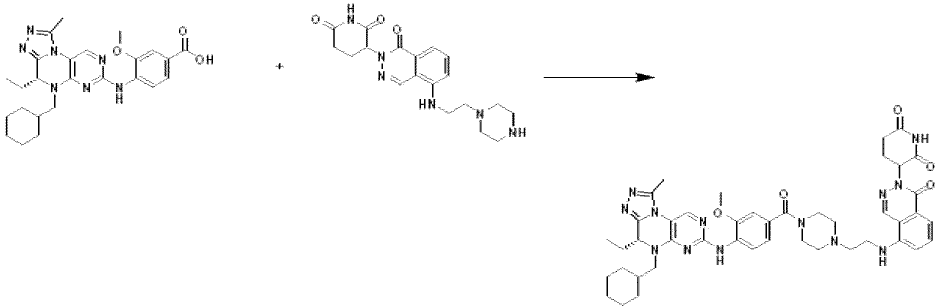

HATU (66.9 mg, 176 umol) is added to (R)-4-((5-(cyclohexylmethyl)-4-ethyl-1-methyl-4,5-dihydro-[1,2,4]triazolo[4,3-f]phteridine-7-yl)amino)-3-methoxybenzoic acid (70 mg, 147 umol) and 3-(1-oxo-5-((2-(piperazine-1-yl)ethyl)amino)phthalazine-2(1H)-yl)piperidine-2,6-dione (61 mg, 147 umol) in 1 ml of DMF at room temperature, followed by ethyl bis(propane-2-yl)amine (37.9 mg, 293 umol). The reactants were stirred for 6 hours. The reactants were poured into water, extracted with ethyl acetate, dried with MgSO₄, and then concentrated under reduced pressure. The residue was purified with MeOH/DCM (0∼10%) MPLC to obtain the title compound (69 mg). MS (ESI, m/z): [M+H]⁺= [845.1]
1H NMR (500 MHz, DMSO-d6) δ ppm 10.96 (s, 1 H) 8.56 (s, 1 H) 8.32 (s, 2 H) 8.21 (d, J=8.24 Hz, 2 H) 7.87 (s, 1 H) 7.54 (t, J=7.93 Hz, 1 H) 7.34 (d, J=7.78 Hz, 1 H) 6.94 - 7.02 (m, 3 H) 6.89 (d, J=8.09 Hz, 2 H) 6.63 (br. s., 1 H) 5.70 (dd, J=11.37, 4.96 Hz, 1 H) 4.82 (dd, J=8.01, 3.74 Hz, 2 H) 4.02 (dd, J=13.50, 6.03 Hz, 2 H) 3.84 (s, 5 H) 2.80 - 2.94 (m, 3 H) 2.00 - 2.06 (m, 2 H) 1.70 - 1.78 (m, 3 H) 1.51 - 1.65 (m, 9 H) 0.95 - 1.08 (m, 5 H) 0.86 (d, J=11.29 Hz, 1 H) 0.67 (t, J=7.32 Hz, 5 H)

### Example B-38: Synthesis of 3-(6-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)1-oxopthalazine-2(1H)-yl)piperidine-2,6-dione (ICT-0001603)

### Step 1) Synthesis of 3-(1-oxo-6-((2-(piperazine-1-yl)ethyl)amino)phthalazine-2(1H)-yl)piperidine-2,6-dione

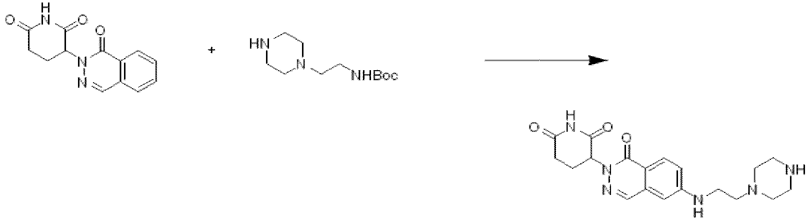

3-(1-oxofthalazine-2(1H)-yl)piperidine-2,6-dione (500 mg, 1.73 mmol), tert-butyl 4-(2-aminoethyl)piperazine-1-carboxylate (595 mg, 2.57 mmol), and DIPEA (447 mg, 3.46 mmol) solutions in NMP (2 mL) were stirred at 120°C for 5 hours. The reaction mixture was purified by column chromatography to obtain the Boc-protected compound, dissolved in 1 ml DCM, followed by 0.2 ml TFA, and stirred at room temperature for 2 hours. The reactants were concentrated under reduced pressure and vacuum-dried. The residues were dissolved in DCM, passed through an NH-DM 1020 chromatorex pad to obtain the heading compound, which was used in the next step without further purification. MS (ESI, m/z): [M+H]⁺ = [385.5].

### Step 2) Synthesis of 3-(6-((2-(4-(4-((((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)1-oxopthalazine-2(1H)-yl)piperidine-2,6-dione (ICT-0001603)

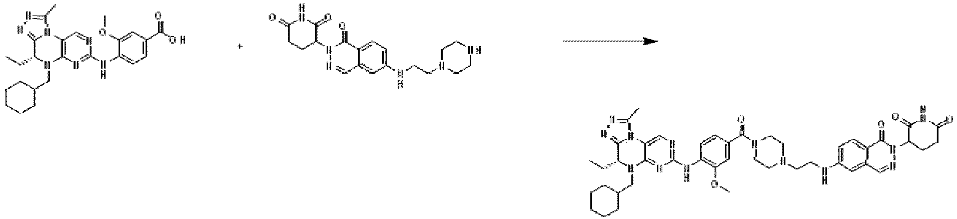

HATU (66.9 mg, 176 umol) is added to (R)-4-((5-(cyclohexylmethyl)-4-ethyl-1-methyl-4,5-dihydro-[1,2,4]triazolo[4,3-f]phteridine-7-yl)amino)-3-methoxybenzoic acid (70 mg, 147 umol) and 3-(1-oxo-6-(((2-(piperazine-1-yl)ethyl)amino)phthalazine-2(1H)-yl)piperidine-2,6-dione (61 mg, 147 umol) in 1 ml of DMF at room temperature, followed by ethyl bis(propane-2-yl)amine (37.9 mg, 293 umol). The reactants were stirred for 6 hours. The reactants were poured into water, extracted with ethyl acetate, dried with MgSO₄, and then concentrated under reduced pressure. The residue was purified with MeOH/DCM (0∼10%) MPLC to obtain the title compound (69 mg). MS (ESI, m/z): [M+H]⁺ = [845.1]
1H NMR (500 MHz, DMSO-d6) δ ppm 10.92 (s, 1 H) 8.32 (s, 2 H) 8.21 (d, J=8.24 Hz, 1 H) 8.11 (s, 2 H) 7.81 - 7.89 (m, 3 H) 7.05 (dd, J=8.85, 1.68 Hz, 1 H) 7.00 (s, 2 H) 6.89 (d, J=8.24 Hz, 1 H) 6.69 - 6.78 (m, 3 H) 5.66 (dd, J=11.67, 4.81 Hz, 1 H) 4.81 (dd, J=8.16, 3.74 Hz, 1 H) 4.02 (dd, J=13.50, 6.03 Hz, 1 H) 3.22 - 3.26 (m, 3 H) 2.77 - 2.94 (m, 3 H) 2.68 (s, 1 H) 2.62 (s, 5 H) 2.50 - 2.56 (m, 4 H) 1.95 - 2.04 (m, 1 H) 1.67 - 1.79 (m, 3 H) 0.92 - 1.11 (m, 7 H) 0.87 (d, J=12.05 Hz, 2 H) 0.67 (t, J=7.32 Hz, 5 H)

### Example B-39: Synthesis of 3-(7-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)1-oxopthalazine-2(1H)-yl)piperidine-2,6-dione (ICT-0001604)

### Step 1) Synthesis of 3-(1-oxo-7-((2-(piperazine-1-yl)ethyl)amino)phthalazine-2(1H)-yl)piperidine-2,6-dione

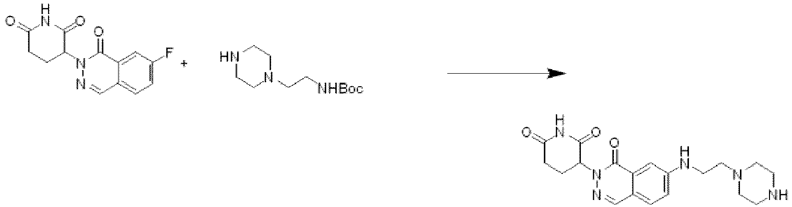

3-(7-fluoro-1-oxopthalazine-2(1H)-yl)piperidine-2,6-dione (500 mg, 1.73 mmol), tert-butyl 4-(2-aminoethyl)piperazine-1-carboxylate (595 mg, 2.57 mmol), and DIPEA (447 mg, 3.46 mmol) solutions in NMP (2 mL) were stirred at 120°C for 5 hours. The reaction mixture was purified by column chromatography to obtain the Boc-protected compound, which was dissolved in 1 ml DCM, followed by 0.2 ml TFA, and stirred at room temperature for 2 hours. The reactants were concentrated under reduced pressure and vacuum-dried. The residue was dissolved, passed through an NH-DM 1020 chromatorex pad in DCM to obtain the heading compound, which was used in the next step without further purification. MS (ESI, m/z): [M+H]⁺ = [385.5].

### Step 2) Synthesis of 3-(7-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)1-oxopthalazine-2(1H)-yl)piperidine-2,6-dione (ICT-0001604)

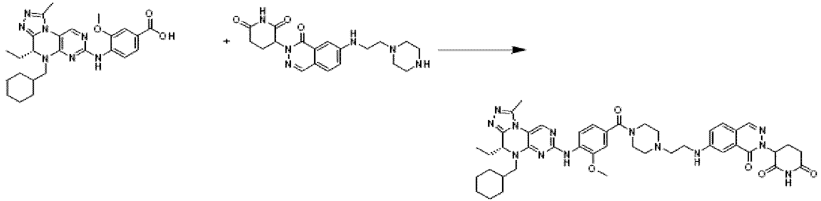

HATU (66.9 mg, 176 umol) is added to (R)-4-((5-(cyclohexylmethyl)-4-ethyl-1-methyl-4,5-dihydro-[1,2,4]triazolo[4,3-f]phteridine-7-yl)amino)-3-methoxybenzoic acid (70 mg, 147 umol) and 3-(1-oxo-7-((2-(piperazine-1-yl)ethyl)amino)phthalazine-2(1H)-yl)piperidine-2,6-dione (61 mg, 147 umol) in 1 ml of DMF, followed by ethyl bis(propane-2-yl)amine (37.9 mg, 293 umol) at room temperature. The reactants were stirred for 6 hours. The reactants were poured into water, extracted with ethyl acetate, dried with MgSO₄, and then concentrated under reduced pressure. The residue was purified with MeOH/DCM (0∼10%) MPLC to obtain the title compound (69 mg). MS (ESI, m/z): [M+H]⁺ = [859.1]
1H NMR (500 MHz, DMSO-d6) δ ppm 10.93 (s, 1 H) 8.32 (s, 2 H) 8.22 (d, J=8.09 Hz, 1 H) 8.05 - 8.12 (m, 2 H) 7.87 (s, 2 H) 7.58 (d, J=9.00 Hz, 1 H) 7.15 (d, J=6.71 Hz, 3 H) 7.00 (s, 2 H) 6.90 (d, J=7.93 Hz, 1 H) 6.75 (br. s., 1 H) 5.68 (d, J=6.56 Hz, 1 H) 4.82 (dd, J=8.09, 3.66 Hz, 1 H) 4.02 (dd, J=13.58, 5.95 Hz, 1 H) 3.84 (s, 5 H) 2.79 - 2.93 (m, 3 H) 2.62 (s, 5 H) 1.97 - 2.05 (m, 2 H) 1.67 - 1.78 (m, 3 H) 1.49 - 1.67 (m, 10 H) 0.87 (d, J=11.14 Hz, 1 H) 0.67 (t, J=7.32 Hz, 5 H)

**Table 1. In vitro degradation analysis and cell viability analysis of WNY-based BRD4 PROTAC compound (see Experimental Examples 1 to 3 described below)**

| ICT-CODE | Structure | Analysis of In vitro cell viability (nM) | | |
|---|---|---|---|---|
| | | RPM12650 | | |
| | | BRD4 (DC50, nM) | BRD4-NUT (DC50, nM) | Cell Viatility (IC50, nM) |
| ICT-0001545 (WNY0824) | 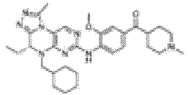 | | | 9290 |
| ICT-0001515 | 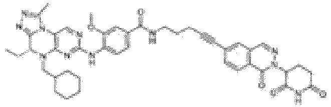 | C | C | C |
| ICT-0001516 | 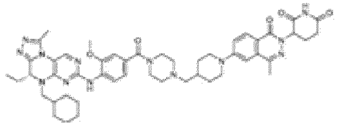 | A | A | C |
| ICT-0001517 | 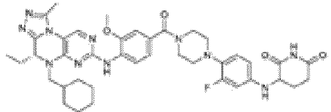 | A | A | A |
| ICT-0001518 | 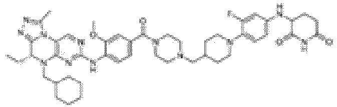 | A | A | B |
| ICT-0001534 | 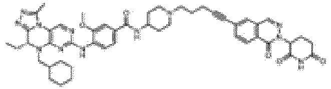 | C | C | C |
| ICT-0001547 | 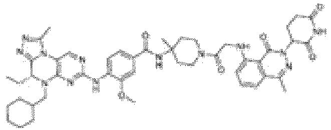 | A | A | A |
| ICT-0001548 | 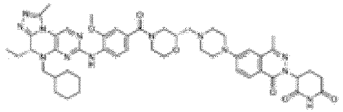 | A | A | C |
| ICT-0001549 | 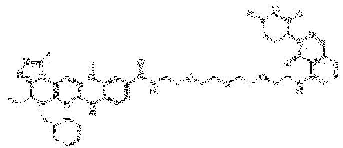 | A | C | B |
| ICT-0001551 | 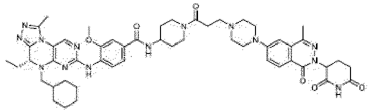 | C | B | C |
| ICT-0001553 | 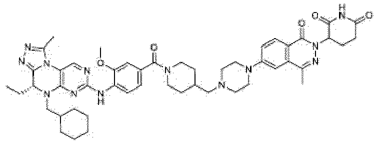 | A | B | C |
| ICT-0001554 | 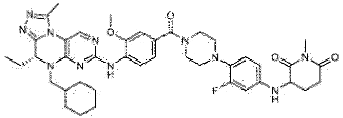 | C | C | C |
| ICT-0001556 | 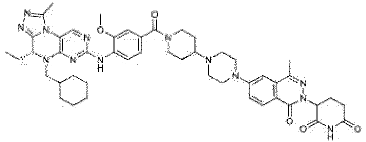 | A | A | C |
| ICT-0001557 | 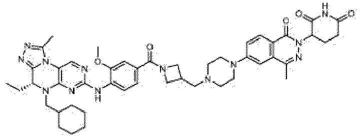 | A | C | C |
| ICT-0001558 | 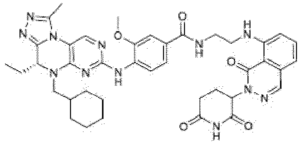 | A | A | C |
| ICT-0001559 | 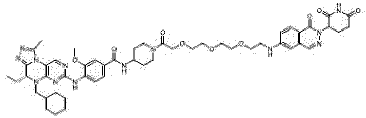 | A | A | C |
| ICT-0001560 | 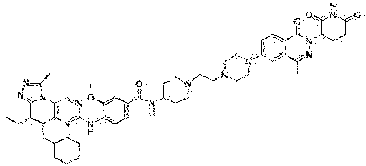 | A | A | C |
| ICT-0001561 | 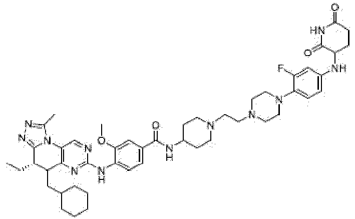 | A | A | C |
| ICT-0001562 | 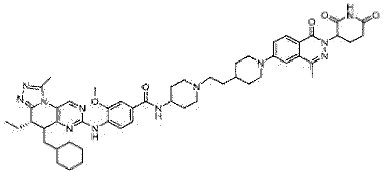 | C | C | B |
| ICT-0001563 | 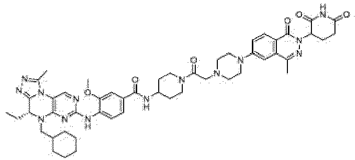 | A | A | C |
| ICT-0001564 | 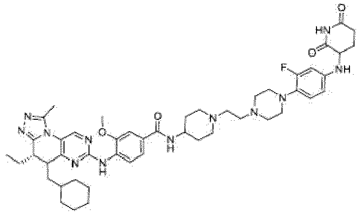 | A | C | C |
| ICT-0001566 | 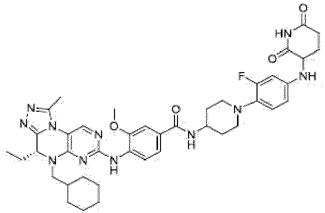 | A | A | C |
| ICT-0001567 | 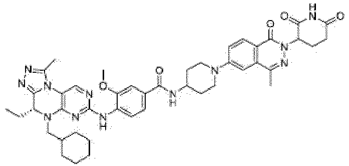 | C | C | C |
| ICT-0001568 | 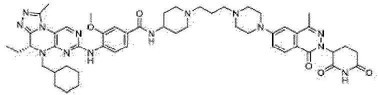 | A | C | C |
| ICT-0001569 | 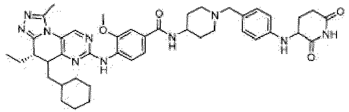 | C | C | C |
| ICT-0001570 | 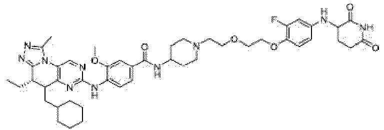 | C | C | C |
| ICT-0001571 | 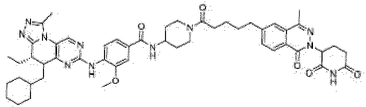 | C | C | C |
| ICT-0001572 | 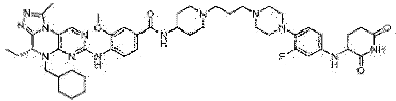 | C | C | C |
| ICT-0001576 | 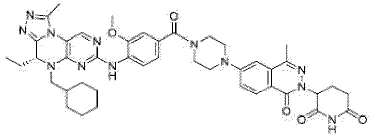 | A | A | C |
| ICT-0001577 | 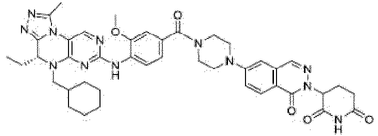 | C | C | C |
| ICT-0001578 | 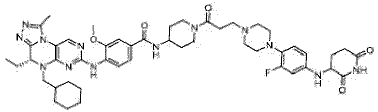 | C | C | B |
| ICT-0001583 | 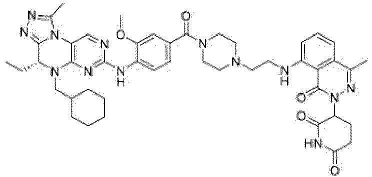 | A | A | C |
| ICT-0001584 | 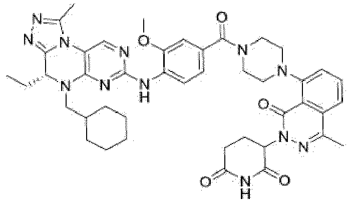 | A | A | A |
| ICT-0001592 | 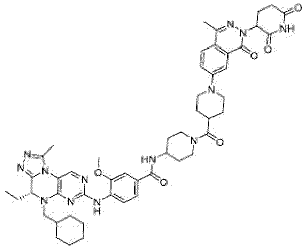 | A | A | A |
| ICT-0001599 | 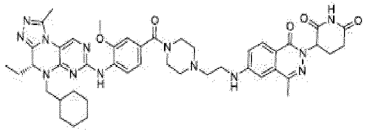 | A | A | C |
| ICT-0001600 | 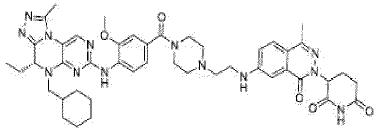 | A | A | A |
| ICT-0001601 | 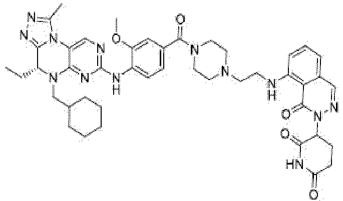 | A | A | A |
| ICT-0001602 | 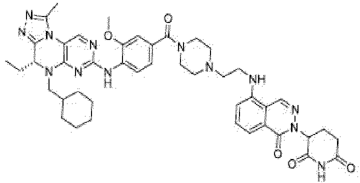 | A | B | C |
| ICT-0001603 | 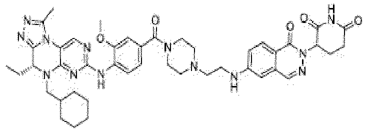 | A | A | C |
| ICT-0001604 | 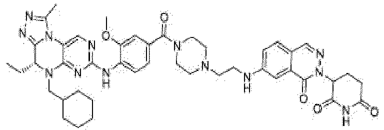 | A | A | A |

| | | | | |
|---|---|---|---|---|
| A: IC50 or DC50 < 100 nM B: 100 nM < IC₅₀ or DC50 < 1 uM C: 1 uM < IC₅₀ or DC50 | | | | |

### Example C: Preparation of a JQ1-based BRD4 PROTAC

### Example C-1: Synthesis of 3-(6-(4-((1-(2-((S)-4-(4-chlorophenyl)-2,3,9-trimethyl-6H-thieno[3,2-f][1,2,4]triazolo[4,3-a][1,4]diazepine-6-il)acetyl)piperidine-4-il)methyl)piperazine-1-il)-7-fluoro-4-methyl-1-oxopthalazine-2(1H)-yl)piperidine-2,6-dione (ICT-0000953)

### Step 1) Synthesis of 3-(7-fluoro-4-methyl-1-oxo-6-(4-(piperidine-4-ylmethyl)piperazine-1-yl)phthalazine-2(1H)-yl)piperidine-2,6-dione and 3-(6-fluoro-4-methyl-1-oxo-7-(4-(piperidine-4-ylmethyl)piperazine-1-yl)phthalazine-2(1H)-yl)piperidine-2,6-dione

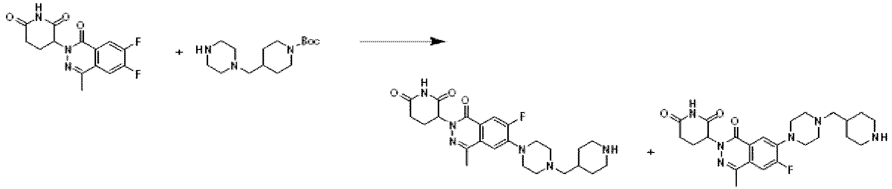

tert-butyl 4-(piperazine-1-monomethyl)piperidine-1-carboxylate (92 mg, 0.35 mmol) was added to 3-(6,7-difluoro-4-methyl-1-oxopthalazine-2(1H)-yl)piperidine-2,6-dione (100 mg, 0.32 mmol) solution in DMA (1 mL) at ambient temperature, followed by ethylbis (propane-2-yl)amine (46.3 mg, 0.35 mmol). The reactant was heated to 120°C for 16 hours. After the end of the reaction, the reactant was poured into water, extracted with ethyl acetate (25 mL x 2). The organic layer was separated. The crude product was obtained by drying, filtering, and concentrating with MgSO₄, purified with MPLC (1 to 5% MeOH in CH2Cl2) to obtain the Boc-protected compound, treated with 50% TFA in DCM (5 mL), and stirred for 5 hours. After the reaction was finished, the reactants were concentrated under reduced pressure and vacuum-dried. The residues were dissolved in DCM, passed through an NH-DM 1020 chromatorex pad to obtain a regio-isomer mixture, and purified into an acetonitrile 0 to 80% C-18 column in water to obtain two compounds in a 3:5 ratio. Compounds with less polarity were designated as 3-(7-fluoro-4-methyl-1-oxo-6-(4-(piperidine-4-yl)piperazine-1-yl)phthalazine-2(1H)-yl)piperidine-2,6-dione, and compounds with greater polarity were designated as 3-(6-fluoro-1-oxo-7-(4-(piperidine-4-ylmethyl)piperazine-1-yl)phthalazine-2(1H)-yl)piperidine-2,6-dione. MS (ESI, m/z): [M+H]⁺ = 471.3, and 471.2

### Step 2) Synthesis of 3-(6-(4-((1-(2-((S)-4-(4-chlorophenyl)-2,3,9-trimethyl-6H-thieno[3,2-f][1,2,4]triazolo[4,3-a][1,4]diazepine-6-il)acetyl)piperidine-4-il)methyl)piperazine-1-yl)7-fluoro-4-methyl-1-oxopthalazine-2(1H)-yl)piperidine-2,6-dione (ICT-0000953)

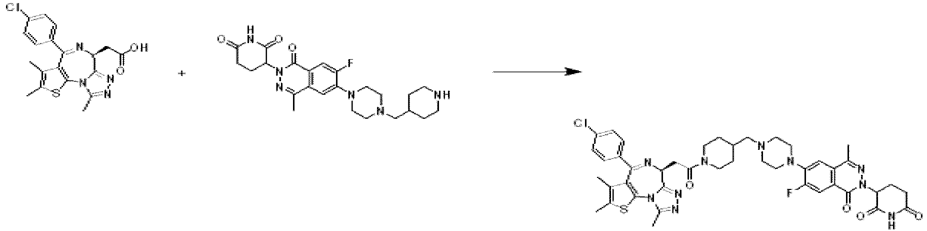

HATU (36.8 mg, 0.1 mmol) is added to the (S)-2-(4-(4-chlorophenyl)-2,3,9-trimethyl-6H-thieno[3,2-f][1,2,4]triazolo[4,3-a][1,4]diazepine-6-yl) acetic acid (50 mg, 0.1 mmol) and 3-(7-fluoro-4-methyl-1-oxo-6-(4-(piperidine-4-ylmethyl)piperazine-1-(4-(piperidine-4-yl)piperazine-2(1H)-yl)piperidine-2,6-dione (40.8 mg, 0.1 mmol) in DMF (5 ml) at room temperature, followed by ethylbis (propane-2-yl)amine (33.7 mg, 0.1 mmol). The reactants were stirred for 6 hours. The reactants were poured into water, extracted with ethyl acetate, dried with MgSO₄, and then concentrated under reduced pressure. The residual compound was obtained by purifying MeOH/DCM (0∼20%) with MPLC (48.2 mg). MS (ESI, m/z): [M+H]⁺ = 854.2
1H NMR (500 MHz, DMSO-d6) δ ppm 1H NMR (400 MHz, DMSO-d 6) d ppm 11.02 (s, 1 H) 7.86 (d, J=13.08 Hz, 1 H) 7.40 - 7.54 (m, 4 H) 7.31 (d, J=8.31 Hz, 1 H) 4.58 (t, J=6.54 Hz, 1 H) 3.29 (br. s., 3 H) 2.54 - 2.65 (m, 11 H) 2.42 (s, 3 H) 2.25 (br. s., 1 H) 1.64 (s, 3 H) 1.11 - 1.29 (m, 17 H)

### Example C-2: Synthesis of 3-(6-(4-((1-(2-((S)-4-(4-chlorophenyl)-2,3,9-trimethyl-6H-thieno[3,2-f][1,2,4]triazolo[4,3-a][1,4]diazepine-6-day)acetyl)piperidine-4-il)methyl)piperazine-1-yl)7-fluoro-1-oxopthalazine-2(1H)-yl)piperidine-2,6-dione (ICT-0000971)

### Step 1) Synthesis of 3-(7-fluoro-1-oxo-6-(4-(piperidine-4-ylmethyl)piperazine-1-yl)phthalazine-2(1H)-yl)piperidine-2,6-dione and 3-(6-fluoro-1-oxo-7-(4-(piperidine-4-ylmethyl)piperazine-1-yl)phthalazine-2(1H)-yl)piperidine-2,6-dione

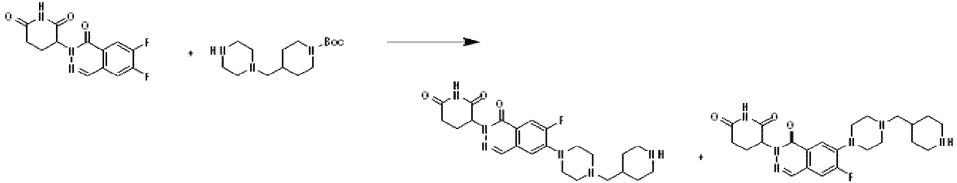

tert-butyl 4-(piperazine-1-yl)piperidine-2,6-dione (100 mg, 0.32 mmol) solution in DMA (3 mL) at ambient temperature was added, followed by ethyl bis(propane-2-yl)amine (46.3 mg, 0.35 mmol). The crude product was obtained by drying, filtering, and concentrating with MgSO₄, purified with MPLC (MeOH 1 to 5 % in CH₂Cl₂) to obtain the Boc-protected compound, which was treated with 50% TFA in DCM (5 mL), and stirred for 5 hours. After the reaction was finished, the reactants were concentrated under reduced pressure and vacuum-dried. The residues were dissolved in DCM, passed through an NH-DM 1020 chromatorex pad to obtain a regio-isomer mixture, and purified into an acetonitrile 0 to 80% C-18 column in water to obtain two compounds in a 3:5 ratio. Compounds with less polarity were defined as 3-(7-fluoro-1-oxo-6-(4-(piperidine-4-ylmethyl)piperazine-1-yl)phthalazine-2(1H)-yl)piperidine-2,6-dione, and compounds with greater polarity were defined as 3-(6-fluoro-1-oxo-7-(4-(piperidine-4-ylmethyl)piperazine-1-yl)phthalazine-2(1H)-yl)piperidine-2,6-dione. MS (ESI, m/z): [M+H]⁺ = 471.3, and 471.2

### Step 2) Synthesis of 3-(6-(4-((1-(2-((S)-4-(4-chlorophenyl)-2,3,9-trimethyl-6H-thieno[3,2-f][1,2,4]triazolo[4,3-a][1,4]diazepine-6-yl)acetyl)piperidine-4-yl)methyl)piperazine-1-yl)-7-fluoro-1-oxopthalazine-2(1H)-yl)piperidine-2,6-dione (ICT-0000971)

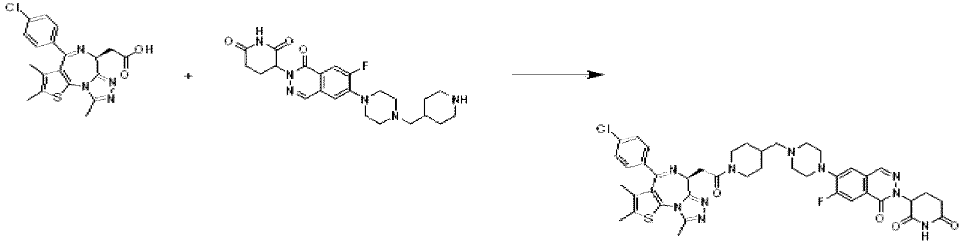

HATU (36.8 mg, 0.1 mmol) is added to (S)-2-(4-(4-chlorophenyl)-2,3,9-trimethyl-6H-thieno[3,2-f][1,2,4]triazolo[4,3-a][1,4]diazepine-6-yl) acetic acid (50 mg, 0.1 mmol) and 3-(7-fluoro-1-oxo-6-(4-(4-(piperidine-4-ylmethyl)piperazine-1-yl)phthalazine-2(1H)-yl)piperazine-2,6-dione (40 mg, 0.1 mmol) in DMF (5 ml) at room temperature, followed by ethylbis (propane-2-yl)amine (33.7 mg, 0.1 mmol). The reactants were stirred for 6 hours. The reactants were poured into water, extracted with ethyl acetate, dried with MgSO₄, and then concentrated under reduced pressure. The residual was purified with MeOH/DCM (0-20%) MPLC to obtain the title compound (48.2 mg). MS (ESI, m/z): [M+H]⁺ = 840.2
1H NMR (500 MHz, DMSO-d6) δ ppm 10.99 (s, 1 H) 8.34 (s, 1 H) 7.87 (d, J=12.72 Hz, 1 H) 7.60 (d, J=8.19 Hz, 1 H) 7.35 - 7.47 (m, 4 H) 5.73 (dd, J=12.10, 4.89 Hz, 1 H) 4.52 (t, J=6.72 Hz, 1 H) 3.64 (br. s., 16 H) 3.20 - 3.38 (m, 4 H) 3.10 (s, 2 H) 2.58 (br. s., 1 H) 2.54 (s, 3 H) 2.46 - 2.52 (m, 1 H) 2.36 (s, 3 H) 1.57 (s, 3 H)

### Example C-3: Synthesis of 3-(7-(4-((1-(2-((S)-4-(4-chlorophenyl)-2,3,9-trimethyl-6H-thieno[3,2-f][1,2,4]triazolo[4,3-a][1,4]diazepine-6-day)acetyl)piperidine-4-il)methyl)piperazine-1-yl)-6-fluoro-1-oxopthalazine-2(1H)-yl)piperidine-2,6-dione (ICT-0000972)

### Step 1) Synthesis of 3-(7-(4-((1-(2-((S)-4-(4-chlorophenyl)-2,3,9-trimethyl-6H-thieno[3,2-f][1,2,4]triazolo[4,3-a][1,4]diazepine-6-yl)acetyl)piperidine-4-il)methyl)piperazine-1-yl)-6-fluoro-1-oxopthalazine-2(1H)-day)piperidine-2,6-dione (ICT-0000972)

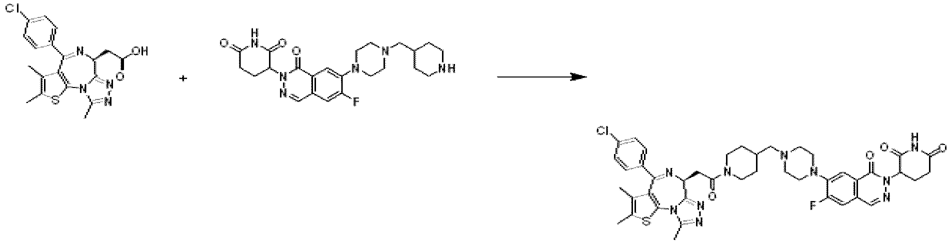

HATU (36.8 mg, 0.1 mmol) is added to (S)-2-(4-(4-chlorophenyl)-2,3,9-trimethyl-6H-thieno[3,2-f][1,2,4]triazolo[4,3-a][1,4]diazepine-6-yl) acetic acid (50 mg, 0.1 mmol) and 3-(6-fluoro-1-oxo-7-(4-(piperidine-4-ylmethyl)piperazine-1-yl)phthalazine-2(1H)-yl)piperidine-2,6-dione (40 mg, 0.1 mmol) in DMF (5 ml) at room temperature, followed by ethylbis (propane-2-yl)amine (33.7 mg, 0.1 mmol). The reactants were stirred for 6 hours. The reactants were poured into water, extracted with ethyl acetate, dried with MgSO₄, and then concentrated under reduced pressure. The residual was purified with MeOH/DCM (0∼20%) MPLC to obtain the title compound (48.2 mg). MS (ESI, m/z): [M+H]⁺ = 840.2
1H NMR (500 MHz, DMSO-d6) δ ppm 11.06 (s, 1 H) 8.37 (s, 1 H) 7.86 (d, J=12.84 Hz, 1 H) 7.77 (d, J=8.44 Hz, 1 H) 7.35 - 7.54 (m, 5 H) 4.59 (t, J=6.72 Hz, 1 H) 3.32 - 3.45 (m, 1 H) 3.30 (br. s., 3 H) 3.17 (s, 3 H) 2.53 - 2.70 (m, 5 H) 2.42 (s, 3 H) 1.64 (s, 3 H)

### Example C-4 : Synthesis of 3-(6-(4-(1-(2-((S)-4-(4-chlorophenyl)-2,3,9-trimethyl-6H-thieno[3,2-f][1,2,4]triazolo[4,3-a][1,4]diazepine-6-day)acetyl)azetidine-3-il)piperazine-1-yl)-1-oxopthalazine-2(1H)-yl)piperidine-2,6-dione (ICT-0000973)

### Step 1) Synthesis of benzyl 4-(1-(tert-butoxycarbonyl)azetidine-3-yl)piperazine-1-carboxylate

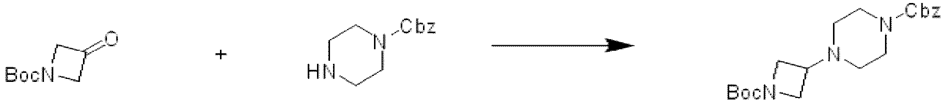

Sodium cyanoborohydride (4.07 g, 102 mmol) was added to tert-butyl 3-oxoazetidine-1-carboxylate (12.8 g, 74.9 mmol) and benzyl piperazine-1-carboxylate (15 g, 68.1 mmol) solutions in methanol (300 ml) and AcOH (6 ml), and stirred at room temperature for 16 hours. The reactants were poured into water, extracted with ethyl acetate, dried with MgSO₄, and then concentrated under reduced pressure. The residue was purified with HX/EA (20~90%) MPLC to obtain the title compound (22 g). MS (ESI, m/z): [M+H]⁺ = 376.0.

### Step 2) Synthesis of tert-butyl 3-(piperazine-1-yl)azetidine-1-carboxylate

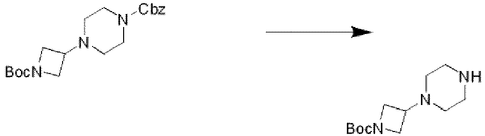

Pd/C (10 wt%, 50 mg) was added to benzyl 4-(1-(tert-butoxycarbonyl)azetidine-3-yl)piperazine-1-carboxylate (1 g, 2.66 mmol) solution in MeOH (10 ml) and stirred at room temperature under H₂ atmosphere for 5 hours. The solution was filtered in Celite 545 phase. The solvent was removed under reduced pressure to obtain the title compound (0.7 g). MS (ESI, m/z): [M+H]⁺ = 242.0.

### Step 3) Synthesis of tert-butyl 3-(4-(2-(2,6-dioxopiperidine-3-yl)-1-oxo-1,2-dihydroptalazine-6-yl)piperazine-1-yl)azetidine-1-carboxylate

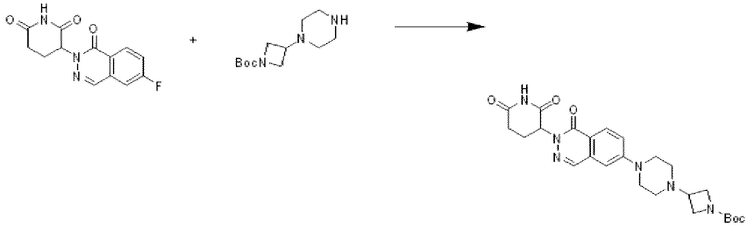

3-(6-fluoro-1-oxopthalazine-2(1H)-yl) piperidine-2,6-dione (642 mg, 2.4 mmol), tert-butyl 3-(piperazine-1-yl)azetidine-1-carboxylate (643 mg, 2.66 mmol), and DIPEA (0.93 ml, 5.33 mmol) solutions in NMP (15 mL) were stirred at 120°C for 20 hours. MS (ESI, m/z): [M+H]⁺ = 497.3.

### Step 4) Synthesis of 3-(6-(4-(azetidine-3-yl)piperazine-1-il)-1-oxopthalazine-2(1H)-yl)piperidine-2,6-dione

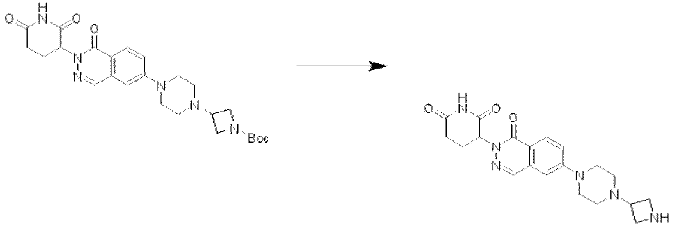

TFA (0.3 ml) was added to tert-butyl 3-(4-(2-(2,6-dioxopiperidin-3-yl)-1-oxo-1,2-dihydroptalazine-6-yl) piperazine-1-yl)azetidine-1-carboxylate (180 mg, 0.4 mmol) solution in DCM (1 ml) and stirred at room temperature for 2 hours. After the reaction was completed, the reactants were concentrated under the reduced pressure and vacuum dried. The residues were dissolved in DCM, passed through an NH-DM 1020 chromatorex pad to obtain the heading compound, which was used in the next step without further purification (140 mg). MS (ESI, m/z): [M+H]⁺ = 397.3.

### Step 5) Synthesis of 3-(6-(4-(1-(2-((S)-4-(4-chlorophenyl)-2,3,9-trimethyl-6H-thieno[3,2-f][1,2,4]triazolo[4,3-a][1,4]diazepine-6-yl)acetyl)azetidine-3-yl)piperazine-1-il)-1-oxopthalazine-2(1H)-day)piperidine-2,6-dione (ICT-0000973)

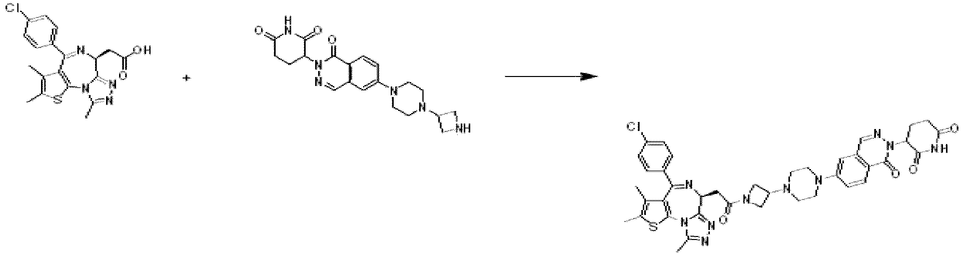

HATU (36.8 mg, 0.1 mmol) is added to (S)-2-(4-(4-chlorophenyl)-2,3,9-trimethyl-6H-thieno[3,2-f][1,2,4]triazolo[4,3-a][1,4]diazepine-6-yl) acetic acid (50 mg, 0.1 mmol) and 3-(6-(4-(azetidine-3-yl)piperazine-1-yl)1-oxopthalazine-2(1H)-yl)piperidine-2,6-dione (38 mg, 0.1 mmol) in DMF (5 ml) at room temperature, followed by ethylbis (propane-2-yl)amine (33.7 mg, 0.1 mmol). The reactants were stirred for 6 hours. The reactants were poured into water, extracted with ethyl acetate, dried with MgSO₄, and then concentrated under reduced pressure. The residual was purified with MeOH/DCM (0∼20%) MPLC to obtain the title compound (48.2 mg). MS (ESI, m/z): [M+H]⁺ = 780.2
1H NMR (500 MHz, DMSO-d6) δ ppm 11.03 (s, 1 H) 8.29 (s, 1 H) 8.12 (d, J=8.93 Hz, 1 H) 7.62 (dd, J=9.23, 2.14 Hz, 1 H) 7.39 - 7.53 (m, 5 H) 5.78 (dd, J=11.98, 5.62 Hz, 1 H) 4.62 (d, J=5.50 Hz, 1 H) 4.52 (td, J=7.03, 2.45 Hz, 1 H) 4.08 - 4.23 (m, 1 H) 3.20 - 3.43 (m, 1 H) 3.11 - 3.20 (m, 1 H) 2.86 - 3.00 (m, 1 H) 2.59 - 2.66 (m, 4 H) 2.53 - 2.59 (m, 1 H) 2.42 (s, 3 H) 1.63 (s, 3 H)

### Example C-5: Synthesis of 3-(6-(4-(1-(2-((S)-4-(4-chlorophenyl)-2,3,9-trimethyl-6H-thieno[3,2-f][1,2,4]triazolo[4,3-a][1,4]diazepine-6-yl)acetyl)azetidine-3-il)piperazine-1-yl)-4-methyl-1-oxopthalazine-2(1H)-yl)piperidine-2,6-dione (ICT-0000974)

### Step 1) Synthesis of tert-butyl 3-(4-(2-(2,6-dioxopiperidin-3-yl)-4-methyl-1-oxo-1,2-dihydroptalazine-6-yl)piperazine-1-yl)azetidine-1-carboxylate

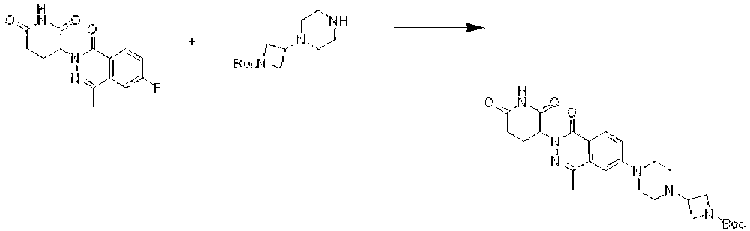

3-(6-fluoro-4-methyl-1-oxopthalazine-2(1H)-yl)piperidine-2,6-dione (642 mg, 2.4 mmol), tert-butyl 3-(piperazine-1-yl)azetidine-1-carboxylate (643 mg, 2.66 mmol), and DIPEA (0.93 ml, 5.33 mmol) solutions in NMP (15 mL) were stirred at 120°C for 20 hours. MS (ESI, m/z): [M+H]⁺ = 411.3.

### Step 2) Synthesis of 3-(6-(4-(azetidine-3-yl)piperazine-1-yl)-4-methyl-1-oxopthalazine-2(1H)-yl)piperidine-2,6-dione

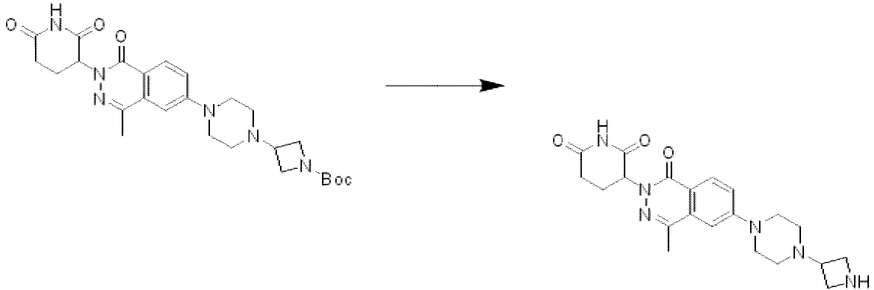

TFA (0.3 ml) was added to tert-butyl 3-(4-(2-(2,6-dioxopiperidine-3-yl)-4-methyl-1-oxo-1,2-dihydroptalazin-6-yl)piperazine-1-yl)azetidine-1-carboxylate (180 mg, 0.4 mmol) solution in DCM (1 ml) and stirred at room temperature for 2 hours. After the end of the reaction, the reactants were depressurized and vacuum-dried. The residues were dissolved in DCM, passed through an NH-DM 1020 Chromatorex pad to obtain the title compound, which was used in the next step without further purification (140 mg). MS (ESI, m/z): [M+H]⁺ = 411.3.

### Step 3) Synthesis of 3-(6-(4-(1-(2-((S)-4-(4-chlorophenyl)-2,3,9-trimethyl-6H-thieno[3,2-f][1,2,4]triazolo[4,3-a][1,4]diazepine-6-yl)acetyl)azetidine-3-il)piperazine-1-yl)4-methyl-1-oxopthalazine-2(1H)-yl)piperidine-2,6-dione (ICT-0000974)

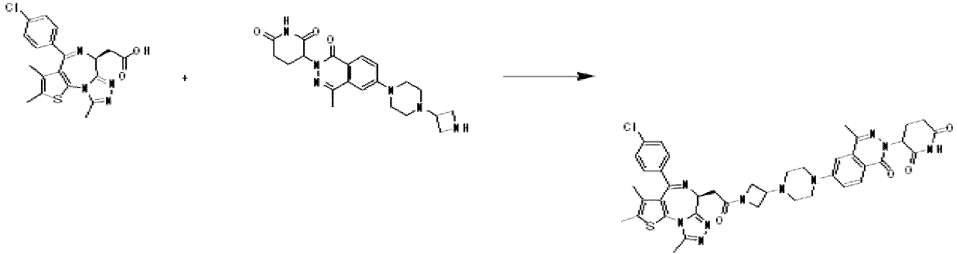

HATU (36.8 mg, 0.1 mmol) is added to (S)-2-(4-(4-chlorophenyl)-2,3,9-trimethyl-6H-thieno[3,2-f][1,2,4]triazolo[4,3-a][1,4]diazepine-6-yl) acetic acid (50 mg, 0.1 mmol) and 3-(6-(4-(azetidine-3-yl)piperazine-1-yl)4-methyl-1-oxopthalazine-2(1H)-yl)piperidine-2,6-dione (40 mg, 0.1 mmol) in DMF (5 ml) at room temperature, followed by ethylbis (propane-2-yl)amine (33.7 mg, 0.1 mmol). The reactants were stirred for 6 hours. The reactants were poured into water, extracted with ethyl acetate, dried with MgSO₄, and then concentrated under reduced pressure. The residual was purified with MeOH/DCM (0∼20%) MPLC to obtain the title compound (48.2 mg). MS (ESI, m/z): [M+H]⁺ = 794.2
1H NMR (500 MHz, DMSO-d6) δ ppm 11.00 (s, 1 H) 8.14 (d, J=8.93 Hz, 1 H) 7.62 (d, J=8.93 Hz, 1 H) 7.41 - 7.54 (m, 4 H) 7.23 (s, 1 H) 5.70 (dd, J=11.86, 4.89 Hz, 1 H) 4.64 (br. s., 1 H) 4.52 (td, J=6.94, 2.26 Hz, 1 H) 3.13 - 3.43 (m, 4 H) 2.90 (d, J=11.49 Hz, 1 H) 2.58 - 2.68 (m, 5 H) 2.42 (s, 3 H) 1.63 (s, 3 H)

### Example C-6: Synthesis of 3-(7-(4-((1-(2-((S)-4-(4-chlorophenyl)-2,3,9-trimethyl-6H-thieno[3,2-f][1,2,4]triazolo[4,3-a][1,4]diazepine-6-day)acetyl)piperidine-4-il)methyl)piperazine-1-yl)4-methyl-1-oxopthalazine-2(1H)-yl)piperidine-2,6-dione (ICT-0000982)

### Step 1) Synthesis of 3-(4-methyl-1-oxo-7-(4-(piperidine-4-ylmethyl)piperazine-1-yl)phthalazine-2(1H)-yl)piperidine-2,6-dione

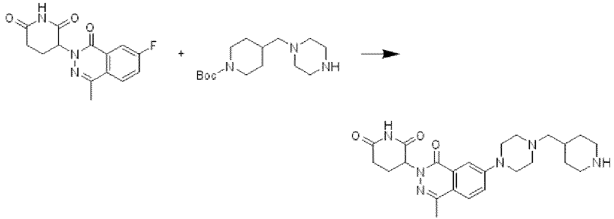

Ethylbis (1.34 g, 10.4 mmol) was added to 3-(7-fluoro-4-methyl-1-oxopthalazine-2(1H)-yl)piperidine-2,6-dione (0.5 g, 1.73 mmol) and tert-butyl 4-(piperazine-1-monomethyl)piperidine-1-carboxylate (980 mg, 3.46 mmol) solutions in 5 ml of DMA at room temperature. The reactants were heated at 120°C for 16 hours. The reactants were poured into water, extracted with ethyl acetate, dried with MgSO₄, and then concentrated under reduced pressure. The residue was purified with MPLC MeOH/DCM (0∼10%) to obtain the protected compound. This was treated with 50% TFA in DCM (5 mL) and stirred for 5 hours. After the reaction was finished, the reactants were concentrated under reduced pressure and vacuum-dried. The residue was dissolved in DCM and passed through an NH-DM 1020 Chromatorex pad to obtain the heading compound (550 mg). MS (ESI, m/z): [M+H]⁺ = 453.6.

### Step 2) Synthesis of 3-(7-(4-((1-(2-((S)-4-(4-chlorophenyl)-2,3,9-trimethyl-6H-thieno[3,2-f][1,2,4]triazolo[4,3-a][1,4]diazepine-6-il)acetyl)piperidine-4-il)methyl)piperazine-1-yl)4-methyl-1-oxopthalazine-2(1H)-yl)piperidine-2,6-dione (ICT-0000982)

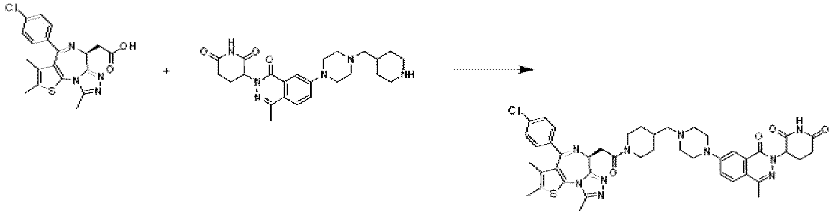

HATU (36.8 mg, 0.1 mmol) is added to (S)-2-(4-(4-chlorophenyl)-2,3,9-trimethyl-6H-thieno[3,2-f][1,2,4]triazolo[4,3-a][1,4]diazepine-6-yl) acetic acid (50 mg, 0.1 mmol) and 3-(4-methyl-1-oxo-7-(4-(piperidine-4-ylmethyl)piperazine-1-yl)phthalazine-2(1H)-yl)piperidine-2,6-dione (42 mg, 0.1 mmol) in DMF (5 ml) at room temperature, followed by ethylbis (propane-2-yl)amine (33.7 mg, 0.1 mmol). The reactants were stirred for 6 hours. The reactants were poured into water, extracted with ethyl acetate, dried with MgSO₄, and then concentrated under reduced pressure. The residual was purified with MeOH/DCM (0-20%) MPLC to obtain the title compound (48.2 mg). MS (ESI, m/z): [M+H]⁺ = 836.2
1H NMR (500 MHz, DMSO-d6) δ ppm 10.99 (s, 1 H) 7.79 (d, J=9.05 Hz, 1 H) 7.61 (d, J=8.93 Hz, 1 H) 7.54 (s, 1 H) 7.41 - 7.52 (m, 4 H) 4.58 (t, J=6.72 Hz, 1 H) 3.41 (br. s., 3 H) 2.62 (br. s., 1 H) 2.52 - 2.62 (m, 8 H) 2.46 (s, 3 H) 2.42 (s, 3 H) 1.88 - 2.02 (m, 3 H) 1.63 (s, 3 H) 1.14 - 1.27 (m, 1 H)

### Example C-7: Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-[4-({5-[2-(2,6-dioxopiperidin-3-yl)-1-oxo-1,2-dihydroptalazine-6-yl]pent-4-phosphorus-1-yl}oxy)phenyl]acetamide (ICT-00001409)

### Step 1) Synthesis 3-[6-(5-hydroxypentyl)-1-oxo-1,2-dihydroptalazine-2-yl]piperidine-2,6-dione

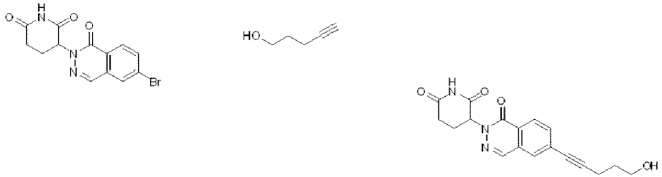

Cul (85 mg, 446 µmol) and Pd(PPh3)2Cl2 (313 mg, 446 µmol) were added to 3-(6-bromo-1-oxo-1,2-dihydrophoptalazin-2-yl) piperidine-2,6-dione (1.5 g, 4.46 mmol) and pent-4-phospho-1-ol (488 mg, 5.8 mmol) solutions in DMF (5 mL), followed by TEA (1.35 g, 13.4 mmol). Ethyl acetate. The organic layer was washed with brine solution, dried with Na₂SO₄, and concentrated. The residue was purified with EA/Hexane (0-10%) MPLC to obtain the title compound (1.3 g). MS (ESI, m/z): [M+H]⁺= [340.4],

### Step 2) Synthesis of 5-[2-(2,6-dioxopiperidin-3-yl)-1-oxo-1,2-dihydroptalazine-6-ylpent-4-phospho-1-ylmethanesulfonate

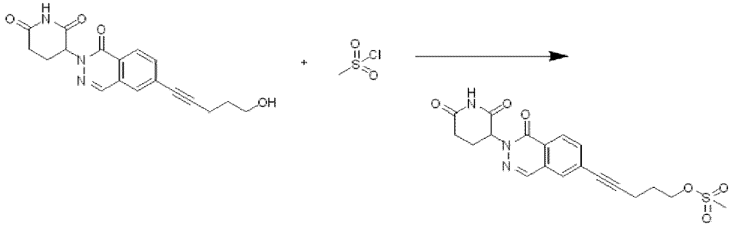

Ethyl bis(propane-2-yl)amine (263 mg, 2.03 mmol) was added to a solution of 3-[6-(5-hydroxypente-1-phosphoin-1-yl)-1-oxo-1,2-dihydroptalazine-2-yl]piperidine-2,6-dione (230 mg, 678 µmol) and methanesulfonyl chloride (93.2 mg, 813 µmol) in DCM (20 ml). The reactants were stirred at 0°C for 1 hour. The mixture was stirred at room temperature for 1 hour. The reactants were poured into the water and extracted with ethyl acetate. The organic layer was dried with MgSO₄, concentrated under reduced pressure. It was then purified by column chromatography to obtain the title compound (220 mg). MS (ESI, m/z): [M+H]⁺ = 418.4

### Step 3) Synthesis of tert-butyl N-[4-({5-[2-(2,6-dioxopiperidin-3-yl)-1-oxo-1,2-dihydroptalazine-6-yl]pent-4-phosphorus-1-yl}oxy)phenyl]carbamate

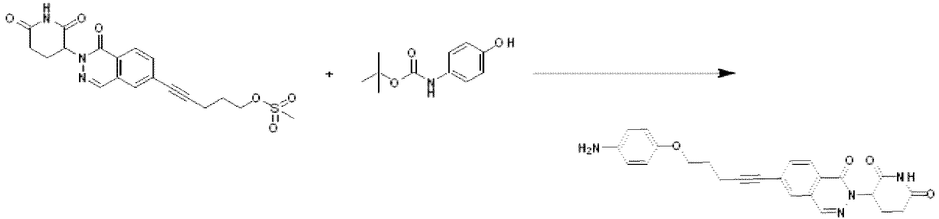

Cesium carbonate (687 mg, 2.11 mmol) and potassium iodide (17.5 mg, 105 µmol) were added to a solution of 5-[2-(2,6-dioxopiperidin-3-yl)-1-oxo-1,2-dihydroptalazine-6-yl]pent-4-phosphorus-1-yl methanesulfonate (220 mg, 527 µmol) and tert-butyl N-(4-hydroxyphenyl)carbamate (110 mg, 527 µmol) in 1 ml of DMF at room temperature. It was dried with MgSO4 and then concentrated under reduced pressure. The residue was purified with MeOH/DCM (0∼10%) MPLC to obtain the Boc-protected compound, which was dissolved in 1 ml DCM, followed by 0.2 ml TFA, and stirred at room temperature for 2 hours. After the reaction was finished, the reactants were concentrated under reduced pressure and vacuum-dried. The residue was dissolved in DCM, passed through an NH-DM 1020 Chromatorex pad to obtain the heading compound, which was used in the next step without further purification. (36 mg) MS (ESI, m/z): [M+H]⁺= [431.5].

### Step 4) Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-[4-({5-[2-(2,6-dioxopiperidin-3-yl)-1-oxo-1,2-dihydroptalazine-6-yl]pent-4-phospho-1-yl}oxy)phenyl]acetamide (ICT-00001409)

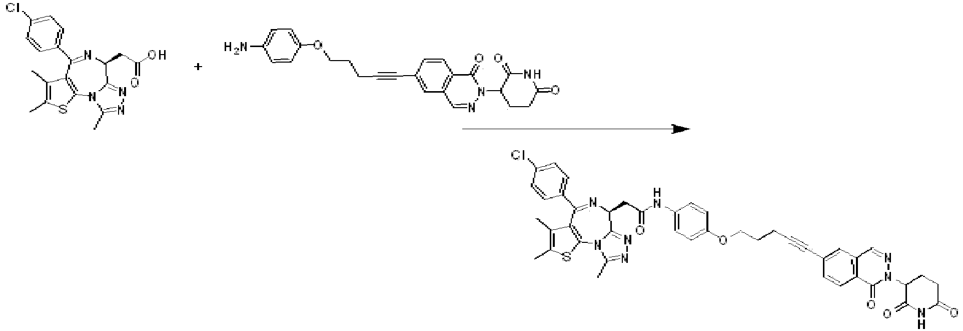

HATU (42.4 mg, 112 µmol) is added to (S)-2-(4-(4-chlorophenyl)-2,3,9-trimethyl-6H-thieno[3,2-f][1,2,4]triazolo[4,3-a][1,4]diazepine-6-yl) acetic acid (22.4 mg, 55.8 µmol) and 3-{6-[5-(4-aminophenoxy)pent-1-phosphoin-1-yl]-1-oxo-1,2-dihydrophopthalazine-2-yl}piperidine-2,6-dione (24 mg, 55.8 µmol) in DMF (5 ml) at room temperature, followed by ethylbis (propane-2-yl)amine (72 mg, 167 µmol). The reactants were stirred for 6 hours. The reactants were poured into water, extracted with ethyl acetate, dried with MgSO₄, and then concentrated under reduced pressure. The residual was purified with MeOH/DCM (0∼20%) MPLC to obtain the title compound (48.2 mg). MS (ESI, m/z): [M+H]⁺ = 814.3
1H NMR (500 MHz, DMSO-d6) δ ppm 11.00 (s, 1 H) 10.12 (s, 1 H) 8.37 (s, 1 H) 8.15 (d, J=7.78 Hz, 3 H) 7.95 (s, 1 H) 7.78 (d, J=8.09 Hz, 1 H) 7.48 (d, J=8.55 Hz, 1 H) 7.42 (m, J=7.93 Hz, 2 H) 7.35 (m, J=8.24 Hz, 2 H) 6.87 (d, J=8.70 Hz, 2 H) 6.47 (s, 1 H) 5.70 - 5.77 (m, 1 H) 4.52 (t, J=7.17 Hz, 1 H) 4.03 (t, J=6.10 Hz, 2 H) 3.38 - 3.46 (m, 2 H) 2.63 (t, J=6.94 Hz, 2 H) 2.57 (br. s., 1 H) 2.54 (s, 4 H) 2.47 (br. s., 1 H) 2.29 (s, 1 H) 2.11 (t, J=7.40 Hz, 1 H) 2.01 - 2.08 (m, 2 H) 1.96 (t, J=6.33 Hz, 2 H) 0.79 (t, J=6.71 Hz, 1 H)

### Example C-8: Synthesis of 2-((S)-4-(4-chlorophenyl)-2,3,9-trimethyl-6H-thieno[3,2-f][1,2,4]triazolo[4,3-a][1,4]diazepine-6-yl)-N-(4-(4-(2-(2,6-dioxopiperidin-3-yl)-4-methyl-1-oxo-1,2-dihydroptalazine-6-yl)piperazine-1-yl)phenyl)acetamide (ICT-00001410) synthesis

### Step 1) 3-(6-(4-(4-aminophenyl)piperazine-1-yl)-4-methyl-1-oxopthalazine-2(1H)-yl)piperidine-2,6-dione

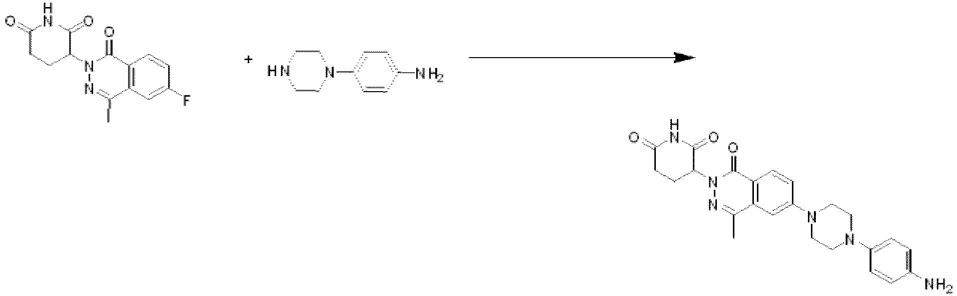

Ethylbis (propane-2-yl)amine (670 mg, 5.19 mmol) was added to 3-(6-fluoro-4-methyl-1-oxopthalazine-2(1H)-yl)piperidine-2,6-dione (0.5 g, 1.73 mmol) and 4-(piperazine-1-yl)aniline (306 mg, 1.73 mmol) solutions in DMA (2 ml) at room temperature. The reactants were stirred at 160°C for 16 hours. The reactants were poured into water, extracted with ethyl acetate, dried with MgSO₄, and then concentrated under reduced pressure. The residue was purified with MeOH/DCM (0∼10%) MPLC to obtain the headline compound (621 mg). MS (ESI, m/z): [M+H]⁺ = 447.6.

### Step 2) Synthesis of 2-((S)-4-(4-chlorophenyl)-2,3,9-trimethyl-6H-thieno[3,2-f][1,2,4]triazolo[4,3-a][1,4]diazepine-6-yl)-N-(4-(4-(2-(2,6-dioxopiperidin-3-yl)-4-methyl-1-oxo-1,2-dihydropetalazine-6-yl)piperazine-1-yl)phenyl)acetamide (ICT-00001410)

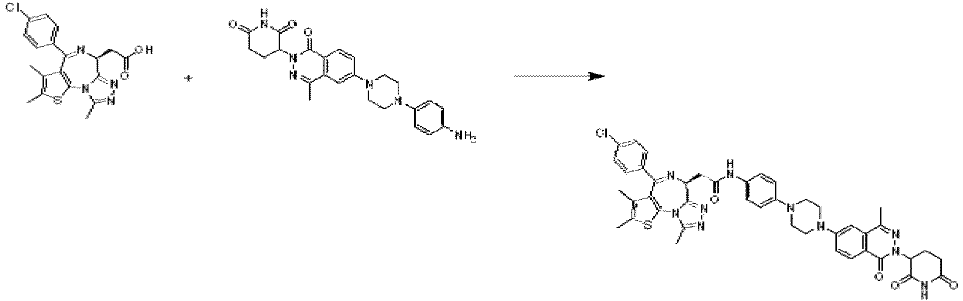

HATU (36.8 mg, 0.1 mmol) is added to (S)-2-(4-(4-chlorophenyl)-2,3,9-trimethyl-6H-thieno[3,2-f][1,2,4]triazolo[4,3-a][1,4]diazepine-6-yl) acetic acid (50 mg, 0.1 mmol) and 3-(6-(4-(4-aminophenyl)piperazine-1-yl)4-methyl-1-oxopthalazine-2(1H)-yl)piperidine-2,6-dione (45 mg, 0.1 mmol) in DMF (5 ml) at room temperature, followed by ethylbis (propane-2-yl)amine (33.7 mg, 0.1 mmol). The reactants were stirred for 6 hours. The reactants were poured into water, extracted with ethyl acetate, dried with MgSO₄, and then concentrated under reduced pressure. The residual was purified with MeOH/DCM (0∼20%) MPLC to obtain the title compound (48.2 mg). MS (ESI, m/z): [M+H]⁺ = 830.2
1H NMR (500 MHz, DMSO-d6) δ ppm 10.91 (s, 1 H) 10.07 (s, 1 H) 7.98 - 8.06 (m, 1 H) 7.51 (dd, J=9.16, 2.14 Hz, 1 H) 7.39 - 7.49 (m, 5 H) 7.36 (d, J=8.55 Hz, 2 H) 7.10 (d, J=1.98 Hz, 1 H) 6.93 (d, J=9.00 Hz, 2 H) 5.62 (d, J=7.48 Hz, 1 H) 4.52 (t, J=7.10 Hz, 1 H) 3.54 (br. s., 4 H) 3.38 - 3.47 (m, 2 H) 3.21 (br. s., 4 H) 2.77 - 2.90 (m, 1 H) 2.49 - 2.59 (m, 5 H) 2.35 (s, 3 H) 1.57 (s, 3 H)

### Example C-9: Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-[4-({5-[2-(2,6-dioxopiperidin-3-yl)-1-oxo-1,2-dihydroptalazine-6-yl]pentyl}oxy)phenyl)acetamide (ICT-00001411)

### Step 1) Synthesis of 3-[6-(5-hydroxypentyl)-1-oxo-1,2-dihydropthalazine-2-yl]piperidine-2,6-dione

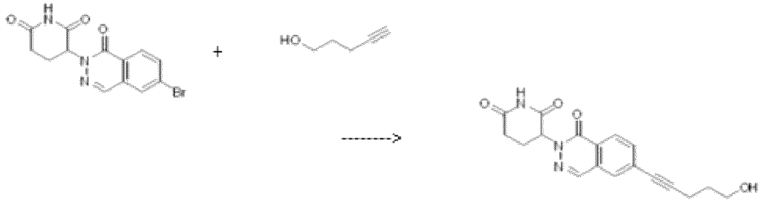

Cul (85 mg, 446 µmol) and Pd(PPh₃)₂Cl₂ (313 mg, 446 µmol) were added to 3-(6-bromo-1-oxo-1,2-dihydroptalazine-2-yl) piperidine-2,6-dione (1.5 g, 4.46 mmol) and pent-4-in-1-ol (488 mg, 5.8 mmol) solution in DMF (5 mL), followed by TEA (1.35 g, 13.4 mmol). The mixture was irradiated in a microwave reactor at 100°C for 2 hours. The reactants were diluted with water and extracted with ethyl acetate. The organic layer was washed with brine solution, dried with Na₂SO₄, and concentrated. The residue was purified with EA/Hexane (0∼10%) MPLC to obtain the title compound (1.3 g). MS (ESI, m/z): [M+H]⁺ = [340.4].

### Step 2) Synthesis of 3-[6-(5-hydroxypentyl)-1-oxo-1,2-dihydroptalazine-2-yl]piperidine-2,6-dione

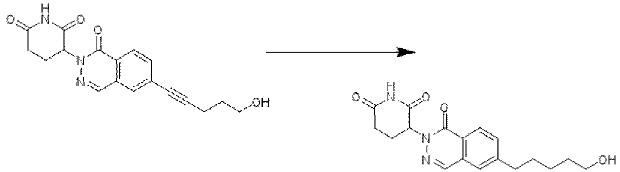

A mixture of 3-[6-(5-hydroxypentyl)-1-oxo-1,2-dihydroptalazine-2-yl]piperidine-2,6-dione (504 mg, 1.60 mmol) and palladium (15.4 mg, 0.142 mmol) solution mixtures in MeOH (10 ml) was stirred for 2 hours at room temperature under nitrogen atmosphere. The solvent was evaporated and vacuum-dried (403 mg). MS (ESI, m/z): [M+H]⁺ = 344.3

### Step 3) Synthesis of 5-[2-(2,6-dioxopiperidin-3-yl)-1-oxo-1,2-dihydroptalazine-6-yl]pentyl methanesulfonate

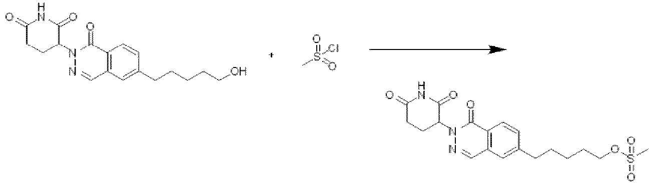

Ethylbis (propane-2-yl)amine (3 equivalent) was added to a solution of 3-[6-(5-hydroxypentyl)-1-oxo-1,2-dihydroptalazine-2-yl]piperidine-2,6-dione (403 mg, 0.79 mmol) and methanesulfonyl chloride (403 mg, 2.12 mmol) in DCM (30 ml). The reactants were stirred at 0°C for 1 hour. The mixture was stirred at room temperature for 1 hour. The reactants were poured into water, extracted with ethyl acetate. The organic layer was dried with MgSO₄, concentrated under reduced pressure, and then purified by column chromatography to obtain the heading compound (530 mg). MS (ESI, m/z): [M+H]⁺ = 422.3

### Step 4) Synthesis of Tert-butyl N-[4-({5-[2-(2,6-dioxopiperidin-3-yl)-1-oxo-1,2-dihydroptalazine-6-yl]pentyl}oxy)phenyl]carbamate

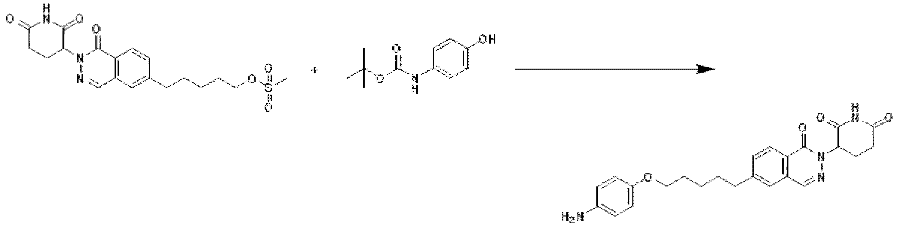

Cesium carbonate (182 mg, 1.10 mmol) and potassium iodide (9.1 mg, 55 µmol) were added at room temperature to a solution of 5-[2-(2,6-dioxopiperidin-3-yl)-1-oxo-1,2-dihydroptalazine-6-yl]pentyl methanesulfonate (116 mg, 275 µmol) and tert-butyl N-(4-hydroxyphenyl)carbamate (57.6 mg, 275 µmol) in 1 ml of DMF. Cesium carbonate (182 mg, 1.10 mmol) and potassium iodide (9.1 mg, 55 µmol) were added at room temperature. The reactants were stirred at 65°C for 6 hours. The reactants were poured into water, extracted with ethyl acetate. It was dried with MgSO₄ and then concentrated under reduced pressure. The residual was purified with MeOH/DCM (0∼10%) MPLC to obtain the Boc-protected compound, which was dissolved in 1 ml DCM, followed by 0.2 ml TFA, and stirred at room temperature for 2 hours. After the reaction was finished, the reactants were concentrated under reduced pressure and vacuum-dried. The residue was dissolved in DCM, passed through an NH-DM 1020 Chromatorex pad to obtain the heading compound, which was used in the next step without further purification. (42 mg) MS (ESI, m/z): [M+H]⁺ = [435.5].

### Step 5) Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-[4-({5-[2-(2,6-dioxopiperidin-3-yl)-1-oxo-1,2-dihydroptalazine-6-yl]pentyl}oxy)phenylamide (ICT-0001411)

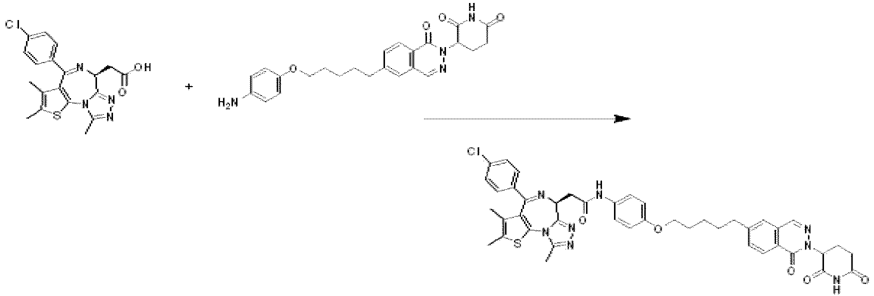

HATU (73.1 mg, 0.2 mmol) is added to 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]acetic acid (50 mg, 0.1 mmol) and 3-{6-[5-(4-aminophenoxy)pentyl]-1-oxo-1,2-dihydroptalazine-2-yl}piperidine-2,6-dione (42 mg, 0.1 mmol) in DMF (5 ml) at room temperature, followed by ethylbis(propane-2-yl)amine (67.4 mg, 0.2 mmol). The reactants were stirred for 6 hours. The reactants were poured into water, extracted with ethyl acetate, dried with MgSO₄, and then concentrated under reduced pressure. The residual was purified with MeOH/DCM (0∼20%) MPLC to obtain the title compound (48.2 mg). MS (ESI, m/z): [M+H]⁺ = 818.4
1H NMR (500 MHz, DMSO-d6) δ ppm 10.98 (s, 1 H) 10.09 (s, 1 H) 8.33 - 8.38 (m, 1 H) 8.13 (d, J=8.24 Hz, 1 H) 7.68 - 7.75 (m, 2 H) 7.39 -7.48 (m, 5 H) 7.33 - 7.36 (m, 2 H) 6.80 (d, J=9.00 Hz, 2 H) 4.52 (t, J=7.17 Hz, 1 H) 3.86 (t, J=6.33 Hz, 2 H) 3.39 (d, J=6.71 Hz, 2 H) 2.77 (t, J=7.78 Hz, 2 H) 2.56 - 2.58 (m, 1 H) 2.54 (s, 4 H) 2.35 (s, 3 H) 2.30 (s, 1 H) 1.91 (br. s., 1 H) 1.87 (br. s., 1 H) 1.63 - 1.70 (m, 4 H) 1.39 (d, J=7.17 Hz, 2 H) 1.17 (s, 2 H)

### Example C-10: Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-[4-({4-[2-(2,6-dioxopiperidin-3-yl)-4-methyl-1-oxo-1,2-dihydroptalazine-6-yl]piperazine-1-yl}methyl)phenyl]acetamide (ICT-0001423)

### Step 1) Synthesis of 3-(4-methyl-6-{4-[(4-nitrophenyl)methyl]piperazine-1-yl}-1-oxo-1,2-dihydroptalazine-2-yl)piperidine-2,6-dione

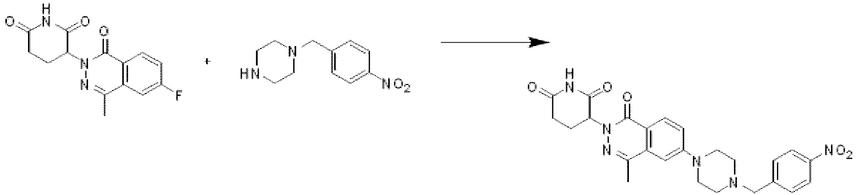

3-(6-fluoro-4-methyl-1-oxo-1,2-dihydroptalazin-2-yl)piperidine-2,6-dione (654 mg, 2.3 mmol) and ethyl bis(propane-2-yl)amine (1.5 g, 11.3 mmol) were added to a 1-[(4-nitrophenyl)methyl]piperazine (0.5 g, 2.3 mmol) solution in NMP (3 mL). The reactants were stirred at 135°C overnight. The reactants were poured into water, extracted with ethyl acetate, dried with MgSO₄, and then concentrated under reduced pressure. The residues were purified by MeOH/DCM (0∼10%) column chromatography to obtain the title compound (0.38 g).

### Step 2) Synthesis of 3-(6-{4-[(4-aminophenyl)methyl]piperazine-1-yl}-4-methyl-1-oxo-1,2-dihydroptalazine-2-yl)piperidine-2,6-dione

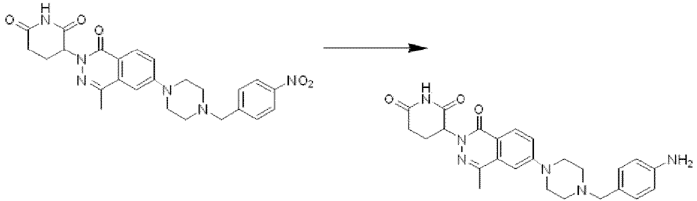

10 mL saturated NH₄Cl was added to a solution of 3-(4-methyl-6-{4-[(4-nitrophenyl)methyl]piperazine-1-yl}-1-oxo-1,2-dihydroptalazin-2-yl) and iron (865 mg, 15.5 mmol) in a THF (10 mL). The reactants were stirred at 100°C for 2 hours. After cooling to room temperature, the reactants were filtered, the filtrate was poured into water, and extracted with ethyl acetate. The organic layer was dried with MgSO₄, concentrated under reduced pressure. The compound was then purified by MeOH/DCM (0∼10%) column chromatography to obtain the title compound (0.31 g).

### Step 3) Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-[4-({4-[2-(2,6-dioxopiperidin-3-yl)-4-methyl-1-oxo-1,2-dihydropthalazine-6-yl]piperazine-1-yl}methyl)phenyl]acetamide (ICT-0001423)

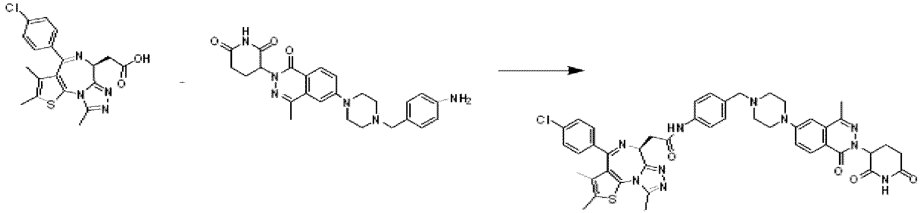

HATU (82.6 mg, 217 µmol) and ethylbis (70.2 mg, 543 µmol) were added to a solution of 2-[(9S)-7-(4-chlorophenyl)methyl-4,5,13-trimethyl-3-thia-1,8,11,12-tetraaztrycyclo [8.3.0.0²,6,6]trideca-2(6)trideca-2(6),4-oxo-1,2-dihydroptalazine-2-yl)piperidine (50 mg, 109 µmol) in DMF (2.0 ml). The reactants were stirred at room temperature for 6 hours. The reactants were poured into water, extracted with ethyl acetate, dried with MgSO₄, and then concentrated under reduced pressure. The residues were purified by MeOH/DCM (0∼10%) column chromatography to obtain the title compound (51 mg).MS (ESI, m/z): [M+H]⁺ = 844.4
1H NMR (500 MHz, DMSO-d6) δ ppm 10.90 (s, 1 H) 10.26 (s, 1 H) 7.99 (d, J=9.16 Hz, 1 H) 7.54 (m, J=8.39 Hz, 2 H) 7.42 (d, J=8.55 Hz, 3 H) 7.36 (d, J=8.39 Hz, 2 H) 7.17 - 7.24 (m, 2 H) 7.01 (s, 1 H) 5.53 - 5.68 (m, 1 H) 4.53 (t, J=7.10 Hz, 1 H) 3.41 - 3.48 (m, 4 H) 3.38 (br. s., 3 H) 2.80 - 2.89 (m, 1 H) 2.46 - 2.55 (m, 8 H) 2.34 - 2.37 (m, 3 H) 1.84 - 2.03 (m, 2 H) 1.57 (s, 3 H) 1.15 - 1.22 (m, 4 H)

### Example C-11: Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-(4-{[(2S)-4-[2-(2,6-dioxopiperidin-3-yl)-4-methyl-1-oxo-1,2-dihydroptalazine-6-yl]morpholine-2-yl]methoxy}phenyl)acetamide (ICT-0001430)

### Step 1) Synthesis of tert-butyl(2S)-2-[(methanesulfonyoxy)methyl]morpholine-4-carboxylate

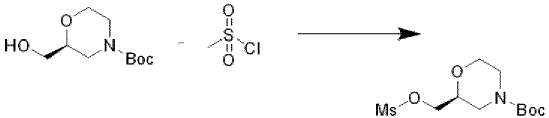

Triethylamine (2.1 g, 1.5 mmol) was added to tert-butyl(2S)-2-(hydroxymethyl)morpholine-4-carboxylate (3 g, 13.8 mmol) solution in DCM (120 mL) at room temperature. The reaction mixture was stirred for 15 minutes. Droplets of diluted methanesulfonyl chloride (1.9 g, 16.6 mmol) in 15 mL DCM were added to the reaction mixture. The reaction mixture was stirred at room temperature for another 30 minutes. After the reaction was completed, the reaction mixture was poured into water, extracted with DCM, dried with MgSO₄, and then concentrated under reduced pressure. The residues were purified by MeOH/DCM (0∼10%) column chromatography to obtain the title compound (4.1 g).

### Step 2) Synthesis of tert-butyl(2S)-2-[(4-{[(tert-butoxy)carbonyl]amino}penoxy)methyl]morpholine-4-carboxylate

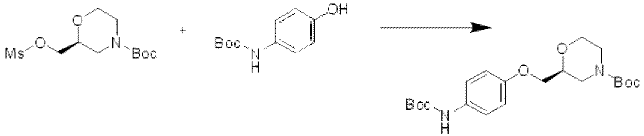

tert-butyl N- (4-hydroxyphenyl)carbamate (3.05 g, 14.6 mmol), disium (1+) carbonate (9.5 g, 29.1 mmol) and potassium iodide (121 mg, 728 µmol) were added to tert-butyl (2S)-2-[(methanesulfonyoxyoxy)methyl]morpholine-4-carboxylate (4.3 g, 14.6 mmol) solution in DMF (10 mL).

### Step 3) Synthesis of 4-[(morpholine-2-yl)methoxy]aniline

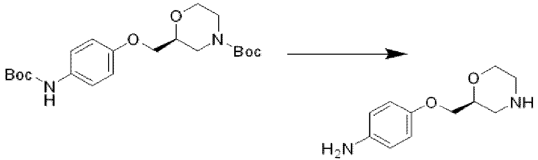

4.0 M HCl in dioxane (140 mL) was added to tert-butyl 2-[(4-{{(tert-butoxy)carbonyl]amino}penoxy)methyl]morpholine-4-carboxylate (6.3 g, 15.4 mmol) solution in dioxane (20 mL). The reaction mixture was stirred at room temperature for 1 hour. After the reaction ended, the reactants were vacuum-concentrated and used in the next step without further purification (6.2 g).

### Step 4) Synthesis of 3-(6-{2-[(4-aminophenoxy)methyl]morpholine-4-yl}-4-methyl-1-oxo-2λ⁴-phthalazine-2-yl)piperidine-2,6-dione

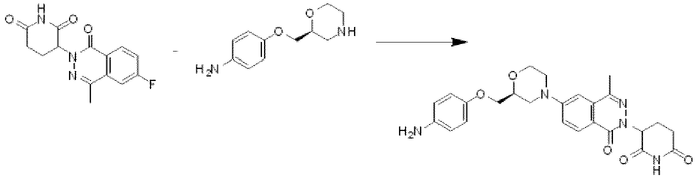

4-[(morpholine-2-yl)methoxy]aniline (3.21 g, 15.4 mmol) were added to the solution in NMP (20 mL), 3-(6-fluoro-4-methyl-1-oxo-2λ⁴-phthalazine-2-yl), piperidine-2,6-dione (4.46 g, 15.4 mmol) and ethylbis(propane-2-yl)amine (9.96 g, 77.1 mmol). The reactants were stirred at 120°C overnight. The reactants were poured into water, extracted with ethyl acetate, dried with MgSO₄, and then concentrated under reduced pressure. The residues were purified and column chromatography yielded with MeOH/DCM (0∼10%) heading compound (6.3 g).

### Step 5) Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-(4-{[(2S)-4-[2-(2,6-dioxopiperidin-3-yl)-4-methyl-1-oxo-1,2-dihydroptalazine-6-yl]morpholine-2-yl]methoxy}phenyl)acetamide (ICT-0001430)

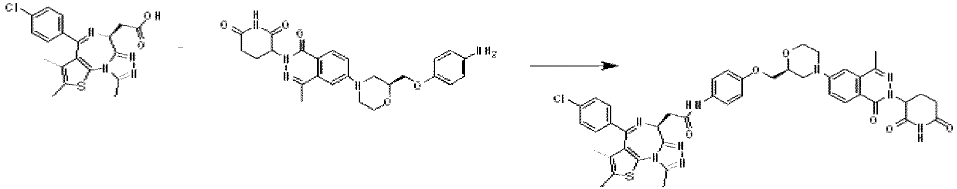

HATU (82.6 mg, 217 µmol) and ethylbis (propane-2-yl)amine (70.2 mg, 543 µmol) were added to a solution of 2-[(9S)-7-(4-chlorophenyl)-4,5,13-trimethyl-3-thia-1,8,11,12-tetraazzatricyclo[8.3.0.0²,6(6),4,7,10,12-pentaene-9-yl]acetic acid (43.5 mg, 109 µmol) and 3-{6-[(2S)-2-[(4-aminophenoxy)methyl]morpholine-4-yl]-4-methyl-1-oxo-1,2-dihydroptallazine-2-yl}piperidine-2,6-dione (51.8 mg, 109 µmol) in DMF (2.0 ml). The reactants were stirred at room temperature for 6 hours. The reactants were poured into water, extracted with ethyl acetate, dried with MgSO₄, and then concentrated under reduced pressure. The residue was purified by MeOH/DCM (0-10%) column chromatography to obtain the title compound (57 mg). MS (ESI, m/z): [M+H]⁺ = 861.4
1H NMR (500 MHz, DMSO-d6) δ ppm 10.92 (s, 1 H) 10.13 (s, 1 H) 8.04 (d, J=9.00 Hz, 1 H) 7.45 - 7.52 (m, 3 H) 7.42 (d, J=8.70 Hz, 3 H) 7.35 (d, J=8.39 Hz, 2 H) 7.05 - 7.09 (m, 1 H) 6.90 (d, J=9.00 Hz, 2 H) 5.63 (dd, J=11.52, 4.35 Hz, 1 H) 4.53 (t, J=7.10 Hz, 1 H) 3.93 - 4.17 (m, 4 H) 3.76 - 3.93 (m, 3 H) 3.62 - 3.69 (m, 1 H) 3.40 (d, J=6.87 Hz, 2 H) 2.76 - 2.92 (m, 3 H) 2.45 - 2.61 (m, 6 H) 2.35 (s, 4 H) 1.96 - 2.03 (m, 1 H) 1.90 - 1.96 (m, 1 H) 1.14 - 1.26 (m, 1 H)

### Example C-12: Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-{4-[(4-{4-[(2,6-dioxopiperidine-3-yl)amino]-2-fluorophenyl}piperazine-1-yl)methyl]phenyl}acetamide (ICT-00001441)

### Step 1) Synthesis of tert-butyl 4-[(4-nitrophenyl)methyl]piperazine-1-carboxylate

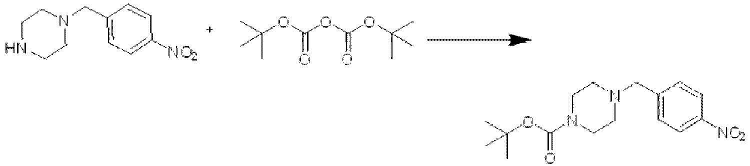

Dissolve 1-[(4-nitrophenyl)methyl]piperazine (0.5 g, 2.26 mmol) in THF (15 mL). Di-tert-butyl dicarbonate (740 mg, 3.39 mmol) dissolved in THF (15 mL) was added to a THF solution of 1-[(4-nitrophenyl)methyl]piperazine. The resulting mixture was stirred at room temperature for 2 hours. The colorless solution was evaporated to remove the THF. Pour colorless oil into water (100 ml). Colorless oil was extracted 3 times with 50 mL CH₂Cl₂. The organic layer was washed with water and brine solution. The organic layer was dried with anhydrous Na₂SO₄. After filtering, the solvent was evaporated. Zansa was used for subsequent reactions without tablets (380 mg). MS (ESI, m/z): [M+H]⁺= [322.3],

### Step 2) Synthesis of tert-butyl 4-[(4-aminophenyl)methyl]piperazine-1-carboxylate

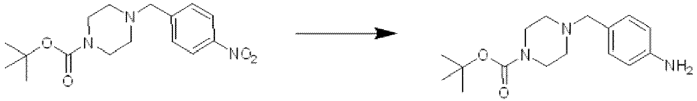

10 mL saturated NH₄Cl was added to tert-butyl 4-[(4-nitrophenyl)methyl]piperazine-1-carboxylate (270 mg, 840 µmol) and iron (938 mg, 16.8 mmol) solution in THF (10 ml). The reactants were stirred at 100°C for 2 hours. After cooling to room temperature, the reactants were filtered, the filtrate was poured into water, and extracted with ethyl acetate. The organic layer was dried with MgSO₄, decompression concentrated, and then purified by column chromatography to obtain the heading compound (158 mg) MS (ESI, m/z): [M+H]⁺ = [292.4].

### Step 3) Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-{4-[(piperazine-1-yl)methyl]phenyl}acetamide

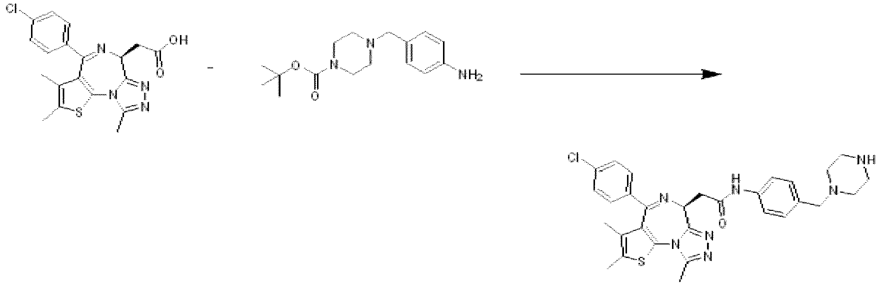

HATU (300 mg, 789 µmol) was added to 2-[(9S)-7-(4-chlorophenyl)-4,5,13-trimethyl-3-thia-1,8,11,12-tetraazatricyclo[8.3.0.0²,6]trideca-2(6),4,7,10,12-pentaene-9-yl]acetic acid (158 mg, 395 µmol) and tert-butyl 4-[(4-aminophenyl)methyl]piperazine-1-carboxylate (115 mg, 395 µmol) in DMF (5 ml) at room temperature, followed by ethylbis (propane-2-yl)amine (255 mg, 1.97 mmol). The reactants were stirred for 6 hours. It was extracted with ethyl acetate, dried with MgSO₄, and then concentrated under reduced pressure. The residuals were purified with MeOH/DCM (0-20%) MPLC to obtain the Boc-protected compound, which was treated with 50% TFA in DCM (5 mL), and stirred for 5 hours. After the reaction was finished, the reactants were concentrated under reduced pressure and vacuum-dried. The residue was dissolved in DCM, passed through an NH-DM 1020 Chromatorex pad to obtain the heading compound, which was used in the next step without further purification. MS (ESI, m/z): [M+H]⁺ = 575.2

### Step 4) Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-(4-{[4-(2-fluoro-4-nitrophenyl)piperazine-1-yl]methyl}phenyl)acetamide

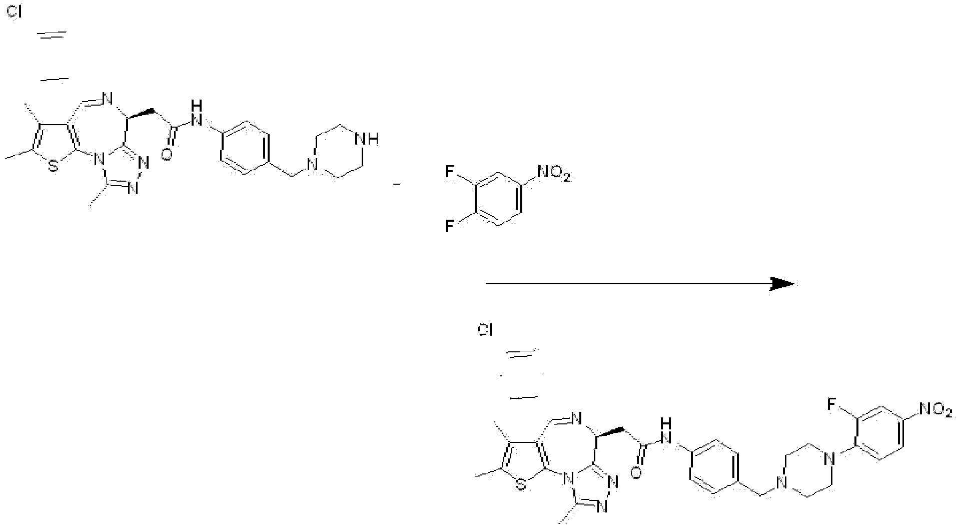

K₂CO₃ (50.6 mg, 366 µmol) was added to acetonitrile (10 ml) in a solution of 2-[(9S)-7-(4-chlorophenyl)-4,5,13-trimethyl-3-thia-1,8,11,12-tetraazatricyclo[8.3.0.0²,6]trideca-2(6),4,7,10,12-pentaene-9-yl]N-{4-[(piperazine-1-yl)methyl]phenyl}acetamide (42 mg, 73.2 µmol) and 1,2-difluoro-4-nitrobenzene (11.6 mg, 73.2 µmol). The reactants were stirred at 80°C for 2 hours. After cooling to room temperature, the reactants were concentrated under reduced pressure. Subsequently, it was purified by column chromatography to obtain the title compound (52.2 mg). MS (ESI, m/z): [M+H]⁺ = [714.2]

### Step 5) Synthesis of N-(4-{[4-(4-amino-2-fluorophenyl)piperazine-1-yl]methyl}phenyl)-2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]acetamide

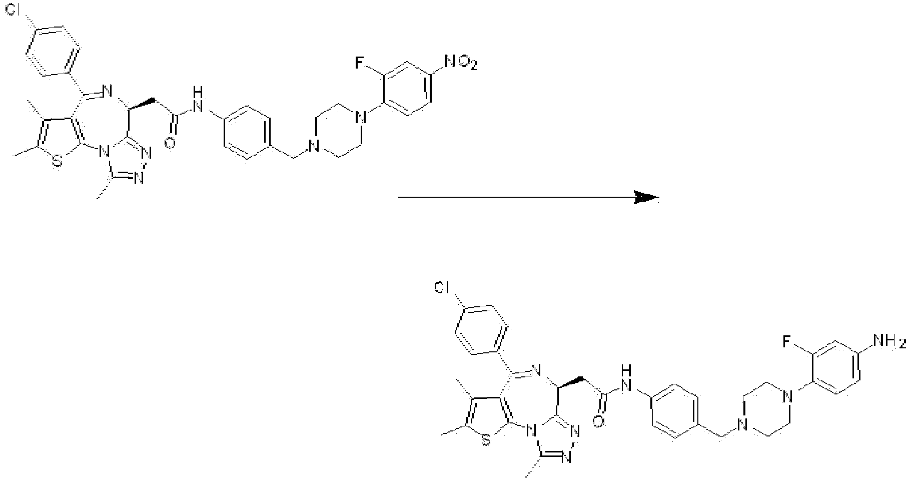

10 mL saturated NH₄Cl was added to a solution of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-(4-{[4-(2-fluoro-4-nitrophenyl)piperazine-1-yl]methyl}phenyl)acetamide (52.2 mg, 73.2 µmol) and iron (81.7 mg, 1.46 mmol) in THF (10 ml). The reactants were stirred at 100°C for 2 hours. After cooling to room temperature, the reactants were filtered, the filtrate was poured into water, and extracted with ethyl acetate. The organic layer was dried with MgSO₄, concentrated under reduced pressure, and then purified by column chromatography to obtain the title compound (20 mg) MS (ESI, m/z): [M+H]⁺= [684.4].

### Step 6) Synthesis of 2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-{4-[(4-{4-[(2,6-dioxopiperidin-3-yl)amino]-2-fluorophenyl}piperazine-1-yl)methyl]phenyl}acetamide (ICT-00001441)

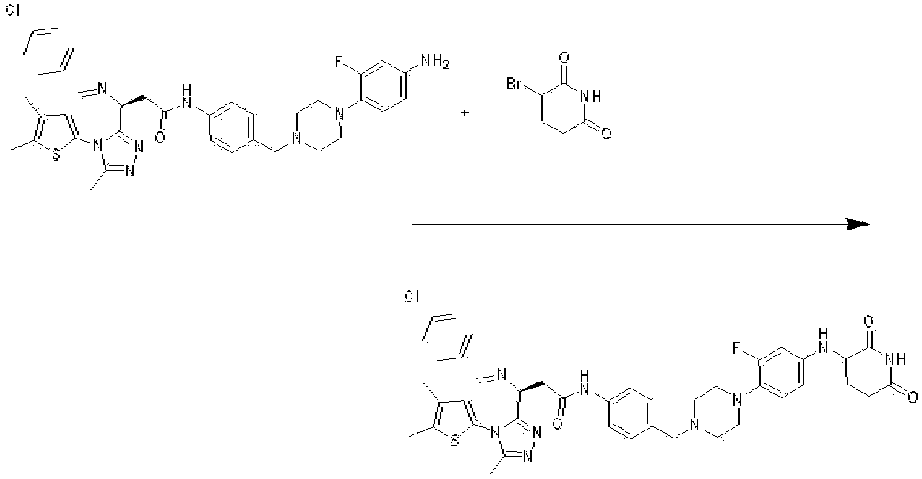

NaHCO3 (14.8 mg, 176 µmol) was added to N-(4-{[4-(4-amino-2-fluorophenyl)piperazine-1-yl]methyl}phenyl)-2-[(9S)-7-(4-chlorophenyl)-4,5,13-trimethyl-3-thia-1,8,11,12-tetraazatricyclo[8.3.0.0² ,6]trideca-2(6),4,7,10,12-pentaene-9-yl]acetamide (20 mg, 29.3 µmol) and 3-bromomorphidine-2,6-dione (16.9 g, 87.8 µmol) in 10 ml of DMF at room temperature. The reactants were stirred at 80°C for 3 hours. The reactants were poured into water. It was extracted with ethyl acetate, dried with MgSO₄, and then concentrated under reduced pressure. The residue was purified with MeOH/DCM (0∼10%) MPLC to obtain the title compound (20 mg) MS (ESI, m/z): [M+H]⁺ = 795.4
1H NMR (500 MHz, DMSO-d6) δ ppm 10.24 (s, 1 H) 7.53 (s, 3 H) 7.42 (s, 4 H) 7.36 (s, 1 H) 7.19 (s, 3 H) 4.53 (t, J=7.02 Hz, 2 H) 2.57 (s, 7 H) 2.54 (s, 7 H) 2.47 (s, 1 H) 2.29 (s, 6 H) 2.02 (s, 5 H) 1.16 (s, 1 H)

**Table 2. In vitro degradation analysis and cell viability analysis of JQ-1-based BRD4 PROTAC compound (see Experimental Examples 1 to 3 described below)**

| ICT-CODE | Structure | Analyis of In vitro Cell viability (nM) | | | | | | |
|---|---|---|---|---|---|---|---|---|
| | | RPMI2650 | | | SNU2972 | | MDA-MB-231 | |
| | | BR04 (DC₅₀) | BRD4-NUT (DC₅₀) | BRD4 (IC₅₀) | BRD4 (DC₅₀) | BRD4 (IC₅₀) | (DC₅₀) | BRD4 (IC₅₀) |
| ICT-0000953 | 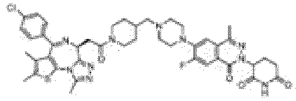 | A | A | A | - | A | A | A |
| ICT-0000971 | 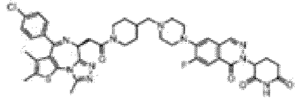 | A | A | A | - | A | A | A |
| ICT-0000972 | 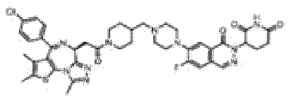 | A | A | B | - | A | A | A |
| ICT-0000973 | 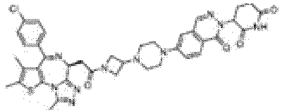 | A | A | B | - | B | A | A |
| ICT-0000974 | 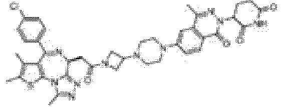 | A | A | A | - | A | A | A |
| ICT-0000982 | 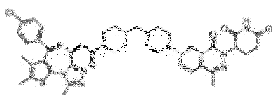 | A | A | A | - | A | A | A |
| ICT-0001409 | 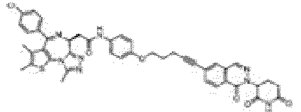 | C | C | - | - | - | - | - |
| ICT-0001410 | 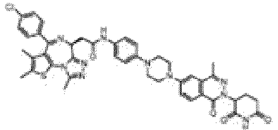 | A | A | A | - | - | A | A |
| ICT-0001411 | 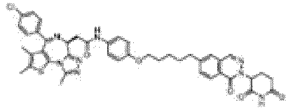 | C | C | - | - | - | - | - |
| ICT-0001423 | 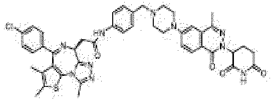 | A | A | A | - | - | A | A |
| ICT-0001430 | 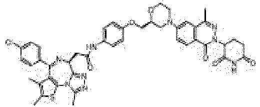 | A | A | B | - | - | - | - |
| ICT-0001441 | 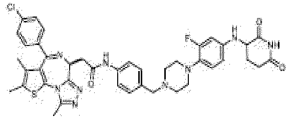 | A | A | A | - | - | - | - |

| | | | | | | | | |
|---|---|---|---|---|---|---|---|---|
| A: IC₅₀ or DC₅₀ < 100 nM, B: 100 nM < IC₅₀ or DC₅₀ < 1 uM, C: 1 uM < IC₅₀ or DC₅₀ | | | | | | | | |

### Example 1: Cell line culture and compound processing

The human breast cancer cell line [MDA-MB-231] was cultured in RPMI medium with the addition of 10% fetal bovine serum, penicillin [100U/ml], and streptomycin [100mg/ml]. The synthesized compounds were prepared with a 10 mM or 50 mM raw material solution dissolved in DMSO [dimethyl sulfoxide], and were diluted sequentially and treated. [RPMI2650] and [SNU2972] were cultivated under specific conditions.

### Example 2: Evaluation of cancer cell antiproliferative activity of BRD4 targeting ligand compound

To observe cell growth inhibition, cells were dispensed in a concentration of 5,000 cells/well in a 96-well plate, and samples prepared at various concentrations were treated for 72 hours. The IC₅₀ value (the concentration of the compound achieving 50% cytostaticism) is the average value of the results of three independent measurements.

### ExperimentExample 3: Evaluation of BRD4 protein degradation by PROTAC targeting BRD4 protein

To evaluate the resolution of PROTAC for BRD4 expressed in cells, cells were dispensed in a concentration of 5 X 105 cells/well in a 6-well plate, and the sample was treated for 24 hours. To perform a western blotting assay to confirm protein expression, cells treated with a protease inhibitor in 70 µL of RIPA buffer with a protease inhibitor were treated for 30 minutes, then the cell lysate was collected using a scraper on ice and centrifuged for 30 min at 15,000 rpm at 4°C to obtain a supernatant containing protein. SDS-PAGE and membrane transfer of proteins were performed using 10 µg of protein for each sample, and anti-BRD4, antibeta-tubulin, and anti-GAPDH antibodies were reacted at 4°C for 16 hours, and secondary antibodies were reacted at room temperature for 1 hour, and the expression of proteins was confirmed using SuperSignal^{™} West Pico PLUS chemiluminescence substrate and image analyzer. Protein expression was normalized based on the expression value of β or GAPDH, and protein expression in each sample was measured as % based on 100% of samples treated with 0.1% DMSO vehicle. Protein expression was quantified using Image J program. Change in protein expression volume by compound treatment (DC50: concentration of compounds where 50% protein expression inhibition is achieved) was calculated using the GraphicPad Prism 8.0 software. The results are shown in Tables 1 and 2 above.

### [Industrial Applicability]

The present invention can be used for anticancer purposes.

## Claims

1. A compound represented by the following chemical formula 1 or a pharmaceutically acceptable salts thereof:
[Chemical Formula 1] BRD4 target protein-binding moiety (P) - {linker (L)}p - E3 ligase-binding moiety (E)
wherein,
E3 ligase binding moiety (E) is a structure denoted by the following chemical formulas 2 or 3;
BRD4 target protein-binding moiety (P) is the structure indicated by the following formula 4 or 5; and
p is an integer of 0 or 1: 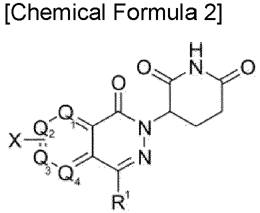 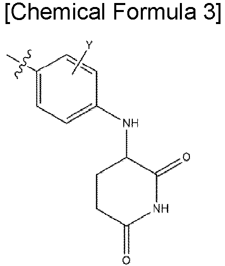 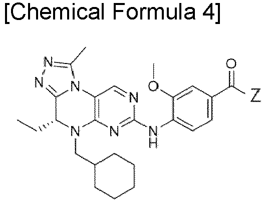 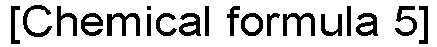 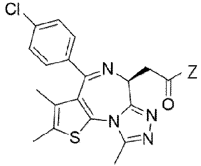
wherein,
X is hydrogen, halogen, amino, nitro, hydroxy, C1 to C6 straight or branched alkyloxy, or a heterocycle of 4 to 8 atoms containing oxygen or nitrogen;
Q₁ to Q₄ are each independently C-F, C-Cl, C-H, C-X, or N, and at least any one of Q₁ to Q₄ is C-X;
Y is hydrogen, halogen, or C1 to C6 straight or branched alkyl that is unsubstituted or optionally substitute with a halogen or cyclic alkyloxy; and
Z is the part where one end of the linker connects,
R¹ is hydrogen, nitro, amino, carbonyl, C1 to C6 straight or branched alkyl, C1 to C6 straight or branched alkyl substituted with halogen, or cycloalkyl.

2. The compound of claim 1, wherein one end of the linker (L) is
(i) substitute X in the structure of the chemical formula 2 and connect with the structure of the chemical formula 1; or
(ii) bonded to a nitrogen atom or oxygen atom of X in the structure of chemical formula 2 and connected to the structure of chemical formula 2.

3. The compound of claim 1, wherein the linker (L) is 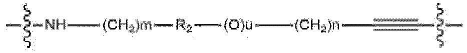 or 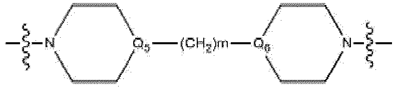 or 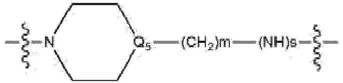 or 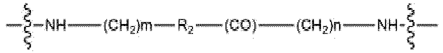 or 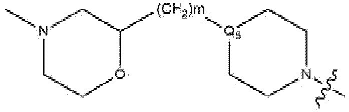 or 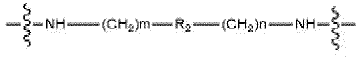 or 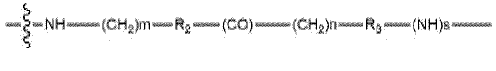 or 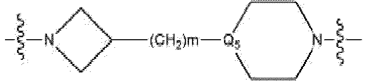 or 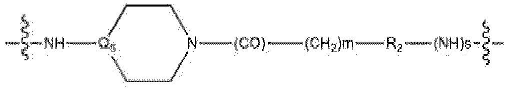 or 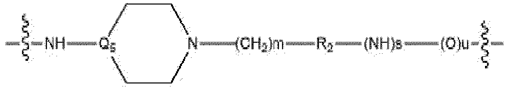 or 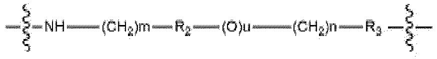 or selected from the following structural formulas: 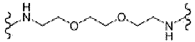 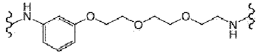 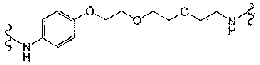 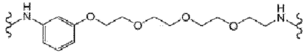 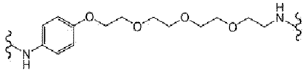 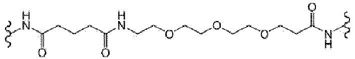
wherein, R₂ and R₃ are each independently unsubstituted or substituted C1 to C4 alkyl, piperidine, piperazine, -(CH₂)s-, -(CH₂)q-[O(C₂H₄)]ᵣ-, -O-(CH₂)-, benzene or morpholine;
Q₅ and Q₆ are independently carbon atoms or nitrogen atoms independently; and
m, n, q, r, s, t, and u are each independently an integer selected from 0 to 7.

4. A compound selected from the group consisting of the following compounds or a pharmaceutically acceptable salt thereof:
4-(((R)-5-(cyclohexylmethyl)-4-ethyl-1-methyl-4,5-dihydro-[1,2,4]triazolo[4,3-f]pteridine-7-yl)amino)-N-(5-(2-(2-(2,6-dioxopiperidin-3-yl)-1-oxo-1,2-dihydrophthalazine-6-yl)pent-4-phospho-1-yl)-3-methoxybenzamide;
3-(6-(4-((4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-il)methyl)piperidine-1-yl)4-methyl-1-oxopthalazine-2(1H)-yl)piperidine-2,6-dione;
3-((4-(4-(4-(R)-5-(cyclohexylmethyl)-4-ethyl-1-methyl-4,5-dihydro-[1,2,4]triazolo[4,3-f]pteridine-7-yl)amino)-3-methoxybenzoyl)piperazine-1-yl)-3-fluorophenyl)amino)piperidine-2,6-dione;
3-((4-(4-((4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-il)methyl)piperidine-1-yl)-3-fluorophenyl)amino)piperidine-2,6-dione;
4-(((R)-5-(cyclohexylmethyl)-4-ethyl-1-methyl-4,5-dihydro-[1,2,4]triazolo[4,3-f]pteridine-7-yl)amino)-N-(1-(5-(2-(2,6-dioxopiperidin-3-yl)-1-oxo-1,2-dihydropthalazine-6-yl)pent-4-phosphorus-1-yl)piperidine-4-yl)-3-methoxybenzamide;
4-(((R)-5-(cyclohexylmethyl)-4-ethyl-1-methyl-4,5-dihydro-[1,2,4]triazolo[4,3-f]pteridine-7-yl)amino)-N-(1-((3-(2,6-dioxopiperidin-3-yl)1-methyl-4-oxo-3,4-dihydropthalazine-5-yl)glycil)-4-methylpiperidine-4-yl)-3-methylpiperidine-4-yl)-3-methoxybenzamide;
3-(6-(4-((S)-4-(4-((R)-5-(cyclohexylmethyl)-4-((R)-5-(cyclohexylmethyl)-4-ethyl-1-methyl-4,5-dihydro-[1,2,4]triazolo[4,3-f]pteridine-7-yl)amino)3-methoxybenzoyl)morpholine-2-yl)methyl)piperazine-1-yl)4-methyl-1-oxopthalazine-2(1H)-yl)piperidine-2,6-dione;
4-(((R)-5-(cyclohexylmethyl)-4-ethyl-1-methyl-4,5-dihydro-[1,2,4]triazolo[4,3-f]pteridine-7-yl)amino)N-(2-(2-(2-(2-(2-(2-(3-(2,6-dioxopiperidin-3-yl)4-oxo-3,4-dihydroptalazin-5-yl)amino)ethoxy)ethoxy)ethoxy)ethoxy)ethoxy)ethyl)ethyl)3-methoxybenzamide;
4-(((R)-5-(cyclohexylmethyl)-4-ethyl-1-methyl-4,5-dihydro-[1,2,4]triazolo[4,3-f]pteridine-7-yl)amino)N-(1-(3-(4-(2-(2,6-dioxopiperidine-3-yl)-4-methyl-1-oxo-1,2-dihydroptalazine-6-yl)piperazine-1-yl)propanoyl)piperidine-4-yl)piperidine-4-yl)-3-methoxybenzamide;
3-(6-(4-(1-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperidine-4-yl)piperazine-1-yl)4-methyl-1-oxopthalazine-2(1H)-yl)piperidine-2,6-dione;
3-({4-[4-(4-{[(4S)-5-(cyclohexylmethyl)-4-ethyl-1-methyl-4H,5H-[1,2,4]triazolo[4,3-f]pteridine-7-yl]amino}-2-methoxybenzoyl)piperazine-1-yl]-3-fluorophenyl}amino)-1-methylpiperidine-2,6-dione;
3-(6-(4-(1-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperidine-4-yl)piperazine-1-yl)4-methyl-1-oxopthalazine-2(1H)-yl)piperidine-2,6-dione;
3-(6-(4-((1-(4-(((R)-5-(cyclohexylmethyl)-4-ethyl-1-methyl-4,5-dihydro-[1,2,4]triazolo[4,3-f]pteridine-7-yl)amino)3-methoxybenzoyl)azetidine-3-yl)methyl)piperazine-1-yl)4-methyl-1-oxopthalazine-2(1H)-yl)piperidine-2,6-dione;
4-(((R)-5-(cyclohexylmethyl)-4-ethyl-1-methyl-4,5-dihydro-[1,2,4]triazolo[4,3-f]pteridine-7-yl)amino)N-(2-((3-(2,6-(2,6-dioxopiperidin-3-yl)4-oxo-3,4-dihydropetalazine-5-yl)amino)ethyl)-3-methoxybenzamide;
4-(((R)-5-(cyclohexylmethyl)-4-ethyl-1-methyl-4,5-dihydro-[1,2,4]triazolo[4,3-f]phteridine-7-yl)amino)N-(1-(2-(2-(2-(2-(2-(2-(2-(2-(2-(2-(2-(2,6-dioxopiperidin-3-yl)1-oxo-1,2-dihydropthalazine-6-yl)amino)ethoxy)ethoxy)ethoxy)ethoxy)acetyl)piperidine-4-yl)-3-methoxybenzamide;
4-{[(4S)-5-(cyclohexylmethyl)-4-ethyl-1-methyl-4H,5H-[1,2,4]triazolo[4,3-f]pteridine-7-yl]amino}-N-[1-(2-{4-[2-(2,6-dioxopiperidin-3-yl)-4-methyl-1-oxo-1,2-dihydroptalazine-6-yl]piperazine-1-yl}ethyl)piperidine-4-yl]-3-methoxybenzamide;
4-{[(4S)-5-(cyclohexylmethyl)-4-ethyl-1-methyl-4H,5H-[1,2,4]triazolo[4,3-f]pteridine-7-yl]amino}-N-{1-[2-(4-{4-[(2,6-dioxopiperidine-3-yl)amino]-2-fluorophenyl}piperazine-1-yl)ethyl]piperidine-4-yl}-3-methoxybenzamide;
4-{[(4S)-5-(cyclohexylmethyl)-4-ethyl-1-methyl-4H,5H-[1,2,4]triazolo[4,3-f]phteridine-7-yl]amino}-N-{1-[2-(1-{4-[(2,6-dioxopiperidine-3-yl)amino]-2-fluorophenyl}piperidine-4-yl)ethyl]piperidine-4-yl}-3-methoxybenzamide;
4-(((R)-5-(cyclohexylmethyl)-4-ethyl-1-methyl-4,5-dihydro-[1,2,4]triazolo[4,3-f]pteridine-7-yl)amino)N-(1-(2-(4-(2-(2-(2-(2,6-dioxopiperidin-3-yl)-4-methyl-1-oxo-1,2-dihydroptalazine-6-yl)piperazine-1-yl)acetyl)piperidine-4-yl)3-methoxybenzamide;
4-{[(4S)-5-(cyclohexylmethyl)-4-ethyl-1-methyl-4H,5H-[1,2,4]triazolo[4,3-f]phteridine-7-yl]amino}-N-{1-[2-(1-{4-[(2,6-dioxopiperidine-3-yl)amino]-2-fluorophenyl}piperidine-4-yl)ethyl]piperidine-4-yl}-3-methoxybenzamide;
4-{[(4S)-5-(cyclohexylmethyl)-4-ethyl-1-methyl-4H,5H-[1,2,4]triazolo[4,3-f]phteridine-7-yl]amino}-N-{1-[2-(1-{4-[(2,6-dioxopiperidine-3-yl)amino]-2-fluorophenyl}piperidine-4-yl)ethyl]piperidine-4-yl}-3-methoxybenzamide;
4-{[(4R)-5-(cyclohexylmethyl)-4-ethyl-1-methyl-4H,5H-[1,2,4]triazolo[4,3-f]pteridine-7-yl]amino}-N-(1-{4-[(2,6-dioxopiperidine-3-yl)amino]-2-fluorophenyl}piperidine-4-yl)-3-methoxybenzamide;
4-{[(4R)-5-(cyclohexylmethyl)-4-ethyl-1-methyl-4H,5H-[1,2,4]triazolo[4,3-f]pteridine-7-yl]amino}-N-{1-[2-(2,6-dioxopiperidin-3-yl)-4-methyl-1-oxo-1,2-dihydroptalazine-6-yl]piperidine-4-yl}-3-methoxybenzamide;
4-{[(4R)-5-(cyclohexylmethyl)-4-ethyl-1-methyl-4H,5H-[1,2,4]triazolo[4,3-f]pteridine-7-yl]amino}-N-[1-(3-{4-[2-(2,6-dioxopiperidin-3-yl)-4-methyl-1-oxo-1,2-dihydropthalazine-6-yl}piperazine-1-yl}propyl)piperidine-4-yl]-3-methoxybenzamide;
4-{[(4R)-5-(cyclohexylmethyl)-4-ethyl-1-methyl-4H,5H-[1,2,4]triazolo[4,3-f]pteridine-7-yl]amino}-N-[1-({4-[(2,6-dioxopiperidin-3-yl)amino]phenyl}methyl)piperidine-4-yl]-3-methoxybenzamide;
4-{[(4R)-5-(cyclohexylmethyl)-4-ethyl-1-methyl-4H,5H-[1,2,4]triazolo[4,3-f]pteridine-7-yl]amino}-N-{1-[2-(2-{4-[(2,6-dioxopiperidine-3-yl)amino]-2-fluorophenoxy}ethoxy)ethyl]piperidine-4-yl}-3-methoxybenzamide;
4-{[(4R)-5-(cyclohexylmethyl)-4-ethyl-1-methyl-4H,5H-[1,2,4]triazolo[4,3-f]pteridine-7-yl]amino}-N-{1-[3-(4-{4-[(2,6-dioxopiperidin-3-yl)amino]-2-fluorophenyl}piperazine-1-day)propanoyl]piperidine-4-yl}-3-methoxybenzamide;
4-{[(4R)-5-(cyclohexylmethyl)-4-ethyl-1-methyl-4H,5H-[1,2,4]triazolo[4,3-f]pteridine-7-yl]amino}-N-{1-[3-(4-{4-[(2,6-dioxopiperidine-3-yl)amino]-2-fluorophenyl}piperazine-1-yl)propyl]piperidine-4-yl}-3-methoxybenzamide;
3-{6-[4-(4-{[(4R)-5-(cyclohexylmethyl)-4-ethyl-1-methyl-4H,5H-[1,2,4]triazolo[4,3-f]pteridine-7-yl]amino}-3-methoxybenzoyl)piperazine-1-yl]-4-methyl-1-oxo-1,2-dihydroptalazine-2-yl}piperidine-2,6-dione;
3-{6-[4-(4-{[(4R)-5-(cyclohexylmethyl)-4-ethyl-1-methyl-4H,5H-[1,2,4]triazolo[4,3-f]pteridine-7-yl]amino}-3-methoxybenzoyl)piperazine-1-yl]-1-oxo-1,2-dihydroptalazine-2-yl}piperidine-2,6-dione;
4-{[(4R)-5-(cyclohexylmethyl)-4-ethyl-1-methyl-4H,5H-[1,2,4]triazolo[4,3-f]pteridine-7-yl]amino}-N-{1-[3-(4-{4-[(2,6-dioxopiperidin-3-yl)amino]-2-fluorophenyl}piperazine-1-day)propanoyl]piperidine-4-yl}-3-methoxybenzamide;
3-(8-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)4-methyl-1-oxopthalazine-2(1H)-yl)piperidine-2,6-dione;
3-(8-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)-4-methyl-1-oxopthalazine-2(1H)-yl)piperidine-2,6-dione;
4-(((R)-5-(cyclohexylmethyl)-4-ethyl-1-methyl-4,5-dihydro-[1,2,4]triazolo[4,3-f]pteridine-7-yl)amino)N-(1-(1-(3-(2,6-dioxopiperidine-3-yl)1-methyl-4-oxo-3,4-dihydropthalazine-6-yl)piperidine-4-carbonyl)piperidine-4-yl)piperidine-4-yl)-3-methoxybenzamide;
3-(6-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)4-methyl-1-oxopthalazine-2(1H)-yl)piperidine-2,6-dione;
3-(7-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)4-methyl-1-oxopthalazine-2(1H)-yl)piperidine-2,6-dione;
3-(8-((2-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)1-oxopthalazine-2(1H)-yl)piperidine-2,6-dione;
3-(5-((2-(4-((((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)1-oxopthalazine-2(1H)-yl)piperidine-2,6-dione;
3-(6-((2-(4-(4-(((R)-5-(cyclohexylmethyl)-4-ethyl-1-methyl-4,5-dihydro-[1,2,4]triazolo[4,3-f]pteridine-7-yl)amino)3-methoxybenzoyl)piperazine-1-yl)ethyl)amino)1-oxopthalazine-2(1H)-yl)piperidine-2,6-dione;
3-(7-((2-(4-(((R)-5-(cyclohexylmethyl)-4-ethyl-1-methyl-4,5-dihydro-[1,2,4]triazolo[4,3-f]pteridine-7-yl)amino)-3-methoxybenzoyl)piperazine-1-yl)ethyl)amino)1-oxopthalazine-2(1H)-yl)piperidine-2,6-dione;
3-(6-(4-((1-(2-((S)-4-(4-chlorophenyl)-2,3,9-trimethyl-6H-thieno[3,2-f][1,2,4]triazolo[4,3-a][1,4]diazepine-6-yl)acetyl)piperidine-4-il)methyl)piperazine-1-yl)7-fluoro-4-methyl-1-oxopthalazine-2(1H)-yl)piperidine-2,6-dione;
3-(6-(4-((1-(2-((S)-4-(4-chlorophenyl)-2,3,9-trimethyl-6H-thieno[3,2-f][1,2,4]triazolo[4,3-a][1,4]diazepine-6-yl)acetyl)piperidine-4-il)methyl)piperazine-1-yl)-7-fluoro-1-oxopthalazine-2(1H)-yl)piperidine-2,6-dione;
3-(7-(4-((1-(2-((S)-4-(4-chlorophenyl)-2,3,9-trimethyl-6H-thieno[3,2-f][1,2,4]triazolo[4,3-a][1,4]diazepine-6-yl)acetyl)piperidine-4-il)methyl)piperazine-1-yl)-6-fluoro-1-oxopthalazine-2(1H)-yl)piperidine-2,6-dione;
3-(6-(4-(1-(2-((S)-4-(4-chlorophenyl)-2,3,9-trimethyl-6H-thieno[3,2-f][1,2,4]triazolo[4,3-a][1,4]diazepine-6-yl)acetyl)azetidin-3-yl)piperazine-1-yl)1-oxopthalazine-2(1H)-yl)piperidine-2,6-dione;
3-(6-(4-(1-(2-((S)-4-(4-chlorophenyl)-2,3,9-trimethyl-6H-thieno[3,2-f][1,2,4]triazolo[4,3-a][1,4]diazepine-6-yl)acetyl)azetidine-3-yl)piperazine-1-yl)-4-methyl-1-oxopthalazine-2(1H)-yl)piperidine-2,6-dione;
3-(7-(4-((1-(2-((S)-4-(4-chlorophenyl)-2,3,9-trimethyl-6H-thieno[3,2-f][1,2,4]triazolo[4,3-a][1,4]diazepine-6-yl)acetyl)piperidine-4-il)methyl)piperazine-1-yl)4-methyl-1-oxopthalazine-2(1H)-yl)piperidine-2,6-dione;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-[4-({5-[2-(2,6-dioxopiperidin-3-yl)-1-oxo-1,2-dihydroptalazine-6-yl]pent-4-phospho-1-yl}oxy)phenylamide;
2-((S)-4-(4-chlorophenyl)-2,3,9-trimethyl-6H-thieno[3,2-f][1,2,4]triazolo[4,3-a][1,4]diazepine-6-yl)-N-(4-(4-(2-(2,6-dioxopiperidin-3-yl)-4-methyl-1-oxo-1,2-dihydroptalazine-6-yl)piperazine-1-yl)phenyl)acetamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-[4-({5-[2-(2,6-dioxopiperidin-3-yl)-1-oxo-1,2-dihydroptalazine-6-yl]pentyl}oxy)phenylamide;
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-[4-({4-[2-(2,6-dioxopiperidin-3-yl)-4-methyl-1-oxo-1,2-dihydroptalazine-6-yl]piperazine-1-yl}methyl)phenylamide (ICT-0001423);
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-(4-{[(2S)-4-[2-(2,6-dioxopiperidin-3-yl)-4-methyl-1-oxo-1,2-dihydroptalazine-6-yl]morpholine-2-yl]methoxy}phenylamide; and
2-[(9S)-7-(4-chlorophenyl)-4,5,13-trimethyl-3-thia-1,8,11,12-tetraazatricyclo[8.3.0.0²,⁶]trideca-2(6),4,7,10,12-pentaene-9-yl]-N-{4-[(4-{4-[(2,6-dioxopiperidin-3-yl)amino]-2-fluorophenyl}piperazine-1-yl)methyl]phenyl}acetamide.

5. An anticancer composition comprising a compound of any of claims 1-4.
